# EUROPEAN PATENT APPLICATION

(11) **EP 4 800 005 A1**
(43) Date of publication of application: **02.09.2026**
(21) Application number: 25160558.0
(22) Date of filing: 27.02.2025
(51) Int. Cl.: C07D 211/70, C07D 277/54, C07D 401/04, C07D 417/12, C07D 417/14, A01N 43/78

(54) **HETEROCYCLIC COMPOUNDS FOR COMBATING INVERTEBRATE PESTS**

(71) Applicant: BASF Agricultural Solutions Deutschland GmbH, 67117 Limburgerhof (DE)
(72) Inventor: KOERBER, Karsten, 67056 Ludwigshafen am Rhein (DE); CHAUDHURI, Rupsha, 67056 Ludwigshafen am Rhein (DE); DEFIEBER, Christian, 67056 Ludwigshafen am Rhein (DE); MAITY, Pulakesh, Navi Mumbai, 400705 (IN); WAKEHAM, Matthew Charles Linford, 67056 Ludwigshafen am Rhein (DE)
(74) Representative: AP-IP

(57) **Abstract**

The invention relates to heterocyclic compounds of formula I 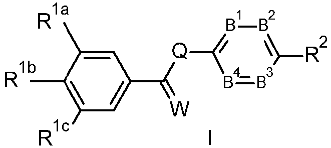 wherein the variables have the meanings as defined in the specification, to compositions comprising them, to active compound combinations comprising them, and to their use for protecting growing plants and animals from attack or infestation by invertebrate pests, furthermore, to seed comprising such compounds.

## Description

The invention relates to heterocyclic compounds of formula I 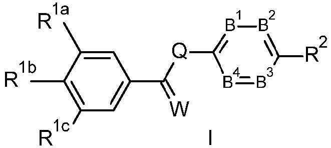 wherein
- B¹: is N or CR^{B1};
- B²: is N or CR^{B2};
- B³: is N or CR^{B3};
- B⁴: is N or CR^{B4};
with the proviso that not more than two of B¹, B², B³, or B⁴ are N;
- W: is O, S, or N-R^{N};
- R^{N}: is H, or C₁-C₄-alkyl, wherein the alkyl moiety is unsubstituted or substituted with halogen;
- Q: is a group Q¹, Q², Q³, Q⁴, or Q⁵ 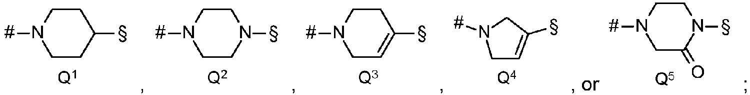 wherein # is the bond to the -C(W)- spacer, and § is the bond to the 6-membered ring;
- R^{B1}, R^{B2}, R^{B3}, and R^{B4}: independently of each other are H, halogen, N₃, OH, CN, NO₂, - SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₁-C₄-alkyl-C₃-C₆-cycloalkoxy, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e}, phenyl, phenoxy, phenylcarbonyl, phenylthio, or -CH₂-phenyl, wherein the phenyl rings are unsubstituted or substituted with R^{f};
- R^{1a}, R^{1b}, and R^{1c}: independently of each other are H, halogen, N₃, OH, CN, NO₂, -SCN, - SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e}, phenyl, phenoxy, phenylcarbonyl, phenylthio or -CH₂-phenyl, wherein phenyl rings are unsubstituted or substituted with R^{f}; or
R^{1a} and R^{1b}, or R^{1b} and R^{1c}, form together with the carbon atoms to which they are bonded a 5- or 6-membered partially unsaturated heterocyclic ring, which is unsubstituted or substituted with one or more halogen, CN, C₁-C₄-alkyl, or C₁-C₄-haloalkyl;
- m: is 0, 1, or 2;
- R²: is a moiety of formula X-Y-Z-T-R³; wherein
X is a single bond;
- (C(R^{xa})₂)ₚ-, wherein p is an integer of 1 to 3, preferably 1 or 2;
- (C(R^{xa})₂)ₒ-NR^{xc}-, wherein o is an integer of 1 or 2 and N is bound to Y; or
- NR^{xc}-;
Y is -CR^{ya}=N-, wherein the N is bound to Z;
- NR^{yc}-C(=O)-, wherein C(=O) is bound to Z; or
- NR^{yc}-C(=S)-, wherein C(=S) is bound to Z;
Z is -NR^{zc}-C(=O)-, wherein C(=O) is bound to T;
- NR^{zc}-C(=S)-, wherein C(=S) is bound to T;
- N=C(S-R^{za})-, wherein T is bound to the carbon atom; or
- NR^{zc}-C(S-R^{za})=, wherein T is bound to the carbon atom;
T is O, N or N-R^{T};
- R³: is aryl, aryl-C₁-C₄-alkyl, hetaryl, or hetaryl-C₁-C₄-alkyl, wherein the aryl or hetaryl rings are unsubstituted or substituted with one or more R^{g} and wherein the hetaryl is a 5- or 6-membered monocyclic hetaryl or a 8-, 9- or 10-membered bicyclic hetaryl; and wherein
- R^{xa}, R^{ya}: are, identical or different, H, halogen, C₁-C₆-alkyl, C₁-C₆-alkoxy, C₂-C₆-alkenyl, C₂-C₆-alkynyl, C₁-C₆-alkoxy-C₁-C₄-alkyl, C₃-C₆-cycloalkyl, C₁-C₄-alkyl-C₃-C₆-cycloalkyl, C₁-C₄-alkyl-C₃-C₆-cycloalkoxy, wherein the alkyl, alkoxy, alkenyl, alkynyl, cycloalkyl and cycloalkoxy moieties are unsubstituted or substituted with halogen;
- R^{xc}, R^{yc}, R^{zc}: are, identical or different, H, C₁-C₆-alkyl, C₂-C₆-alkenyl, C₂-C₆-alkynyl, C₁-C₄-alkyl-C₁-C₆-alkoxy, C₃-C₆-cycloalkyl, C₁-C₄-alkyl-C₃-C₆-cycloalkyl, C₁-C₄-alkyl-C₃-C₆-cycloalkoxy, or -NR^{b}R^{c}, wherein the alkyl, alkoxy, alkenyl, alkynyl, cycloalkyl and cycloalkoxy moieties are unsubstituted or substituted with halogen;
- R^{T}: is H, C₁-C₆-alkyl, C₂-C₆-alkenyl, C₂-C₆-alkynyl, C₁-C₄-alkyl-C₁-C₆-alkoxy, C₃-C₆-cycloalkyl, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen;
- R^{za}: is H, C₁-C₆-alkyl, C₁-C₆-alkoxy, C₂-C₆-alkenyl, tri-C₁-C₆-alkylsilyl, C₂-C₆-alkynyl, C₁-C₄-alkyl-C₁-C₆-alkoxy, C₃-C₆-cycloalkyl, C₁-C₄-alkyl-C₃-C₆-cycloalkoxy, C₁-C₄-alkyl-C₃-C₆-cycloalkyl, wherein the alkyl, alkoxy, alkenyl, alkynyl, cycloalkyl and cycloalkoxy moieties are unsubstituted or substituted with halogen; C₁-C₆-alkylene-NR^{b}R^{c}, C₁-C₆-alkylene-CN, C(=O)-NR^{b}R^{c}, C(=O)-R^{d}, phenyl, phenylcarbonyl, or -CH₂-phenyl, wherein the phenyl rings are unsubstituted or substituted with R^{f};
- R^{za}: together with R^{T} or R^{zc} if present, may form a linear C₁-C₆-alkylene or a linear C₂-C₆-alkenylene group, where in the linear C₁-C₆-alkylene and the linear C₂-C₆-alkenylene a CH₂ moiety is optionally replaced by a carbonyl and/or wherein 1 or 2 CH₂ moieties are optionally replaced by O or S and/or wherein the linear C₁-C₆-alkylene and the linear C₂-C₆-alkenylene are unsubstituted or substituted with one or more R^{h};
- R^{a}, R^{b} and R^{c}: are, identical or different, H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloal koxy-C₁-C₄-alkyl, wherein the alkyl, alkoxy, alkenyl, alkynyl, cycloalkyl and cycloalkoxy moieties are unsubstituted or substituted with halogen, C₁-C₆-alkylene-CN, phenyl, or -CH₂-phenyl, wherein the phenyl rings are unsubstituted or substituted with one or more R^{f};
- R^{d}: is H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen; phenyl, or -CH₂-phenyl, wherein the phenyl rings are unsubstituted or substituted with one or more R^{f};
- R^{e}: is C₁-C₆-alkyl, C₃-C₆-cycloalkyl, C₃-C₆-cycloalkyl-C₁-C₄-alkyl, wherein the alkyl, cycloalkyl moieties are unsubstituted or substituted with halogen; phenyl or -CH₂-phenyl, wherein the phenyl rings are unsubstituted or substituted with one or more R^{f};
- R^{f}: is halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, wherein the alkyl, alkoxy, alkenyl, alkynyl, cycloalkyl and cycloalkoxy moieties are unsubstituted or substituted with halogen;
- R^{g}: is halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₄-alkyl-C₁-C₆-alkoxy, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e};
- R^{h}: is halogen, OH, C₁-C₆-alkyl, C₃-C₆-cycloalkyl, or CN; and
the N-oxides, stereoisomers, tautomers, and agriculturally or veterinarily acceptable salts thereof.

The invention also provides an agricultural composition comprising at least one compound of formula I, a stereoisomer thereof and/or an agriculturally acceptable salt thereof and at least one liquid and/or solid carrier, especially at least one inert liquid and/or solid agriculturally acceptable carrier.

The invention also provides a veterinary composition comprising at least one compound of formula I, a stereoisomer thereof and/or a veterinarily acceptable salt thereof and at least one liquid and/or solid carrier, especially at least one inert veterinarily liquid and/or solid acceptable carrier.

The invention also provides a method for controlling invertebrate pests which method comprises treating the pests, their food supply, their habitat or their breeding ground or a cultivated plant, plant propagation materials (such as seed), soil, area, material or environment in which the pests are growing or may grow, or the materials, cultivated plants, plant propagation materials (such as seed), soils, surfaces or spaces to be protected from pest attack or infestation with a pesticidally effective amount of a compound of formula I or a salt thereof as defined herein.

The present invention also relates to plant propagation material, in particular seed, comprising at least one compound of formula I and/or an agriculturally acceptable salt thereof. The invention further relates to a method for treating or protecting an animal from infestation or infection by parasites which comprises bringing the animal in contact with a parasiticidally effective amount of a compound of formula I or a veterinarily acceptable salt thereof. Bringing the animal in contact with the compound I, its salt or the veterinary composition of the invention means applying or administering it to the animal.

WO 2013/009791, WO 2020/163146, WO2021/013561, and WO/2024/061768 describe structurally closely related active compounds. These compounds are mentioned to be useful for combating invertebrate pests.

Nevertheless, there remains a need for highly effective and versatile agents for combating invertebrate pests. It is therefore an object of the present invention to provide compounds having a good pesticidal activity and showing a broad activity spectrum against a large number of different invertebrate pests, especially against difficult to control pests, such as insects.

It has been found that these objects can be achieved by compounds of formula I as depicted and defined below, and by their stereoisomers, salts, tautomers and N-oxides, in particular their agriculturally acceptable salts.

Compounds of formula I, in particular wherein R² is R²-2 (compounds of formula II), can be obtained from Compounds III via a carbonyl substitution reaction with compound i. 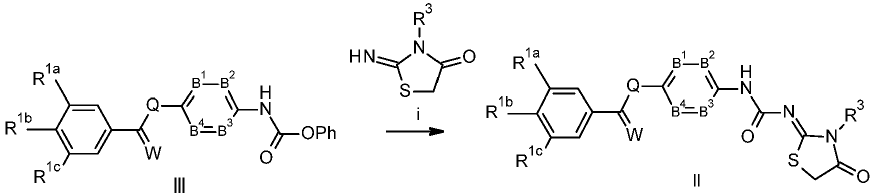 wherein Q is Q₁, Q₂, Q₃, or Q₄

This transformation is usually carried out at temperatures of from -20°C to +120 °C, preferably from 0°C to 25°C, in an inert solvent, in the presence of a base [cf. WO2020163146].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably ethers such as THF. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide, and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal carbonates, such as potassium carbonate. The bases are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of II, based on III.

Compounds II can be obtained from Compounds IV via a carbonyl addition reaction. Suitable reagents are carbonyl transfer reagents such as phosgene, diphosgene or carbonyldiimidazole. 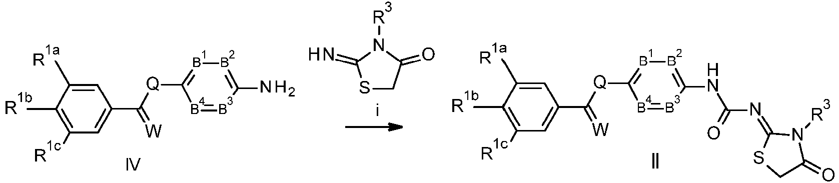 wherein Q is Q₅

This transformation is usually carried out at temperatures of from -20°C to +120°C, preferably from 0°C to 50°C, in an inert solvent, in the presence of a base [cf. WO2024061768].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, ketons such as acetone, methyl ethyl ketone, diethyl ketone, and tert.-butyl methyl ketone, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably nitrils such as acetonitrile. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide, and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal carbonates, such as potassium carbonate. The bases are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of I and/or the carbonylating agent, based on IV.

Compounds III can be obtained from Compounds IV via an acylation reaction. Suitable reagents are aryl chloroformates such as phenyl chloroformate. 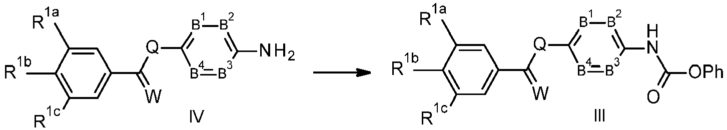 wherein Q is Q₁, Q₂, Q₃, or Q₄

This transformation is usually carried out at temperatures of from -20°C to +120°C, preferably from 0°C to 25°C, in an inert solvent, in the presence of a base [cf. WO2024061768].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, ketons such as acetone, methyl ethyl ketone, diethyl ketone, and tert.-butyl methyl ketone, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably ethers such as tetrahydrofuran (THF). It is also possible to use mixtures of the solvents mentioned.

Suitable bases are, in general, inorganic compounds, such as alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to organic bases, such as triethylamine. The bases are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of the chloroformate, based on IV.

Compounds IV can be obtained from Compounds V via a Suzuki reaction. Suitable reagents are compounds ii.

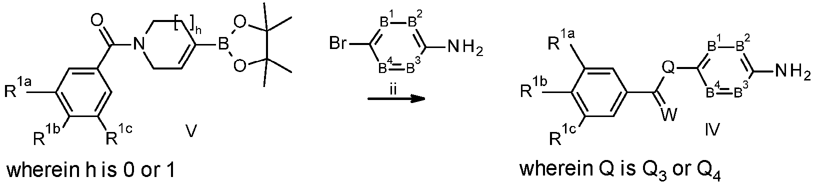

This transformation is usually carried out at temperatures of from 25°C to 120°C, preferably from 50°C to 110°C, in an inert solvent, in the presence of a base and a catalyst [cf. WO2013147183].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofura (THF), nitrils such as acetonitrile, and propionitrile, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), and water, preferably aromatic hydrocarbons such as toluene and water. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide, and magnesium oxide, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal carbonates, such potassium carbonate. The bases are generally employed in catalytic amounts; however, they can also be used in equimolar amounts, in excess or, if appropriate, as solvent.

Suitable catalysts are in general Palladium catalyst systems - preferably in combination with a phosphine ligand such as palladium acetate and triphenylphosphine, tetrakis(triphenylphosphine)palladium, dichloro[1,1'-bis(diphenylphosphino)ferrocene]palladium(II), dichlorobis(triphenylphosphine)palladium. The catalyst systems are generally employed in catalytic amounts; however, they can also be used in equimolar amounts, or in excess.

The starting materials are generally reacted with one another in equimolar amounts. In terms of yield, it may be advantageous to employ an excess of ii, based on V.

Compounds VI can be obtained from Compounds VII via an amide coupling reaction. Suitable reagents are compounds iii.

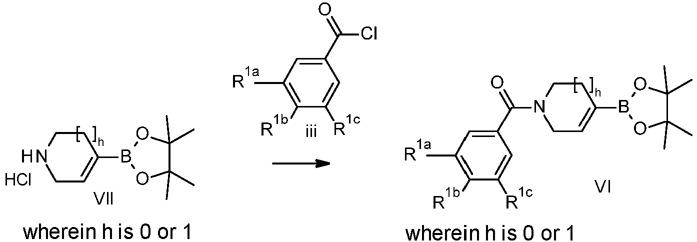

This transformation is usually carried out at temperatures of from -20°C to +120°C, preferably from 0°C to 80°C, in an inert solvent, in the presence of a base [cf. WO2021262684].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, moreover dimethyl sulphoxide (DMSO), dimethyl formamide (DMF), and dimethylacetamide (DMA), preferably aromatic hydrocarbons such as toluene and halogenated hydrocarbons such as methylene chloride. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also

alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal carbonates, such as potassium carbonate. The bases are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of iii, based on VII.

Compounds IV can be obtained from Compounds VIII via a reduction reaction. Suitable reducing agents are hydrogen gas in the presence of a metal catalyst or a nonprecious metal in the presence of acid, or other reducing agents such as sodium ditihionite. 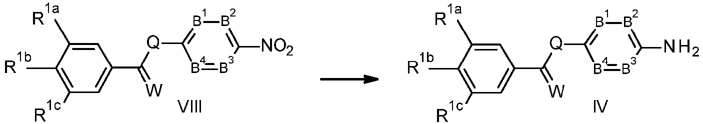 wherein Q is Q₁, Q₂, or Q₅

Conditions for Q₁ and Q₂:
This transformation is usually carried out at temperatures of from 0°C to 120°C, preferably from 25°C to 100°C, in an inert solvent, in the presence of a catalyst [cf. Long et al, European Journal of Medicinal Chemistry (2021), 213, 113215].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, ketons such as acetone, methyl ethyl ketone, diethyl ketone, and tert.-butyl methyl ketone, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), and water, preferably alcohols such as methanol. It is also possible to use mixtures of the solvents mentioned.

Suitable metal catalysts are, in general, metal catalysts, such as Palladium and platinum on charcoal, or Raney nickel. Particular preference is given to Raney nickel. The catalysts are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of catalyst, based on VII.

### Conditions for Q₅:

This transformation is usually carried out at temperatures of from -20°C to 120°C, preferably from 0°C to 110°C, in an inert solvent, in the presence of a reducing agent [cf. WO2013080222].

Suitable solvents are aliphatic hydrocarbons such as pentane, hexane, cyclohexane, and petrol ether, aromatic hydrocarbons such as toluene, o-, m-, and p-xylene, ethers such as diethylether, diisopropylether, tert.-butylmethylether (MTBE), dioxane, anisole, and tetrahydrofuran (THF), nitrils such as acetonitrile, and propionitrile, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), and water, preferably alcohols such as methanol. It is also possible to use mixtures of the solvents mentioned.

Suitable reducing agents are, in general, nonprecious metals in the presence of acid or ammonium chloride such as the following combinations Iron and zinc in the presence of ammonium chloride and acetic acid. The reducing agents are generally employed in stoichiometric amounts; however, they can also be used in excess or, if appropriate, as solvent.

Compounds IX can be obtained from Compounds X or from Compounds XI via an amide coupling reaction. Suitable reagents are acid chlorides such as compounds iv.

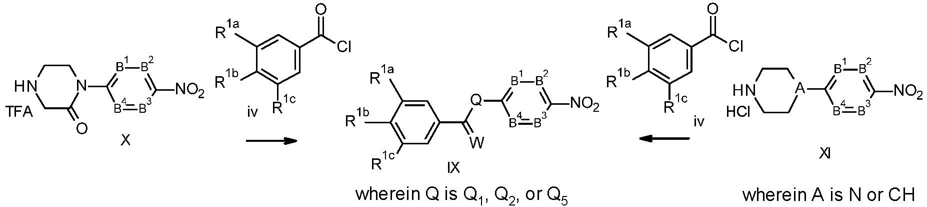

This transformation is usually carried out at temperatures of from -20°C to +120°C, preferably from 0°C to 80°C, in an inert solvent, in the presence of a base [cf. WO2021262684].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, moreover dimethyl sulphoxide (DMSO), dimethyl formamide (DMF), and dimethylacetamide (DMA), preferably aromatic hydrocarbons such as toluene and halogenated hydrocarbons such as methylene chloride. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also

alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal carbonates, such as potassium carbonate. The bases are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of iv, based on X or XI.

Compounds X can be obtained from Compounds XII via an Ullman coupling reaction. Suitable reagents are compounds v.

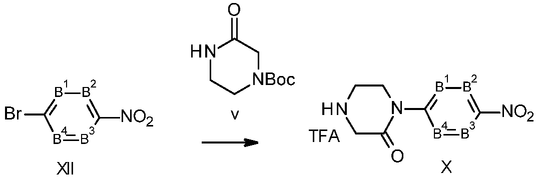

This transformation is usually carried out at temperatures of from 0°C to 120°C, preferably from 40°C to 120°C, in an inert solvent, in the presence of a base and a catalyst [cf. WO2023098656].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, ketons such as acetone, methyl ethyl ketone, diethyl ketone, and tert.-butyl methyl ketone, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably ethers such as dioxane. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide, and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, alkali metal phosphates such as sodium phosphate or potassium phosphate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal phosphates such as sodium phosphate. The bases are generally employed in catalytic amounts; however, they can also be used in equimolar amounts, in excess or, if appropriate, as solvent.

Suitable catalysts are in general Cuprous iodide, cuprous cyanide or cuprous oxide. The catalysts are generally employed in catalytic amounts; however, they can also be used in equimolar amounts, or in excess.

The starting materials are generally reacted with one another in equimolar amounts. In terms of yield, it may be advantageous to employ an excess of v, based on XII.

Compounds XI can be obtained from Compounds XIII via a nucleophilic aromatic substitution reaction. Suitable reagents are compounds vi. 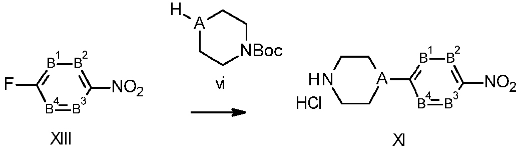 wherein A is N

This transformation is usually carried out at temperatures of from 25°C to 150°C, preferably from 50°C to 120°C, in an inert solvent, in the presence of a base [cf. Russell et al, RSC Advances (2015), 5(113), 93433-93437].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, ketons such as acetone, methyl ethyl ketone, diethyl ketone, and tert.-butyl methyl ketone, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably nitrils such as acetonitrile. It is also possible to use mixtures of the solvents mentioned.

Suitable bases are, in general, inorganic compounds, such as alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal carbonates, such as potassium carbonate. The bases are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of vi, based on XIII.

Compounds XIV can be obtained from Compounds XV via a cyclization reaction. Suitable reagents are alpha halogenated acetic acid derivatives.

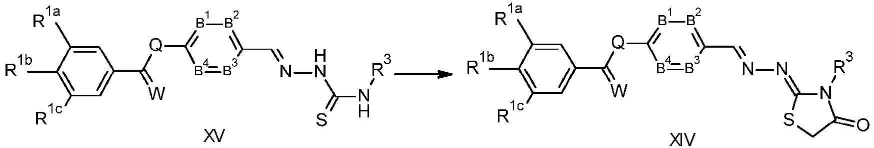

This transformation is usually carried out at temperatures of from -20°C to 120°C, preferably from 25°C to 110°C, in an inert solvent, in the presence of a base [cf. Trotsko et al, Molecules (2019), 24(17), 3029].

Suitable solvents are aliphatic hydrocarbons such as pentane, hexane, cyclohexane, and petrol ether, aromatic hydrocarbons such as toluene, o-, m-, and p-xylene, halogenated hydrocarbons such as methylene chloride, chloroform, and chlorobenzene, ethers such as diethylether, diisopropylether, tert.-butylmethylether (MTBE), dioxane, anisole, and tetrahydrofuran (THF), nitrils such as acetonitrile, and propionitrile, ketons such as acetone, methyl ethyl ketone, diethyl ketone, and tert.-butyl methyl ketone, alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably alcohols such as methanol. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal hydrides, such as lithium hydride, sodium hydride, potassium hydride and calcium hydride, alkali metal acetates such as sodium acetate, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to alkali metal acetates such as sodium acetate. The bases are generally employed in catalytic amounts; however, they can also be used in equimolar amounts, in excess or, if appropriate, as solvent.

The starting materials are generally reacted with one another in equimolar amounts. In terms of yield, it may be advantageous to employ an excess of alpha halogenated acetic acid derivatives, based on XV.

The starting materials required for preparing the compounds I are commercially available or known from the literature [cf. WO2020163146] or can be prepared in accordance with the literature cited.

The reaction mixtures are worked up in a customary manner, for example by mixing with water, separating the phases and, if appropriate, chromatographic purification of the crude products. Some of the intermediates and end products are obtained in the form of colorless or slightly brownish viscous oils which are purified or freed from volatile components under reduced pressure and at moderately elevated temperature. If the intermediates and end products are obtained as solids, purification can also be carried out by recrystallization or digestion.

If individual compounds I cannot be obtained by the routes described above, they can be prepared by derivatization of other compounds I.

However, if the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the invertebrate pest to be controlled.

The organic moieties groups mentioned in the above definitions of the variables are - like the term halogen - collective terms for individual listings of the individual group members. The prefix Cn-Cm indicates in each case the possible number of carbon atoms in the group.

The term "partially or fully substituted" by a radical means that in general the group is substituted with same or different radicals.

The term "halogen" denotes in each case fluorine, bromine, chlorine, or iodine, in particular fluo-rine, chlorine, or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl (Me), ethyl (Et), n-propyl (n-Pr), iso-propyl, n-butyl, 2-butyl, isobutyl, tert-butyl, n-pentyl, 1-me¬thylbutyl, 2 methylbutyl, 3 methylbutyl, 2,2-di-methylpropyl, 1 ethylpropyl, n-hexyl, 1,1-dimethyl-propyl, 1,2-dimethylpropyl, 1-methylpentyl, 2-methylpentyl, 3-methylpentyl, 4-methylpentyl, 1,1-dimethyl¬butyl, 1,2-dimethylbutyl, 1,3-dimethyl-butyl, 2,2-dimethylbutyl, 2,3-dimethylbutyl, 3,3-dimethyl¬butyl, 1-ethylbutyl, 2-ethylbutyl, 1,1,2-trimethylpropyl, 1,2,2-trimethylpropyl, 1-ethyl-1-methyl-propyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₄-C₄-halo-alkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2 difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 10 carbon atoms, frequently from 1 to 6 carbon atoms, preferably 1 to 4 carbon atoms. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to alkyl usually comprising 1 to 10, frequently 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 10 carbon atoms, frequently from 1 to 6 carbon atoms, preferably 1 to 4 carbon atoms, wherein the hydrogen atoms of this group are partially or totally replaced with halogen atoms, in particular fluorine atoms. Preferred haloalkoxy moieties include C₁-C₄-haloalkoxy, in particular C₁-C₂-fluoroalkoxy, such as fluoromethoxy, difluoromethoxy, trifluoromethoxy, 1 fluoroethoxy, 2-fluoroethoxy, 2,2 difluoroethoxy, 2,2,2-trifluoroethoxy, 2-chloro-2-fluoroethoxy, 2-chloro-2,2-difluoro-ethoxy, 2,2dichloro-2-fluorethoxy, 2,2,2-trichloroethoxy, penta-fluoroethoxy and the like.

The term "alkylthio "(alkylsulfanyl: alkyl-S-)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom.

The term "haloalkylthio" as used herein refers to an alkylthio group as mentioned above wherein the hydrogen atoms are partially or fully substituted by fluorine, chlorine, bromine and/or iodine.

The term "alkylsulfinyl" (alkylsulfoxyl: C₁-C₆-alkyl-S(O)-), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "haloalkylsulfinyl" as used herein refers to an alkylsulfinyl group as mentioned above wherein the hydrogen atoms are partially or fully substituted by fluorine, chlorine, bromine and/or iodine.

The term "alkylsulfonyl" (alkyl-S(O)₂-) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "haloalkylsulfonyl" as used herein refers to an alkylsulfonyl group as mentioned above wherein the hydrogen atoms are partially or fully substituted by fluorine, chlorine, bromine and/or iodine.

The term "alkylcarbonyl" refers to an alkyl group as defined above, which is bonded via the car-bon atom of a carbonyl group (C=O) to the remainder of the molecule.

The term "haloalkylcarbonyl" refers to an alkylcarbonyl group as mentioned above, wherein the hydrogen atoms are partially or fully substituted by fluorine, chlorine, bromine and/or iodine.

The term "alkoxycarbonyl" refers to an alkylcarbonyl group as defined above, which is bonded via an oxygen atom to the remainder of the molecule.

The term "haloalkoxycarbonyl" refers to an alkoxycarbonyl group as mentioned above, wherein the hydrogen atoms are partially or fully substituted by fluorine, chlorine, bromine and/or iodine.

The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2 propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the hydrogen atoms are partially or totally replaced with halogen atoms.

The term "alkynyl" as used herein denotes in each case a singly unsaturated hydrocarbon radi-cal having usually 2 to 10, frequently 2 to 6, preferably 2 to 4 carbon atoms, e.g. ethynyl, propar-gyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like.

The term "haloalkynyl" as used herein refers to an alkynyl group as defined above, wherein the hydrogen atoms are partially or totally replaced with halogen atoms.

The term "cycloalkyl" as used herein and in the cycloalkyl moieties of cycloalkoxy and cyclo-alkylthio denotes in each case a monocyclic cycloaliphatic radical having usually from 3 to 10 or from 3 to 6 carbon atoms, such as cyclopropyl (c-C₃H₅), cyclobutyl, cyclopentyl, cyclohexyl, cy-cloheptyl, cyclooctyl, cyclononyl and cyclodecyl or cyclopropyl, cyclobutyl, cyclopentyl and cyclo-hexyl.

The term "halocycloalkyl" as used herein and in the halocycloalkyl moieties of halocycloalkoxy and halocycloalkylthio denotes in each case a monocyclic cycloaliphatic radical having usually from 3 to 10 C atoms or 3 to 6 C atoms, wherein at least one, e.g. 1, 2, 3, 4 or 5 of the hydrogen atoms, are replaced by halogen, in particular by fluorine or chlorine. Examples are 1- and 2-flu-orocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "halocycloalkenyl" as used herein and in the halocycloalkenyl moieties of halocycloalkenyloxy and halocycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms, wherein at least one, e.g. 1, 2, 3, 4 or 5 of the hydrogen atoms, are replaced by halogen, in particular by fluorine or chlorine. Examples are 3,3-difluorocyclopropen-1-yl and 3,3-dichlorocyclopropen-1-yl.

The term "cycloalkenylalkyl" refers to a cycloalkenyl group as defined above which is bonded via an alkyl group, such as a C₁-C₅-alkyl group or a C₁-C₄-alkyl group, in particular a methyl group (= cycloalkenylmethyl), to the remainder of the molecule.

The term "carbocycle" or "carbocyclyl" includes in general a 3- to 12-membered, preferably a 3- to 8-membered or a 5- to 8-membered, more preferably a 5- or 6-membered monocyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 3- to 6-membered, in particular 6-membered monocyclic heterocyclic non-aromatic radicals. The heterocyclic non-aromatic radicals usually comprise 1, 2, 3, 4 or 5, preferably 1, 2 or 3 hetero¬atoms selected from N, O and S as ring members, wherein S-atoms as ring members may be present as S, SO or SO₂. Examples of 5- or 6-membered heterocyclic radicals comprise satu¬rated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxo-thiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxodihydrothiopyranyl, S-dioxodihydrothiopyranyl, tetrahydrothiopyranyl, S-oxotetrahydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-only, and the like.

The term "hetaryl" includes monocyclic 5- or 6-membered heteroaromatic radicals comprising as ring members 1, 2, 3 or 4 heteroatoms selected from N, O and S. N- or S-containing hetaryl groups may be present as positively charged onium, and form together with a neighbouring atom a mesoionic entity. Examples of 5- or 6 membered heteroaromatic radicals include pyridyl, i.e. 2-, 3-, or 4 pyridyl, pyrimidinyl, i.e. 2 , 4- or 5-pyrimidinyl, pyrazinyl, pyridazinyl, i.e. 3- or 4 pyridazinyl, thienyl, i.e. 2- or 3-thienyl, furyl, i.e. 2-or 3-furyl, pyrrolyl, i.e. 2- or 3 pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5 isothiazolyl, pyrazolyl, i.e. 1-, 3-, 4- or 5-pyrazolyl, i.e. 1-, 2-, 4- or 5-imidazolyl, oxadiazolyl, e.g. 2- or 5 [1,3,4]oxadiazolyl, 4- or 5-(1,2,3-oxa¬diazol)yl, 3- or 5-(1,2,4-oxadiazol)yl, 2- or 5 (1,3,4-thiadiazol)yl, thiadiazolyl, e.g. 2- or 5-(1,3,4-thia¬diazol)yl, 4- or 5 (1,2,3 thiadiazol)yl, 3- or 5-(1,2,4-thiadiazol)yl, triazolyl, e.g. 1H-, 2H- or 3H 1,2,3 triazol-4-yl, 2H-triazol-3-yl, 1H-, 2H-, or 4H-1,2,4-triazolyl and tetrazolyl, i.e. 1H- or 2H tetrazolyl. The term "hetaryl" also includes bicyclic 8 to 10-membered heteroaromatic radicals comprising as ring members 1, 2 or 3 heteroatoms selected from N, O and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzo-thienyl, indolyl, ind¬azolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2 d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety.

The terms "heterocyclylalkyl" and "hetarylalkyl" refer to heterocyclyl or hetaryl, respectively, as defined above which are bonded via a C₁-C₅-alkyl group or a C₁-C₄-alkyl group, in particular a methyl group (= heterocyclylmethyl or hetarylmethyl, respectively), to the remainder of the mole-cule.

The term "arylalkyl" and "phenylalkyl" refer to aryl as defined above and phenyl, respectively, which are bonded via C₁-C₅-alkyl group or a C₁-C₄-alkyl group, in particular a methyl group (= arylmethyl or phenylmethyl), to the remainder of the molecule, examples including benzyl, 1-phenylethyl, 2-phenylethyl, 2-phenoxyethyl etc.

The terms "alkylene", "cycloalkylene", "heterocycloalkylene", "alkenylene", "cycloalkenylene", "heterocycloalkenylene" and "alkynylene" refer to alkyl, cycloalkyl, heterocycloalkyl, alkenyl, cy-cloalkenyl, heterocycloalkenyl and alkynyl as defined above, respectively, which are bonded to the remainder of the molecule, via two atoms, preferably via two carbon atoms, of the respective group, so that they represent a linker between two moieties of the molecule.

In a particular embodiment, the variables of the compounds of the formula I have the following meanings, these meanings, both on their own and in combination with one another, being particular embodiments of the compounds of the formula I.

Embodiments and preferred compounds of the invention for use in pesticidal methods and for insecticidal application purposes are outlined in the following paragraphs.

With respect to the variables, embodiments of the intermediates correspond to those of the compounds of the formula I.

In various embodiments, R² is R²-1, and W is O. Such compounds correspond to compounds of formula II.A

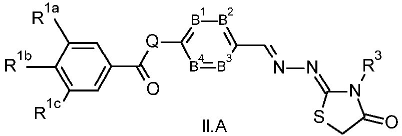

In various embodiments, R² is R²-2, and W is O. Such compounds correspond to compounds of formula II.B

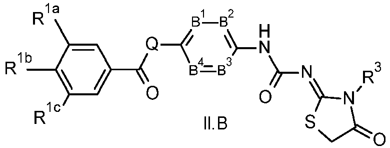

In various embodiments, R² is R²-4, and W is O. Such compounds correspond to compounds of formula II.C

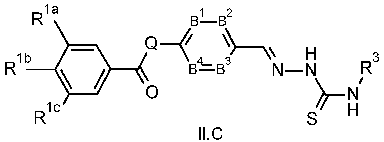

In various embodiments, B¹ is CR^{B1}, B² is CR^{B2}, B³ is CR^{B3}, and B⁴ is CR^{B4}.

In various embodiments, B¹ is N, B² is CR^{B2}, B³ is CR^{B3}, and B⁴ is CR^{B4}.

In various embodiments, B¹ is CR^{B1}, B² is N, B³ is CR^{B3}, and B⁴ is CR^{B4}.

In various embodiments, B¹ is N, B² is CR^{B2}, B³ is CR^{B3}, and B⁴ is N.

In various embodiments, B¹ is N, B² is CR^{B2}, B³ is N, and B⁴ is CR^{B4}.

In various embodiments, B¹ is CR^{B1}, B² is N, B³ is N, and B⁴ is CR^{B4}.

In various embodiments, B¹ is CH, B² is CH, B³ is CH, and B⁴ is CH.

In various embodiments, B¹ is N, B² is CH, B³ is CH, and B⁴ is CH.

In various embodiments, B¹ is CH, B² is N, B³ is CH, and B⁴ is CH.

In various embodiments, B¹ is N, B² is CH, B³ is CH, and B⁴ is N.

In various embodiments, B¹ is N, B² is CH, B³ is N, and B⁴ is CH.

In various embodiments, B¹ is CH, B² is N, B³ is N, and B⁴ is CH.

In various embodiments, B¹ is CCl, B² is CH, B³ is CH, and B⁴ is CH.

In various embodiments, B¹ is CH, B² is CCI, B³ is CH, and B⁴ is CH.

In various embodiments, B¹ is CF, B² is CH, B³ is CH, and B⁴ is CH.

In various embodiments, B¹ is CH, B² is CF, B³ is CH, and B⁴ is CH.

In various embodiments, X is a single bond, Y is -CR^{ya}=N-, wherein the N is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and R^{ya} is preferably H.

In various embodiments, X is a single bond, Y is -NR^{yc}-C(=O)-, wherein C(=O) is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and R^{yc} is preferably H.

In various embodiments, X is -(C(R^{xa})₂)ₚ-, wherein p is 2, Y is -NR^{yc}-C(=O)-, wherein C(=O) is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and all R^{xa} and R^{yc} are preferably H.

In various embodiments, X is -(C(R^{xa})₂)ₒ-NR^{xc}-, wherein o is 1 and N is bound to Y, Y is -NR^{yc}-C(=O)-, wherein C(=O) is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, wherein one R^{xa}, R^{xc} and R^{yc} are preferably H and the other R^{xa} is preferably methyl, or wherein one R^{xa} and R^{yc} are preferably H and the other R^{xa} and R^{xc} are preferably methyl.

In various embodiments, X is -(C(R^{xa})₂)ₒ-NR^{xc}-, wherein o is 1 and N is bound to Y, Y is -NR^{yc}-C(=O)-, wherein C(=O) is bound to Z, Z is -NR^{zc}-C(=S)-, wherein C(=S) is bound to T, and T is N-R^{T}, wherein one R^{xa}, R^{xc}, R^{yc}, R^{zc} and R^{T} are preferably H and the other R^{xa} is preferably methyl, or wherein one R^{xa}, R^{yc}, R^{zc} and R^{T} are preferably H and the other R^{xa} and R^{xc} are preferably methyl.

In various embodiments, X is -(C(R^{xa})₂)ₚ-, wherein p is 1, Y is -NR^{yc}-C(=O)-, wherein C(=O) is bound to Z, Z is -NR^{zc}-C(=S)-, wherein C(=S) is bound to T, and T is N-R^{T}, wherein one R^{xa}, R^{zc} and R^{T} are preferably H, the other R^{xa} is preferably methyl, and R^{yc} is preferably -NH₂.

In various embodiments, X is -(C(R^{xa})₂)ₒ-NR^{xc}-, wherein o is 1 and N is bound to Y, Y is -NR^{yc}-C(=O)-, wherein C(=O) is bound to Z, Z is -NR^{zc}-C(=S)-, wherein C(=S) is bound to T, and T is N-R^{T}, wherein one R^{xa}, R^{yc}, R^{zc} and R^{T} are preferably H and the other R^{xa} and R^{xc} are preferably methyl.

In various embodiments, X is -(C(R^{xa})₂)ₒ-NR^{xc}-, wherein o is 1 and N is bound to Y, Y is -NR^{yc}-C(=O)-, wherein C(=O) is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and one R^{xa} and R^{yc} are preferably H and the other R^{xa} and R^{xc} are preferably methyl.

In various embodiments, X is a single bond, Y is -CR^{ya}=N-, wherein the N is bound to Z, Z is -NR^{zc}-C(=S)-, wherein C(=S) is bound to T and T is N-R^{T}, wherein R^{ya}, R^{zc}, and R^{T} are preferably H.

In various embodiments, X is a single bond, Y is -NR^{yc}-C(=O)-, wherein C(=O) is bound to Z, Z is -NR^{zc}-C(=S)-, wherein C(=S) is bound to T and T is N-R^{T}, wherein R^{yc}, R^{zc}, and R^{T} are preferably H.

In various embodiments, R³ is monocyclic or bicyclic aryl substituted with one or more R^{g}, preferably phenyl, naphthyl, or pyridinyl optionally substituted with one or more R^{g}.

In various embodiments, R^{B1}, R^{B2}, R^{B3}, and R^{B4} independently of each other are H, halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₄-alkyl-C₁-C₆-alkoxy, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₁-C₄-alkyl-C₃-C₆-cycloalkoxy, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e}, phenyl, phenoxy, phenylcarbonyl, phenylthio, or -CH₂-phenyl, wherein the phenyl rings are unsubstituted or substituted with R^{f};

In various embodiments, R^{1a}, R^{1b}, and R^{1c} independently of each other are H, halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₄-alkyl-C₁-C₆-alkoxy, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e}, phenyl, phenoxy, phenylcarbonyl, phenylthio or -CH₂-phenyl, wherein phenyl rings are unsubstituted or substituted with R^{f};

In various embodiments, R^{1a} and R^{1b}, or R^{1b} and R^{1c}, form together with the carbon atoms to which they are bonded a 5- or 6-membered partially unsaturated heterocyclic ring, which contains 1 or 2 heteroatoms selected from N, O, S, and which is unsubstituted or substituted with one or more halogen, CN, C₁-C₄-alkyl, or C₁-C₄-haloalkyl; preferably wherein the heterocyclic ring contains two O atoms.

In various embodiments, R^{1a}, R^{1b}, and R^{1c} independently of each other are halogen, OH, C₁-C₆-alkyl, C₁-C₆-haloalkyl, C₁-C₆-alkoxy, C₁-C₆-haloalkoxy, or S-R^{e}.

In various embodiments, R^{e} is C₁-C₆-alkyl, C₃-C₆-cycloalkyl, C₃-C₆-cycloalkyl-C₁-C₄-alkyl, wherein the alkyl, cycloalkyl moieties are unsubstituted or substituted with halogen.

In various embodiments, R^{B1}, R^{B2}, R^{B3}, and R^{B4} independently of each other are H, halogen, C₁-C₆-alkyl, C₁-C₆-alkoxy, C₂-C₆-alkenyl, C₂-C₆-alkynyl, wherein the alkyl, alkoxy, alkenyl, alkynyl, moieties are unsubstituted or substituted with halogen, preferably H, F, Cl, Br, OCF₃, OCHF₂, OCH₃, or OCH₂CH₃.

In various embodiments, R^{1a}, R^{1b}, and R^{1c} independently of each other are halogen, OH, C₁-C₆-alkyl, C₁-C₆-haloalkyl, C₁-C₆-alkoxy, C₁-C₆-haloalkoxy, or S-R^{e}; and
R^{e} is C₁-C₆-alkyl, C₃-C₆-cycloalkyl, C₃-C₆-cycloalkyl-C₁-C₄-alkyl, wherein the alkyl, cycloalkyl moieties are unsubstituted or substituted with halogen; and
R^{B1}, R^{B2}, R^{B3}, and R^{B4} independently of each other are H, halogen, C₁-C₆-alkyl, C₁-C₆-alkoxy, C₂-C₆-alkenyl, C₂-C₆-alkynyl, wherein the alkyl, alkoxy, alkenyl, alkynyl, moieties are unsubstituted or substituted with halogen.

In various embodiments, R² is selected from the following groups

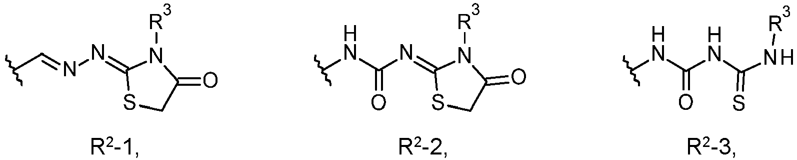

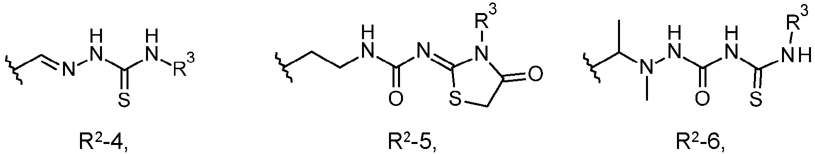

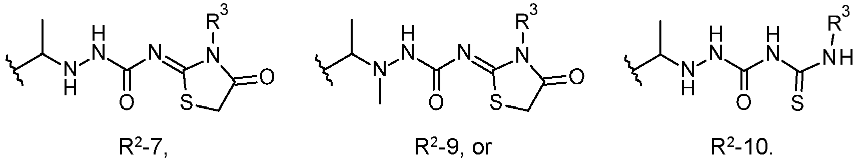

In various embodiments, R³ is selected from the following groups 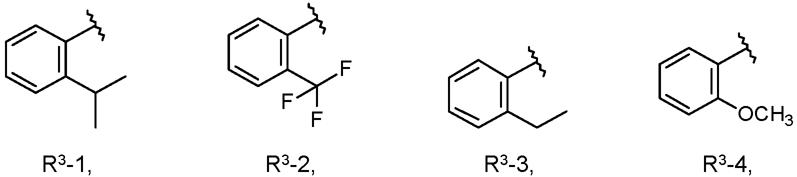 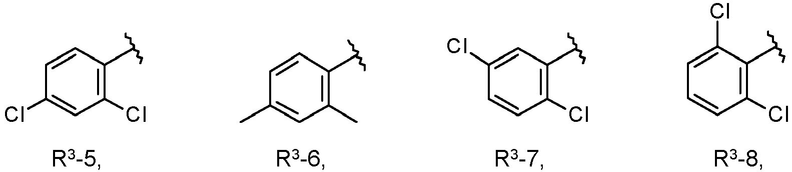 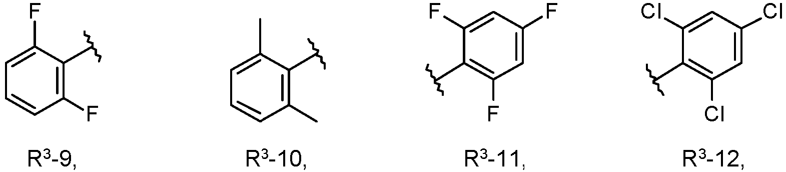 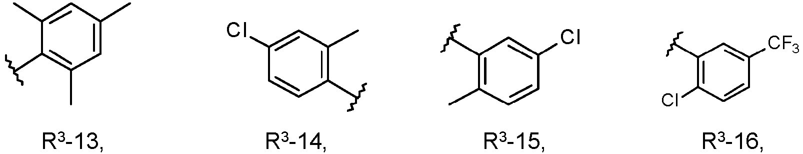 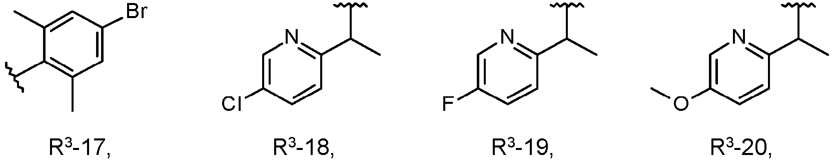 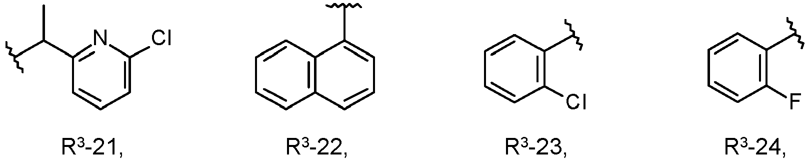 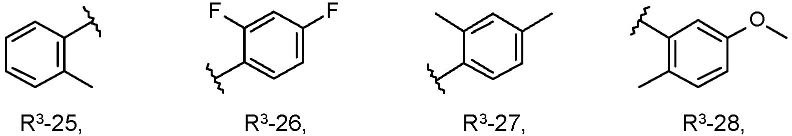 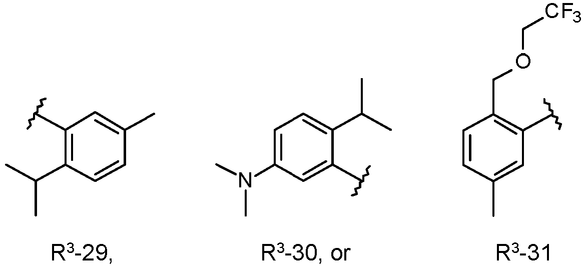 preferably, wherein R³ is R³-1, R³-29, R³-30, or R³-31.

In one preferred embodiment, the invention relates to compounds of Table I.

In particular with a view to their use, preference is given to the compounds of formula I compiled in the tables below. Each of the groups mentioned for a substituent in the tables is furthermore per se, independently of the combination in which it is mentioned, a particularly preferred aspect of the substituent in question.

Table 1 : Compounds of formula I in which R^{1a} is H, R^{1c} is H, W is O, R² is R²-1, R³ is R³-1, and the combination of R^{1b}, Q, B¹, B², B³, and B⁴ for a compound corresponds in each case to one row of Table A.

Table 2 : Compounds of formula I in which R^{1a} is H, R^{1c} is H, W is O, R² is R²-2, R³ is R³-1, and the combination of R^{1b}, Q, B¹, B², B³, and B⁴ for a compound corresponds in each case to one row of Table A.

Table 3 : Compounds of formula I in which R^{1a} is H, R^{1c} is H, W is O, R² is R²-3, R³ is R³-1, and the combination of R^{1b}, Q, B¹, B², B³, and B⁴ for a compound corresponds in each case to one row of Table A.

Table 4 : Compounds of formula I in which R^{1a} is H, R^{1c} is H, W is O, R² is R²-4, R³ is R³-1, and the combination of R^{1b}, Q, B¹, B², B³, and B⁴ for a compound corresponds in each case to one row of Table A.

Table 5 : Compounds of formula I in which R^{1a} is H, R^{1c} is H, W is O, R² is R²-5, R³ is R³-1, and the combination of R^{1b}, Q, B¹, B², B³, and B⁴ for a compound corresponds in each case to one row of Table A.

Table 6 : Compounds of formula I in which R^{1a} is H, R^{1c} is H, W is O, R² is R²-6, R³ is R³-1, and the combination of R^{1b}, Q, B¹, B², B³, and B⁴ for a compound corresponds in each case to one row of Table A.

Table 7 : Compounds of formula I in which R^{1a} is H, R^{1c} is H, W is O, R² is R²-7, R³ is R³-1, and the combination of R^{1b}, Q, B¹, B², B³, and B⁴ for a compound corresponds in each case to one row of Table A.

Table 8 : Compounds of formula I in which R^{1a} is H, R^{1c} is H, W is O, R² is R²-8, R³ is R³-1, and the combination of R^{1b}, Q, B¹, B², B³, and B⁴ for a compound corresponds in each case to one row of Table A.

Table 9 : Compounds of formula I in which R^{1a} is H, R^{1c} is H, W is O, R² is R²-9, R³ is R³-1, and the combination of R^{1b}, Q, B¹, B², B³, and B⁴ for a compound corresponds in each case to one row of Table A.

Table 10 : Compounds of formula I in which R^{1a} is H, R^{1c} is H, W is O, R² is R²-10, R³ is R³-1, and the combination of R^{1b}, Q, B¹, B², B³, and B⁴ for a compound corresponds in each case to one row of Table A.

Table 11 : Compounds of formula I in which R^{1a} is H, R^{1c} is H, W is O, R² is R²-1, R³ is R³-29, and the combination of R^{1b}, Q, B¹, B², B³, and B⁴ for a compound corresponds in each case to one row of Table A.

Table 12 : Compounds of formula I in which R^{1a} is H, R^{1c} is H, W is O, R² is R²-2, R³ is R³-29, and the combination of R^{1b}, Q, B¹, B², B³, and B⁴ for a compound corresponds in each case to one row of Table A.

Table 13 : Compounds of formula I in which R^{1a} is H, R^{1c} is H, W is O, R² is R²-3, R³ is R³-29, and the combination of R^{1b}, Q, B¹, B², B³, and B⁴ for a compound corresponds in each case to one row of Table A.

Table 14 : Compounds of formula I in which R^{1a} is H, R^{1c} is H, W is O, R² is R²-4, R³ is R³-29, and the combination of R^{1b}, Q, B¹, B², B³, and B⁴ for a compound corresponds in each case to one row of Table A.

Table 15 : Compounds of formula I in which R^{1a} is H, R^{1c} is H, W is O, R² is R²-5, R³ is R³-29, and the combination of R^{1b}, Q, B¹, B², B³, and B⁴ for a compound corresponds in each case to one row of Table A.

Table 16 : Compounds of formula I in which R^{1a} is H, R^{1c} is H, W is O, R² is R²-6, R³ is R³-29, and the combination of R^{1b}, Q, B¹, B², B³, and B⁴ for a compound corresponds in each case to one row of Table A.

Table 17 : Compounds of formula I in which R^{1a} is H, R^{1c} is H, W is O, R² is R²-7, R³ is R³-29, and the combination of R^{1b}, Q, B¹, B², B³, and B⁴ for a compound corresponds in each case to one row of Table A.

Table 18 : Compounds of formula I in which R^{1a} is H, R^{1c} is H, W is O, R² is R²-8, R³ is R³-29, and the combination of R^{1b}, Q, B¹, B², B³, and B⁴ for a compound corresponds in each case to one row of Table A.

Table 19 : Compounds of formula I in which R^{1a} is H, R^{1c} is H, W is O, R² is R²-9, R³ is R³-29, and the combination of R^{1b}, Q, B¹, B², B³, and B⁴ for a compound corresponds in each case to one row of Table A.

Table 20 : Compounds of formula I in which R^{1a} is H, R^{1c} is H, W is O, R² is R²-10, R³ is R³-29, and the combination of R^{1b}, Q, B¹, B², B³, and B⁴ for a compound corresponds in each case to one row of Table A.

Table 21 : Compounds of formula I in which R^{1a} is H, R^{1c} is H, W is O, R² is R²-1, R³ is R³-30, and the combination of R^{1b}, Q, B¹, B², B³, and B⁴ for a compound corresponds in each case to one row of Table A.

Table 22 : Compounds of formula I in which R^{1a} is H, R^{1c} is H, W is O, R² is R²-2, R³ is R³-30, and the combination of R^{1b}, Q, B¹, B², B³, and B⁴ for a compound corresponds in each case to one row of Table A.

Table 23 : Compounds of formula I in which R^{1a} is H, R^{1c} is H, W is O, R² is R²-3, R³ is R³-30, and the combination of R^{1b}, Q, B¹, B², B³, and B⁴ for a compound corresponds in each case to one row of Table A.

Table 24 : Compounds of formula I in which R^{1a} is H, R^{1c} is H, W is O, R² is R²-4, R³ is R³-30, and the combination of R^{1b}, Q, B¹, B², B³, and B⁴ for a compound corresponds in each case to one row of Table A.

Table 25 : Compounds of formula I in which R^{1a} is H, R^{1c} is H, W is O, R² is R²-5, R³ is R³-30, and the combination of R^{1b}, Q, B¹, B², B³, and B⁴ for a compound corresponds in each case to one row of Table A.

Table 26 : Compounds of formula I in which R^{1a} is H, R^{1c} is H, W is O, R² is R²-6, R³ is R³-30, and the combination of R^{1b}, Q, B¹, B², B³, and B⁴ for a compound corresponds in each case to one row of Table A.

Table 27 : Compounds of formula I in which R^{1a} is H, R^{1c} is H, W is O, R² is R²-7, R³ is R³-30, and the combination of R^{1b}, Q, B¹, B², B³, and B⁴ for a compound corresponds in each case to one row of Table A.

Table 28 : Compounds of formula I in which R^{1a} is H, R^{1c} is H, W is O, R² is R²-8, R³ is R³-30, and the combination of R^{1b}, Q, B¹, B², B³, and B⁴ for a compound corresponds in each case to one row of Table A.

Table 29 : Compounds of formula I in which R^{1a} is H, R^{1c} is H, W is O, R² is R²-9, R³ is R³-30, and the combination of R^{1b}, Q, B¹, B², B³, and B⁴ for a compound corresponds in each case to one row of Table A.

Table 30 : Compounds of formula I in which R^{1a} is H, R^{1c} is H, W is O, R² is R²-10, R³ is R³-30, and the combination of R^{1b}, Q, B¹, B², B³, and B⁴ for a compound corresponds in each case to one row of Table A.

Table 31 : Compounds of formula I in which R^{1a} is H, R^{1c} is H, W is O, R² is R²-1, R³ is R³-31, and the combination of R^{1b}, Q, B¹, B², B³, and B⁴ for a compound corresponds in each case to one row of Table A.

Table 32 : Compounds of formula I in which R^{1a} is H, R^{1c} is H, W is O, R² is R²-2, R³ is R³-31, and the combination of R^{1b}, Q, B¹, B², B³, and B⁴ for a compound corresponds in each case to one row of Table A.

Table 33 : Compounds of formula I in which R^{1a} is H, R^{1c} is H, W is O, R² is R²-3, R³ is R³-31, and the combination of R^{1b}, Q, B¹, B², B³, and B⁴ for a compound corresponds in each case to one row of Table A.

Table 34 : Compounds of formula I in which R^{1a} is H, R^{1c} is H, W is O, R² is R²-4, R³ is R³-31, and the combination of R^{1b}, Q, B¹, B², B³, and B⁴ for a compound corresponds in each case to one row of Table A.

Table 35 : Compounds of formula I in which R^{1a} is H, R^{1c} is H, W is O, R² is R²-5, R³ is R³-31, and the combination of R^{1b}, Q, B¹, B², B³, and B⁴ for a compound corresponds in each case to one row of Table A.

Table 36 : Compounds of formula I in which R^{1a} is H, R^{1c} is H, W is O, R² is R²-6, R³ is R³-31, and the combination of R^{1b}, Q, B¹, B², B³, and B⁴ for a compound corresponds in each case to one row of Table A.

Table 37 : Compounds of formula I in which R^{1a} is H, R^{1c} is H, W is O, R² is R²-7, R³ is R³-31, and the combination of R^{1b}, Q, B¹, B², B³, and B⁴ for a compound corresponds in each case to one row of Table A.

Table 38 : Compounds of formula I in which R^{1a} is H, R^{1c} is H, W is O, R² is R²-8, R³ is R³-31, and the combination of R^{1b}, Q, B¹, B², B³, and B⁴ for a compound corresponds in each case to one row of Table A.

Table 39 : Compounds of formula I in which R^{1a} is H, R^{1c} is H, W is O, R² is R²-9, R³ is R³-31, and the combination of R^{1b}, Q, B¹, B², B³, and B⁴ for a compound corresponds in each case to one row of Table A.

Table 40 : Compounds of formula I in which R^{1a} is H, R^{1c} is H, W is O, R² is R²-10, R³ is R³-31, and the combination of R^{1b}, Q, B¹, B², B³, and B⁴ for a compound corresponds in each case to one row of Table A.

Table 41 : Compounds of formula I in which R^{1a} is F, R^{1c} is H, W is O, R² is R²-1, R³ is R³-1, and the combination of R^{1b}, Q, B¹, B², B³, and B⁴ for a compound corresponds in each case to one row of Table A.

Table 42 : Compounds of formula I in which R^{1a} is F, R^{1c} is H, W is O, R² is R²-2, R³ is R³-1, and the combination of R^{1b}, Q, B¹, B², B³, and B⁴ for a compound corresponds in each case to one row of Table A.

Table 43 : Compounds of formula I in which R^{1a} is F, R^{1c} is H, W is O, R² is R²-3, R³ is R³-1, and the combination of R^{1b}, Q, B¹, B², B³, and B⁴ for a compound corresponds in each case to one row of Table A.

Table 44 : Compounds of formula I in which R^{1a} is F, R^{1c} is H, W is O, R² is R²-4, R³ is R³-1, and the combination of R^{1b}, Q, B¹, B², B³, and B⁴ for a compound corresponds in each case to one row of Table A.

Table 45 : Compounds of formula I in which R^{1a} is F, R^{1c} is H, W is O, R² is R²-5, R³ is R³-1, and the combination of R^{1b}, Q, B¹, B², B³, and B⁴ for a compound corresponds in each case to one row of Table A.

Table 46 : Compounds of formula I in which R^{1a} is F, R^{1c} is H, W is O, R² is R²-6, R³ is R³-1, and the combination of R^{1b}, Q, B¹, B², B³, and B⁴ for a compound corresponds in each case to one row of Table A.

Table 47 : Compounds of formula I in which R^{1a} is F, R^{1c} is H, W is O, R² is R²-7, R³ is R³-1, and the combination of R^{1b}, Q, B¹, B², B³, and B⁴ for a compound corresponds in each case to one row of Table A.

Table 48 : Compounds of formula I in which R^{1a} is F, R^{1c} is H, W is O, R² is R²-8, R³ is R³-1, and the combination of R^{1b}, Q, B¹, B², B³, and B⁴ for a compound corresponds in each case to one row of Table A.

Table 49 : Compounds of formula I in which R^{1a} is F, R^{1c} is H, W is O, R² is R²-9, R³ is R³-1, and the combination of R^{1b}, Q, B¹, B², B³, and B⁴ for a compound corresponds in each case to one row of Table A.

Table 50 : Compounds of formula I in which R^{1a} is F, R^{1c} is H, W is O, R² is R²-10, R³ is R³-1, and the combination of R^{1b}, Q, B¹, B², B³, and B⁴ for a compound corresponds in each case to one row of Table A.

Table 51 : Compounds of formula I in which R^{1a} is F, R^{1c} is H, W is O, R² is R²-1, R³ is R³-29, and the combination of R^{1b}, Q, B¹, B², B³, and B⁴ for a compound corresponds in each case to one row of Table A.

Table 52 : Compounds of formula I in which R^{1a} is F, R^{1c} is H, W is O, R² is R²-2, R³ is R³-29, and the combination of R^{1b}, Q, B¹, B², B³, and B⁴ for a compound corresponds in each case to one row of Table A.

Table 53 : Compounds of formula I in which R^{1a} is F, R^{1c} is H, W is O, R² is R²-3, R³ is R³-29, and the combination of R^{1b}, Q, B¹, B², B³, and B⁴ for a compound corresponds in each case to one row of Table A.

Table 54 : Compounds of formula I in which R^{1a} is F, R^{1c} is H, W is O, R² is R²-4, R³ is R³-29, and the combination of R^{1b}, Q, B¹, B², B³, and B⁴ for a compound corresponds in each case to one row of Table A.

Table 55 : Compounds of formula I in which R^{1a} is F, R^{1c} is H, W is O, R² is R²-5, R³ is R³-29, and the combination of R^{1b}, Q, B¹, B², B³, and B⁴ for a compound corresponds in each case to one row of Table A.

Table 56 : Compounds of formula I in which R^{1a} is F, R^{1c} is H, W is O, R² is R²-6, R³ is R³-29, and the combination of R^{1b}, Q, B¹, B², B³, and B⁴ for a compound corresponds in each case to one row of Table A.

Table 57 : Compounds of formula I in which R^{1a} is F, R^{1c} is H, W is O, R² is R²-7, R³ is R³-29, and the combination of R^{1b}, Q, B¹, B², B³, and B⁴ for a compound corresponds in each case to one row of Table A.

Table 58 : Compounds of formula I in which R^{1a} is F, R^{1c} is H, W is O, R² is R²-8, R³ is R³-29, and the combination of R^{1b}, Q, B¹, B², B³, and B⁴ for a compound corresponds in each case to one row of Table A.

Table 59 : Compounds of formula I in which R^{1a} is F, R^{1c} is H, W is O, R² is R²-9, R³ is R³-29, and the combination of R^{1b}, Q, B¹, B², B³, and B⁴ for a compound corresponds in each case to one row of Table A.

Table 60 : Compounds of formula I in which R^{1a} is F, R^{1c} is H, W is O, R² is R²-10, R³ is R³-29, and the combination of R^{1b}, Q, B¹, B², B³, and B⁴ for a compound corresponds in each case to one row of Table A.

Table 61 : Compounds of formula I in which R^{1a} is F, R^{1c} is H, W is O, R² is R²-1, R³ is R³-30, and the combination of R^{1b}, Q, B¹, B², B³, and B⁴ for a compound corresponds in each case to one row of Table A.

Table 62 : Compounds of formula I in which R^{1a} is F, R^{1c} is H, W is O, R² is R²-2, R³ is R³-30, and the combination of R^{1b}, Q, B¹, B², B³, and B⁴ for a compound corresponds in each case to one row of Table A.

Table 63 : Compounds of formula I in which R^{1a} is F, R^{1c} is H, W is O, R² is R²-3, R³ is R³-30, and the combination of R^{1b}, Q, B¹, B², B³, and B⁴ for a compound corresponds in each case to one row of Table A.

Table 64 : Compounds of formula I in which R^{1a} is F, R^{1c} is H, W is O, R² is R²-4, R³ is R³-30, and the combination of R^{1b}, Q, B¹, B², B³, and B⁴ for a compound corresponds in each case to one row of Table A.

Table 65 : Compounds of formula I in which R^{1a} is F, R^{1c} is H, W is O, R² is R²-5, R³ is R³-30, and the combination of R^{1b}, Q, B¹, B², B³, and B⁴ for a compound corresponds in each case to one row of Table A.

Table 66 : Compounds of formula I in which R^{1a} is F, R^{1c} is H, W is O, R² is R²-6, R³ is R³-30, and the combination of R^{1b}, Q, B¹, B², B³, and B⁴ for a compound corresponds in each case to one row of Table A.

Table 67 : Compounds of formula I in which R^{1a} is F, R^{1c} is H, W is O, R² is R²-7, R³ is R³-30, and the combination of R^{1b}, Q, B¹, B², B³, and B⁴ for a compound corresponds in each case to one row of Table A.

Table 68 : Compounds of formula I in which R^{1a} is F, R^{1c} is H, W is O, R² is R²-8, R³ is R³-30, and the combination of R^{1b}, Q, B¹, B², B³, and B⁴ for a compound corresponds in each case to one row of Table A.

Table 69 : Compounds of formula I in which R^{1a} is F, R^{1c} is H, W is O, R² is R²-9, R³ is R³-30, and the combination of R^{1b}, Q, B¹, B², B³, and B⁴ for a compound corresponds in each case to one row of Table A.

Table 70 : Compounds of formula I in which R^{1a} is F, R^{1c} is H, W is O, R² is R²-10, R³ is R³-30, and the combination of R^{1b}, Q, B¹, B², B³, and B⁴ for a compound corresponds in each case to one row of Table A.

Table 71 : Compounds of formula I in which R^{1a} is F, R^{1c} is H, W is O, R² is R²-1, R³ is R³-31, and the combination of R^{1b}, Q, B¹, B², B³, and B⁴ for a compound corresponds in each case to one row of Table A.

Table 72 : Compounds of formula I in which R^{1a} is F, R^{1c} is H, W is O, R² is R²-2, R³ is R³-31, and the combination of R^{1b}, Q, B¹, B², B³, and B⁴ for a compound corresponds in each case to one row of Table A.

Table 73 : Compounds of formula I in which R^{1a} is F, R^{1c} is H, W is O, R² is R²-3, R³ is R³-31, and the combination of R^{1b}, Q, B¹, B², B³, and B⁴ for a compound corresponds in each case to one row of Table A.

Table 74 : Compounds of formula I in which R^{1a} is F, R^{1c} is H, W is O, R² is R²-4, R³ is R³-31, and the combination of R^{1b}, Q, B¹, B², B³, and B⁴ for a compound corresponds in each case to one row of Table A.

Table 75 : Compounds of formula I in which R^{1a} is F, R^{1c} is H, W is O, R² is R²-5, R³ is R³-31, and the combination of R^{1b}, Q, B¹, B², B³, and B⁴ for a compound corresponds in each case to one row of Table A.

Table 76 : Compounds of formula I in which R^{1a} is F, R^{1c} is H, W is O, R² is R²-6, R³ is R³-31, and the combination of R^{1b}, Q, B¹, B², B³, and B⁴ for a compound corresponds in each case to one row of Table A.

Table 77 : Compounds of formula I in which R^{1a} is F, R^{1c} is H, W is O, R² is R²-7, R³ is R³-31, and the combination of R^{1b}, Q, B¹, B², B³, and B⁴ for a compound corresponds in each case to one row of Table A.

Table 78 : Compounds of formula I in which R^{1a} is F, R^{1c} is H, W is O, R² is R²-8, R³ is R³-31, and the combination of R^{1b}, Q, B¹, B², B³, and B⁴ for a compound corresponds in each case to one row of Table A.

Table 79 : Compounds of formula I in which R^{1a} is F, R^{1c} is H, W is O, R² is R²-9, R³ is R³-31, and the combination of R^{1b}, Q, B¹, B², B³, and B⁴ for a compound corresponds in each case to one row of Table A.

Table 80 : Compounds of formula I in which R^{1a} is F, R^{1c} is H, W is O, R² is R²-10, R³ is R³-31, and the combination of R^{1b}, Q, B¹, B², B³, and B⁴ for a compound corresponds in each case to one row of Table A.

Table 81 : Compounds of formula I in which R^{1a} is CI, R^{1c} is H, W is O, R² is R²-1, R³ is R³-1, and the combination of R^{1b}, Q, B¹, B², B³, and B⁴ for a compound corresponds in each case to one row of Table A.

Table 82 : Compounds of formula I in which R^{1a} is Cl, R^{1c} is H, W is O, R² is R²-2, R³ is R³-1, and the combination of R^{1b}, Q, B¹, B², B³, and B⁴ for a compound corresponds in each case to one row of Table A.

Table 83 : Compounds of formula I in which R^{1a} is CI, R^{1c} is H, W is O, R² is R²-3, R³ is R³-1, and the combination of R^{1b}, Q, B¹, B², B³, and B⁴ for a compound corresponds in each case to one row of Table A.

Table 84 : Compounds of formula I in which R^{1a} is Cl, R^{1c} is H, W is O, R² is R²-4, R³ is R³-1, and the combination of R^{1b}, Q, B¹, B², B³, and B⁴ for a compound corresponds in each case to one row of Table A.

Table 85 : Compounds of formula I in which R^{1a} is Cl, R^{1c} is H, W is O, R² is R²-5, R³ is R³-1, and the combination of R^{1b}, Q, B¹, B², B³, and B⁴ for a compound corresponds in each case to one row of Table A.

Table 86 : Compounds of formula I in which R^{1a} is Cl, R^{1c} is H, W is O, R² is R²-6, R³ is R³-1, and the combination of R^{1b}, Q, B¹, B², B³, and B⁴ for a compound corresponds in each case to one row of Table A.

Table 87 : Compounds of formula I in which R^{1a} is Cl, R^{1c} is H, W is O, R² is R²-7, R³ is R³-1, and the combination of R^{1b}, Q, B¹, B², B³, and B⁴ for a compound corresponds in each case to one row of Table A.

Table 88 : Compounds of formula I in which R^{1a} is Cl, R^{1c} is H, W is O, R² is R²-8, R³ is R³-1, and the combination of R^{1b}, Q, B¹, B², B³, and B⁴ for a compound corresponds in each case to one row of Table A.

Table 89 : Compounds of formula I in which R^{1a} is Cl, R^{1c} is H, W is O, R² is R²-9, R³ is R³-1, and the combination of R^{1b}, Q, B¹, B², B³, and B⁴ for a compound corresponds in each case to one row of Table A.

Table 90 : Compounds of formula I in which R^{1a} is CI, R^{1c} is H, W is O, R² is R²-10, R³ is R³-1, and the combination of R^{1b}, Q, B¹, B², B³, and B⁴ for a compound corresponds in each case to one row of Table A.

Table 91 : Compounds of formula I in which R^{1a} is CI, R^{1c} is H, W is O, R² is R²-1, R³ is R³-29, and the combination of R^{1b}, Q, B¹, B², B³, and B⁴ for a compound corresponds in each case to one row of Table A.

Table 92 : Compounds of formula I in which R^{1a} is CI, R^{1c} is H, W is O, R² is R²-2, R³ is R³-29, and the combination of R^{1b}, Q, B¹, B², B³, and B⁴ for a compound corresponds in each case to one row of Table A.

Table 93 : Compounds of formula I in which R^{1a} is CI, R^{1c} is H, W is O, R² is R²-3, R³ is R³-29, and the combination of R^{1b}, Q, B¹, B², B³, and B⁴ for a compound corresponds in each case to one row of Table A.

Table 94 : Compounds of formula I in which R^{1a} is CI, R^{1c} is H, W is O, R² is R²-4, R³ is R³-29, and the combination of R^{1b}, Q, B¹, B², B³, and B⁴ for a compound corresponds in each case to one row of Table A.

Table 95 : Compounds of formula I in which R^{1a} is CI, R^{1c} is H, W is O, R² is R²-5, R³ is R³-29, and the combination of R^{1b}, Q, B¹, B², B³, and B⁴ for a compound corresponds in each case to one row of Table A.

Table 96 : Compounds of formula I in which R^{1a} is CI, R^{1c} is H, W is O, R² is R²-6, R³ is R³-29, and the combination of R^{1b}, Q, B¹, B², B³, and B⁴ for a compound corresponds in each case to one row of Table A.

Table 97 : Compounds of formula I in which R^{1a} is CI, R^{1c} is H, W is O, R² is R²-7, R³ is R³-29, and the combination of R^{1b}, Q, B¹, B², B³, and B⁴ for a compound corresponds in each case to one row of Table A.

Table 98 : Compounds of formula I in which R^{1a} is CI, R^{1c} is H, W is O, R² is R²-8, R³ is R³-29, and the combination of R^{1b}, Q, B¹, B², B³, and B⁴ for a compound corresponds in each case to one row of Table A.

Table 99 : Compounds of formula I in which R^{1a} is CI, R^{1c} is H, W is O, R² is R²-9, R³ is R³-29, and the combination of R^{1b}, Q, B¹, B², B³, and B⁴ for a compound corresponds in each case to one row of Table A.

Table 100 : Compounds of formula I in which R^{1a} is Cl, R^{1c} is H, W is O, R² is R²-10, R³ is R³-29, and the combination of R^{1b}, Q, B¹, B², B³, and B⁴ for a compound corresponds in each case to one row of Table A.

Table 101 : Compounds of formula I in which R^{1a} is Cl, R^{1c} is H, W is O, R² is R²-1, R³ is R³-30, and the combination of R^{1b}, Q, B¹, B², B³, and B⁴ for a compound corresponds in each case to one row of Table A.

Table 102 : Compounds of formula I in which R^{1a} is Cl, R^{1c} is H, W is O, R² is R²-2, R³ is R³-30, and the combination of R^{1b}, Q, B¹, B², B³, and B⁴ for a compound corresponds in each case to one row of Table A.

Table 103 : Compounds of formula I in which R^{1a} is Cl, R^{1c} is H, W is O, R² is R²-3, R³ is R³-30, and the combination of R^{1b}, Q, B¹, B², B³, and B⁴ for a compound corresponds in each case to one row of Table A.

Table 104 : Compounds of formula I in which R^{1a} is Cl, R^{1c} is H, W is O, R² is R²-4, R³ is R³-30, and the combination of R^{1b}, Q, B¹, B², B³, and B⁴ for a compound corresponds in each case to one row of Table A.

Table 105 : Compounds of formula I in which R^{1a} is Cl, R^{1c} is H, W is O, R² is R²-5, R³ is R³-30, and the combination of R^{1b}, Q, B¹, B², B³, and B⁴ for a compound corresponds in each case to one row of Table A.

Table 106 : Compounds of formula I in which R^{1a} is Cl, R^{1c} is H, W is O, R² is R²-6, R³ is R³-30, and the combination of R^{1b}, Q, B¹, B², B³, and B⁴ for a compound corresponds in each case to one row of Table A.

Table 107 : Compounds of formula I in which R^{1a} is Cl, R^{1c} is H, W is O, R² is R²-7, R³ is R³-30, and the combination of R^{1b}, Q, B¹, B², B³, and B⁴ for a compound corresponds in each case to one row of Table A.

Table 108 : Compounds of formula I in which R^{1a} is Cl, R^{1c} is H, W is O, R² is R²-8, R³ is R³-30, and the combination of R^{1b}, Q, B¹, B², B³, and B⁴ for a compound corresponds in each case to one row of Table A.

Table 109 : Compounds of formula I in which R^{1a} is Cl, R^{1c} is H, W is O, R² is R²-9, R³ is R³-30, and the combination of R^{1b}, Q, B¹, B², B³, and B⁴ for a compound corresponds in each case to one row of Table A.

Table 110 : Compounds of formula I in which R^{1a} is Cl, R^{1c} is H, W is O, R² is R²-10, R³ is R³-30, and the combination of R^{1b}, Q, B¹, B², B³, and B⁴ for a compound corresponds in each case to one row of Table A.

Table 111 : Compounds of formula I in which R^{1a} is Cl, R^{1c} is H, W is O, R² is R²-1, R³ is R³-31, and the combination of R^{1b}, Q, B¹, B², B³, and B⁴ for a compound corresponds in each case to one row of Table A.

Table 112 : Compounds of formula I in which R^{1a} is Cl, R^{1c} is H, W is O, R² is R²-2, R³ is R³-31, and the combination of R^{1b}, Q, B¹, B², B³, and B⁴ for a compound corresponds in each case to one row of Table A.

Table 113 : Compounds of formula I in which R^{1a} is Cl, R^{1c} is H, W is O, R² is R²-3, R³ is R³-31, and the combination of R^{1b}, Q, B¹, B², B³, and B⁴ for a compound corresponds in each case to one row of Table A.

Table 114 : Compounds of formula I in which R^{1a} is Cl, R^{1c} is H, W is O, R² is R²-4, R³ is R³-31, and the combination of R^{1b}, Q, B¹, B², B³, and B⁴ for a compound corresponds in each case to one row of Table A.

Table 115 : Compounds of formula I in which R^{1a} is Cl, R^{1c} is H, W is O, R² is R²-5, R³ is R³-31, and the combination of R^{1b}, Q, B¹, B², B³, and B⁴ for a compound corresponds in each case to one row of Table A.

Table 116 : Compounds of formula I in which R^{1a} is Cl, R^{1c} is H, W is O, R² is R²-6, R³ is R³-31, and the combination of R^{1b}, Q, B¹, B², B³, and B⁴ for a compound corresponds in each case to one row of Table A.

Table 117 : Compounds of formula I in which R^{1a} is Cl, R^{1c} is H, W is O, R² is R²-7, R³ is R³-31, and the combination of R^{1b}, Q, B¹, B², B³, and B⁴ for a compound corresponds in each case to one row of Table A.

Table 118 : Compounds of formula I in which R^{1a} is Cl, R^{1c} is H, W is O, R² is R²-8, R³ is R³-31, and the combination of R^{1b}, Q, B¹, B², B³, and B⁴ for a compound corresponds in each case to one row of Table A.

Table 119 : Compounds of formula I in which R^{1a} is Cl, R^{1c} is H, W is O, R² is R²-9, R³ is R³-31, and the combination of R^{1b}, Q, B¹, B², B³, and B⁴ for a compound corresponds in each case to one row of Table A.

Table 120 : Compounds of formula I in which R^{1a} is Cl, R^{1c} is H, W is O, R² is R²-10, R³ is R³-31, and the combination of R^{1b}, Q, B¹, B², B³, and B⁴ for a compound corresponds in each case to one row of Table A.

Table 121 : Compounds of formula I in which R^{1a} is OCF₃, R^{1c} is H, W is O, R² is R²-1, R³ is R³-1, and the combination of R^{1b}, Q, B¹, B², B³, and B⁴ for a compound corresponds in each case to one row of Table A.

Table 122 : Compounds of formula I in which R^{1a} is OCF₃, R^{1c} is H, W is O, R² is R²-2, R³ is R³-1, and the combination of R^{1b}, Q, B¹, B², B³, and B⁴ for a compound corresponds in each case to one row of Table A.

Table 123 : Compounds of formula I in which R^{1a} is OCF₃, R^{1c} is H, W is O, R² is R²-3, R³ is R³-1, and the combination of R^{1b}, Q, B¹, B², B³, and B⁴ for a compound corresponds in each case to one row of Table A.

Table 124 : Compounds of formula I in which R^{1a} is OCF₃, R^{1c} is H, W is O, R² is R²-4, R³ is R³-1, and the combination of R^{1b}, Q, B¹, B², B³, and B⁴ for a compound corresponds in each case to one row of Table A.

Table 125 : Compounds of formula I in which R^{1a} is OCF₃, R^{1c} is H, W is O, R² is R²-5, R³ is R³-1, and the combination of R^{1b}, Q, B¹, B², B³, and B⁴ for a compound corresponds in each case to one row of Table A.

Table 126 : Compounds of formula I in which R^{1a} is OCF₃, R^{1c} is H, W is O, R² is R²-6, R³ is R³-1, and the combination of R^{1b}, Q, B¹, B², B³, and B⁴ for a compound corresponds in each case to one row of Table A.

Table 127 : Compounds of formula I in which R^{1a} is OCF₃, R^{1c} is H, W is O, R² is R²-7, R³ is R³-1, and the combination of R^{1b}, Q, B¹, B², B³, and B⁴ for a compound corresponds in each case to one row of Table A.

Table 128 : Compounds of formula I in which R^{1a} is OCF₃, R^{1c} is H, W is O, R² is R²-8, R³ is R³-1, and the combination of R^{1b}, Q, B¹, B², B³, and B⁴ for a compound corresponds in each case to one row of Table A.

Table 129 : Compounds of formula I in which R^{1a} is OCF₃, R^{1c} is H, W is O, R² is R²-9, R³ is R³-1, and the combination of R^{1b}, Q, B¹, B², B³, and B⁴ for a compound corresponds in each case to one row of Table A.

Table 130 : Compounds of formula I in which R^{1a} is OCF₃, R^{1c} is H, W is O, R² is R²-10, R³ is R³-1, and the combination of R^{1b}, Q, B¹, B², B³, and B⁴ for a compound corresponds in each case to one row of Table A.

Table 131 : Compounds of formula I in which R^{1a} is OCF₃, R^{1c} is H, W is O, R² is R²-1, R³ is R³-29, and the combination of R^{1b}, Q, B¹, B², B³, and B⁴ for a compound corresponds in each case to one row of Table A.

Table 132 : Compounds of formula I in which R^{1a} is OCF₃, R^{1c} is H, W is O, R² is R²-2, R³ is R³-29, and the combination of R^{1b}, Q, B¹, B², B³, and B⁴ for a compound corresponds in each case to one row of Table A.

Table 133 : Compounds of formula I in which R^{1a} is OCF₃, R^{1c} is H, W is O, R² is R²-3, R³ is R³-29, and the combination of R^{1b}, Q, B¹, B², B³, and B⁴ for a compound corresponds in each case to one row of Table A.

Table 134 : Compounds of formula I in which R^{1a} is OCF₃, R^{1c} is H, W is O, R² is R²-4, R³ is R³-29, and the combination of R^{1b}, Q, B¹, B², B³, and B⁴ for a compound corresponds in each case to one row of Table A.

Table 135 : Compounds of formula I in which R^{1a} is OCF₃, R^{1c} is H, W is O, R² is R²-5, R³ is R³-29, and the combination of R^{1b}, Q, B¹, B², B³, and B⁴ for a compound corresponds in each case to one row of Table A.

Table 136 : Compounds of formula I in which R^{1a} is OCF₃, R^{1c} is H, W is O, R² is R²-6, R³ is R³-29, and the combination of R^{1b}, Q, B¹, B², B³, and B⁴ for a compound corresponds in each case to one row of Table A.

Table 137 : Compounds of formula I in which R^{1a} is OCF₃, R^{1c} is H, W is O, R² is R²-7, R³ is R³-29, and the combination of R^{1b}, Q, B¹, B², B³, and B⁴ for a compound corresponds in each case to one row of Table A.

Table 138 : Compounds of formula I in which R^{1a} is OCF₃, R^{1c} is H, W is O, R² is R²-8, R³ is R³-29, and the combination of R^{1b}, Q, B¹, B², B³, and B⁴ for a compound corresponds in each case to one row of Table A.

Table 139 : Compounds of formula I in which R^{1a} is OCF₃, R^{1c} is H, W is O, R² is R²-9, R³ is R³-29, and the combination of R^{1b}, Q, B¹, B², B³, and B⁴ for a compound corresponds in each case to one row of Table A.

Table 140 : Compounds of formula I in which R^{1a} is OCF₃, R^{1c} is H, W is O, R² is R²-10, R³ is R³-29, and the combination of R^{1b}, Q, B¹, B², B³, and B⁴ for a compound corresponds in each case to one row of Table A.

Table 141 : Compounds of formula I in which R^{1a} is OCF₃, R^{1c} is H, W is O, R² is R²-1, R³ is R³-30, and the combination of R^{1b}, Q, B¹, B², B³, and B⁴ for a compound corresponds in each case to one row of Table A.

Table 142 : Compounds of formula I in which R^{1a} is OCF₃, R^{1c} is H, W is O, R² is R²-2, R³ is R³-30, and the combination of R^{1b}, Q, B¹, B², B³, and B⁴ for a compound corresponds in each case to one row of Table A.

Table 143 : Compounds of formula I in which R^{1a} is OCF₃, R^{1c} is H, W is O, R² is R²-3, R³ is R³-30, and the combination of R^{1b}, Q, B¹, B², B³, and B⁴ for a compound corresponds in each case to one row of Table A.

Table 144 : Compounds of formula I in which R^{1a} is OCF₃, R^{1c} is H, W is O, R² is R²-4, R³ is R³-30, and the combination of R^{1b}, Q, B¹, B², B³, and B⁴ for a compound corresponds in each case to one row of Table A.

Table 145 : Compounds of formula I in which R^{1a} is OCF₃, R^{1c} is H, W is O, R² is R²-5, R³ is R³-30, and the combination of R^{1b}, Q, B¹, B², B³, and B⁴ for a compound corresponds in each case to one row of Table A.

Table 146 : Compounds of formula I in which R^{1a} is OCF₃, R^{1c} is H, W is O, R² is R²-6, R³ is R³-30, and the combination of R^{1b}, Q, B¹, B², B³, and B⁴ for a compound corresponds in each case to one row of Table A.

Table 147 : Compounds of formula I in which R^{1a} is OCF₃, R^{1c} is H, W is O, R² is R²-7, R³ is R³-30, and the combination of R^{1b}, Q, B¹, B², B³, and B⁴ for a compound corresponds in each case to one row of Table A.

Table 148 : Compounds of formula I in which R^{1a} is OCF₃, R^{1c} is H, W is O, R² is R²-8, R³ is R³-30, and the combination of R^{1b}, Q, B¹, B², B³, and B⁴ for a compound corresponds in each case to one row of Table A.

Table 149 : Compounds of formula I in which R^{1a} is OCF₃, R^{1c} is H, W is O, R² is R²-9, R³ is R³-30, and the combination of R^{1b}, Q, B¹, B², B³, and B⁴ for a compound corresponds in each case to one row of Table A.

Table 150 : Compounds of formula I in which R^{1a} is OCF₃, R^{1c} is H, W is O, R² is R²-10, R³ is R³-30, and the combination of R^{1b}, Q, B¹, B², B³, and B⁴ for a compound corresponds in each case to one row of Table A.

Table 151 : Compounds of formula I in which R^{1a} is OCF₃, R^{1c} is H, W is O, R² is R²-1, R³ is R³-31, and the combination of R^{1b}, Q, B¹, B², B³, and B⁴ for a compound corresponds in each case to one row of Table A.

Table 152 : Compounds of formula I in which R^{1a} is OCF₃, R^{1c} is H, W is O, R² is R²-2, R³ is R³-31, and the combination of R^{1b}, Q, B¹, B², B³, and B⁴ for a compound corresponds in each case to one row of Table A.

Table 153 : Compounds of formula I in which R^{1a} is OCF₃, R^{1c} is H, W is O, R² is R²-3, R³ is R³-31, and the combination of R^{1b}, Q, B¹, B², B³, and B⁴ for a compound corresponds in each case to one row of Table A.

Table 154 : Compounds of formula I in which R^{1a} is OCF₃, R^{1c} is H, W is O, R² is R²-4, R³ is R³-31, and the combination of R^{1b}, Q, B¹, B², B³, and B⁴ for a compound corresponds in each case to one row of Table A.

Table 155 : Compounds of formula I in which R^{1a} is OCF₃, R^{1c} is H, W is O, R² is R²-5, R³ is R³-31, and the combination of R^{1b}, Q, B¹, B², B³, and B⁴ for a compound corresponds in each case to one row of Table A.

Table 156 : Compounds of formula I in which R^{1a} is OCF₃, R^{1c} is H, W is O, R² is R²-6, R³ is R³-31, and the combination of R^{1b}, Q, B¹, B², B³, and B⁴ for a compound corresponds in each case to one row of Table A.

Table 157 : Compounds of formula I in which R^{1a} is OCF₃, R^{1c} is H, W is O, R² is R²-7, R³ is R³-31, and the combination of R^{1b}, Q, B¹, B², B³, and B⁴ for a compound corresponds in each case to one row of Table A.

Table 158 : Compounds of formula I in which R^{1a} is OCF₃, R^{1c} is H, W is O, R² is R²-8, R³ is R³-31, and the combination of R^{1b}, Q, B¹, B², B³, and B⁴ for a compound corresponds in each case to one row of Table A.

Table 159 : Compounds of formula I in which R^{1a} is OCF₃, R^{1c} is H, W is O, R² is R²-9, R³ is R³-31, and the combination of R^{1b}, Q, B¹, B², B³, and B⁴ for a compound corresponds in each case to one row of Table A.

Table 160 : Compounds of formula I in which R^{1a} is OCF₃, R^{1c} is H, W is O, R² is R²-10, R³ is R³-31, and the combination of R^{1b}, Q, B¹, B², B³, and B⁴ for a compound corresponds in each case to one row of Table A.

**Table A**

| No. | R^{1b} | Q | B¹ | B² | B³ | B⁴ |
|---|---|---|---|---|---|---|
| A-1 | F | Q¹ | CH | CH | CH | CH |
| A-2 | Cl | Q¹ | CH | CH | CH | CH |
| A-3 | Br | Q¹ | CH | CH | CH | CH |
| A-4 | OCF₃ | Q¹ | CH | CH | CH | CH |
| A-5 | OCHF₂ | Q¹ | CH | CH | CH | CH |
| A-6 | CN | Q¹ | CH | CH | CH | CH |
| A-7 | OCH₃ | Q¹ | CH | CH | CH | CH |
| A-8 | OCH₂CH₃ | Q¹ | CH | CH | CH | CH |
| A-9 | F | Q² | CH | CH | CH | CH |
| A-10 | Cl | Q² | CH | CH | CH | CH |
| A-11 | Br | Q² | CH | CH | CH | CH |
| A-12 | OCF₃ | Q² | CH | CH | CH | CH |
| A-13 | OCHF₂ | Q² | CH | CH | CH | CH |
| A-14 | CN | Q² | CH | CH | CH | CH |
| A-15 | OCH₃ | Q² | CH | CH | CH | CH |
| A-16 | OCH₂CH₃ | Q² | CH | CH | CH | CH |
| A-17 | F | Q³ | CH | CH | CH | CH |
| A-18 | Cl | Q³ | CH | CH | CH | CH |
| A-19 | Br | Q³ | CH | CH | CH | CH |
| A-20 | OCF₃ | Q³ | CH | CH | CH | CH |
| A-21 | OCHF₂ | Q³ | CH | CH | CH | CH |
| A-22 | CN | Q³ | CH | CH | CH | CH |
| A-23 | OCH₃ | Q³ | CH | CH | CH | CH |
| A-24 | OCH₂CH₃ | Q³ | CH | CH | CH | CH |
| A-25 | F | Q⁴ | CH | CH | CH | CH |
| A-26 | Cl | Q⁴ | CH | CH | CH | CH |
| A-27 | Br | Q⁴ | CH | CH | CH | CH |
| A-28 | OCF₃ | Q⁴ | CH | CH | CH | CH |
| A-29 | OCHF₂ | Q⁴ | CH | CH | CH | CH |
| A-30 | CN | Q⁴ | CH | CH | CH | CH |
| A-31 | OCH₃ | Q⁴ | CH | CH | CH | CH |
| A-32 | OCH₂CH₃ | Q⁴ | CH | CH | CH | CH |
| A-33 | F | Q⁵ | CH | CH | CH | CH |
| A-34 | Cl | Q⁵ | CH | CH | CH | CH |
| A-35 | Br | Q⁵ | CH | CH | CH | CH |
| A-36 | OCF₃ | Q⁵ | CH | CH | CH | CH |
| A-37 | OCHF₂ | Q⁵ | CH | CH | CH | CH |
| A-38 | CN | Q⁵ | CH | CH | CH | CH |
| A-39 | OCH₃ | Q⁵ | CH | CH | CH | CH |
| A-40 | OCH₂CH₃ | Q⁵ | CH | CH | CH | CH |
| A-41 | F | Q¹ | N | CH | CH | CH |
| A-42 | Cl | Q¹ | N | CH | CH | CH |
| A-43 | Br | Q¹ | N | CH | CH | CH |
| A-44 | OCF₃ | Q¹ | N | CH | CH | CH |
| A-45 | OCHF₂ | Q¹ | N | CH | CH | CH |
| A-46 | CN | Q¹ | N | CH | CH | CH |
| A-47 | OCH₃ | Q¹ | N | CH | CH | CH |
| A-48 | OCH₂CH₃ | Q¹ | N | CH | CH | CH |
| A-49 | F | Q² | N | CH | CH | CH |
| A-50 | Cl | Q² | N | CH | CH | CH |
| A-51 | Br | Q² | N | CH | CH | CH |
| A-52 | OCF₃ | Q² | N | CH | CH | CH |
| A-53 | OCHF₂ | Q² | N | CH | CH | CH |
| A-54 | CN | Q² | N | CH | CH | CH |
| A-55 | OCH₃ | Q² | N | CH | CH | CH |
| A-56 | OCH₂CH₃ | Q² | N | CH | CH | CH |
| A-57 | F | Q³ | N | CH | CH | CH |
| A-58 | Cl | Q³ | N | CH | CH | CH |
| A-59 | Br | Q³ | N | CH | CH | CH |
| A-60 | OCF₃ | Q³ | N | CH | CH | CH |
| A-61 | OCHF₂ | Q³ | N | CH | CH | CH |
| A-62 | CN | Q³ | N | CH | CH | CH |
| A-63 | OCH₃ | Q³ | N | CH | CH | CH |
| A-64 | OCH₂CH₃ | Q³ | N | CH | CH | CH |
| A-65 | F | Q⁴ | N | CH | CH | CH |
| A-66 | Cl | Q⁴ | N | CH | CH | CH |
| A-67 | Br | Q⁴ | N | CH | CH | CH |
| A-68 | OCF₃ | Q⁴ | N | CH | CH | CH |
| A-69 | OCHF₂ | Q⁴ | N | CH | CH | CH |
| A-70 | CN | Q⁴ | N | CH | CH | CH |
| A-71 | OCH₃ | Q⁴ | N | CH | CH | CH |
| A-72 | OCH₂CH₃ | Q⁴ | N | CH | CH | CH |
| A-73 | F | Q⁵ | N | CH | CH | CH |
| A-74 | Cl | Q⁵ | N | CH | CH | CH |
| A-75 | Br | Q⁵ | N | CH | CH | CH |
| A-76 | OCF₃ | Q⁵ | N | CH | CH | CH |
| A-77 | OCHF₂ | Q⁵ | N | CH | CH | CH |
| A-78 | CN | Q⁵ | N | CH | CH | CH |
| A-79 | OCH₃ | Q⁵ | N | CH | CH | CH |
| A-80 | OCH₂CH₃ | Q⁵ | N | CH | CH | CH |
| A-81 | F | Q¹ | CH | N | CH | CH |
| A-82 | Cl | Q¹ | CH | N | CH | CH |
| A-83 | Br | Q¹ | CH | N | CH | CH |
| A-84 | OCF₃ | Q¹ | CH | N | CH | CH |
| A-85 | OCHF₂ | Q¹ | CH | N | CH | CH |
| A-86 | CN | Q¹ | CH | N | CH | CH |
| A-87 | OCH₃ | Q¹ | CH | N | CH | CH |
| A-88 | OCH₂CH₃ | Q¹ | CH | N | CH | CH |
| A-89 | F | Q² | CH | N | CH | CH |
| A-90 | Cl | Q² | CH | N | CH | CH |
| A-91 | Br | Q² | CH | N | CH | CH |
| A-92 | OCF₃ | Q² | CH | N | CH | CH |
| A-93 | OCHF₂ | Q² | CH | N | CH | CH |
| A-94 | CN | Q² | CH | N | CH | CH |
| A-95 | OCH₃ | Q² | CH | N | CH | CH |
| A-96 | OCH₂CH₃ | Q² | CH | N | CH | CH |
| A-97 | F | Q³ | CH | N | CH | CH |
| A-98 | Cl | Q³ | CH | N | CH | CH |
| A-99 | Br | Q³ | CH | N | CH | CH |
| A-100 | OCF₃ | Q³ | CH | N | CH | CH |
| A-101 | OCHF₂ | Q³ | CH | N | CH | CH |
| A-102 | CN | Q³ | CH | N | CH | CH |
| A-103 | OCH₃ | Q³ | CH | N | CH | CH |
| A-104 | OCH₂CH₃ | Q³ | CH | N | CH | CH |
| A-105 | F | Q⁴ | CH | N | CH | CH |
| A-106 | Cl | Q⁴ | CH | N | CH | CH |
| A-107 | Br | Q⁴ | CH | N | CH | CH |
| A-108 | OCF₃ | Q⁴ | CH | N | CH | CH |
| A-109 | OCHF₂ | Q⁴ | CH | N | CH | CH |
| A-110 | CN | Q⁴ | CH | N | CH | CH |
| A-111 | OCH₃ | Q⁴ | CH | N | CH | CH |
| A-112 | OCH₂CH₃ | Q⁴ | CH | N | CH | CH |
| A-113 | F | Q⁵ | CH | N | CH | CH |
| A-114 | Cl | Q⁵ | CH | N | CH | CH |
| A-115 | Br | Q⁵ | CH | N | CH | CH |
| A-116 | OCF₃ | Q⁵ | CH | N | CH | CH |
| A-117 | OCHF₂ | Q⁵ | CH | N | CH | CH |
| A-118 | CN | Q⁵ | CH | N | CH | CH |
| A-119 | OCH₃ | Q⁵ | CH | N | CH | CH |
| A-120 | OCH₂CH₃ | Q⁵ | CH | N | CH | CH |
| A-121 | F | Q¹ | N | CH | CH | N |
| A-122 | Cl | Q¹ | N | CH | CH | N |
| A-123 | Br | Q¹ | N | CH | CH | N |
| A-124 | OCF₃ | Q¹ | N | CH | CH | N |
| A-125 | OCHF₂ | Q¹ | N | CH | CH | N |
| A-126 | CN | Q¹ | N | CH | CH | N |
| A-127 | OCH₃ | Q¹ | N | CH | CH | N |
| A-128 | OCH₂CH₃ | Q¹ | N | CH | CH | N |
| A-129 | F | Q² | N | CH | CH | N |
| A-130 | Cl | Q² | N | CH | CH | N |
| A-131 | Br | Q² | N | CH | CH | N |
| A-132 | OCF₃ | Q² | N | CH | CH | N |
| A-133 | OCHF₂ | Q² | N | CH | CH | N |
| A-134 | CN | Q² | N | CH | CH | N |
| A-135 | OCH₃ | Q² | N | CH | CH | N |
| A-136 | OCH₂CH₃ | Q² | N | CH | CH | N |
| A-137 | F | Q³ | N | CH | CH | N |
| A-138 | Cl | Q³ | N | CH | CH | N |
| A-139 | Br | Q³ | N | CH | CH | N |
| A-140 | OCF₃ | Q³ | N | CH | CH | N |
| A-141 | OCHF₂ | Q³ | N | CH | CH | N |
| A-142 | CN | Q³ | N | CH | CH | N |
| A-143 | OCH₃ | Q³ | N | CH | CH | N |
| A-144 | OCH₂CH₃ | Q³ | N | CH | CH | N |
| A-145 | F | Q⁴ | N | CH | CH | N |
| A-146 | Cl | Q⁴ | N | CH | CH | N |
| A-147 | Br | Q⁴ | N | CH | CH | N |
| A-148 | OCF₃ | Q⁴ | N | CH | CH | N |
| A-149 | OCHF₂ | Q⁴ | N | CH | CH | N |
| A-150 | CN | Q⁴ | N | CH | CH | N |
| A-151 | OCH₃ | Q⁴ | N | CH | CH | N |
| A-152 | OCH₂CH₃ | Q⁴ | N | CH | CH | N |
| A-153 | F | Q⁵ | N | CH | CH | N |
| A-154 | Cl | Q⁵ | N | CH | CH | N |
| A-155 | Br | Q⁵ | N | CH | CH | N |
| A-156 | OCF₃ | Q⁵ | N | CH | CH | N |
| A-157 | OCHF₂ | Q⁵ | N | CH | CH | N |
| A-158 | CN | Q⁵ | N | CH | CH | N |
| A-159 | OCH₃ | Q⁵ | N | CH | CH | N |
| A-160 | OCH₂CH₃ | Q⁵ | N | CH | CH | N |
| A-161 | F | Q¹ | CH | N | N | CH |
| A-162 | Cl | Q¹ | CH | N | N | CH |
| A-163 | Br | Q¹ | CH | N | N | CH |
| A-164 | OCF₃ | Q¹ | CH | N | N | CH |
| A-165 | OCHF₂ | Q¹ | CH | N | N | CH |
| A-166 | CN | Q¹ | CH | N | N | CH |
| A-167 | OCH₃ | Q¹ | CH | N | N | CH |
| A-168 | OCH₂CH₃ | Q¹ | CH | N | N | CH |
| A-169 | F | Q² | CH | N | N | CH |
| A-170 | Cl | Q² | CH | N | N | CH |
| A-171 | Br | Q² | CH | N | N | CH |
| A-172 | OCF₃ | Q² | CH | N | N | CH |
| A-173 | OCHF₂ | Q² | CH | N | N | CH |
| A-174 | CN | Q² | CH | N | N | CH |
| A-175 | OCH₃ | Q² | CH | N | N | CH |
| A-176 | OCH₂CH₃ | Q² | CH | N | N | CH |
| A-177 | F | Q³ | CH | N | N | CH |
| A-178 | Cl | Q³ | CH | N | N | CH |
| A-179 | Br | Q³ | CH | N | N | CH |
| A-180 | OCF₃ | Q³ | CH | N | N | CH |
| A-181 | OCHF₂ | Q³ | CH | N | N | CH |
| A-182 | CN | Q³ | CH | N | N | CH |
| A-183 | OCH₃ | Q³ | CH | N | N | CH |
| A-184 | OCH₂CH₃ | Q³ | CH | N | N | CH |
| A-185 | F | Q⁴ | CH | N | N | CH |
| A-186 | Cl | Q⁴ | CH | N | N | CH |
| A-187 | Br | Q⁴ | CH | N | N | CH |
| A-188 | OCF₃ | Q⁴ | CH | N | N | CH |
| A-189 | OCHF₂ | Q⁴ | CH | N | N | CH |
| A-190 | CN | Q⁴ | CH | N | N | CH |
| A-191 | OCH₃ | Q⁴ | CH | N | N | CH |
| A-192 | OCH₂CH₃ | Q⁴ | CH | N | N | CH |
| A-193 | F | Q⁵ | CH | N | N | CH |
| A-194 | Cl | Q⁵ | CH | N | N | CH |
| A-195 | Br | Q⁵ | CH | N | N | CH |
| A-196 | OCF₃ | Q⁵ | CH | N | N | CH |
| A-197 | OCHF₂ | Q⁵ | CH | N | N | CH |
| A-198 | CN | Q⁵ | CH | N | N | CH |
| A-199 | OCH₃ | Q⁵ | CH | N | N | CH |
| A-200 | OCH₂CH₃ | Q⁵ | CH | N | N | CH |
| A-201 | F | Q¹ | N | CH | N | CH |
| A-202 | Cl | Q¹ | N | CH | N | CH |
| A-203 | Br | Q¹ | N | CH | N | CH |
| A-204 | OCF₃ | Q¹ | N | CH | N | CH |
| A-205 | OCHF₂ | Q¹ | N | CH | N | CH |
| A-206 | CN | Q¹ | N | CH | N | CH |
| A-207 | OCH₃ | Q¹ | N | CH | N | CH |
| A-208 | OCH₂CH₃ | Q¹ | N | CH | N | CH |
| A-209 | F | Q² | N | CH | N | CH |
| A-210 | Cl | Q² | N | CH | N | CH |
| A-211 | Br | Q² | N | CH | N | CH |
| A-212 | OCF₃ | Q² | N | CH | N | CH |
| A-213 | OCHF₂ | Q² | N | CH | N | CH |
| A-214 | CN | Q² | N | CH | N | CH |
| A-215 | OCH₃ | Q² | N | CH | N | CH |
| A-216 | OCH₂CH₃ | Q² | N | CH | N | CH |
| A-217 | F | Q³ | N | CH | N | CH |
| A-218 | Cl | Q³ | N | CH | N | CH |
| A-219 | Br | Q³ | N | CH | N | CH |
| A-220 | OCF₃ | Q³ | N | CH | N | CH |
| A-221 | OCHF₂ | Q³ | N | CH | N | CH |
| A-222 | CN | Q³ | N | CH | N | CH |
| A-223 | OCH₃ | Q³ | N | CH | N | CH |
| A-224 | OCH₂CH₃ | Q³ | N | CH | N | CH |
| A-225 | F | Q⁴ | N | CH | N | CH |
| A-226 | Cl | Q⁴ | N | CH | N | CH |
| A-227 | Br | Q⁴ | N | CH | N | CH |
| A-228 | OCF₃ | Q⁴ | N | CH | N | CH |
| A-229 | OCHF₂ | Q⁴ | N | CH | N | CH |
| A-230 | CN | Q⁴ | N | CH | N | CH |
| A-231 | OCH₃ | Q⁴ | N | CH | N | CH |
| A-232 | OCH₂CH₃ | Q⁴ | N | CH | N | CH |
| A-233 | F | Q⁵ | N | CH | N | CH |
| A-234 | Cl | Q⁵ | N | CH | N | CH |
| A-235 | Br | Q⁵ | N | CH | N | CH |
| A-236 | OCF₃ | Q⁵ | N | CH | N | CH |
| A-237 | OCHF₂ | Q⁵ | N | CH | N | CH |
| A-238 | CN | Q⁵ | N | CH | N | CH |
| A-239 | OCH₃ | Q⁵ | N | CH | N | CH |
| A-240 | OCH₂CH₃ | Q⁵ | N | CH | N | CH |
| A-241 | F | Q¹ | CCl | CH | CH | CH |
| A-242 | Cl | Q¹ | CCl | CH | CH | CH |
| A-243 | Br | Q¹ | CCl | CH | CH | CH |
| A-244 | OCF₃ | Q¹ | CCl | CH | CH | CH |
| A-245 | OCHF₂ | Q¹ | CCl | CH | CH | CH |
| A-246 | CN | Q¹ | CCl | CH | CH | CH |
| A-247 | OCH₃ | Q¹ | CCl | CH | CH | CH |
| A-248 | OCH₂CH₃ | Q¹ | CCl | CH | CH | CH |
| A-249 | F | Q² | CCl | CH | CH | CH |
| A-250 | Cl | Q² | CCl | CH | CH | CH |
| A-251 | Br | Q² | CCl | CH | CH | CH |
| A-252 | OCF₃ | Q² | CCl | CH | CH | CH |
| A-253 | OCHF₂ | Q² | CCl | CH | CH | CH |
| A-254 | CN | Q² | CCl | CH | CH | CH |
| A-255 | OCH₃ | Q² | CCl | CH | CH | CH |
| A-256 | OCH₂CH₃ | Q² | CCl | CH | CH | CH |
| A-257 | F | Q³ | CCl | CH | CH | CH |
| A-258 | Cl | Q³ | CCl | CH | CH | CH |
| A-259 | Br | Q³ | CCl | CH | CH | CH |
| A-260 | OCF₃ | Q³ | CCl | CH | CH | CH |
| A-261 | OCHF₂ | Q³ | CCl | CH | CH | CH |
| A-262 | CN | Q³ | CCl | CH | CH | CH |
| A-263 | OCH₃ | Q³ | CCl | CH | CH | CH |
| A-264 | OCH₂CH₃ | Q³ | CCl | CH | CH | CH |
| A-265 | F | Q⁴ | CCl | CH | CH | CH |
| A-266 | Cl | Q⁴ | CCl | CH | CH | CH |
| A-267 | Br | Q⁴ | CCl | CH | CH | CH |
| A-268 | OCF₃ | Q⁴ | CCl | CH | CH | CH |
| A-269 | OCHF₂ | Q⁴ | CCl | CH | CH | CH |
| A-270 | CN | Q⁴ | CCl | CH | CH | CH |
| A-271 | OCH₃ | Q⁴ | CCl | CH | CH | CH |
| A-272 | OCH₂CH₃ | Q⁴ | CCl | CH | CH | CH |
| A-273 | F | Q⁵ | CCl | CH | CH | CH |
| A-274 | Cl | Q⁵ | CCl | CH | CH | CH |
| A-275 | Br | Q⁵ | CCl | CH | CH | CH |
| A-276 | OCF₃ | Q⁵ | CCl | CH | CH | CH |
| A-277 | OCHF₂ | Q⁵ | CCl | CH | CH | CH |
| A-278 | CN | Q⁵ | CCl | CH | CH | CH |
| A-279 | OCH₃ | Q⁵ | CCl | CH | CH | CH |
| A-280 | OCH₂CH₃ | Q⁵ | CCl | CH | CH | CH |
| A-281 | F | Q¹ | CH | CCl | CH | CH |
| A-282 | Cl | Q¹ | CH | CCl | CH | CH |
| A-283 | Br | Q¹ | CH | CCl | CH | CH |
| A-284 | OCF₃ | Q¹ | CH | CCl | CH | CH |
| A-285 | OCHF₂ | Q¹ | CH | CCl | CH | CH |
| A-286 | CN | Q¹ | CH | CCl | CH | CH |
| A-287 | OCH₃ | Q¹ | CH | CCl | CH | CH |
| A-288 | OCH₂CH₃ | Q¹ | CH | CCl | CH | CH |
| A-289 | F | Q² | CH | CCl | CH | CH |
| A-290 | Cl | Q² | CH | CCl | CH | CH |
| A-291 | Br | Q² | CH | CCl | CH | CH |
| A-292 | OCF₃ | Q² | CH | CCl | CH | CH |
| A-293 | OCHF₂ | Q² | CH | CCl | CH | CH |
| A-294 | CN | Q² | CH | CCl | CH | CH |
| A-295 | OCH₃ | Q² | CH | CCl | CH | CH |
| A-296 | OCH₂CH₃ | Q² | CH | CCl | CH | CH |
| A-297 | F | Q³ | CH | CCl | CH | CH |
| A-298 | Cl | Q³ | CH | CCl | CH | CH |
| A-299 | Br | Q³ | CH | CCl | CH | CH |
| A-300 | OCF₃ | Q³ | CH | CCl | CH | CH |
| A-301 | OCHF₂ | Q³ | CH | CCl | CH | CH |
| A-302 | CN | Q³ | CH | CCl | CH | CH |
| A-303 | OCH₃ | Q³ | CH | CCl | CH | CH |
| A-304 | OCH₂CH₃ | Q³ | CH | CCl | CH | CH |
| A-305 | F | Q⁴ | CH | CCl | CH | CH |
| A-306 | Cl | Q⁴ | CH | CCl | CH | CH |
| A-307 | Br | Q⁴ | CH | CCl | CH | CH |
| A-308 | OCF₃ | Q⁴ | CH | CCl | CH | CH |
| A-309 | OCHF₂ | Q⁴ | CH | CCl | CH | CH |
| A-310 | CN | Q⁴ | CH | CCl | CH | CH |
| A-311 | OCH₃ | Q⁴ | CH | CCl | CH | CH |
| A-312 | OCH₂CH₃ | Q⁴ | CH | CCl | CH | CH |
| A-313 | F | Q⁵ | CH | CCl | CH | CH |
| A-314 | Cl | Q⁵ | CH | CCl | CH | CH |
| A-315 | Br | Q⁵ | CH | CCl | CH | CH |
| A-316 | OCF₃ | Q⁵ | CH | CCl | CH | CH |
| A-317 | OCHF₂ | Q⁵ | CH | CCl | CH | CH |
| A-318 | CN | Q⁵ | CH | CCl | CH | CH |
| A-319 | OCH₃ | Q⁵ | CH | CCl | CH | CH |
| A-320 | OCH₂CH₃ | Q⁵ | CH | CCl | CH | CH |
| A-321 | F | Q¹ | CF | CH | CH | CH |
| A-322 | Cl | Q¹ | CF | CH | CH | CH |
| A-323 | Br | Q¹ | CF | CH | CH | CH |
| A-324 | OCF₃ | Q¹ | CF | CH | CH | CH |
| A-325 | OCHF₂ | Q¹ | CF | CH | CH | CH |
| A-326 | CN | Q¹ | CF | CH | CH | CH |
| A-327 | OCH₃ | Q¹ | CF | CH | CH | CH |
| A-328 | OCH₂CH₃ | Q¹ | CF | CH | CH | CH |
| A-329 | F | Q² | CF | CH | CH | CH |
| A-330 | Cl | Q² | CF | CH | CH | CH |
| A-331 | Br | Q² | CF | CH | CH | CH |
| A-332 | OCF₃ | Q² | CF | CH | CH | CH |
| A-333 | OCHF₂ | Q² | CF | CH | CH | CH |
| A-334 | CN | Q² | CF | CH | CH | CH |
| A-335 | OCH₃ | Q² | CF | CH | CH | CH |
| A-336 | OCH₂CH₃ | Q² | CF | CH | CH | CH |
| A-337 | F | Q³ | CF | CH | CH | CH |
| A-338 | Cl | Q³ | CF | CH | CH | CH |
| A-339 | Br | Q³ | CF | CH | CH | CH |
| A-340 | OCF₃ | Q³ | CF | CH | CH | CH |
| A-341 | OCHF₂ | Q³ | CF | CH | CH | CH |
| A-342 | CN | Q³ | CF | CH | CH | CH |
| A-343 | OCH₃ | Q³ | CF | CH | CH | CH |
| A-344 | OCH₂CH₃ | Q³ | CF | CH | CH | CH |
| A-345 | F | Q⁴ | CF | CH | CH | CH |
| A-346 | Cl | Q⁴ | CF | CH | CH | CH |
| A-347 | Br | Q⁴ | CF | CH | CH | CH |
| A-348 | OCF₃ | Q⁴ | CF | CH | CH | CH |
| A-349 | OCHF₂ | Q⁴ | CF | CH | CH | CH |
| A-350 | CN | Q⁴ | CF | CH | CH | CH |
| A-351 | OCH₃ | Q⁴ | CF | CH | CH | CH |
| A-352 | OCH₂CH₃ | Q⁴ | CF | CH | CH | CH |
| A-353 | F | Q⁵ | CF | CH | CH | CH |
| A-354 | Cl | Q⁵ | CF | CH | CH | CH |
| A-355 | Br | Q⁵ | CF | CH | CH | CH |
| A-356 | OCF₃ | Q⁵ | CF | CH | CH | CH |
| A-357 | OCHF₂ | Q⁵ | CF | CH | CH | CH |
| A-358 | CN | Q⁵ | CF | CH | CH | CH |
| A-359 | OCH₃ | Q⁵ | CF | CH | CH | CH |
| A-360 | OCH₂CH₃ | Q⁵ | CF | CH | CH | CH |
| A-361 | F | Q¹ | CH | CF | CH | CH |
| A-362 | Cl | Q¹ | CH | CF | CH | CH |
| A-363 | Br | Q¹ | CH | CF | CH | CH |
| A-364 | OCF₃ | Q¹ | CH | CF | CH | CH |
| A-365 | OCHF₂ | Q¹ | CH | CF | CH | CH |
| A-366 | CN | Q¹ | CH | CF | CH | CH |
| A-367 | OCH₃ | Q¹ | CH | CF | CH | CH |
| A-368 | OCH₂CH₃ | Q¹ | CH | CF | CH | CH |
| A-369 | F | Q² | CH | CF | CH | CH |
| A-370 | Cl | Q² | CH | CF | CH | CH |
| A-371 | Br | Q² | CH | CF | CH | CH |
| A-372 | OCF₃ | Q² | CH | CF | CH | CH |
| A-373 | OCHF₂ | Q² | CH | CF | CH | CH |
| A-374 | CN | Q² | CH | CF | CH | CH |
| A-375 | OCH₃ | Q² | CH | CF | CH | CH |
| A-376 | OCH₂CH₃ | Q² | CH | CF | CH | CH |
| A-377 | F | Q³ | CH | CF | CH | CH |
| A-378 | Cl | Q³ | CH | CF | CH | CH |
| A-379 | Br | Q³ | CH | CF | CH | CH |
| A-380 | OCF₃ | Q³ | CH | CF | CH | CH |
| A-381 | OCHF₂ | Q³ | CH | CF | CH | CH |
| A-382 | CN | Q³ | CH | CF | CH | CH |
| A-383 | OCH₃ | Q³ | CH | CF | CH | CH |
| A-384 | OCH₂CH₃ | Q³ | CH | CF | CH | CH |
| A-385 | F | Q⁴ | CH | CF | CH | CH |
| A-386 | Cl | Q⁴ | CH | CF | CH | CH |
| A-387 | Br | Q⁴ | CH | CF | CH | CH |
| A-388 | OCF₃ | Q⁴ | CH | CF | CH | CH |
| A-389 | OCHF₂ | Q⁴ | CH | CF | CH | CH |
| A-390 | CN | Q⁴ | CH | CF | CH | CH |
| A-391 | OCH₃ | Q⁴ | CH | CF | CH | CH |
| A-392 | OCH₂CH₃ | Q⁴ | CH | CF | CH | CH |
| A-393 | F | Q⁵ | CH | CF | CH | CH |
| A-394 | Cl | Q⁵ | CH | CF | CH | CH |
| A-395 | Br | Q⁵ | CH | CF | CH | CH |
| A-396 | OCF₃ | Q⁵ | CH | CF | CH | CH |
| A-397 | OCHF₂ | Q⁵ | CH | CF | CH | CH |
| A-398 | CN | Q⁵ | CH | CF | CH | CH |
| A-399 | OCH₃ | Q⁵ | CH | CF | CH | CH |
| A-400 | OCH₂CH₃ | Q⁵ | CH | CF | CH | CH |
| A-401 | H | Q¹ | CH | CH | CH | CH |
| A-402 | H | Q² | CH | CH | CH | CH |
| A-403 | H | Q³ | CH | CH | CH | CH |
| A-404 | H | Q⁴ | CH | CH | CH | CH |
| A-405 | H | Q⁵ | CH | CH | CH | CH |
| A-406 | H | Q¹ | N | CH | CH | CH |
| A-407 | H | Q² | N | CH | CH | CH |
| A-408 | H | Q³ | N | CH | CH | CH |
| A-409 | H | Q⁴ | N | CH | CH | CH |
| A-410 | H | Q⁵ | N | CH | CH | CH |
| A-411 | H | Q¹ | CH | N | CH | CH |
| A-412 | H | Q² | CH | N | CH | CH |
| A-413 | H | Q³ | CH | N | CH | CH |
| A-414 | H | Q⁴ | CH | N | CH | CH |
| A-415 | H | Q⁵ | CH | N | CH | CH |
| A-416 | H | Q¹ | N | CH | CH | N |
| A-417 | H | Q² | N | CH | CH | N |
| A-418 | H | Q³ | N | CH | CH | N |
| A-419 | H | Q⁴ | N | CH | CH | N |
| A-420 | H | Q⁵ | N | CH | CH | N |
| A-421 | H | Q¹ | CH | N | N | CH |
| A-422 | H | Q² | CH | N | N | CH |
| A-423 | H | Q³ | CH | N | N | CH |
| A-424 | H | Q⁴ | CH | N | N | CH |
| A-425 | H | Q⁵ | CH | N | N | CH |
| A-426 | H | Q¹ | N | CH | N | CH |
| A-427 | H | Q² | N | CH | N | CH |
| A-428 | H | Q³ | N | CH | N | CH |
| A-429 | H | Q⁴ | N | CH | N | CH |
| A-430 | H | Q⁵ | N | CH | N | CH |
| A-431 | H | Q¹ | CCl | CH | CH | CH |
| A-432 | H | Q² | CCl | CH | CH | CH |
| A-433 | H | Q³ | CCl | CH | CH | CH |
| A-434 | H | Q⁴ | CCl | CH | CH | CH |
| A-435 | H | Q⁵ | CCl | CH | CH | CH |
| A-436 | H | Q¹ | CH | CCl | CH | CH |
| A-437 | H | Q² | CH | CCl | CH | CH |
| A-438 | H | Q³ | CH | CCl | CH | CH |
| A-439 | H | Q⁴ | CH | CCl | CH | CH |
| A-440 | H | Q⁵ | CH | CCl | CH | CH |
| A-441 | H | Q¹ | CF | CH | CH | CH |
| A-442 | H | Q² | CF | CH | CH | CH |
| A-443 | H | Q³ | CF | CH | CH | CH |
| A-444 | H | Q⁴ | CF | CH | CH | CH |
| A-445 | H | Q⁵ | CF | CH | CH | CH |
| A-446 | H | Q¹ | CH | CF | CH | CH |
| A-447 | H | Q² | CH | CF | CH | CH |
| A-448 | H | Q³ | CH | CF | CH | CH |
| A-449 | H | Q⁴ | CH | CF | CH | CH |
| A-450 | H | Q⁵ | CH | CF | CH | CH |

As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

### Mixtures

The invention also relates to a mixture of at least one compound of the invention with at least one mixing partner. Preferred are binary mixtures of one compound of the invention as component I with one mixing partner herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides, and fungicides.

The following list M of pesticides, grouped according to the Mode of Action Classification of the Insecticide Resistance Action Committee (IRAC), together with which the compounds of the invention can be used and with which potential synergistic effects might be produced, illustrates the possible combinations:
M.1 AChE inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifosmethyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
M.2. GABA-gated chloride channel antagonists: cyclodiene organochlorine compounds: endosulfan, chlordane; phenylpyrazoles: ethiprole, fipronil, flufiprole, pyrafluprole, pyriprole;
M.3 Sodium channel modulators: pyrethroids: acrinathrin, allethrin, d-cis-trans allethrin, d-trans allethrin, bifenthrin, kappa-bifenthrin, bioallethrin, bioallethrin S-cylclopentenyl, bio-resmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; sodium channel modulators, e.g.: DDT, methoxychlor;
M.4 nAChR agonists: neonicotinoids: acetamiprid, clothianidin, cycloxaprid, dinotefuran, imidacloprid, nitenpyram, thiacloprid, thiamethoxam; 4,5-dihydro-N-nitro-1-(2-oxiranylmethyl)-1H-imidazol-2-amine, (2E-)-1-[(6-Chloropyridin-3-yl)methyl]-N'-nitro-2-pentylidenehydrazine-carboximidamide; 1-[(6-Chloropyridin-3-yl)methyl]-7-methyl-8-nitro-5-propoxy-1,2,3,5,6,7-hexahydroimidazo[1,2-a]pyridine; nicotine; sulfoxaflor; flupyradifurone; triflumezopyrim, fenmezoditiaz, flupyrimin, 1-[(2-chlorothiazol-5-yl)methyl]-3-(3,5-dimethylisoxazol-4-yl)pyrido[1,2-a]pyrimidine-2,4-dione;
M.5 Nicotinic acetylcholine receptor allosteric activators: spinosyns, e.g. spinosad or spinetoram;
M.6 Chloride channel activators from the class of avermectins and milbemycins, e.g. abamectin, emamectin benzoate, ivermectin, lepimectin, or milbemectin;
M.7 Juvenile hormone mimics, such as hydroprene, kino-prene, methoprene; fenoxycarb, or pyriproxyfen;
M.8 miscellaneous multi-site inhibitors: CH₃Br, other alkyl halides, chloropicrin, sulfuryl fluoride, borax, tartar emetic;
M.9 Chordotonal organ TRPV channel modulators: afidopyropen, pymetrozine; pyrifluquinazon;
M.10 Mite growth inhibitors: clofentezine, hexythiazox, diflovidazin, etoxazole;
M.11 Microbial disruptors of insect midgut membranes: *bacillus thuringiensis, bacillus sphaericus,* and insecticdal proteins they produce e.g.: *bacillus thuringiensis* subsp. *israelensis, bacillus sphaericus, bacillus thuringiensis* subsp. *aizawai, bacillus thuringiensis* subsp. *kurstaki, bacillus thuringiensis* subsp. *tenebrionis*, Bt crop proteins: Cry1Ab, Cry1Ac, Cry1Fa, Cry2Ab, mCry3A, Cry3Ab, Cry3Bb, Cry34/35Ab1;
M.12 Inhibitors of mitochondrial ATP synthase: diafenthiuron, organotin miticides, e.g.: azocyclotin, cyhexatin, fenbutatin oxide, propargite, tetradifon;
M.13 Uncouplers of oxidative phosphorylation via disruption of the proton gradient: chlorfenapyr, DNOC, sulfluramid;
M.14 nAChR channel blockers: nereistoxin analogues bensultap, cartap hydrochloride, thiocyclam, thiosultap-sodium;
M.15 Inhibitors of the chitin biosynthesis type 0, e.g.: bistrifluron, chlorfluazuron, diflubenzuron, flucycloxuron, flufenoxuron, hexaflumuron, lufenuron, novaluron, noviflumuron, teflubenzuron, triflumuron;
M.16 Inhibitors of the chitin biosynthesis type 1: buprofezin;
M.17 Moulting disruptors: Dipteran, cyromazine;
M.18 Ecdyson receptor agonists, e.g.: methoxyfenozide, tebufenozide, halofenozide, fufenozide, chromafenozide;
M.19 Octopamin receptor agonists: amitraz;
M.20 Mitochondrial complex III electron transport inhibitors: hydramethylnon, acequinocyl, fluacrypyrim; bifenazate;
M.21 METI acaricides and insecticides, e.g.: fenazaquin, fenpyroximate, pyrimidifen, pyridaben, tebufenpyrad, tolfenpyrad, rotenone;
M.22 Voltage-dependent sodium channel blockers: indoxacarb, metaflumizone, N-(3-chloro-2-methyl-phenyl)-2-[(4-chlorophenyl)[4-[methyl(methylsulfonyl)amino]phenyl]-methylene]-hydrazinecarboxamide, N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide, 2-[2-(4-cyanophenyl)-1-[3-(trifluoromethyl)phenyl]ethylidene]-N-[4-(difluoromethoxy)phenyl]-hydrazinecarboxamide;
M.23 Inhibitors of the of acetyl CoA carboxylase, e.g.: spirodiclofen, spiromesifen, spirotetramat; spiropidion; spirobudifen, 11-(4-chloro-2,6-dimethylphenyl)-12-hydroxy-1,4-dioxa-9-azadispiro[4.2.4.2]tetradec-11-en-10-one, spidoxamat;
M.24 Mitochondrial complex IV electron transport inhibitors: e.g. aluminium phosphide, calcium phosphide, zinc phosphide, cyanide;
M.25 Mitochondrial complex II electron transport inhibitors, e.g.: cyenopyrafen, cyflumetofen, cyetpyrafen, pyflubumide;
M.28 Ryanodine receptor-modulators: chlorantraniliprole, cyantraniliprole, cyclaniliprole, flubendiamide, fluchlordiniliprole, (R)-3-chloro-N1-{2-methyl-4-[1,2,2,2-tetrafluoro-1-(trifluoromethyl)ethyl]phenyl}-N2-(1-methyl-2-methylsulfonylethyl)phthalamid, (S)-3-chloro-N1-{2-methyl-4-[1,2,2,2-tetrafluoro-1-(trifluoromethyl)ethyl]phenyl}-N2-(1-methyl-2-methylsulfonylethyl)phthalamide, methyl-2-[3,5-dibromo-2-({[3-bromo-1-(3-chlorpyridin-2-yl)-1H-pyrazol-5-yl]carbonyl}amino)benzoyl]-1,2-dimethylhydrazine-carboxylate; N-[2-(5-amino-1,3,4-thiadiazol-2-yl)-4-chloro-6-methyl-phenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; tetrachlorantraniliprole; tetraniliprole; tiorantraniliprole; N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide; cyhalodiamide; N-[2-(5-amino-1,3,4-thiadiazol-2-yl)-4-chloro-6-methylphenyl]-3-bromo-1-(3-chloro-2-pyridinyl)-1H-pyrazole-5-carboxamide, pioxaniliprole;
M.29: Chordotonal organ Modulators: flonicamid, flumetnicam;
M.30: broflanilide; fluxametamide, isocycloseram, piperflanilide;
M.33 acynonapyr;
M.UN. Unknown mode of action: afoxolaner, azadirachtin, amidoflumet, ben-zoximate, bromopropylate, chinomethionat, cryolite, cyproflanilid, dicloromezotiaz, dicofol, dimpropyridaz, flufenerim, flometoquin, fluensulfone, fluhexafon, fluopyram, fluralaner, metaldehyde, metoxadiazone, mivorilaner, modoflaner, piperonyl butoxide, pyridalyl, tioxazafen, trifluenfuronate, umifoxolaner, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1H-1,2,4-triazole-5-amine, actives on basis of *bacillus firmus* (Votivo, I-1582); fluazaindolizine; N-[5-[[2-bromo-6-chloro-4-[1,2,2,3,3,3-hexafluoro-1-(trifluoromethyl)-propyl]phenyl]carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)-propyl]phenyl]carbamoyl]phenyl]-2-methyl-benzamide; 4-cyano-N-[2-cyano-5-[[2,6-dichloro-4-[1,2,2,2-tetrafluoro-1-(trifluoromethyl)ethyllphenyl]carbamoyl]phenyl]-2-methyl-benzamide; N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide;
1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; 1-[(6-chloro-3-pyridinyl)methyl]-1,2,3,5,6,7-hexahydro-5-methoxy-7-methyl-8-nitro-imidazo[1,2-a]pyridine; 2-(3-pyridinyl)-N-(2-pyrimidinylmethyl )-2H-indazole-5-carboxamide; tyclopyrazoflor; sarolaner, lotilaner; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; 2-(3-ethylsulfonyl-2-pyridyl)-3-methyl-6-(tri-fluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; N-[4-chloro-3-(cyclopropylcarbamoyl)phenyl]-2-methyl-5-(1,1,2,2,2-pentafluoroethyl)-4-(trifluoromethyl)pyrazole-3-carboxamide, N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; benzpyrimoxan; tigolaner; oxazosulfyl; [(2S,3R,4R,5S,6S)-3,5-dimethoxy-6-methyl-4-propoxy-tetrahydropyran-2-yl] N-[4-[1-[4-(trifluoromethoxy)phenyl]-1,2,4-triazol-3-yl]phenyl]carbamate; [(2S,3R,4R,5S,6S)-3,4,5-trimethoxy-6-methyltetrahydropyran-2-yl] N-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine; N-[[2-fluoro-4-[(2S,3S)-2-hydroxy-3-(3,4,5-trichlorophenyl)-3-(trifluoromethyl)pyrrolidin-1-yl]phenyl]methyl]cyclopropanecarboxamide; sulfiflumin; flupentiofenox, N-[3-chloro-1-(3-pyridyl)pyrazol-4-yl]-2-methylsulfonyl-propanamide, cyclobutrifluram; N-[4-chloro-3-[(1-cyanocyclopropyl)carbamoyl]phenyl]-2-methyl-4-methylsulfonyl-5-(1,1,2,2,2-pentafluoroethyl)pyrazole-3-carboxamide, cyproflanilide, nicofluprole; 1,4-dimethyl-2-[2-(pyridin-3-yl)-2h-indazol-5-yl]-1,2,4-triazolidine-3,5-dione, indazapyroxamet, tiapyrachlor, N-cyclopropyl-5-[(5S)-5-(3,5-dichloro-4-fluoro-phenyl)-5-(trifluoromethyl)-4H-isoxazol-3-yl]isoquinoline-8-carboxamide, 5-[(5S)-5-(3,5-dichloro-4-fluoro-phenyl)-5-(trifluoromethyl)-4H-isoxazol-3-yl]-N-(pyrimidin-2-ylmethyl)isoquinoline-8-carboxamide, N-[1-(2,6-difluorophenyl)pyrazol-3-yl]-2-(trifluoromethyl)benzamide, 5-((1R,3R)-3-(3,5-Bis(trifluoromethyl)phenyl)-2,2-dichlorocyclopropane-1-carboxamido)-2-chloro-N-(3-(2,2-difluoroacetamido)-2,4-difluorophenyl)benzamide, 1-[6-(2,2-difluoro-7-methyl-[1,3]dioxolo[4,5-f]benzimidazol-6-yl)-5-ethylsulfonyl-3-pyridyl]cyclopropanecarbonitrile, 6-(5-cyclopropyl-3-ethylsulfonyl-2-pyridyl)-2,2-difluoro-7-methyl-[1,3]dioxolo[4,5-f]benzimidazole, Ledprona. Flupyroxystrobin, 3,5-bis(trifluoromethyl)-N-[(1S)-1-[1-[6-(trifluoromethyl)-4-pyrimidinyl]-1H-1,2,4-triazol-5-yl]ethyl]-benzamide, 2-(3-ethylsulfonyl-2-pyridyl)-5-(2,2,3,3,3-pentafluoropropoxy)pyrazine, 2-[3-ethylsulfonyl-6-(trifluoromethyl)pyrazolo[1,5-a]pyridin-2-yl]-3-methyl-6-(trifluoromethyl)imidazo[4,5-b]pyridine, 9-(methoxymethyl)-5-(3-pyridyl)-2-oxa-5,6,9,14-tetrazatricyclo[8.4.0.0^{3,7}]tetradeca-1(10),3,6,11,13-pentaen-8-one, bisulfufen, 2-[5-[(E)-2-chloro-3,3,3-trifluoro-prop-1-enyl]-1-methyl-imidazol-2-yl]-5-cyclopropyl-3-ethylsulfonyl-pyridine, cybenzoxasulfyl, isoflualanam;
Bentioflumin, Vadescana, (3Z)-1-[2-fluoro-4-[1-[4-(trifluoromethoxy)phenyl]-1,2,4-triazol-3-yl]phenyl]-3-[3-[5-methyl-2-(2,2,2-trifluoroethoxymethyl)phenyl]-4-oxo-thiazolidin-2-ylidene]urea, 1-[6-(2,2-difluoro-8-oxo-[1,3]dioxolo[4,5-g]chromen-7-yl)-5-ethylsulfonyl-3-pyridyl]cyclopropanecarbonitrile, 2-chloro-N-[(1S)-2-ethyl-1-methyl-butyl]furan-3-carboxamide, galquin, [5-cyclopropyl-2-[3-methyl-6-(trifluoromethyl)imidazo[4,5-b]pyridin-2-yl]-3-pyridyl]-ethyl-hydroxy-oxo-λ⁶-sulfane, 4-[5-(3,5-dichloro-4-fluoro-phenyl)-5-(trifluoromethyl)-4H-isoxazol-3-yl]-2-methyl-N-[1-(2,2,2-trifluoroethylcarbamoyl)cyclopropyl]benzamide, 3-[3-ethylsulfonyl-7-(trifluoromethyl)imidazo[1,2-a]pyridin-2-yl]-7-(trifluoromethyl)chromen-4-one,

The commercially available compounds M listed above may be found in The Pesticide Manual, 18th Edition, C. MacBean, British Crop Protection Council (2018), or http://bcpcdata.com/pesticide-manual.html, http://www.alanwood.net/pesticides.

The active compounds described by IUPAC nomenclature are known from CN103814937; WO2013/003977, WO2007/101369, WO2018/177970, CN10171577, CN102126994, WO2007/101540, WO2007/043677, WO2011/085575, WO2008/134969, WO2012/034403, WO2006/089633, WO2008/067911, WO2006/043635, WO2009/124707, WO2013/050317, WO2010/060379, WO2010/127926, WO2010/006713, WO2012/000896, WO2007/101369, WO2012/143317, WO2015/038503, EP2910126, WO2015/059039, WO2015/190316, WO2012/126766, WO2009/102736, WO2013/116053, WO2018/052136, WO2015150252, WO2020055955, WO2021158455, WO2013092350, WO201811111, EP3608311, WO2019236274, WO2013092350, WO 2018052136, WO2009102736, WO2016174049, WO2012126766, CN106554335, WO2017054524, CN105153113, WO2022072650; WO2018071327, WO2022101502, WO2012158396, WO2007079162, WO2020013147, WO2020097414, EP242081, WO2023058748, WO2017065228, EP3428166, WO2021029308, WO2022009058, WO2017067500, WO2020090585, WO2017146226, WO2021043115, WO2023016278, WO2021011722, WO2024126388, WO2024137594, EP4389739, WO2016096584, WO2024120137, WO2024188755, CN115925576, WO2024087613.

The following list of fungicides, in conjunction with which the compounds of the invention can be used, illustrates the possible combinations:
A) Respiration (C)
   - complex III at Qₒ site (Qol, C3): azoxystrobin (A.1.1), bifemetstrobin (A.1.24), bifujunzhi (A.1.37), coumethoxystrobin (A.1.2), coumoxystrobin (A.1.3), dimoxystrobin (A.1.4), enestroburin (A.1.5), famoxadone (A.1.21), fenamidone (A.1.23), fenaminstrobin (A.1.6), flufenoxystrobin (A.1.7), fluoxastrobin (A.1.8), kresoxim-methyl (A.1.9), mandestrobin (A.1.10), metominostrobin (A.1.11), metyltetraprole (A.1.25; member of MoA subgroup A), orysastrobin (A.1.12), picoxystrobin (A.1.13), pyraclostrobin (A.1.14), pyrametostrobin (A.1.15), pyraoxystrobin (A.1.16), pyribencarb (A.1.19), pyriminostrobin (A.1.36), triclopyricarb (A.1.20), trifloxystrobin (A.1.17), 2-(2-(3-(2,6-dichlorophenyl)-1-methyl-allylidene-aminooxymethyl)-phenyl)-2-methoxyimino-*N*-methyl-acetamide (A.1.18), methyl-*N*-[2-[(1,4-dimethyl-5-phenyl-pyrazol-3-yl)oxylmethyl]phenyl]-*N*-methoxy-carbamate (A.1.22), (*Z*,2*E*)-5-[1-(2,4-dichlorophenyl)pyrazol-3-yl]-oxy-2-methoxyimino-*N*,3-dimethylpent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), 2-(ortho-((2,5-dimethylphenyl-oxymethylen)phenyl)-3-methoxy-acrylic acid methylester (A.1.38), methyl (*Z*)-3-methoxy-2-[2-methyl-5-(3-propylpyrazol-1-yl)phenoxy]prop-2-enoate, methyl (*Z*)-2-[5-(3-isopropylpyrazol-1-yl)-2-methyl-phenoxy]-3-methoxy-prop-2-enoate, methyl (*Z*)-3-methoxy-2-[2-methyl-5-[3-(trifluoromethyl)pyrazol-1-yl]phenoxy]prop-2-enoate, methyl (*Z*)-3-methoxy-2-[2-methyl-5-(4-propyltriazol-2-yl)phenoxy]prop-2-enoate, methyl (*Z*)-3-methoxy-2-[2-methyl-5-[4-(trifluoromethyl)triazol-2-yl]phenoxy]prop-2-enoate, methyl (*Z*)-2-[5-(4-isopropyltriazol-2-yl)-2-methyl-phenoxy]-3-methoxy-prop-2-enoate, methyl (*Z*)-2-(5-cyclobutyl-2-methyl-phenoxy)-3-methoxy-prop-2-enoate, methyl (*Z*)-2-(5-cyclopentyl-2-methyl-phenoxy)-3-methoxy-prop-2-enoate, methyl (*Z*)-2-(5-cyclopropyl-2-methyl-phenoxy)-3-methoxy-prop-2-enoate, methyl (*Z*)-2-(5-cyclohexyl-2-methyl-phenoxy)-3-methoxy-prop-2-enoate, methyl (*E*)-3-methoxy-2-[(2-methyl-5-phenyl-phenyl)methyl]prop-2-enoate, methyl (*E*)-3-methoxy-2-[[5-[(*E*)-*N*-methoxy-*C*-methyl-carbonimidoyl]-2,4-dimethyl-phenyl]methyl]prop-2-enoate; methyl (*E*)-2-[[5-(2-cyclopropyl-ethynyl)-2,4-dimethyl-phenyl]methyl]-3-methoxy-prop-2-enoate; methyl (*E*)-3-methoxy-2-(2-phenyl-1,3-benzoxazol-4-yl)prop-2-enoate; methyl (*E*)-3-methoxy-2-(2-phenyl-1,3-benzothiazol-4-yl)prop-2-enoate; methyl (*E*)-3-methoxy-2-(2-phenyl-1,3-benzoxazol-7-yl)prop-2-enoate; methyl (*Z*)-2-[6-(2-cyclopropylethynyl)benzimidazol-1-yl]-3-methoxy-prop-2-enoate; methyl (*Z*)-3-methoxy-2-(6-phenylbenzimidazol-1-yl)prop-2-enoate; methyl (*Z*)-2-(3-chloro-6-phenyl-indol-1-yl)-3-methoxy-prop-2-enoate; methyl (*Z*)-2-(2,3-dichloro-6-phenyl-indol-1-yl)-3-methoxy-prop-2-enoate; methyl (Z)-3-methoxy-2-[6-[(*E*)-methoxy-iminomethyl]indol-1-yl]prop-2-enoate; methyl (*Z*)-3-methoxy-2-[6-[(E)-N-methoxy-*C*-methyl-carbonimidoyl]indol-1-yl]prop-2-enoate, methyl *N*-[[5-[1-(2,6-difluoro-4-isopropylphenyl)pyrazol-3-yl]-2-methyl-phenyl]methyl]carbamate, methyl *N*-[[5-[1-(4-cyclopropyl-2,6-difluoro-phenyl)pyrazol-3-yl]-2-methyl-phenyl]methyl]carbamate, methyl *N*-[[5-[1-(4-chloro-2,6-difluoro-phenyl)pyrazol-3-yl]-2-methyl-phenylmethyl]carbamate, methyl *N*-[[5-[1-[2,6-difluoro-4-(trifluoromethyl)phenyl]pyrazol-3-yl]-2-methylphenyl]methyl]carbamate;
   - complex III at Qᵢ site (Qil, C4): cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-dioxo-1,5-dioxonan-7-yl]-2-methylpropanoate (A.2.3), fenpicoxamid (A.2.4), florylpicoxamid (A.2.5), metarylpicoxamid (A.2.6);
   - complex II (SDHI, C2): benodanil (A.3.1), benzovindiflupyr (A.3.2), bixafen (A.3.3), boscalid (A.3.4), carboxin (A.3.5), cyclobutrifluram (A.3.24), fenfuram (A.3.6), fluopyram (A.3.7), flutolanil (A.3.8), fluxapyroxad (A.3.9), furametpyr (A.3.10), inpyrfluxam (A.3.22), isofetamid (A.3.11), isoflucypram (A.3.31), isopyrazam (A.3.12), mepronil (A.3.13), oxycarboxin (A.3.14), penflufen (A.3.15), penthiopyrad (A.3.16), pydiflumetofen (A.3.17), pyrapropoyne (A.3.23), pyraziflumid (A.3.18), sedaxane (A.3.19), tecloftalam (A.3.20), thifluzamide (A.3.21), fluindapyr (A.3.28), *N*-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]lphenyl]-3-methoxy-prop-2-enoate (A.3.30), 2-(difluoromethyl)-*N*-(1,1,3-trimethyl-indan-4-yl)pyridine-3-carboxamide (A.3.32), 2-(difluoromethyl)-*N*-[(3*R*)-1,1,3-trimethylindan-4-yl]pyridine-3-carboxamide (A.3.33), 2-(difluoromethyl)-*N*-(3-ethyl-1,1-dimethyl-indan-4-yl)pyridine-3-carboxamide (A.3.34), 2-(difluoromethyl)-*N*-[(3*R*)-3-ethyl-1,1-dimethyl-indan-4-yl]pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]-pyridine-3-carboxamide (A.3.37), 2-(difluoromethyl)-*N*-(3-isobutyl-1,1-dimethyl-indan-4-yl)-pyridine-3-carboxamide (A.3.38), 2-(difluoromethyl)-*N*-[(3*R*)-3-isobutyl-1,1-dimethyl-indan-4-yl]pyridine-3-carboxamide (A.3.39);
   - complex I NADH oxido-reductase (C1): diflumetorim (A.4.1);
   - uncouplers (C5): binapacryl (A.4.2), dinobuton (A.4.3), dinocap (A.4.4), fluazinam (A.4.5), meptyldinocap (A.4.6), ferimzone (A.4.7);
   - inhibitors of ox. phosphorylation (C6): fentin salts, e.g. fentin-acetate (A.4.8), fentin chloride (A.4.9) or fentin hydroxide (A.4.10);
   - ATP transport: silthiofam (A.4.11);
   - quinone inside and outside inhibitor stigmatellin binding type (QioSI; C8): ametoctradin (A.5.1);
B) Sterol biosynthesis (G)
   - C14 demethylase (DMI, G1): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluoxytioconazole (B.1.33), fluquinconazole (B.1.10), flusilazole (B.1.11), flutriafol (B.1.12), hexaconazole (B.1.13), imibenconazole (B.1.14), ipconazole (B.1.15), ipfentrifluconazole (B.1.37), mefentrifluconazole (B.1.38), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), 2-(chloromethyl)-2-methyl-5-(*p*-tolylmethyl)-1-(1,2,4-triazol-1-ylmethyl)cyclopentanol (B.1.43), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55), (2*R*)-2-[4-(4-chlorophenoxy)-2-(trifluoromethyl)phenyl]-1-(1,2,4-triazol-1-yl)propan-2-ol, (2*S*)-2-[4-(4-chlorophenoxy)-2-(trifluoromethyl)phenyl]-1-(1,2,4-triazol-1-yl)propan-2-ol, methyl 2-[2-chloro-4-(4-chlorophenoxy)phenyl]-2-hydroxy-3-(1,2,4-triazol-1-yl)propanoate (B.1.56), 2-[2-chloro-4-(4-chlorophenoxy)phenyl]-2-hydroxy-3-(1,2,4-triazol-1-yl)propanoic acid (B.1.57); imidazoles: imazalil (B.1.44), pefurazoate (B.1.45), prochloraz (B.1.46), triflumizole (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52);
   - delta14-reductase (G2): aldimorph (B.2.1), dodemorph (B.2.2), dodemorph-acetate (B.2.3), fenpropidin (B.2.6), fenpropimorph (B.2.4), piperalin (B.2.7), spiroxamine (B.2.8), tridemorph (B.2.5);
   - 3-keto reductase (G3): fenhexamid (B.3.1), fenpyrazamine (B.3.2);
   - other: chlorphenomizole (B.4.1);
C)Nucleic acids metabolism (A)
   - RNA polymerase I (A1): benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - adenosine deaminase (A2): bupirimate (C.2.4), 5-fluoro-2-(*p*-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4-amine (C.2.8);
   - DNA/RNA synthesis (A3): 5-fluorocytosine (C.2.5), hymexazole (C.2.1), octhilinone (C.2.2),
   - gyrase (A4): oxolinic acid (C.2.3);
   - dihydroorotate dehydrogenase (DHODH; A5): ipflufenoquin (C.5.1), quinofumelin (C.5.2), feneptamidoquin (C.5.3);
D)Cytoskeleton and motor protein (B)
   - tubulin polymerization (MBC; B1): benomyl (D.1.1), carbendazim (D.1.2), fuberidazole (D.1.3), pyridachlometyl (D.1.6), thiabendazole (D.1.4), thiophanate-methyl (D.1.5), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluoro-phenyl)-*N*-(2-chloro-6-fluoro-phenyl)-2,5-dimethyl-pyrazol-3-amine (D.1.16), 4-(2-bromo-4-fluorophenyl)-*N*-(2-fluoro-6-nitrophenyl)-1,3-dimethyl-1*H*-pyrazol-5-amine, 4-(2-chloro-4,6-difluorophenyl)-*N*-(2-fluoro-6-nitrophenyl)-1,3-dimethyl-1*H*-pyrazol-5-amine, 4-(2-chloro-4-fluorophenyl)-*N*-(2-fluoro-6-nitrophenyl)-3-ethyl-1-methyl-1*H*-pyrazol-5-amine, 4-(2-chloro-4-fluorophenyl)-*N*-(2-fluoro-4-methyl-6-nitrophenyl)-1,3-dimethyl-1*H*-pyrazol-5-amine, 4-(2-chloro-4-fluorophenyl)-*N*-(2-fluoro-6-nitrophenyl)-1,3-dimethyl-1*H*-pyrazol-5-amine, 4-(2,4-difluorophenyl)-*N*-(2-fluoro-6-nitrophenyl)-1,3-dimethyl-1*H*-pyrazol-5-amine;
   - tubulin polymerization (B2): diethofencarb (D.2.1),
   - tubulin polymersation (B3): ethaboxam (D.2.2), zoxamide (D.2.5);
   - cell division (B4): pencycuron (D.2.3);
   - spectrin-like proteins (B5): fluopicolide (D.2.4), fluopimomide (D.2.9);
   - actin/myosin/fimbrin function (B6): metrafenone (D.2.6), phenamacril (D.2.8), pyriofenone (D.2.7);
E)Amino acids and protein synthesis (D)
   - methionine synthesis (D1): cyprodinil (E.1.1), mepanipyrim (E.1.2), pyrimethanil (E.1.3);
   - ribosome, termination step (D2): blasticidin-S (E.2.1);
   - ribosome initiation step (D3): kasugamycin (E.2.2), kasugamycin hydrochloride-hydrate (E.2.3);
   - ribosome initiation step (D4): streptomycin (E.2.5);
   - ribosome elongation step (D5): mildiomycin (E.2.4), oxytetracyclin (E.2.6);
F) Signal transduction
   - mechanism unknown (E1): proquinazid (F.2.2), quinoxyfen (F.2.1);
   - MAP/histidine kinase os-2 (E2): fludioxonil (F.1.5);
   - MAP/histidine kinase os-1 (E3): iprodione (F.1.2), procymidone (F.1.3), vinclozolin (F.1.4);
G) Lipid synthesis or transport / membrane (F)
   - methyl transferase (F2): edifenphos (G.1.1), iprobenfos (G.1.2), isoprothiolane (G.1.4); pyrazophos (G.1.3);
   - cell peroxidation (F3): biphenyl (G.2.5), chloroneb (G.2.6), dicloran (G.2.1), etridiazole (G.2.7), quintozene (G.2.2), tecnazene (G.2.3), tolclofos-methyl (G.2.4);
   - cell membrane permeability (F4): propamocarb (G.4.1);
   - ergosterol binding (F8): natamycin;
   - oxysterol binding protein (F9): fluoxapiprolin (G.5.3), oxathiapiprolin (G.5.1), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11), (1-(4-(4-(5-(2,6-dichlorophenyl)-4,5-dihydroisoxazol-3-yl)thiazol-2-yl)piperidin-1-yl)-2-((3-trifluoromethyl)pyrazin-2-yl)oxy)ethan-1-one, 1-(4-(4-(5-(2-chloro-6-fluorophenyl)-4,5-dihydroisoxazol-3-yl)thiazol-2-yl)piperidin-1-yl)-2-((3-trifluoromethyl)pyridin-2-yl)oxy)ethan-1-one, *tert*-butyl 4-(4-(5-(2-bromo-6-fluorophenyl)-4,5-dihydroisoxazol-3-yl)thiazol-2-yl)piperidin-1-carboxylate, ((2-(3-(2-(1-(2-(3,5-bis(trifluoromethyl)-1*H*-pyrazol-1-yl)acetyl)piperidin-4-yl)thiazol-4-yl)-4,5-dihydroisoxazo-5-yl)-3-fluorophenyl)imino)dimethyl-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-fluorophenyl)imino)dimethyl-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-chorophenyl)imino)(isopropyl)(methyl)-λ⁶-sulfanone, ((2-(3-(2-(1-(2-(3,5-bis(trifluoromethyl)-1*H*-pyrazol-1-yl)acetyl)piperidin-4-yl)thiazol-4-yl)-4,5-dihydroisoxazo-5-yl)-3-fluorophenyl)imino)(isopropyl)(methyl)-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-(trifluoromethyl)phenyl)imino)dimethyl-λ⁶-sulfanone, ((3-fluoro-2-(3-(2-(1-(2-(5-methyl-3-(trifluoromethyl)-1*H*-pyrazol-1-yl)acetyl)piperidin-4-yl)thiazol-4-yl)-4,5-dihydroisoxazo-5-yl)-phenyl)imino)dimethyl-λ⁶-sulfanone;
H)Multi Site Activity (M)
   - inorganics (M01): Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - dithiocarbamates and relatives: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9), zinc thiazole (H.2.10);
   - organochlorine compounds (M04, M05, M06, M08): anilazine (H.3.1), captafol (H.3.3), captan (H.3.4), chlorothalonil (H.3.2), dichlofluanid (H.3.6), dichlorophen (H.3.7), folpet (H.3.5), hexachlorobenzene (H.3.8), pentachlorphenole (H.3.9) and its salts, phthalide (H.3.10), tolylfluanid (H.3.11);
   - guanidines, quinones, quinoxalines, maleimides, thiocarbamates (M07, M09, M10, M11, M12): chinomethionat (H.4.13), dithianon (H.4.9), fluoroimide (H.4.11), guanidine (H.4.1), guazatine (H.4.4), guazatine-acetate (H.4.5), iminoctadine (H.4.6), iminoctadine-triacetate (H.4.7), iminoctadine-tris(albesilate) (H.4.8), methasulfocarb (H.4.12), 2,6-dimethyl-1*H*,5*H*-[1,4]dithiino[2,3-c:5,6-c']dipyrrole-1,3,5,7(2*H*,6*H*)-tetraone (H.4.10),
I) Cell wall biosynthesis (H) and melanin synthesis in cell wall (I)
   - chitin synthase (H4): polyoxin B (I.1.2);
   - cellulose synthase (H5): benthiavalicarb (I.3.5), dimethomorph (I.3.1), flumorph (I.3.2), iprovalicarb (I.3.6), mandipropamid (I.3.3), pyrimorph (I.3.4), valifenalate (I.3.7);
   - reductase in melanin synthesis (MBI-R; I1) pyroquilon (I.2.1), tricyclazole (I.2.2);
   - dehydratase in melanin synthesis (MBI-D, I2); carpropamid (I.2.3), dicyclomet (I.2.4), fenoxanil (I.2.5);
   - polyketide synthase in melanin synthesis (MBI-P, I3): tolprocarb (I.2.6);
J) Plant defence induction (P1 to P8)
   - salicylate-related (P01-P03, P08): acibenzolar-S-methyl (J.1.1), probenazole (J.1.2), isotianil (J.1.3), tiadinil (J.1.4), dichlobentiazox (J.1.13); phosphonates (P07): fosetyl (J.1.6), fosetyl-aluminum (J.1.7), phosphorous acid and its salts (J.1.8), calcium phosphonate (J.1.11), potassium phosphonate (J.1.12); others: potassium or sodium bicarbonate (J.1.9), 4-cyclopropyl-*N*-(2,4-di¬methoxy¬phenyl)thiadiazole-5-carboxamide (J.1.10);
K) Unknown mode of action (U)
   - aminopyrifen (K.1.54), benziothiazolinone (K.1.48), bromothalonil (K.1.49), bronopol (K.1.1), cyflufenamid (K.1.3), cymoxanil (K.1.4), dazomet (K.1.5), debacarb (K.1.6), diclomezine (K.1.8), difenzoquat (K.1.9), difenzoquat-methylsulfate (K.1.10), diphenylamin (K.1.11), dodine, dodine free base (K.1.18), fenitropan (K.1.12), flufenoxadiazam (K.1.58) [MoA proposed: class II histone deacetylase inhibitor], flumetover (K.1.14), flumetylsulforim (K.1.60), flusulfamide (K.1.15), flutianil (K.1.16), harpin (K.1.17), nitrapyrin (K.1.19), nitrothal-isopropyl (K.1.20), oxine-copper (K.1.22), picarbutrazox (K.1.41), pyrisoxazole (K.1.37), seboctylamine (K.1.61), tebufloquin (K.1.24), tecloftalam (K.1.25), triazoxide (K.1.26), validamycin (K.1.2); *N'*-(4-(4-chloro-3-trifluoromethyl-phenoxy)-2,5-dimethylphenyl)-*N*-ethyl-*N*-methyl formamidine (K.1.27), *N'*-(4-(4-fluoro-3-trifluoromethyl-phenoxy)-2,5-dimethyl-phenyl)-*N*-ethyl-*N*-methyl formamidine (K.1.28), *N*'-[4-[[3-[(4-chlorophenyl)methyl]-1,2,4-thiadiazol-5-yl]oxy]-2,5-dimethyl-phenyl]-*N*-ethyl-*N*-methyl-formamidine (K.1.29), *N*'-(5-bromo-6-indan-2-yloxy-2-methyl-3-pyridyl)-*N*-ethyl-*N*-methyl-formamidine (K.1.30), *N'*-[5-bromo-6-[1-(3,5-difluorophenyl)ethoxy]-2-methyl-3-pyridyl]-*N*-ethyl-*N*-methyl-formamidine (K.1.31), *N'*-[5-bromo-6-(4-isopropylcyclohexoxy)-2-methyl-3-pyridyl]-*N*-ethyl-*N*-methyl-formamidine (K.1.32), *N'*-[5-bromo-2-methyl-6-(1-phenylethoxy)-3-pyridyl]-N-ethyl-N-methyl-formamidine (K.1.33), *N*'-(2-methyl-5-trifluoromethyl-4-(3-trimethylsilanyl-propoxy)-phenyl)-*N*-ethyl-*N*-methyl formamidine (K.1.34), *N'*-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-di-methoxy-phenyl)-isoxazol-5-yl]-2-prop-2-ynyloxy-acetamide (K.1.36), 3-[5-(4-methylphenyl)-2,3-dimethyl-isoxazolidin-3-yl]-pyridine (K.1.38), 5-chloro-1-(4,6-di-methoxy-pyrimidin-2-yl)-2-methyl-1*H*-benzoimidazole (K.1.39), ethyl (Z)-3-amino-2-cyano-3-phenyl-prop-2-enoate (K.1.40), pentyl *N*-[6-[[(*Z*)-[(1-methyltetrazol-5-yl)-phenylmethylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), 2-(6-benzyl-2-pyridyl)quinazoline (K.1.50), 2-[6-(3-fluoro-4-methoxy-phenyl)-5-methyl-2-pyridyl]quinazoline (K.1.51), *N*'-(2,5-dimethyl-4-phenoxyphenyl)-*N*-ethyl-*N*-methyl-formamidine (K.1.53), *N*'-[5-bromo-2-methyl-6-(1-methyl-2-propoxy-ethoxy)-3-pyridyl]-*N*-ethyl-*N*-methyl-formamidine (K.1.56), *N*'-[4-(4,5-dichlorothiazol-2-yl)oxy-2,5-dimethyl-phenyl]-*N*-ethyl-*N*-methyl-formamidine (K.1.57), *N*-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropanecarboxamide (K.1.61), *N*-((4-[5-(trifluoromethyl)-1,2,4-oxadiazol-3-yl]phenyl)methyl)propanamide (K.1.62), 3,3,3-trifluoro-*N*-[[3-fluoro-4-[5-(trifluoromethyl)-1,2,4-oxadiazol-3-yl]phenyl]methyl]propanamide (K.1.63), 3,3,3-trifluoro-*N*-[[2-fluoro-4-[5-(trifluoromethyl)-1,2,4-oxadiazol-3-yl]phenyl]methyl]propanamide (K.1.64), *N*-[2,3-difluoro-4-[5-(trifluoromethyl)-1,2,4-oxadiazol-3-yl]benzyl]butanamide (K.1.65), *N*-[[2,3-difluoro-4-[5-(trifluoromethyl)-1,2,4-oxadiazol-3-yl]phenyl]methyl]-3,3,3-trifluoro-propanamide (K.1.66), 1-methoxy-1-methyl-3-[[4-[5-(trifluoromethyl)-1,2,4-oxadiazol-3-yl]phenyl]methyl]urea (K.1.67), 1,1-diethyl-3-[[4-[5-[trifluoromethyl]-1,2,4-oxadiazol-3-yl]phenyl]methyl]urea (K.1.68), *N*,2-dimethoxy-*N*-[[4-[5-(trifluoromethyl)-1,2,4-oxadiazol-3-yl]phenyl]methyl]propanamide (K.1.69), *N*-ethyl-2-methyl-*N*-[[4-[5-(trifluoromethyl)-1,2,4-oxadiazol-3-yl]phenyl]methyl]propanamide (K.1.70), 1-methoxy-3-methyl-1-[[4-[5-(trifluoromethyl)-1,2,4-oxadiazol-3-yl]phenyl]methyl]urea (K.1.71), 1-[[4-[5-(trifluoromethyl)-1,2,4-oxadiazol-3-yl]phenyl]methyl]pyrrolidin-2-one (K.1.72), 1-[[4-[5-(trifluoromethyl)-1,2,4-oxadiazol-3-yl]phenyl]methyl]piperidin-2-one (K.1.73), 4-[[4-[5-(trifluoromethyl)-1,2,4-oxadiazol-3-yl]phenyl]methyl]morpholin-3-one (K.1.74), 4,4-dimethyl-2-[[4-[5-(trifluoromethyl)-1,2,4-oxadiazol-3-yl]phenyl]methyl]isoxazolidin-3-one (K.1.75), 2-[[4-[5-(trifluoromethyl)-1,2,4-oxadiazol-3-yl]phenyl]methyl]isoxazolidin-3-one (K.1.76), 5,5-dimethyl-2-[[4-[5-(trifluoromethyl)-1,2,4-oxadiazol-3-yl]phenyl]methyl]isoxazolidin-3-one (K.1.77), 3,3-dimethyl-1-[[4-[5-(trifluoromethyl)-1,2,4-oxadiazol-3-yl]phenyl]methyl]piperidin-2-one (K.1.78), 2-[[4-[5-(trifluoromethyl)-1,2,4-oxadiazol-3-yl]phenyl]methyl]oxazinan-3-one (K.1.79), 1-[[3-fluoro-4-[5-(trifluoromethyl)-1,2,4-oxadiazol-3-yl]phenyl]methyl]azepan-2-one (K.1.80), 4,4-dimethyl-1-[[4-[5-(trifluoromethyl)-1,2,4-oxadiazol-3-yl]-phenyl]methyl]pyrrolidin-2-one (K.1.81), 5-methyl-1-[[4-[5-(trifluoromethyl)-1,2,4-oxadiazol-3-yl]phenyl]methyl]pyrrolidin-2-one (K.1.82), ethyl 1-[[4-[5-(trifluoromethyl)-1,2,4-oxadiazol-3-yl]phenyl]methyl]pyrazole-4-carboxylate (K.1.83), *N*-methyl-1-[[4-[5-(trifluoromethyl)-1,2,4-oxadiazol-3-yl]phenyl]methyl]pyrazole-4-carboxamide (K.1.84), *N,N*-dimethyl-1-[4-[5-(trifluoromethyl)-1,2,4-oxadiazol-3-yl]benzyl]-1*H*-1,2,4-triazol-3-amine (K.1.85), *N*-methoxy-*N*-methyl-1-[[4-[5-(trifluoromethyl)-1,2,4-oxadiazol-3-yl]phenyl]methyl]pyrazole-4-carboxamide (K.1.86), propyl-1-[[4-[5-(trifluoromethyl)-1,2,4-oxadiazol-3-yl]phenyl]methyl]pyrazole-4-carboxamide (K.1.87), *N*-methoxy-1-[[4-[5-(trifluoromethyl)-1,2,4-oxadiazol-3-yl]phenyl]methyl]pyrazole-4-carboxamide (K.1.88), *N*-allyl-*N*-[[4-[5-(trifluoromethyl)-1,2,4-oxadiazol-3-yl]phenyl]methyl]propanamide (K.1.89), 3-ethyl-1-methoxy-1-[[4-[5-(trifluoromethyl)-1,2,4-oxadiazol-3-yl]phenyl]methyl]urea (K.1.90), 1,3-dimethoxy-1-[[4-[5-(trifluoromethyl)-1,2,4-oxadiazol-3-yl]phenyl]methyl]urea (K.1.91), *N*-allyl-*N*-[[4-[5-(trifluoromethyl)-1,2,4-oxadiazol-3-yl]phenyl]methyl]acetamide (K.1.92), *N*-[4-[5-(trifluoromethyl)-1,2,4-oxadiazol-3-yl]benzyl]cyclopropanecarboxamide (K.1.93), 1-methyl-3-[[4-[5-(trifluoromethyl)-1,2,4-oxadiazol-3-yl]phenyl]methyl]urea (K.1.94), *N*'-[2-chloro-4-(2-fluorophenoxy)-5-methyl-phenyl]-*N*-ethyl-*N*-methyl-formamidine (K.1.95), *N*'-[2-chloro-4-[(4-methoxy-phenyl)methyl]-5-methyl-phenyl]-*N*-ethyl-*N*-methyl-formamidine (K.1.96), *N'*-[2-chloro-4-[(4-cyano-phenyl)methyl]-5-methyl-phenyl]-*N*-ethyl-*N*-methyl-formamidine (K.1.97), *N*'-[2,5-dimethyl-4-(*o*-tolylmethyl)phenyl]-*N*-ethyl-N-methyl-formamidine (K.1.98), 6-chloro-3-(3-cyclopropyl-2-fluoro-phenoxy)-*N*-[2-(2,4-dimethylphenyl)-2,2-difluoro-ethyl]-5-methyl-pyridazine-4-carboxamide (K.1.99), 3-(3-bromo-2-fluoro-phenoxy)-6-chloro-*N*-[2-(2-chloro-4-methyl-phenyl)-2,2-difluoro-ethyl]-5-methyl-pyridazine-4-carboxamide (K.1.100), 6-chloro-*N*-[2-(2-chloro-4-methyl-phenyl)-2,2-difluoro-ethyl]-3-(3-cyclopropyl-2-fluoro-phenoxy)-5-methyl-pyridazine-4-carboxamide (K.1.101), 6-chloro-3-(3-cyclopropyl-2-fluoro-phenoxy)-*N*-[2-(3,4-dimethylphenyl)-2,2-difluoro-ethyl]-5-methyl-pyridazine-4-carboxamide (K.1.102), 6-chloro-3-(3-chloro-2-fluorophenoxy)-*N*-[2-(2,4-dimethylphenyl)-2,2-difluoro-ethyl]-5-methyl-pyridazine-4-carboxamide (K.1.103), *N*-[2-(2-bromo-4-methyl-phenyl)-2,2-difluoro-ethyl]-6-chloro-3-(3-cyclopropyl-2-fluoro-phenoxy)-5-methyl-pyridazine-4-carboxamide (K.1.104), 2-[cyano-(2,6-difluoro-4-pyridyl)amino]-5-methyl-*N*-spiro[3.4]octan-3-yl-thiazole-4-carboxamide, 2-[acetyl-(2,6-difluoro-4-pyridyl)amino]-*N*-(2,2-dimethylcyclobutyl)-5-methylthiazole-4-carboxamide, 2-[(2,6-difluoro-4-pyridyl)-(2-methoxyacetyl)amino]-*N*-(2,2-dimethylcyclobutyl)-5-methyl-thiazole-4-carboxamide, 2-[cyano-(2,6-difluoro-4-pyridyl)amino]-*N*-(2,2-dimethylcyclobutyl)-5-methyl-thiazole-4-carboxamide, *N*-[1-[[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]methyl]-2-methyl-propyl]-2-methyl-propanamide, *N*-[1-[[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]methyl]-2-methyl-propyl]-2,2-dimethyl-propanamide, *N*-[2-[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]-1-methyl-ethyl]-2-methyl-propanamide, *N-*[2-[3-[2-hydroxy-2-(2-methoxyphenyl)ethyl]-5-[(*Z*)-*N*-isopropoxy-*C*-methyl-carbonimidoyl]-2,6-dioxo-pyrimidin-1-yl]-1-methyl-ethyl]-2-methyl-propanamide, *N*-[2-[3-[2-(2-cyanoethoxy)-2-(5-fluoro-2-methoxy-phenyl)ethyl]-5-[(*Z*)-*N*-isopropoxy-C-methyl-carbonimidoyl]-2,6-dioxo-pyrimidin-1-yl]-1-methyl-ethyl]-2-methyl-propanamide, *rac*-3-[3-(3-chloro-2-fluoro-phenoxy)-6-methyl-pyridazin-4-yl]-5-[(2-chloro-4-methyl-phenyl)methyl]-5,6-dihydro-4*H*-1,2,4-oxadiazine, (5*S*)-3-[3-(3-chloro-2-fluoro-phenoxy)-6-methyl-pyridazin-4-yl]-5-[(2-chloro-4-methylphenyl)methyl]-5,6-dihydro-4*H*-1,2,4-oxadiazine, (5*R*)-3-[3-(3-chloro-2-fluoro-phenoxy)-6-methyl-pyridazin-4-yl]-5-[(2-chloro-4-methyl-phenyl)methyl]-5,6-dihydro-4*H*-1,2,4-oxadiazine, 2-(4-fluorophenoxy)-1-[4-[5-(trifluoromethyl)-1,2,4-oxadiazol-3-yl]phenyl]ethanone, 2-[(6-fluoro-3-pyridyl)oxy]-1-[4-[5-(trifluoromethyl)-1,2,4-oxadiazol-3-yl]phenyl]ethanone, 2-(4-fluoroanilino)-1-[4-[5-(trifluoromethyl)-1,2,4-oxadiazol-3-yl]phenyl]ethenone, ethyl 1-[[4-[[2-(trifluoromethyl)-1,3-dioxolan-2-yl]methoxy]-phenyl]methyl]-1*H*-pyrazole-4-carboxylate, ethyl 1-[[4-[[(1Z)-2-ethoxy-3,3,3-trifluoro-1-propen-1-yl]oxy]phenyl]methyl]-1*H*-pyrazole-4-carboxylate;

The fungicides described by common names, their preparation and their activity e.g. against harmful fungi is known (cf.: http://www.alanwood.net/pesticides/); these substances are commercially available.

The active substances, their preparation and their activity e.g. against fungi is known (www.bcpcpesticidecompendium.bcpc.org/); many of them are commercially available. Compounds defined by IUPAC nomenclature, their preparation and pesticidal activity are also known (e.g. Can. J. Plant Sci. 48(6), 587-94, 1968; EP-141 317; EP-152 031; EP-226 917; EP-243 970; EP-256 503; EP-428 941; EP-532 022; EP-1 028 125; EP-1 035 122; EP-1 201 648; EP-1 122 244, JP2002316902; DE19650197; DE10021412; DE102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441, WO 16/156241, WO 16/162265, WO 23/99460, WO 21/244950, WO 21/244951, WO 22/130188, WO 22/243810, WO 21/255070, WO 21/176057, WO 21/153754, JP2023064110, JP2022153603, JP2022046550, JP2022031355, WO 22/249074, WO 23/046861, WO 18/177894, WO 20/212513, WO 20/097012, US 2023/0069915.

Suitable mixing partners for the compounds of the invention also include biopesticides. Biopesticides have been defined as a form of pesticides based on micro-organisms (bacteria, fungi, viruses, nematodes, etc.) or natural products (compounds, e.g. metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (e.g. growth or developmental regulation, attractants, repellents or defence activators (e.g. induced resistance) and are relatively non-toxic to mammals.

The following list of biopesticides, in conjunction with which the compounds of the invention can be used, illustrates the possible combinations:
L) Biopesticides
L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. amyloliquefaciens* ssp. *plantarum* (also referred to as B. *velezensis), B. megaterium, B. mojavensis, B. mycoides, B. pumilus, B. simplex, B. solisalsi, B. subtilis, B. subtilis var. amyloliquefaciens, B. velezensis, Candida oleophila,* C. *saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea* f. *catenulate* (also named *Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus, P. polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes myco-parasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum*, *T. stromaticum, T. virens, T. viride, Typhula phacorrhiza*, *Ulocladium oudemansii, Verticillium dahliae,* zucchini yellow mosaic virus (avirulent strain);
L2) Biochemical pesticides with fungicidal, bactericidal, viricidal and/or plant defense activator activity: harpin protein, plant oils (BM3): tea tree oil, orange oil (L.2.1), eugenol, limonene (L.2.2), geraniol (L.2.3), thymol (L.2.4); Reynoutria sachalinensis extract, aureobasidin (in particular aureobasidin A (L.2.5)), ambruticin (L.2.6), bafilomycin (L.2.7) (in particular bafilomycin A1, B1 and C1), chlorflavonin (L.2.8), cinnamaldehyde (L.2.9), natamycin (L.2.10; F8);
L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae*, *B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae, M. anisopliae var. anisopliae, M. anisopliae var. acridum, Nomuraea rileyi, Paecilomyces fumosoroseus, P. lilacinus, Paenibacillus popilliae, Pasteuria* spp., *P. nishizawae, P. penetrans, P. ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microflavus;*
L4) Biochemical pesticides with insecticidal, acaricidal, molluscidal, pheromone and/or nematicidal activity: L-carvone, citral, (*E,Z*)-7,9-dodecadien-1-yl acetate, ethyl formate, (*E,Z*)-2,4-ethyl decadienoate (pear ester), (*Z,Z,E*)-7, 11, 13-hexadecatrienal, heptyl butyrate, isopropyl myristate, lavanulyl senecioate, cis-jasmone, 2-methyl-1-butanol, methyl eugenol, methyl jasmonate, (*E,Z*)-2,13-octadecadien-1-ol, (*E,Z*)-2,13-octadecadien-1-ol acetate, (*E,Z*)-3,13-octadecadien-1-ol, (*R*)-1-octen-3-ol, pentatermanone, (*E,Z,Z*)-3,8,11-tetradecatrienyl acetate, (*Z,E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes,* Neem oil, Quillay extract;
L5) Microbial pesticides with plant stress reducing, plant growth regulator, plant growth promoting and/or yield enhancing activity: *Azospirillum amazonense, A. brasilense*, *A. lipoferum, A. irakense, A. halopraeferens*, *Bradyrhizobium* spp., *B. elkanii, B. japonicum, B. liaoningense*, *B. lupini, Delftia acidovorans, Glomus intraradices*, *Mesorhizobium* spp., *Rhizobium leguminosarum* bv. *phaseoli, R. I.* bv. *trifolii, R. I.* bv. *viciae, R. tropici, Sinorhizobium meliloti.*

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices e.g. ATCC or DSM refer to the acronym of the respective culture collection, for details see e.g. here: http://www. wfcc.info/ccinfo/collection/by_acronym/), are referred to in literature, registered and/or are commercially available: mixtures of Aureobasidium pullulans DSM 14940 and DSM 14941 isolated in 1989 in Konstanz, Germany (e.g. blastospores in BlossomProtect^{®} from bio-ferm GmbH, Austria), Azospirillum brasilense Sp245 originally isolated in wheat reagion of South Brazil (Passo Fundo) at least prior to 1980 (BR 11005; e.g. GELFIX^{®} Gramineas from BASF Agricultural Specialties Ltd., Brazil), A. brasilense strains Ab-V5 and Ab-V6 (e.g. in AzoMax from Novozymes BioAg Produtos papra Agricultura Ltda., Quattro Barras, Brazil or Simbiose-Maiz^{®} from Simbiose-Agro, Brazil; Plant Soil 331, 413-425, 2010), Bacillus amyloliquefaciens strain AP-188 (NRRL B-50615 and B-50331; US8,445,255); B. amylo-liquefaciens ssp. plantarum strains formerly also sometimes referred to as B. subtilis, recently together with B. methylotrophicus, and B. velezensis classified as B. velezensis (Int. J. Syst. Evol. Microbiol. 66, 1212-1217, 2016): B. a. ssp. plantarum or B. velezensis D747 isolated from air in Kikugawashi, Japan (US 20130236522 A1; FERM BP 8234; e.g. Double Nickel^{™} 55 WDG from Certis LLC, USA), B. a. ssp. plantarum or B. velezensis FZB24 isolated from soil in Brandenburg, Germany (also called SB3615; DSM 96-2; J. Plant Dis. Prot. 105, 181-197, 1998; e.g. Taegro^{®} from Novozyme Biologicals, Inc., USA), B. a. ssp. plantarum or B. velezensis FZB42 isolated from soil in Brandenburg, Germany (DSM 23117; J. Plant Dis. Prot. 105, 181-197, 1998; e.g. RhizoVital^{®} 42 from AbiTEP GmbH, Germany), B. a. ssp. plantarum or B. vele-zensis MBI600 isolated from faba bean in Sutton Bonington, Nottinghamshire, U.K. at least before 1988 (also called 1430; NRRL B 50595; US 2012/0149571 A1; e.g. Integral^{®} from BASF Corp., USA), B. a. ssp. plantarum or B. velezensis QST-713 isolated from peach orchard in 1995 in California, U.S.A. (NRRL B 21661; e.g. Serenade^{®} MAX from Bayer Crop Science LP, USA), B. a. ssp. plantarum or B. velezensis TJ1000 isolated in 1992 in South Dakoda, U.S.A. (also called 1BE; ATCC BAA-390; CA 2471555 A1; e.g. QuickRoots^{™} from TJ Technologies, Watertown, SD, USA); B. firmus CNCM I-1582, a variant of parental strain EIP-N1 (CNCM I-1556) isolated from soil of central plain area of Israel (WO 2009/126473, US6,406,690; e.g. Votivo^{®} from Bayer CropScience LP, USA), B. pumilus GHA 180 isolated from apple tree rhizo-sphere in Mexico (IDAC 260707-01; e.g. PRO-MIX^{®} BX from Premier Horticulture, Quebec, Canada), B. pumilus INR-7 otherwise referred to as BU F22 and BU-F33 isolated at least be-fore 1993 from cucumber infested by Erwinia tracheiphila (NRRL B-50185, NRRL B-50153; US 8,445,255), B. pumilus KFP9F isolated from the rhizosphere of grasses in South Africa at least before 2008 (NRRL B-50754; WO 2014/029697; e.g. BAC-UP or FUSION-P from BASF Agricultural Specialities (Pty) Ltd., South Africa), B. pumilus QST 2808 was isolated from soil collected in Pohnpei, Federated States of Micronesia, in 1998 (NRRL B 30087; e.g. Sonata^{®} or Ballad^{®} Plus from Bayer Crop Science LP, USA), B. simplex ABU 288 (NRRL B-50304; US8,445,255), B. subtilis FB17 also called UD 1022 or UD10-22 isolated from red beet roots in North America (ATCC PTA-11857; System. Appl. Microbiol. 27, 372-379, 2004; US2010/0260735; WO 2011/109395); B. thuringiensis ssp. aizawai ABTS-1857 isolated from soil taken from a lawn in Ephraim, Wisconsin, U.S.A., in 1987 (also called ABG 6346; ATCC SD-1372; e.g. XenTari^{®} from BioFa AG, Münsingen, Germany), B. t. ssp. kurstaki ABTS-351 identical to HD-1 isolated in 1967 from diseased Pink Bollworm black larvae in Brownsville, Texas, U.S.A. (ATCC SD-1275; e.g. Dipel^{®} DF from Valent BioSciences, IL, USA), B. t. ssp. kurstaki SB4 isolated from E. saccharina larval cadavers (NRRL B-50753; e.g. Beta Pro^{®} from BASF Agricultural Specialities (Pty) Ltd., South Africa), B. t. ssp. tenebrionis NB-176-1, a mutant of strain NB-125, a wild type strain isolated in 1982 from a dead pupa of the beetle Tenebrio molitor (DSM 5480; EP 585 215 B1; e.g. Novodor^{®} from Valent BioSciences, Switzerland), Beauveria bassiana GHA (ATCC 74250; e.g. BotaniGard^{®} 22WGP from Laverlam Int. Corp., USA), B. bassiana JW-1 (ATCC 74040; e.g. Naturalis^{®} from CBC (Europe) S.r.l., Italy), B. bassiana PPRI 5339 isolated from the larva of the tortoise beetle Conchyloctenia punctata (NRRL 50757; e.g. BroadBand^{®} from BASF Agricultural Specialities (Pty) Ltd., South Africa), Bradyrhizobium elkanii strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e.g. GELFIX 5 from BASF Agricultural Specialties Ltd., Brazil), B. japonicum 532c isolated from Wisconsin field in U.S.A. (Nitragin 61A152; Can. J. Plant. Sci. 70, 661-666, 1990; e.g. in Rhizoflo^{®}, Histick^{®}, Hicoat^{®} Super from BASF Agricultural Specialties Ltd., Canada), B. japonicum E-109 variant of strain USDA 138 (INTA E109, SEMIA 5085; Eur. J. Soil Biol. 45, 28-35, 2009; Biol. Fertil. Soils 47, 81-89, 2011); B. japonicum strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e.g. GELFIX 5 or ADHERE 60 from BASF Agricultural Specialties Ltd., Brazil), B. japonicum SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e.g. GELFIX 5 or ADHERE 60 from BASF Agricultural Specialties Ltd., Brazil); Burkholderia sp. A396 isolated from soil in Nikko, Japan, in 2008 (NRRL B-50319; WO 2013/032693; Marrone Bio Innovations, Inc., USA), Coniothyrium minitans CON/M/91-08 isolated from oilseed rape (WO 1996/021358; DSM 9660; e.g. Contans^{®} WG, Intercept^{®} WG from Bayer CropScience AG, Germany), harpin (alpha-beta) protein (Science 257, 85-88, 1992; e.g. Messenger^{™} or HARP-N Tek from Plant Health Care plc, U.K.), Helicoverpa armigera nucleopolyhedrovirus (HearNPV) (J. Invertebrate Pathol. 107, 112-126, 2011; e.g. Helicovex^{®} from Adermatt Biocontrol, Switzerland; Diplomata^{®} from Koppert, Brazil; Vivus^{®} Max from AgBiTech Pty Ltd., Queensland, Australia), Helicoverpa zea single capsid nucleopolyhedrovirus (HzSNPV) (e.g. Gemstar^{®} from Certis LLC, USA), Helicoverpa zea nucleopolyhedrovirus ABA-NPV-U (e.g. Heligen^{®} from AgBiTech Pty Ltd., Queensland, Australia), Heterorhabditis bacteriophora (e.g. Nemasys^{®} G from BASF Agricultural Specialities Limited, UK), Isaria fumosorosea Apopka-97 isolated from mealy bug on gynura in Apopka, Florida, U.S.A. (ATCC 20874; Biocontrol Science Technol. 22(7), 747-761, 2012; e.g. PFR-97^{™} or PreFeRal^{®} from Certis LLC, USA), Metarhizium anisopliae var. anisopliae F52 also called 275 or V275 isolated from codling moth in Austria (DSM 3884, ATCC 90448; e.g. Met52^{®} Novozymes Biologicals BioAg Group, Canada), Metschnikowia fructicola 277 isolated from grapes in the central part of Israel (US 6,994,849; NRRL Y-30752; e.g. formerly Shemer^{®} from Agrogreen, Israel), Paecilomyces ilacinus 251 isolated from infected nematode eggs in the Philippines (AGAL 89/030550; WO1991/02051; Crop Protection 27, 352-361, 2008; e.g. BioAct^{®}from Bayer CropScience AG, Germany and MeloCon^{®} from Certis, USA), Paenibacillus alvei NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), Paenibacillus strains isolated from soil samples from a variety of European locations including Germany: P. epiphyticus Lu17015 (WO 2016/020371; DSM 26971), P. polymyxa ssp. plantarum Lu16774 (WO 2016/020371; DSM 26969), P. p. ssp. plantarum strain Lu17007 (WO 2016/020371; DSM 26970); Pasteuria nishizawae Pn1 isolated from a soybean field in the mid-2000s in Illinois, U.S.A. (ATCC SD 5833; Federal Register 76(22), 5808, February 2, 2011; e.g. Clariva^{™} PN from Syngenta Crop Protection, LLC, USA), Penicillium bilaiae (also called P. bilaii) strains ATCC 18309 (= ATCC 74319), ATCC 20851 and/or ATCC 22348 (=ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e.g. Jump Start^{®}, Provide^{®} from Novozymes Biologicals BioAg Group, Canada), Reynoutria sachalinensis extract (EP 0307510 B1; e.g. Regalia^{®} SC from Marrone Biolnnovations, Davis, CA, USA or Milsana^{®} from BioFa AG, Germany), Steinernema carpocapsae (e.g. Millenium^{®} from BASF Agricultural Specialities Limited, UK), S. feltiae (e.g. Nemashield^{®} from BioWorks, Inc., USA; Nemasys^{®} from BASF Agricultural Specialities Limited, UK), Streptomyces microflavus NRRL B-50550 (WO 2014/124369; Bayer CropScience, Germany), Trichoderma asperelloides JM41R isolated in South Africa (NRRL 50759; also referred to as T. fertile; e.g. Trichoplus^{®} from BASF Agricultural Specialities (Pty) Ltd., South Africa), T. harzianum T-22 also called KRL-AG2 (ATCC 20847; BioControl 57, 687-696, 2012; e.g. Plantshield^{®} from BioWorks Inc., USA or SabrEx^{™} from Advanced Biological Marketing Inc., Van Wert, OH, USA).

According to the invention, the solid material (dry matter) of the biopesticides (with the exception of oils e.g. Neem oil) are considered as active components (e.g. to be obtained after drying or evaporation of the extraction or suspension medium in case of liquid formulations of the microbial pesticides).

In accordance with the invention, the weight ratios and percentages used herein for a biological extract e.g. Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1×10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, e.g. Steinernema feltiae.

When mixtures comprising microbial pesticides are employed in crop protection, the application rates range from 1×10⁶ to 5×10¹⁶ (or more) CFU/ha, preferably from 1×10⁸ to 1×10¹³ CFU/ha, and even more preferably from 1×10⁹ to 5×10¹⁵ CFU/ha and in particular from 1×10¹² to 5×10¹⁴ CFU/ha. In the case of nematodes as microbial pesticides (e.g. Steinernema feltiae), the application rates regularly range from 1×10⁶ to 1×10¹² (or more), preferably from 1×10⁸ to 1×10¹¹, more preferably from 5×10⁸ to 1×10¹⁰ individuals (e.g. in the form of eggs, juvenile or any other live stages, preferably in an infetive juvenile stage) per ha.

When mixtures comprising microbial pesticides are employed in seed treatment, the application rates generally range from 1×10⁶ to 1×10¹² (or more) CFU/seed, preferably from 1×10⁶ to 1×10⁹ CFU/seed. Furthermore, the application rates with respect to seed treatment generally range from 1×10⁷ to 1×10¹⁴ (or more) CFU per 100 kg of seed, preferably from 1×10⁹ to 1×10¹² CFU per 100 kg of seed.

### Formulations

The invention also relates to agrochemical compositions comprising an auxiliary and at least one compound of the invention or a mixture thereof.

An agrochemical composition comprises a pesticidally effective amount of a compound of the invention or a mixture thereof.

The compounds of the invention or the mixtures thereof can be converted into customary types of agro-chemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

The compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, e.g. mineral oil fractions of medium to high boiling point, e.g. kerosene, diesel oil; oils of vegetable or animal origin; hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, CaSO₄, MgSO₄, MgO; polysaccharide powders, e.g. cellulose, starch; fertilizers, e.g. (NH₄)₂SO₄, (NH₄)₃PO₄, NH₄NO₃, ureas; products of vegetable origin, e.g. cereal meal, tree bark meal, wood meal, nutshell meal, and mixtures thereof.

Suitable surfactants are surface-active compounds, e.g. anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds e.g. alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines, or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives e.g. alkylisothiazolinones and benzisothiazolinones.

Suitable anti-freezing agents are ethylene glycol, propylene glycol, urea, and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e.g. polyvinylpyrrolidone) are dissolved in up to 100 wt% organic solvent (e.g. cyclohexanone). Dilution with water gives a dispersion.
iii) Emulsifiable concentrates (EC)
   15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active sub-stance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e.g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. di-phenylme-thene-4,4'-diisocyanatae) are dispersed into an aqueous solution of a protective colloid (e.g. polyvinyl alcohol). The addition of a polyamine (e.g. hexamethylenediamine) results in the for-ation of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, e.g. 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agro-chemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition of the invention e.g. parts of a kit or parts of a binary or ternary mixture may be mixed by the user himself in a spray tank and further auxiliaries may be added, if appropriate.

In a further embodiment, either individual components of the composition according to the invention or partially premixed components, e.g. components comprising compounds of the invention and/or mixing partners as defined above, may be mixed by the user in a spray tank and further auxiliaries and additives may be added.

In a further embodiment, either individual components of the composition according to the invention or partially premixed components, e.g. components comprising compounds of the invention and/or mixing partners as defined above, can be applied jointly (e.g. after tank mix) or consecutively.

### Application methods

The compounds of the invention are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound of the invention.

The compounds of the invention are effective through both contact and ingestion. Furthermore, the compounds of the invention can be applied to any and all developmental stages, e.g. egg, larva, pupa, and adult.

The compounds of the invention can be applied as such or in form of compositions comprising them as defined above. Furthermore, the compounds of the invention can be applied together with a mixing partner or in form of compositions comprising said mixtures. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, e.g. seeds, soil, or the area, material or environment by the pests.

Suitable application methods include i.a. soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known and publicly available from databases of pheromones and semiochemicals, e.g. http://www.pherobase.com.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material, or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grapefruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to ALS inhibitor herbicides by conventional methods of mutagenesis and breeding comprise plant varieties commercially available under the name Clearfield^{®}.

Herbicide tolerance has been created to glyphosate, glufosinate, 2,4-D, dicamba, oxynil herbicides, like bromoxynil and ioxynil, sulfonylurea herbicides, ALS inhibitor herbicides and HPPD inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03. Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are e.g., but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of Bacillus spec. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are known (http://www.isaaa.org/gmapprovaldatabase) and (http://cera-gmc.org/GMCropDatabase).

Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531(MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects may comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

It has been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant e.g. seeds and vegetative plant material e.g. cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises e.g. seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants e.g. potatoes, sugar cane, tobacco, grapes, petunias, geranium/pelargoniums, pansies and impatiens.

In addition, the active compound may also be used for the treatment of seeds from plants, which have been modified by mutagenisis or genetic engineering, and which e.g. tolerate the action of herbicides or fungicides or insecticides.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40% by weight.

In a preferred embodiment a FS formulation is used for seed treatment. Typically, a FS formulation may comprise 1-800 g/l of active ingredient, 1-200 g/l Surfactant, 0 to 200 g/l anti-freezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20% by weight (1 to 200 g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker/adhesion agent), optionally up to 5% by weight, e.g. from 0.1 to 5% by weight of a thickener, optionally from 0.1 to 2% of an anti-foam agent, and optionally a preservative e.g. a biocide, antioxidant or the like, e.g. in an amount from 0.01 to 1% by weight and a filler/vehicle up to 100% by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops e.g. lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

"Plant health" is defined as a condition of the plant and/or its products which is determined by several aspects alone or in combination with each other e.g. yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, e.g. ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects e.g. ants, termites, wasps, flies, mosquitoes, crickets etc. or cockroaches to eat it. The attractiveness can be manipulated by using feeding stimulants or sex pheromones. Food stimulants are preferably chosen from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are known (http://www.pherobase.com).

For use in bait compositions, the typical content of active ingredient is from 0.001 wt% to 15 wt%, desirably from 0.001 wt% to 5 wt% of active compound.

Formulations of the compounds of the invention as aerosols (e.g in spray cans), oil sprays or pump sprays are highly suitable for professional or non-professional users for controlling pests e.g. flies, fleas, ticks, bed bugs, mosquitoes or cockroaches. Aerosol recipes are preferably composed of the active compound, solvents, furthermore auxiliaries e.g. emulsifiers, perfume oils, if appropriate stabilizers, and, if required, propellants.

The oil spray formulations differ from the aerosol recipes in that no propellants are used. For use in spray compositions, the content of active ingredient is from 0.001 to 80 wt%, preferably from 0.01 to 50 wt% and most preferably from 0.01 to 15 wt%.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its re-spective compositions also comprise treating surfaces of huts and houses, air spraying and impregnation of curtains, tents, clothing items, bed nets, tsetse-fly trap. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials e.g. trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 wt%, preferably from 0.1 to 45 wt%, and more preferably from 1 to 25 wt% of at least one repellent and/or insecticide.

### Digital application

The compounds of the invention and the compositions containing them may be applied in combination with, or by utilizing smart agricultural technologies, such as precision agriculture, remote and proximate imaging and image recognition, or smart agricultural site management programs. These smart agricultural technologies typically include models, e.g. computer programs, that support the user by considering information from a wide variety of sources to increase the quality and yield of harvested material, reduce damage by pests including the prediction of pest pressure and smart application of crop protection products, secure environmental protection, support quick and reliable agronomic decision making, reduce usage of fertilizers and crop protection products, reduce product residues in consumables increase spatial and temporal precision of agronomical measures, automate processes, and enable traceability of measures.

Commercially available systems which include agronomic models are e.g. FieldScripts^{™} from The Climate Corporation, Xarvio^{™} from BASF, AGLogic^{™} from John Deere, etc.

Information input for these models include but is not limited to soil data, information on the plants that are currently growing or that may grow at the area of interest including crop plants and/or unwanted vegetation, weather information, information on the location of the area and directly derivable information thereof, information on pest pressure, information on beneficial organisms, and / or historic information of any of the aforementioned.

The information usable for precision agriculture may be based on input by at least one user, be accessible from external data sources and databases, or be based on sensor data. Data sources typically includes proximate-detection systems like soil-borne sensors and remote sensing as may be achieved by imaging with unmanned airborne vehicles like drones, or satellites. Sensors may be included in an Internet-of-Things system and may be directly or indirectly connected to the processing unit, e.g. via a wireless network and/or cloud applications. The information is typically taken into account by at least one processing unit and used to provide recommendations and generate control signals.

Typical technologies that are used in smart agricultural technologies include self-steering robots (such as tractors, harvesters, drones), artificial intelligence (e.g. machine learning), imaging technologies (e.g. image segmentation technologies), big data analysis, and model generation, cloud computing, and machine-to-machine communication.

Precision agriculture such as precision farming is characterized by spatially and/or temporally resolved, targeted application of active ingredients like pesticides, plant-growth-regulators, fertilizers, and/or water including the variation of application rates over the agronomic site, zone or spot application, and of the spatially and/or temporally resolved, targeted planting or seeding of desired plant propagation material to a agronomic site. Precision farming typically includes the use of geo-positioning technologies like GPS for gaining information on the location and boundaries of the area of interest, the utilized application equipment, sensing equipment and recorded data, and to control the actions of farm vehicles such as spraying. By combining geo-positioning data with (digital) maps, it is possible to (semi)-automate agricultural measures at the site of interest, e.g. by using (semi)-autonomous spraying or seeding equipment.

Precision farming may typically include the application of smart spraying equipment, e.g. spot spraying, and precision spraying at a farm, e.g. by irrigation systems, tractors, robots, helicopters, airplanes, unmanned aerial vehicles, such as drones. Such equipment usually includes input sensors (such as e.g. a camera) and a processing unit configured to analyze the input data and configured to provide a recommendation or decision based on the analysis of the input data to apply the compounds of the invention or compositions comprising them to the agronomic site, e.g. the soil, the crop plants, or to control pests in a specific and precise manner. For example, pests may be detected, identified, and/or classified from imagery acquired by a camera. Such identification and/ classification can make use of image processing algorithms, which may utilize artificial intelligence (e.g. machine learning algorithms), or decision trees. In this manner, the compounds or compositions described herein can be applied only at the required location, point in time and dose rate.

### Pests

The compounds of the invention are especially suitable for efficiently combating animal pests e.g. arthropods, gastropods and nematodes including:
insects from the order of Lepidoptera, e.g. Achroia grisella, Acleris spp. e.g. A. fimbriana, A. gloverana, A. variana; Acrolepiopsis assectella, Acronicta major, Adoxophyes spp. e.g. A. cyrtosema, A. orana; Aedia leucomelas, Agrotis spp. e.g. A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia) spp. e.g. A. gemmatalis; Apamea spp., Aproaerema modicella, Archips spp. e.g. A. argyrospila, A. fuscocupreanus, A. rosana, A. xyloseanus; Argyresthia conjugella, Argyroploce spp., Argyrotaenia spp. e.g. A. velutinana; Athetis mindara, Austroasca viridigrisea, Autographa gamma, Autographa nigrisigna, Barathra brassicae, Bedellia spp., Bonagota salubricola, Borbo cinnara, Bucculatrix thurberiella, Bupalus piniarius, Busseola spp., Cacoecia spp. e.g. C. murinana, C. podana; Cactoblastis cactorum, Cadra cautella, Calingo braziliensis, Caloptilis theivora, Capua reticulana, Carposina spp. e.g. C. niponensis, C. sasakii; Cephus spp., Chaetocnema aridula, Cheimatobia brumata, Chilo spp. e.g. C. Indicus, C. suppressalis, C. partellus; Choreutis pariana, Choristoneura spp. e.g. C. conflictana, C. fumiferana, C. longicellana, C. murinana, C. occidentalis, C. rosaceana; Chrysodeixis (=Pseudoplusia) spp., e.g. C. eriosoma, C. includens; Cirphis unipuncta, Clysia ambiguella, Cnaphalocerus spp., Cnaphalocrocis medinalis, Cnephasia spp., Cochylis hospes, Coleophora spp., Colias eurytheme, Conopomorpha spp., Conotrachelus spp., Copitarsia spp., Corcyra cephalonica, Crambus caliginosellus, Crambus teterrellus, Crocidosema (=Epinotia) aporema, Cydalima (=Diaphania) perspectalis, Cydia (=Carpocapsa) spp., e.g. C. pomonella, C. latiferreana; Dalaca noctuides, Datana integerrima, Dasychira pinicola, Dendrolimus spp., e.g. D. pini, D. spectabilis, D. sibiricus; Desmia funeralis, Diaphania spp., e.g. D. nitidalis, D. hyalinata; Diatraea grandiosella, Diatraea saccharalis, Diphthera festiva, Earias spp. e.g. E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia spp., e.g. E. cautella, E. elutella, E. kuehniella; Epinotia aporema, Epiphyas postvittana, Erannis tiliaria, Erionota thrax, Etiella spp., Eulia spp., Eupoecilia ambiguella, Euproctis chrysorrhoea, Euxoa spp., Evetria bouliana, Faronta albilinea, Feltia spp. e.g. F. subterranean; Galleria mellonella, Gracillaria spp., Grapholita spp. e.g. G. funebrana, G. molesta, G. inopinata; Halysidota spp., Harrisina americana, Hedylepta spp., Helicoverpa spp. e.g. H. armigera (=Heliothis armigera), H. zea (=Heliothis zea); Heliothis spp. e.g. H. assulta, H. subflexa, H. virescens; Hellula spp. e.g. H. undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera spp. e.g. L. coffeella, L. scitella; Leuminivora lycinivorella, Lithocolletis blancardella, Lithophane antennata, Llattia octo (=Amyna axis), Lobesia botrana, Lophocampa spp., Loxagrotis albicosta, Loxostege spp. e.g. L. sticticalis, L. cereralis; Lymantria spp., e.g. L. dispar, L. monacha; Lyonetia clerkella, Lyonetia prunifoliella, Malacosoma spp., e.g. M. americanum, M. californicum, M. constrictum, M. neu-stria; Mamestra spp., e.g. M. brassicae, M. configurata; Mamstra brassicae, Manduca spp. e.g. M. quinquemaculata, M. sexta; Marasmia spp, Marmara spp., Maruca testulalis, Megalopyge lanata, Melanchra picta, Melanitis leda, Mocis spp., e.g. M. lapites, M. repanda; Mocis latipes, Monochroa fragariae, Mythimna separata, Nemapogon cloacella, Neoleucinodes elegantalis, Nepytia spp., Nymphula spp., Oiketicus spp., Omiodes indicata, Omphisa anastomosalis, Operophtera brumata, Orgyia pseudotsugata, Oria spp., Orthaga thyrisalis, Ostrinia spp. e.g. O. nubilalis; Oulema oryzae, Paleacrita vernata, Panolis flammea, Parnara spp., Papaipema nebris, Papilio cresphontes, Paramyelois transitella, Paranthrene regalis, Paysandisia archon, Pectinophora spp. e.g. P. gossypiella; Peridroma saucia, Perileucoptera spp., e.g. P. coffeella; Phalera bucephala, Phryganidia californica, Phthorimaea spp. e.g. P. operculella; Phyllocnistis citrella, Phyllonorycter spp. e.g. P. blancardella, P. crataegella, P. issikii, P. ringoniella; Pieris spp. e.g. P. brassicae, P. rapae, P. napi; Pilocrocis tripunctata, Plathypena scabra, Platynota spp. e.g. P. flavedana, P. idaeusalis, P. stultana; Platyptilia carduidactyla, Plebejus argus, Plo-dia interpunctella, Plusia spp, Plutella maculipennis, Plutella xylostella, Pontia protodica, Prays spp., Prodenia spp., Proxenus lepigone, Pseudaletia spp. e.g. P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius spp., Schreckensteinia festaliella, Scirpophaga spp. e.g. S. incertulas, S. innotata; Scotia segetum, Sesamia spp. e.g. S. inferens, Seudyra subflava, Sitotroga cerealella, Sparganothis pilleriana, Spilonota lechriaspis, S. ocelli-na, Spodoptera (=Lamphygma) spp. e.g. S. cosmoides, S. eridania, S. exigua, S. frugiperda, S. latisfascia, S. littoralis, S. litura, S. omithogalli; Stigmella spp., Stomopteryx subsecivella, Strymon bazochii, Sylepta derogata, Synanthedon spp. e.g. S. exitiosa, Tecia solanivora, Telehin licus, Thaumatopoea pityocampa, Thaumatotibia (=Cryptophlebia) leucotreta, Thaumetopoea pityocampa, Thecla spp., Theresimima ampelophaga, Thyrinteina spp, Tildenia inconspicuella, Tinea spp. e.g. T. cloacella, T. pellionella; Tineola bisselliella, Tortrix spp. e.g. T. viridana; Trichophaga tapetzella, Trichoplusia spp. e.g. T. ni; Tuta (=Scrobipalpula) absoluta, Udea spp. e.g. U. rubigalis, U. rubigalis; Virachola spp., Yponomeuta padella, and Zeiraphera canadensis;
insects from the order of Coleoptera, e.g. Acalymma vittatum, Acanthoscehdes obtectus, Adoretus spp., Agelastica alni, Agrilus spp. e.g. A. anxius, A. planipennis, A. sinuatus; Agriotes spp. e.g. A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora spp. e.g. A. glabripennis; Anthonomus spp. e.g. A. eugenii, A. grandis, A. pomorum; Anthrenus spp., Aphthona euphoridae, Apion spp., Apogonia spp., Athous haemorrhoidalis, Atomaria spp. e.g. A. linearis; Attagenus spp., Aulacophora femoralis, Blastophagus piniperda, Blitophaga undata, Bruchidius obtectus, Bruchus spp. e.g. B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus spp. e.g. C. assimilis, C. napi; Chaetocnema tibialis, Cleonus mendicus, Conoderus spp. e.g. C. vespertinus; Conotrachelus nenuphar, Cosmopolites spp., Costelytra zealandica, Crioceris asparagi, Cryptolestes ferrugineus, Cryptorhynchus lapathi, Ctenicera spp. e.g. C. destructor; Curculio spp., Cylindrocopturus spp., Cyclocephala spp., Dac-tylispa balyi, Dectes texanus, Dermestes spp., Diabrotica spp. e.g. D. undecimpunctata, D. speciosa, D. longicornis, D. semipunctata, D. virgifera; Diaprepes abbreviates, Dichocrocis spp., Dicladispa armigera, Diloboderus abderus, Diocalandra frumenti (Diocalandra stigmaticollis), Enaphalodes rufulus, Epilachna spp. e.g. E. varivestis, E. vigintioctomaculata; Epitrix spp. e.g. E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera spp., e.g. H. brunneipennis, H. postica; Hypomeces squamosus, Hypothenemus spp., Ips typographus, Lachnosterna consanguinea, Lasioderma serricorne, Latheticus oryzae, Lathridius spp., Lema spp. e.g. L. bilineata, L. melanopus; Leptinotarsa spp. e.g. L. decemlineata; Leptispa pygmaea, Limonius californi-cus, Lissorhoptrus oryzophilus, Lixus spp., Luperodes spp., Lyctus spp. e.g. L. bruneus; Liogenys fuscus, Macrodactylus spp. e.g. M. subspinosus; Maladera matrida, Megaplatypus mutates, Megascelis spp., Melanotus communis, Meligethes spp. e.g. M. aeneus; Melolontha spp. e.g. M. hippocastani, M. melolontha; Metamasius hemipterus, Microtheca spp., Migdolus spp. e.g. M. fryanus, Monochamus spp. e.g. M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon spp. e.g. P. brassicae, P. cochleariae; Phoracantha recurva, Phyllobius pyri, Phyllopertha horticola, Phyllophaga spp. e.g. P. helleri; Phyllotreta spp. e.g. P. chrysocephala, P. nemorum, P. striolata, P. vittula; Phyllopertha horticola, Popillia japonica, Premnotrypes spp., Psacothea hilaris, Psylliodes chrysocephala, Prostephanus truncates, Psylliodes spp., Ptinus spp., Pulga saltona, Rhizopertha dominica, Rhynchophorus spp. e.g. R. billineatus, R. ferrugineus, R. palmarum, R. phoenicis, R. vulneratus; Saperda candida, Scolytus schevyrewi, Scyphophorus acupunctatus, Sitona lineatus, Sitophilus spp. e.g. S. granaria, S. oryzae, S. zeamais; Sphenophorus spp. e.g. S. levis; Stegobium paniceum, Sternechus spp. e.g. S. subsignatus; Strophomorphus ctenotus, Symphyletes spp., Tanymecus spp., Tenebrio molitor, Tenebrioides mauretanicus, Tribolium spp. e.g. T. castaneum; Trogoderma spp., Tychius spp., Xylotrechus spp. e.g. X. pyrrhoderus; and, Zabrus spp. e.g. Z. tenebrioides;
insects from the order of Diptera e.g. Aedes spp. e.g. A. aegypti, A. albopictus, A. vexans; Anastrepha ludens, Anopheles spp. e.g. A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia spp. e.g. C. bezziana, C. hominivorax, C. macellaria; Chrysops atlanticus, Chrysops discalis, Chrysops silacea, Cochliomyia spp. e.g. C. hominivorax; Contarinia spp. e.g. C. sorghicola; Cordylobia anthropophaga, Culex spp. e.g. C. nigripalpus, C. pipiens, C. quinquefasciatus, C. tarsalis, C. tritaeniorhynchus; Culicoides furens, Culiseta inornata, Culiseta melanura, Cuterebra spp., Dacus cucurbitae, Dacus oleae, Dasineura brassicae, Dasineura oxycoccana, Delia spp. e.g. D. antique, D. coarctata, D. platura, D. radicum; Dermatobia hominis, Drosophila spp. e.g. D. suzukii, Fannia spp. e.g. F. canicularis; Gastraphilus spp. e.g. G. intestinalis; Geomyza tipunctata, Glossina spp. e.g. G. fuscipes, G. morsitans, G. palpalis, G. tachinoides; Haematobia irritans, Haplodiplosis equestris, Hippelates spp., Hylemyia spp. e.g. H. platura; Hypoderma spp. e.g. H. lineata; Hyppobosca spp., Hydrellia philippina, Leptoconops torrens, Liriomyza spp. e.g. L. sativae, L. trifolii; Lucilia spp. e.g. L. caprina, L. cuprina, L. sericata; Lycoria pectoralis, Mansonia titillanus, Mayetiola spp. e.g. M. destructor; Musca spp. e.g. M. autumnalis, M. domestica; Muscina stabulans, Oestrus spp. e.g. O. ovis; Opomyza florum, Oscinella spp. e.g. O. frit; Orseolia oryzae, Pegomya hysocyami, Phlebotomus argentipes, Phorbia spp. e.g. P. antiqua, P. brassicae, P. coarctata; Phytomyza gymnostoma, Prosimulium mixtum, Psila rosae, Psorophora columbiae, Psorophora discolor, Rhagoletis spp. e.g. R. cerasi, R. cingulate, R. indifferens, R. mendax, R. pomonella; Rivellia quadrifasciata, Sarcophaga spp. e.g. S. haemorrhoidalis; Simulium vittatum, Sitodiplosis mosellana, Stomoxys spp. e.g. S. calcitrans; Tabanus spp. e.g. T. atratus, T. bovinus, T. lineola, T. similis; Tannia spp., Thecodiplo-sis japonensis, Tipula oleracea, Tipula paludosa, Wohlfahrtia spp, and Zaprionus indianus;
insects from the order of Thysanoptera e.g., Baliothrips biformis, Dichromothrips corbetti, Dichromothrips ssp., Echinothrips americanus, Enneothrips flavens, Frankliniella spp. e.g. F. fusca, F. occidentalis, F. tritici; Heliothrips spp., Hercinothrips femoralis, Kakothrips spp., Microcephalothrips abdominalis, Neohydatothrips samayunkur, Pezothrips kellyanus, Rhipiphorothrips cruentatus, Scirtothrips spp. e.g. S. citri, S. dorsalis, S. perseae; Stenchaetothrips spp, Taeniothrips cardamoni, Taeniothrips inconsequens, Thrips spp. e.g. T. imagines, T. hawaiiensis, T. oryzae, T. palmi, T. parvispinus, T. tabaci;
insects from the order of Hemiptera e.g., Acizzia jamatonica, Acrosternum spp., e.g. A. hilare; Acyrthosipon spp., e.g. A. onobrychis, A. pisum; Adelges laricis, Adelges tsugae, Adelphocoris spp., e.g. A. rapidus, A. superbus; Aeneolamia spp., Agonoscena spp., Aulacorthum solani, Aleurocanthus woglumi, Aleurodes spp., Aleurodicus disperses, Aleurolobus barodensis, Aleurothrixus spp., Amrasca spp., Anasa tristis, Antestiopsis spp., Anuraphis cardui, Aonidiella spp., Aphanostigma piri, Aphidula nasturtii, Aphis spp. e.g. A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella spp., Aspidiotus spp., Atanus spp., Aulacaspis yasumatsui, Aulacorthum solani, Bactericera cockerelli (Paratrioza cockerelli), Bemisia spp. e.g. B. argentifolii, B. tabaci (Aleurodes tabaci); Blissus spp. e.g. B. leucopterus; Brachycaudus spp. e.g. B. cardui, B. helichrysi, B. persicae, B. prunicola; Brachycolus spp., Brachycorynella as-paragi, Brevicoryne brassicae, Cacopsylla spp. e.g. C. fulguralis, C. pyricola (Psylla piri); Calligypona marginata, Calocoris spp., Campylomma livida, Capitophorus horni, Carneocephala fulgida, Cavelerius spp., Ceraplastes spp., Ceratovacuna lanigera, Ceroplastes ceriferus, Cerosipha gossypii, Chaetosiphon fragaefolii, Chionaspis tegalensis, Chlorita onukii, Chromaphis juglandicola, Chrysomphalus ficus, Cicadulina mbila, Cimex spp. e.g. C. hemipterus, C. lectularius; Circulifer tenellus, Coccomytilus halli, Coccus spp. e.g. C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus spp., Dasynus piperis, Dialeurodes spp. e.g. D. citrifolii; Dalbulus maidis, Diaphorina spp. e.g. D. citri; Diaspis spp. e.g. D. bromeliae; Dichelops furcatus, Diconocoris hewetti, Doralis spp., Dreyfusia nordmannianae, Dreyfusia piceae, Drosicha spp., Dysaphis spp. e.g. D. plantaginea, D. pyri, D. radicola; Dysaulacorthum pseudosolani, Dysdercus spp. e.g. D. cingulatus, D. intermedius; Dysmicoccus spp., Edessa spp., Geocoris spp., Empoasca spp. e.g. E. fabae, E. solana; Epidiaspis leperii, Eriosoma spp. e.g. E. lanigerum, E. pyricola; Erythroneura spp., Eurygaster spp. e.g. E. integriceps; Euscelis bilobatus, Euschistus spp. e.g. E. heros, E. impictiventris, E. servus; Fiorinia theae, Geococcus coffeae, Glycaspis brimblecombei, Halyomorpha spp. e.g. H. halys; Heliopeltis spp., Homalodisca vitripennis (=H. coagulata), Horcias nobilellus, Hyalopterus pruni, Hyperomyzus lactucae, Icerya spp. e.g. I. purchase; Idiocerus spp., Idioscopus spp., Laodelphax striatellus, Lecanium spp., Lecanoideus floccissimus, Lepidosaphes spp. e.g. L. ulmi; Leptocorisa spp., Leptoglossus phyllopus, Lipaphis erysimi, Lygus spp. e.g. L. hesperus, L. lineolaris, L. praten-sis; Maconellicoccus hirsutus, Marchalina hellenica, Macropes excavatus, Macrosiphum spp. e.g. M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella spp., Metopolophium dirhodum, Monellia costalis, Monelliopsis pecanis, Myzocallis coryli, Murgantia spp., Myzus spp. e.g. M. ascalonicus, M. cerasi, M. nicotianae, M. persicae, M. varians; Nasonovia ribisnigri, Neotoxoptera formosana, Neomegalotomus spp, Nephotettix spp. e.g. N. malayanus, N. nigropictus, N. parvus, N. virescens; Nezara spp. e.g. N. viridula; Nilaparvata lugens, Nysius huttoni, Oebalus spp. e.g. O. pugnax; Oncometopia spp., Orthezia praelonga, Oxycaraenus hyalinipennis, Parabemisia myricae, Parlatoria spp., Parthenolecanium spp. e.g. P. corni, P. persicae; Pemphigus spp. e.g. P. bursarius, P. populivenae; Peregrinus maidis, Perkinsiella saccharicida, Phenacoccus spp. e.g. P. aceris, P. gossypii; Phloeomyzus passerinii, Phorodon humuli, Phylloxera spp. e.g. P. devastatrix, Piesma quadrata, Piezodorus spp. e.g. P. guildinii; Pinnaspis aspidistrae, Planococcus spp. e.g. P. citri, P. ficus; Prosapia bicincta, Protopulvinaria pyriformis, Psallus seriatus, Pseudacysta persea, Pseudaulacaspis pentagona, Pseudococcus spp. e.g. P. comstocki; Psylla spp. e.g. P. mali; Pteromalus spp., Pulvinaria amygdali, Pyrilla spp., Quadraspidiotus spp., e.g. Q. perniciosus; Quesada gigas, Rastrococcus spp., Reduvius senilis, Rhizoecus americanus, Rhodnius spp., Rhopalomyzus ascalonicus, Rhopalosiphum spp. e.g. R. pseudobrassicas, R. insertum, R. maidis, R. padi; Sagatodes spp., Sahlbergella singularis, Saissetia spp., Sappaphis mala, Sappaphis mali, Scaptocoris spp., Scaphoideus titanus, Schizaphis graminum, Schizoneura lanuginosa, Scotinophora spp., Selenaspidus articulatus, Sitobion avenae, Sogata spp., Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta spp. e.g. T. accerra, T. perditor; Tibraca spp., Tomaspis spp., Toxoptera spp. e.g. T. aurantii; Trialeurodes spp. e.g. T. abutilonea, T. ricini, T. vaporariorum; Triatoma spp., Trioza spp., Typhlocyba spp., Unaspis spp. e.g. U. citri, U. yanonensis; and Viteus vitifolii,
Insects from the order Hymenoptera e.g. Acanthomyops interjectus, Athalia rosae, Atta spp. e.g. A. capiguara, A. cephalotes, A. cephalotes, A. laevigata, A. robusta, A. sexdens, A. texana, Bombus spp., Brachymyrmex spp., Camponotus spp. e.g. C. floridanus, C. pennsylvanicus, C. modoc; Cardiocondyla nuda, Chalibion sp, Crematogaster spp., Dasymutilla occidentalis, Diprion spp., Dolichovespula maculata, Dorymyrmex spp., Dryocosmus kuriphilus, Formica spp., Hoplocampa spp. e.g. H. minuta, H. testudinea; Iridomyrmex humilis, Lasius spp. e.g. L. niger, Linepithema humile, Liometopum spp., Leptocybe invasa, Monomorium spp. e.g. M. pharaonis, Monomorium, Nylandria fulva, Pachycondyla chinensis, Paratrechina longicornis, Paravespula spp., e.g. P. germanica, P. pennsylvanica, P. vulgaris; Pheidole spp. e.g. P. megacephala; Pogonomyrmex spp. e.g. P. barbatus, P. californicus, Polistes rubiginosa, Prenolepis impairs, Pseudomyrmex gracilis, Schelipron spp., Sirex cyaneus, Solenopsis spp. e.g. S. geminata, S.invicta, S. molesta, S. richteri, S. xyloni, Sphecius speciosus, Sphex spp., Tapinoma spp. e.g. T. melanocephalum, T. sessile; Tetramorium spp., e.g. T. caespitum, T. bicarinatum, Vespa spp., e.g. V. crabro; Vespula spp., e.g. V. squamosal; Wasmannia auropunctata, Xylocopa sp;
Insects from the order Orthoptera e.g. Acheta domesticus, Calliptamus italicus, Chortoicetes terminifera, Ceuthophilus spp., Diastrammena asynamora, Dociostaurus maroccanus, Gryllotalpa spp. e.g. G. africana, G. gryllotalpa; Gryllus spp., Hieroglyphus daganensis, Kraussaria angulifera, Locusta spp. e.g. L. migratoria, L. pardalina; Melanoplus spp. e.g. M. bivittatus, M. femurrubrum, M. mexicanus, M. sanguinipes, M. spretus; Nomadacris septemfasciata, Oedaleus senegalensis, Scapteriscus spp., Schistocerca spp. e.g. S. americana, S. gregaria, Stemopelmatus spp., Tachycines asynamorus, and Zonozerus variegatus;
Pests from the Class Arachnida e.g. Acari,e.g. of the families Argasidae, Ixodidae and Sarcoptidae, e.g. Amblyomma spp. (e.g. A. americanum, A. variegatum, A. maculatum), Argas spp. e.g. A. persicu), Boophilus spp. e.g. B. annulatus, B. decoloratus, B. microplus, Dermacentor spp. e.g. D.silvarum, D. andersoni, D. variabilis, Hyalomma spp. e.g. H. truncatum, Ixodes spp. e.g. I. ricinus, I. rubicundus, I. scapularis, I. holocyclus, I. pacificus, Rhipicephalus sanguineus, Ornithodorus spp. e.g. O. moubata, O. hermsi, O. turicata, Ornithonyssus bacoti, Otobius megnini, Dermanyssus gallinae, Psoroptes spp. e.g. P. ovis, Rhipicephalus spp. e.g. R. sanguineus, R. appendiculatus, Rhipicephalus evertsi, Rhizoglyphus spp., Sarcoptes spp. e.g. S. Scabiei; and Family Eriophyidae including Aceria spp. e.g. A. sheldoni, A. anthocoptes, Acallitus spp., Aculops spp. e.g. A. lycopersici, A. pelekassi; Aculus spp. e.g. A. schlechtendali; Colomerus vitis, Epitrimerus pyri, Phyllocoptruta oleivora; Eriophytes ribis and Eriophyes spp. e.g. Eriophyes sheldoni; Family Tarsonemidae including Hemitarsonemus spp., Phytonemus pallidus and Polyphagotarsonemus latus, Stenotarsonemus spp. Steneotarsonemus spinki; Family Tenuipalpidae including Brevipalpus spp. e.g. B. phoenicis; Family Tetranychidae including Eotetranychus spp., Eutetranychus spp., Oligonychus spp., Petrobia latens, Tetranychus spp. e.g. T. cinnabarinus, T. evansi, T. kanzawai, T, pacificus, T. phaseulus, T. telarius and T. urticae; Bryobia praetiosa; Panonychus spp. e.g. P. ulmi, P. citri; Metatetranychus spp. and Oligonychus spp. e.g. O. pratensis, O. perseae, Vasates lycopersici; Raoiella indica, Family Carpoglyphidae including Carpoglyphus spp.; Penthaleidae spp. e.g. Halotydeus destructor; Family Demodicidae with species e.g. Demodex spp.; Family Trombicidea including Trombicula spp.; Family Macronyssidae including Ornothonyssus spp.; Family Pyemotidae including Pyemotes tritici; Tyrophagus putrescentiae; Family Acaridae includ-ing Acarus siro; Family Araneida including Latrodectus mactans, Eratigena agrestis, Cheiracanthium sp, Lycosa sp Achaearanea tepidariorum and Loxosceles reclusa;
Pests from the Phylum Nematoda, e.g. plant parasitic nematodes e.g. root-knot nematodes, Meloidogyne spp. e.g. M. hapla, M. incognita, M. javanica; cyst-forming nematodes, Globodera spp. e.g. G. rostochiensis; Heterodera spp. e.g. H. avenae, H. glycines, H. schachtii, H. trifolii; Seed gall nematodes, Anguina spp.; Stem and foliar nematodes, Aphelenchoides spp. e.g. A. besseyi; Sting nematodes, Belonolaimus spp. e.g. B. longicaudatus; Pine nematodes, Bursaphelenchus spp. e.g. B. lignicolus, B. xylophilus; Ring nematodes, Criconema spp., Criconemella spp. e.g. C. xenoplax and C. ornata; and, Criconemoides spp. e.g. Criconemoides informis; Mesocriconema spp.; Stem and bulb nematodes, Ditylenchus spp. e.g. D. destructor, D. dipsaci; Awl nematodes, Dolichodorus spp.; Spiral nematodes, Heliocotylenchus multicinctus; Sheath and sheathoid nematodes, Hemicycliophora spp. and Hemicriconemoides spp.; Hirshmanniella spp.; Lance nematodes, Hoploaimus spp.; False rootknot nematodes, Nacobbus spp.; Needle nematodes, Longidorus spp. e.g. L. elongatus; Lesion nematodes, Pratylenchus spp. e.g. P. brachyurus, P. neglectus, P. penetrans, P. curvitatus, P. goodeyi; Burrowing nema-todes, Radopholus spp. e.g. R. similis; Rhadopholus spp.; Rhodopholus spp.; Reniform nematodes, Rotylenchus spp. e.g. R. robustus, R. reniformis; Scutellonema spp.; Stubby-root nematode, Trichodorus spp. e.g. T. obtusus, T. primitivus; Paratrichodorus spp. e.g. P. minor; Stunt nematodes, Tylenchorhynchus spp. e.g. T. claytoni, T. dubius; Citrus nematodes, Tylenchulus spp. e.g. T. semipenetrans; Dagger nematodes, Xiphinema spp.; and other plant parasitic nematode species;
Insects from the order Blattodea e.g. Macrotermes spp. e.g. M. natalensis; Cornitermes cumulans, Procornitermes spp., Globitermes sulfureus, Neocapritermes spp. e.g. N. opacus, N. parvus; Odontotermes spp., Nasutitermes spp. e.g. N. corniger; Coptotermes spp. e.g. C. formosanus, C. gestroi, C. acinaciformis; Reticulitermes spp. e.g. R. hesperus, R. tibialis, R. speratus, R. flavipes, R. grassei, R. lucifugus, R. virginicus; Heterotermes spp. e.g. H. aureus, H. longiceps, H. tenuis; Cryptotermes spp. e.g. C. brevis, C. cavifrons; Incisitermes spp. e.g. I. minor, I. snyderi; Marginitermes hubbardi, Kalotermes flavicollis, Neotermes spp. e.g. N. castaneus, Zootermopsis spp. e.g. Z. angusticollis, Z. nevadensis, Mastotermes spp. e.g. M. darwiniensis; Blatta spp. e.g. B. orientalis, B. lateralis; Blattella spp. e.g. B. asahinae, B. germanica; Rhyparobia maderae, Panchlora nivea, Periplaneta spp. e.g. P. americana, P. australasiae, P. brunnea, P. fuliginosa, P. japonica; Supella longipalpa, Parcoblatta pennsylvanica, Eurycotis floridana, Pycnoscelus surinamensis,
Insects from the order Siphonoptera e.g. Cediopsylla simples, Ceratophyllus spp., Ctenocephalides spp. e.g. C. felis, C. canis, Xenopsylla cheopis, Pulex irritans, Trichodectes canis, Tunga penetrans, and Nosopsyllus fasciatus,
Insects from the order Thysanura e.g. Lepisma saccharina, Ctenolepisma urbana, and Thermobia domestica,
Pests from the class Chilopoda e.g. Geophilus spp., Scutigera spp. e.g. Scutigera coleoptrata;
Pests from the class Diplopoda e.g. Blaniulus guttulatus, Julus spp., Narceus spp.,
Pests from the class Symphyla e.g. Scutigerella immaculata,
Insects from the order Dermaptera, e.g. Forficula auricularia,
Insects from the order Collembola, e.g. Onychiurus spp., e.g. Onychiurus armatus,
Pests from the order Isopoda, e.g. Armadillidium vulgare, Oniscus asellus, Porcellio scaber, Insects from the order Phthiraptera, e.g. Damalinia spp., Pediculus spp. e.g. Pediculus humanus capitis, Pediculus humanus corporis, Pediculus humanus humanus; Pthirus pubis, Haematopinus spp. e.g. Haematopinus eurysternus, Haematopinus suis; Linognathus spp. e.g. Linognathus vituli; Bovicola bovis, Menopon gallinae, Menacanthus stramineus and Solenopotes capillatus, Trichodectes spp.,
Further pest species which may be controlled by compounds I include: from the Phylum Mollusca, class Bivalvia, e.g., Dreissena spp.; class Gastropoda, e.g., Arion spp., Biomphalaria spp., Bulinus spp., Deroceras spp., Galba spp., Lymnaea spp., Oncomelania spp., Pomacea canaliclata, Succinea spp.; from the class of the helminths, e.g., Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp. e.g. Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuel-leborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

The compounds of the invention are particularly suitable for efficiently combating
insects from the sub-order of Auchenorrhyncha, e.g. Amrasca biguttula, Empoasca spp., Nephotettix virescens, Sogatella furcifera, Mahanarva spp., Laodelphax striatellus, Nilaparvata lugens, Diaphorina citri, Lycorma delicatula, Pentastiridus leporinus;
Lepidoptera, e.g. Helicoverpa spp., Heliothis virescens, Lobesia botrana, Ostrinia nubilalis, Plutella xylostella, Pseudoplusia includens, Scirpophaga incertulas, Spodoptera spp., Trichoplusia ni, Tuta absoluta, Cnaphalocrocis medialis, Cydia pomonella, Chilo suppressalis, Anticarsia gemmatalis, Agrotis ipsilon, Chrysodeixis includens;
True bugs, e.g. Lygus spp., Stink bugs such as Euschistus spp., Halyomorpha halys, Nezara viridula, Piezodorus guildinii, Dichelops furcatus;
Thrips, e.g. Frankliniella spp., Thrips spp., Dichromothrips corbettii;
Aphids, e.g. Acyrthosiphon pisum, Aphis spp., Myzus persicae, Rhopalosiphum spp., Schizaphis graminum, Megoura viciae;
Whiteflies, e.g. Trialeurodes vaporariorum, Bemisia spp.;
Coleoptera, e.g. Phyllotreta spp., Melanotus spp., Meligethes aeneus, Leptinotarsa decimlineata, Ceutorhynchus spp., Diabrotica spp., Anthonomus grandis, Atomaria linearia, Agriotes spp., Epilachna spp.;
Flies, e.g. Delia spp., Ceratitis capitate, Bactrocera spp., Liriomyza spp.;
Coccoidea, e.g. Aonidiella aurantia, Ferrisia virgate;
Anthropods of class Arachnida (Mites), e.g. Penthaleus major, Tetranychus spp.;
Nematodes, e.g. Heterodera glycines, Meloidogyne sp., Pratylenchus spp., Caenorhabditis elegans.

### Animal health

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or con-trolling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics e.g. Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at its locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
fleas (Siphonaptera), e.g. Ctenocephalides felis, C. canis, Xenopsylla cheopis, Pulex irritans, Tunga penetrans, and Nosopsyllus fasciatus; cockroaches (Blattaria - Blattodea), e.g. Blattella germanica, B. asahinae, Periplaneta americana, P. japonica, P. brunnea, P. fuligginosa, P. australasiae, and Blatta orientalis; flies, mosquitoes (Diptera), e.g. Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus, Calliphora vicina, Chrysomya bezziana, C. hominivorax, C. macellaria, Chrysops discalis, C. silacea, C. atlanticus, Cochliomyia hominivorax, Cordylobia anthropophaga, Culicoides furens, Culex pipiens, C. nigripalpus, C. quinquefasciatus, C. tarsalis, Culiseta inornata, C. melanura, Dermatobia hominis, Fannia canicularis, Gasterophilus intestinalis, Glossina morsitans, G. palpalis, G. fuscipes, G. tachinoides, Haematobia irritans, Haplodiplosis equestris, Hippelates spp., Hypoderma line-ata, Leptoconops torrens, Lucilia caprina, L. cuprina, L. sericata, Lycoria pectoralis, Mansonia spp., Musca domestica, M. stabulans, Oestrus ovis, Phlebotomus argentipes, Psorophora columbiae, P. discolor, Prosimulium mixtum, Sarcophaga spp., S. haemorrhoidalis, Simulium vittatum, Stomoxys calcitrans, Tabanus bovinus, T. atratus, T. lineola, and T. similis; lice (Phthiraptera), e.g. Pediculus humanus capitis, P. humanus humanus, Pthirus pubis, Haematopinus eurysternus, H. suis, Linognathus vituli, Bovicola bovis, Menopon gallinae, Menacanthus stramineus, and Solenopotes capillatus; ticks and parasitic mites (Parasitiformes): ticks (Ixodida), e.g. Ixodes scapularis, I. holocyclus, I. pacificus, Rhiphicephalus sanguineus, Dermacentor andersoni, D. variabilis, Amblyomma americanum, A. maculatum, Ornithodorus hermsi, O. turicata and parasitic mites (Mesostigmata), e.g. Ornithonyssus bacoti, Dermanyssus gallinae; Actinedida (Prostigmata) and Acaridida (Astigmata), e.g. Acarapis spp., Cheyletiella spp., Ornithocheyletia spp., Myobia spp., Psorergates spp., Demo-dex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., and Laminosioptes spp; Bugs (Het-eropterida): Cimex lectularius, C. hemipterus, Reduvius senilis, Triatoma spp., Rhodnius ssp., Panstrongylus ssp., and Arilus critatus; Anoplurida, e.g. Haematopinus spp., Linognathus spp., Pediculus spp., Phtirus spp., and Solenopotes spp.; Mallophagida (suborders Arnblycerina and Ischnocerina), e.g. Trimenopon spp., Menopon spp., Trinoton spp., Bovicola spp., Werneckiella spp., Lepikentron spp., Trichodectes spp., and Felicola spp.; Roundworms Nematoda: Wipeworms and Trichinosis (Trichosyringida), e.g. Trichinellidae (Trichinella spp.), (Trichuridae) Trichuris spp., Capillaria spp.; Rhabditida, e.g. Rhabditis spp., Strongyloides spp., Helicephalobus spp.; Strongylida, e.g. Strongylus spp., Ancylostoma spp., Necator americanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus, and Dioctophyma renale; Intestinal roundworms (Ascaridida), e.g. Ascaris lumbricoides, Ascaris suum, Ascaridia galli, Parascaris equorum, Enterobius vermicularis (Threadworm), Toxocara canis, Toxascaris leonine, Skrjabinema spp., and Oxyuris equi; Camallanida, e.g. Dracunculus medinensis (guinea worm); Spirurida, e.g. Thelazia spp., Wuchereria spp., Brugia spp., Onchocerca spp., Dirofilari spp.a, Dipetalonema spp., Setaria spp., Elaeophora spp., Spirocerca lupi, and Habronema spp.; Thorny headed worms (Acanthocephala), e.g. Acanthocephalus spp., Macracanthorhynchus hirudinaceus and Oncicola spp.; Planarians (Plathelminthes): Flukes (Trematoda), e.g. Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp., and Nanocyetes spp.; Cercomeromorpha, in particular Cestoda (Tapeworms), e.g. Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp., and Hymenolepis spp..

The term "animal" includes warm-blooded animals (including humans) and fish. Preferred are mammals, e.g. cattle, sheep, swine, camels, deer, horses, pigs, poultry, rabbits, goats, dogs and cats, water buffalo, donkeys, fallow deer and reindeer, and also in fur-bearing animals e.g. mink, chinchilla and raccoon, birds e.g. hens, geese, turkeys and ducks and fish e.g. fresh- and salt-water fish e.g. trout, carp and eels. Particularly preferred are domestic animals, e.g. dogs or cats.

Generally, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions e.g. desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. In addition, the compounds I may be ad-ministered to the animals in their drinking water. For oral administration, the dosage form chosen should provide the animal with 0.01 mg/kg to 100 mg/kg of animal body weight per day of the compounds I, preferably with 0.5 mg/kg to 100 mg/kg of animal body weight per day.

Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Suitable preparations are:
- Solutions e.g. oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations e.g. powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries e.g. acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dis-solving, suspending or emulsifying the active compound in suitable skin-compatible solvents or solvent mixtures. If appropriate, other auxiliaries e.g. colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries e.g. colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries e.g. wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Topical application may be conducted with compound-containing shaped articles e.g. collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### A. Preparation Examples

The compounds were characterized by melting point determination, by NMR spectroscopy or by the mass-to-charge ratio ([m/z]) and retention time (RT; [min.]), as determined by mass spectrometry (MS) coupled with HPLC analysis (HPLC-MS = high performance liquid chromatography-coupled mass spectrometry),LC analysis (LC-MS = liquid chromatography-coupled mass spectrometry) or chiral SFC (SFC = supercritical fluid chromatography).

Method D: Column: X-Bridge C18, 5u (4.6mmX100mm) Mobile Phase A: 10 mM Ammonium Bicarbonate in Water, B: 100% ACN, Gradient (T%B): 0/10, 8/95, 11/95, 13/10,15/10, Flow Rate: 1.0 ml/min Column Temp: 40°C, Sample Diluent: ACN+WATER

Method E: Acquity UPLC BEH C18, 1.7µm (50mmX2.1mm), Mobile Phase A: 0.1% Formic Acid in Water, B: 0.1% Formic Acid in ACN, Gradient (T%B): 0/3, 1.0/3, 7.0/95, 7.5/95, 9.0/3, 10.0/3, Flow Rate: 0.5ml/min, Column Temp: 40°C Mass range (m/z): 100-700

Method F: Shimadzu Nexera UHPLC + Shimadzu LCMS 20-20,ESI, Column: C-18, 50 mm , 4.6 mm, 5 micron, Mobile Phase: A: 10mM ammonium Formate + B:ACN, Column Oven Temperature: 40°C, Gradient: 10% B to 100% B in 1,50min; 100% B 1.0 min, Flow: 1.2 ml/min, MS method: ESI Positive - Negative (Dual lonisation), mass range (m/z): 100-700

Method G: Analytical SFC Condition: Column/dimensions:DCPAK P4VP (4.6x250)mm, 5µ % CO₂ :70%, % Co solvent :30% (0.5% DEA in MeOH), Flow: 3mL/min Back Pressure:1500 psi;Temperature: 30°C

### Example 1: Synthesis of 2-((6-(1-(4-chlorobenzoyl) piperidin-4-yl) pyridin-3-yl) methylene)-N-(2-isopropyl-5-methylphenyl) hydrazine-1-carbothioamide (compound I-2)

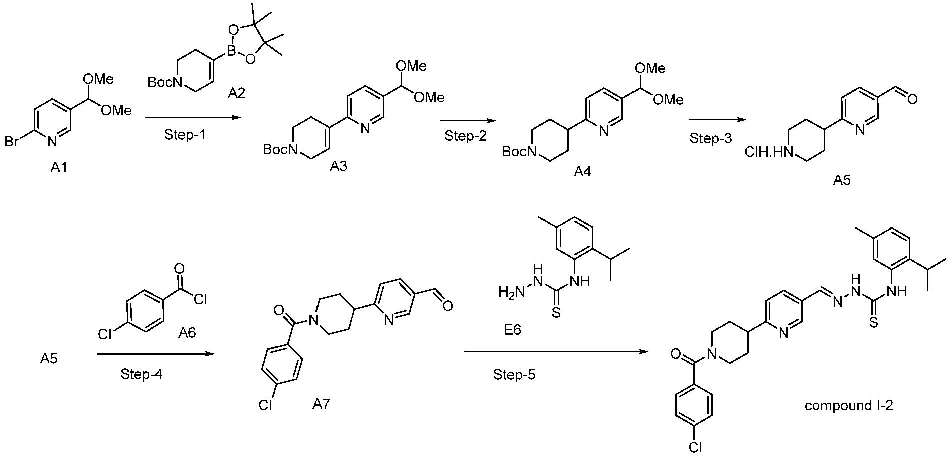

### Step 1: Synthesis of tert-butyl 4-[5-(dimethoxymethyl)-2-pyridyl]-3,6-dihydro-2H-pyridine-1-carboxylate (A3):

To a stirred solution of 2-bromo-5-(dimethoxymethyl)pyridine (A1) (4.8 g, 20.683 mmol, 1 equiv.), tert-butyl 4-(4,4,5,5-tetramethyl-1,3,2-dioxaborolan-2-yl)-3,6-dihydropyridine-1(2H)-carboxylate (A2) (7.035 g, 22.751 mmol, 1.1 equiv.) and sodium carbonate (5.480 g, 51.707 mmol, 2.5 equiv.) in mixture of 1,4-dioxane (38.400 mL) and water (9.600 mL), was degassed with nitrogen for 5 min. After that, Pd(PPh₃)₄ (1.195 g, 1.034 mmol, 0.05 equiv.) was added and the resulting mixture was again degassed with N₂ for 5 minutes and stirred at 100 °C for 16 h. After completion of the reaction, the reaction mass was quenched with water (100 mL) and extracted with ethyl acetate (3 × 100 mL). The combined organic layer was washed with brine (100 mL), dried over sodium sulphate, filtered and concentrated under reduced pressure to get the crude product. The crude product was purified by silica gel (100-200 mesh) column chromatography using 50% ethyl acetate in pet ether as an eluent to afford tert-butyl 5-(dimethoxymethyl)-3',6'-dihydro-[2,4'-bipyridine]-1'(2'H)-carboxylate (A3) (4 g, 57%).
LC-MS (m/z): 335.32 [M+H] + ion present

### Step 2: Synthesis of tert-butyl 4-(5-(dimethoxy methyl) pyridin-2-yl) piperidine-1-carboxylate (A4):

To a stirred solution of tert-butyl 5-(dimethoxymethyl)-3',6'-dihydro-[2,4'-bipyridine]-1'(2'H)-carboxylate (A3) (4 g, 11.961 mmol, 1 equiv.) in ethyl acetate (80 mL) was added 10% Pd/C (1.2 g, 0.3 w/w) under H₂ (60 Psi) atmosphere at room temperature and continued the stirring for 16 h (the progress of the reaction was monitored by TLC). After completion of the reaction, the reaction mass was diluted with ethyl acetate (100 mL), filtered through celite bed and the filtrate was concentrated under reduced pressure to afford tert-butyl 4-(5-(dimethoxy methyl) pyridin-2-yl) piperidine-1-carboxylate (A4) (4 g crude). The crude product was used in the next step without further purification.
LC-MS (m/z): 337.33 [M+H] + ion present

### Step 3: Synthesis of 6-(piperidin-4-yl)nicotinaldehyde hydrochloride (A5):

To a stirred solution of tert-butyl 4-(5-(dimethoxy methyl) pyridin-2-yl) piperidine-1-carboxylate (A4) (4 g, 11.889 mmol, 1 equiv.) in DCM (40 mL) was added 4M HCl in 1,4-dioxane (40 mL) dropwise at room temperature. The resulting mixture was stirred for 4 hours under same condition. After completion of the reaction, reaction mass was concentrated under reduced pressure to get 6-(piperidin-4-yl)nicotinaldehyde hydrochloride (A5) (3 g crude). The crude product was used in the next step without further purification.
LC-MS (m/z): 191.10 [M+H] + ion present

### Step 4: Synthesis of 6-(1-(4-chlorobenzoyl) piperidin-4-yl) nicotinaldehyde (A7):

To a stirred solution of 6-(piperidin-4-yl)nicotinaldehyde hydrochloride (A5) (3 g, 13.233 mmol, 1 equiv.) and DIPEA (9.245 mL, 52.932 mmol, 4 equiv.) in THF (60 mL) was added 4-chlorobenzoyl chloride (A6) (2.316 g, 13.233 mmol, 1 equiv.) at room temperature. The resulting mixture was stirred for 16 h under same condition. After completion of the reaction, the reaction mass was quenched with water (100 mL) and extracted with ethyl acetate (3 x 100 mL). The combined organic layer was washed with brine (50 mL), dried over sodium sulphate, filtered and concentrated under reduced pressure to get the crude product. The crude product was purified by silica gel (100-200 mesh) column chromatography using 60% ethyl acetate in pet ether as an eluent to afford 6-(1-(4-chlorobenzoyl) piperidin-4-yl) nicotinaldehyde (A7) (2 g, 46%).
LC-MS (m/z): 329.25 [M+H] + ion present

### Step 5: Synthesis of 2-((6-(1-(4-chlorobenzoyl) piperidin-4-yl) pyridin-3-yl) methylene)-N-(2-isopropyl-5-methylphenyl) hydrazine-1-carbothioamide (compound I-2):

To a stirred solution of 6-(1-(4-chlorobenzoyl)piperidin-4-yl)nicotinaldehyde (A7) (2 g, 6.083 mmol, 1 equiv.) and N-(2-isopropyl-5-methylphenyl)hydrazinecarbothioamide (E6) (1.359 g, 6.083 mmol, 1 equiv.) in methanol (20 mL) was added acetic acid (1.0 mL, 0.5 V) at room temperature. The resulting mixture was stirred for 16 h under same condition (progress of the reaction was monitored TLC and LCMS). After completion of the reaction, the precipitated solid was filtered and dried to afford 2-((6-(1-(4-chlorobenzoyl) piperidin-4-yl) pyridin-3-yl) methylene)-N-(2-isopropyl-5-methylphenyl) hydrazine-1-carbothioamide (1.7 g) as a white solid. 0.8 g (HPLC purity: 89%) of compound was purified by reverse phase preparative HPLC and lyophilized to afford 2-((6-(1-(4-chlorobenzoyl) piperidin-4-yl) pyridin-3-yl) methylene)-N-(2-isopropyl-5-methylphenyl) hydrazine-1-carbothioamide (compound I-2) (0.54 g).
LC-MS (m/z): 534.32 [M+H] + ion present;
¹H NMR (400 MHz, DMSO-*d*₆): δ 11.86 (s, 1H), 9.98 (s, 1H), 8.85 (d, *J =* 1.6 Hz, 1H), 8.36 (dd, *J* = 8.4 & 2.0 Hz, 1H), 8.13 (s, 1H), 7.52 (d, *J* = 8.4 Hz, 2H), 7.45 (d, *J* = 8.8 Hz, 2H), 7.38 (d, *J =* 8.4 Hz, 1H), 7.23 (d, *J* = 8.0 Hz, 1H), 7.11 (d, *J* = 7.6 Hz, 1H), 7.00 (s, 1H), 4.70-4.50 (m, 1H), 3.81-3.50 (m, 1H), 3.25-3.10 (m, 1H), 3.09-3.01 (m, 2H), 2.98-2.80 (s, 1H), 2.28 (s, 3H), 1.98 -1.62 (m, 4H), 1.15 (d, *J* = 6.8 Hz, 6H).

### Example 2: Synthesis of compound (E/Z)-2-(((E)-(6-(1-(4-chlorobenzoyl)piperidin-4-yl)pyridin-3-yl)methylene)hydrazineylidene)-3-(2-isopropyl-5-methyl phenyl)thiazolidin-4-one (compound I-1)

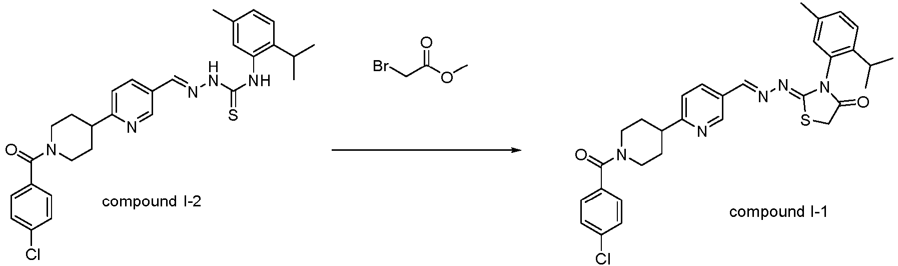

To a stirred solution of (E)-2-((6-(1-(4-chlorobenzoyl)piperidin-4-yl)pyridin-3-yl)methylene)-N-(2-isopropyl-5-methylphenyl)hydrazine-1-carbothioamide (compound I-2) (0.8 g, 1.498 mmol, 1 equiv.) and sodium acetate (0.491 g, 5.991 mmol, 4 equiv.) in ethanol (16.0 mL) was added methyl 2-bromoacetate (0.344 g, 2.247 mmol, 1.5 equiv.) at rt. The resulting mixture was stirred at 70 °C for 16 hours. After completion of the reaction, the reaction mass was diluted with water (100 mL) and extracted with ethyl acetate (2 X 100 mL). The combined organic layer was washed with brine (50 mL), dried over sodium sulfate, filtered and concentrated under reduced pressure to get the crude compound. The crude compound was purified by silica gel (100-200 mesh) column chromatography using 60% ethyl acetate in pet ether as an eluent to afford (E/Z)-2-(((E)-(6-(1-(4-chlorobenzoyl)piperidin-4-yl)pyridin-3-yl)methylene)hydrazineylidene)-3-(2-isopropyl-5-methyl phenyl)thiazolidin-4-one (compound I-1) (0.62 g, 72%).
LC-MS (m/z): 574.30 [M+H]⁺ ion present;
¹H-NMR (400 MHz, DMSO-*d*₆): δ 8.77 (d, *J =* 1.6 Hz, 1H), 8.36 (s, 1H), 8.04-8.06 (dd, *J =* 8.4 & 2.4 Hz, 1H), 7.52 (d, *J* = 8.4 Hz, 2H), 7.46-7.37 (m, 4H), 7.27 (d, *J* = 8.4 Hz, 1H), 7.06 (s, 1H), 4.62-4.54 (m, 1H), 4.24 (d, *J =* 17.2 Hz, 1H), 4.12 (d, *J =* 17.2 Hz, 1H), 3.66-3.58 (m, 1H), 3.22-3.14 (m, 1H), 3.07-3.01 (m, 1H), 2.96-2.84 (m, 1H), 2.76-2.71 (m, 1H), 2.31 (s, 3H), 1.93-1.68 (m, 4H), 1.13-1.09 (m, 6H).

### Example 3: Synthesis of 1-(4-(4-(4-chlorobenzoyl)-2-oxopiperazin-1-yl)phenyl)-3-(3-(2-isopropyl-5-methylphenyl)-4-oxothiazolidin-2-ylidene)urea (compound I-3)

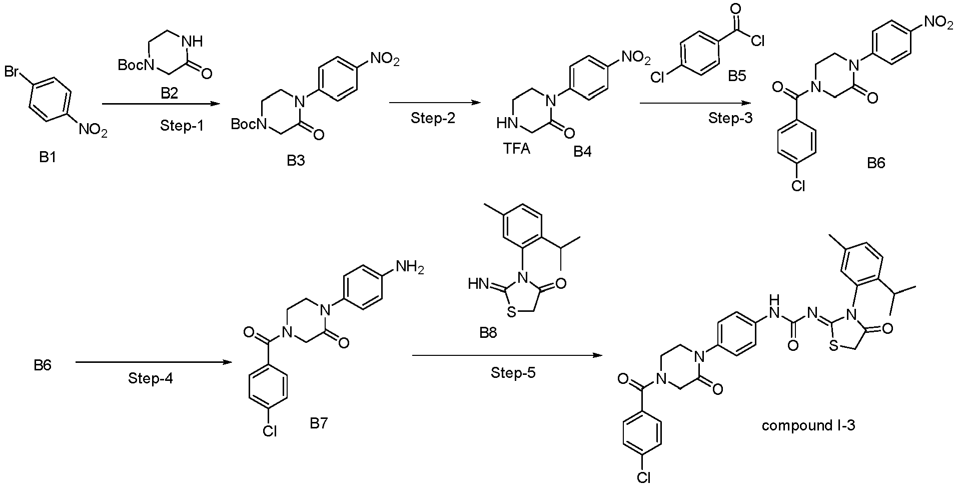

### Step-1: Synthesis of tert-butyl 4-(4-nitrophenyl)-3-oxopiperazine-1-carboxylate (B3):

A solution of 1-bromo-4-nitrobenzene (B1) (5 g, 24.751 mmol) and tert-butyl 3-oxopiperazine-1-carboxylate (B2) (5.947 g, 29.702 mmol) and cesium carbonate (16.129 g, 49.502 mmol) in 1,4-dioxane was degassed with nitrogen for 10 minutes followed by addition of palladium (II) acetate (1.667 g, 2.475 mmol) and xantphos (1.432 g, 2.475 mmol). The resulting mixture was degassed again for 5 min. The resulting mixture heated at 80 °C for 6 h. After completion, water (200 mL) was added and extracted with ethyl acetate (3 x 200 mL). The combined organic layers and washed with brine (200 mL), dried over anhydrous sodium sulfate, filtered and concentrated under reduced pressure. The crude product was adsorbed on a plug of silica gel and purified by flash column chromatography using 50-70% ethyl acetate in pet ether as an eluent to afford tert-butyl 4-(4-nitrophenyl)-3-oxopiperazine-1-carboxylate (B3) (4 g, 50%).
LC-MS (m/z): 322.28 [M+H] ⁺ ion present

### Step-2: Synthesis of 1-(4-nitrophenyl)piperazin-2-one trifluoroacetic acid salt (B4):

To a stirred solution of tert-butyl 4-(4-nitrophenyl)-3-oxopiperazine-1-carboxylate (B3) (4 g, 12.448 mmol) in dichloromethane (40 mL) was added trifluoroacetic acid (14.193 g, 124.481 mmol) at RT. The resulting mixture was stirred at RT for 3 h. After completion of the reaction, concentrated the reaction mixture under reduced pressure. The crude product thus obtained was triturated in diethyl ether (2 x 5 mL) to afford 1-(4-nitrophenyl)piperazin-2-one trifluoroacetic acid salt (B4) (2.5 g).
LC-MS (m/z): 222.12 [M-TFA+H] + ion present

### Step-3: Synthesis of 4-(4-chlorobenzoyl)-1-(4-nitrophenyl)piperazin-2-one (B6):

To a stirred solution of 1-(4-nitrophenyl)piperazin-2-one trifluoroacetic acid salt (B4) (2.5 g, 7.831 mmol) and triethylamine (1.189 g, 11.447 mmol) in THF (25 mL) was added 4-chlorobenzoyl chloride (B5) (1.371 g, 7.831 mmol) dropwise at 0 °C. The resulting mixture was stirred at RT for 2 h. After completion, the reaction mass was quenched with water (100 mL) and extracted with ethyl acetate (3 x 100 mL). The combined organic layer was washed with brine (100 mL), dried over sodium sulfate, filtered and concentrated under reduced pressure. The crude product thus obtained was triturated in diethyl ether (2 x 25 mL) to afford 4-(4-chlorobenzoyl)-1-(4-nitrophenyl)piperazin-2-one (B6) (3 g).
LC-MS (m/z): 360.20 [M+H] ⁺ ion present

### Step-4: Synthesis of 1-(4-aminophenyl)-4-(4-chlorobenzoyl)piperazin-2-one (B7):

To a stirred solution of 4-(4-chlorobenzoyl)-1-(4-nitrophenyl)piperazin-2-one (B6) (3 g, 8.339 mmol) in mixture of THF: water (30 mL, 10V, 7:3 ratio) was added sodium dithionite (7.259 g, 41.694 mmol) at RT. The resulting mixture was stirred at 55 °C for 3 h. After completion, the reaction mass was diluted with water (100 mL) and extracted with ethyl acetate (2 x 100 mL). The combined organic layer was washed with brine (100 mL), dried over sodium sulfate, filtered and concentrated under reduced pressure. The crude product thus obtained was triturated in diethyl ether (2 x 10 mL) to afford 1-(4-aminophenyl)-4-(4-chlorobenzoyl)piperazin-2-one (B7) (1.2 g, 44%). LC-MS (m/z): 330.23 [M+H] ⁺ ion present

### Step-5: Synthesis of 1-(4-(4-(4-chlorobenzoyl)-2-oxopiperazin-1-yl)phenyl)-3-(3-(2-isopropyl-5-methylphenyl)-4-oxothiazolidin-2-ylidene)urea (compound I-3):

To a stirred solution of 1-(4-aminophenyl)-4-(4-chlorobenzoyl) piperazin-2-one (7) (1.2 g, 3.639 mmol) and N,N'-disuccinimidyl carbonate (1.119 g, 4.366 mmol) in acetonitrile (12 mL) was added N,N-diisopropylethylamine (0.941 g, 7.277 mmol) at rt. The resulting reaction mixture stirred for 30 min at room temperature. After that, 2-imino-3-(2-isopropyl-5-methylphenyl) thiazolidin-4-one (B8) (0.904 g, 3.639 mmol) was added at room temperature and continue the reaction at same temperature for 4 h. After completion, the reaction mass was quenched with water (100 mL) and extracted with ethyl acetate (3 x 100 mL). The combined organic layer was washed with brine (50 mL), dried over sodium sulfate, filtered and concentrated under reduced pressure. The crude product was purified by a SFC purification to afford 1-(4-(4-(4-chlorobenzoyl)-2-oxopiperazin-1-yl)phenyl)-3-(3-(2-isopropyl-5-methylphenyl)-4-oxothiazolidin-2-ylidene)urea (compound I-3) (0.312 g, 14%). LC-MS (m/z): 604.33 [M+H] ⁺ ion present
¹H NMR (400 MHz, DMSO-*d*₆): δ 9.82 (s, 1H), 7.64 (d, *J* = 8.8 Hz, 2H), 7.60-7.52 (m, 4H), 7.39 (d, *J =* 8.0 Hz, 1H), 7.27-7.22 (m, 3H), 7.05 (s, 1H), 4.28-4.04 (m, 4H), 3.93-3.70 (m, 4H), 2.69-2.62 (m, 1H), 2.31 (s, 3H), 1.15 (d, *J =* 6.8 Hz, 3H), 1.08 (d, *J =* 6.8 Hz, 3H).

### Example 4: Synthesis of (E/Z)-1-(4-(1-(4-chlorobenzoyl)-1,2,3,6-tetrahydropyridin-4-yl)-2-fluorophenyl)-3-(3-(2-isopropyl-5-methylphenyl)-4-oxothiazolidin-2-ylidene)urea (compound I-15)

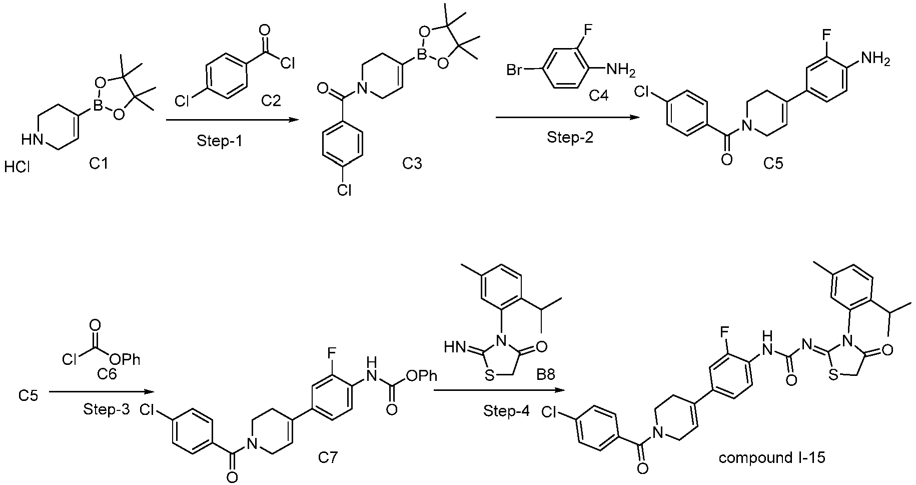

### Step 1: Synthesis of 3

To a stirred solution of 4-(4,4,5,5-tetramethyl-1,3,2-dioxaborolan-2-yl)-1,2,3,6-tetrahydropyridine hydrochloride (C1) (8 g, 32.579 mmol, 1 equiv.) in dichloromethane (80 mL) was added triethylamine (9.89 g, 97.738 mmol, 3 equiv.) followed by 4-chlorobenzoyl chloride (C2) (6.84 g, 39.095 mmol, 1.2 equiv.) at 0°C, and the reaction mixture was stirred at room temperature for 16 hours. After completion of reaction, the reaction mixture was quenched with water (300 mL) and extracted with DCM (2 x 300 mL). The organic layer was washed with brine (200 mL) and dried over anhydrous sodium sulfate, filtered, and concentrated under reduced pressure to get the crude product. The crude product was triturated with diethyl ether (30 mL), filtered and dried to afford (4-chlorophenyl)(4-(4,4,5,5-tetramethyl-1,3,2-dioxaborolan-2-yl)-3,6-dihydropyridin-1(2H)-yl)methanone (C3) (9.5 g, 83.8%).
LC-MS (m/z): 348.69 [M+H]⁺ ion present

### Step 2: Synthesis of (4-(4-amino-3-fluorophenyl)-3,6-dihydropyridin-1(2H)-yl)(4-chlorophenyl)methanone (C5)

To a stirred solution of (4-chlorophenyl)(4-(4,4,5,5-tetramethyl-1,3,2-dioxaborolan-2-yl)-3,6-dihydropyridin-1(2H)-yl)methanone (C3) (2 g, 5.753 mmol, 1 equiv.) in 1,4-dioxane (17 mL) and water (3 mL) were added 4-bromo-2-fluoroaniline (C4) (1.202 g, 6.328 mmol, 1.1 equiv.) and potassium carbonate (1.988 g, 14.382 mmol, 2.5 equiv.) at room temperature and degassed with nitrogen for 10 minutes. Then was added tetrakis(triphenylphosphine)-palladium(0), (0.5 g, 0.575 mmol, 0.1 equiv.) at same temperature and again degassed with nitrogen for 10 minutes. The resultant reaction mixture was heated to 100 °C and stirred for 16 hours. After completion of the reaction, the reaction mass was concentrated under reduced pressure. To the obtained crude mass, water (200 mL) was added and extracted with ethyl acetate (2 x 200 mL). The combined organic layer was washed with saturated brine solution (200 mL), dried over anhydrous sodium sulphate and concentrated to get the crude product. The crude product was purified by column chromatography using silica gel (230-400 mesh) and 25% ethyl acetate in petroleum ether to get (4-(4-amino-3-fluorophenyl)-3,6-dihydropyridin-1(2H)-yl)(4-chlorophenyl)methanone (C5) (1.2 g, 63%). LC-MS (m/z): 331.63 [M+H]⁺ ion present

### Step 3: Synthesis of phenyl (4-(1-(4-chlorobenzoyl)-1,2,3,6-tetrahydropyridin-4-yl)-2-fluorophenyl)carbamate (C7)

To a stirred solution of (4-(4-amino-3-fluorophenyl)-3,6-dihydropyridin-1(2H)-yl)(4-chlorophenyl)methanone (C5) (1.2 g, 3.628 mmol, 1 equiv.) in dichloromethane (18 mL) was added pyridine (0.439 mL, 5.442 mmol, 1.5 equiv.) at room temperature. The resultant reaction mixture was cooled to 0 °C, then was added phenyl carbonochloridate (C6) (0.625 g, 3.990 mmol, 1.1 equiv.) in dichloromethane (6 mL). The resultant reaction mixture was warm to room temperature and stirred for 2 hours (reaction followed by TLC). After completion of reaction, reaction mass was concentrated. To the obtained crude product was dissolved in ethyl acetate (200 mL) and the organic layer was washed with 1N hydrochloric acid (50 mL), saturated sodium bicarbonate (30 mL) and saturated brine solution (50 mL). Then the organic layer was dried over anhydrous sodium sulphate and concentrated to get the crude product. To the obtained crude product was added diethyl ether (5 mL) and stirred for 5 minutes at room temperature. After filtration, the solid was dried for 30 minutes to obtain phenyl (4-(1-(4-chlorobenzoyl)-1,2,3,6-tetrahydropyridin-4-yl)-2-fluorophenyl)carbamate (C7) (0.6 g, 37%), which was used directly for next step without purification.

### Step 4: Synthesis of (Z)-1-(4-(1-(4-chlorobenzoyl)-1,2,3,6-tetrahydropyridin-4-yl)-2-fluorophenyl)-3-(3-(2-isopropyl-5-methylphenyl)-4-oxothiazolidin-2-ylidene)urea (compound I-15)

To a stirred solution of phenyl (4-(1-(4-chlorobenzoyl)-1,2,3,6-tetrahydropyridin-4-yl)-2-fluorophenyl)carbamate (C7) (0.6 g, 1.331 mmol, 1 equiv.) in dry tetrahydrofuran (12 mL) were added 2-imino-3-(2-isopropyl-5-methylphenyl)thiazolidin-4-one (B8) (0.330 g, 1.331 mmol, 1 equiv.) and N,N-diisopropylethylamine (0.695 mL, 3.992 mmol, 3 equiv.) at room temperature. The reaction mixture was heated to 80 °C and stirred at same temperature for 24 hours. After completion of the reaction, the mass was concentrated. To the obtained crude product was added water (100 mL) and extracted the compound with ethyl acetate (2 x 50 mL). Combined organic layer was washed with saturated brine solution (100 mL) then dried over anhydrous sodium sulphate and concentrated to get the crude product. The crude product was purified by silica gel (230-400 mesh) column chromatography using 30% ethyl acetate in petroleum ether and further purified with reverse phase preparative HPLC to get (E/Z)-1-(4-(1-(4-chlorobenzoyl)-1,2,3,6-tetrahydropyridin-4-yl)-2-fluorophenyl)-3-(3-(2-isopropyl-5-methylphenyl)-4-oxothiazolidin-2-ylidene)urea (compound I-15) (0.13 g, 16%).

LC-MS (m/z): 605.30 [M+H]⁺ ion present; ¹H NMR (400 MHz, DMSO-*d*₆): δ 9.34 (s, 1H), 7.59-7.45 (m, 5H), 7.41-7.36 (m, 1H), 7.31-7.18 (m, 3H), 7.05 (s, 1H), 6.32-6.08 (m, 1H), 4.35-3.98 (m, 4H), 3.91-3.48 (m, 2H), 2.67-2.61 (m, 1H), 2.57-2.52 (m, 2H), 2.30 (s, 3H), 1.21-1.04 (m, 6H).

### Example 5: Synthesis of (E/Z)-1-(5-(1-(4-chlorobenzoyl)-1,2,3,6-tetrahydropyridin-4-yl)pyrazin-2-yl)-3-(3-(2-isopropyl-5-methylphenyl)-4-oxothiazolidin-2-ylidene)urea (compound I-12)

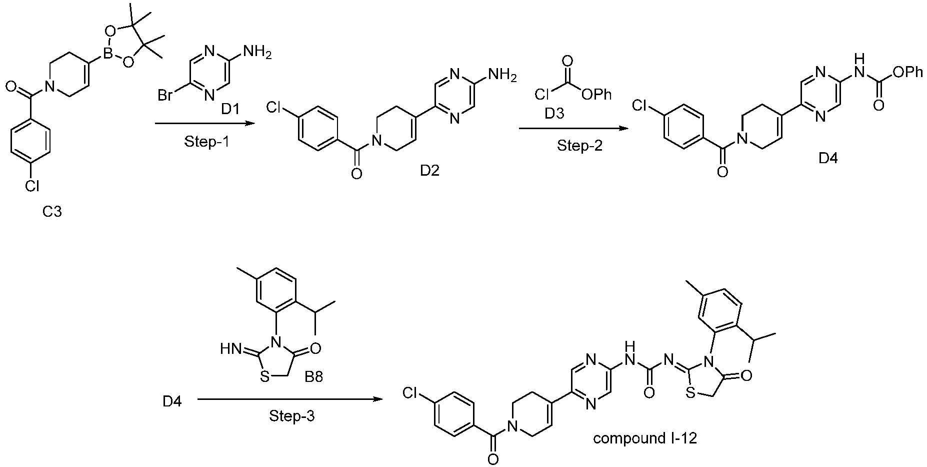

### Step 1: Synthesis of (4-(5-aminopyrazin-2-yl)-3,6-dihydropyridin-1(2H)-yl)(4-chlorophenyl)methanone (D2)

To a stirred solution of (4-chlorophenyl)(4-(4,4,5,5-tetramethyl-1,3,2-dioxaborolan-2-yl)-3,6-dihydropyridin-1(2H)-yl)methanone (C3) (1.5 g, 4.315 mmol, 1.0 equiv.) in 1,4-dioxane (12.75 mL, 8.5 V) and water (2.25 mL, 1.5 V) were added 5-bromopyrazin-2-amine (D1) (0.826 g, 4.746 mmol, 1.1 equiv.) and potassium carbonate (1.491 g, 10.787 mmol, 2.5 equiv.) at room temperature. The reaction mass was purged with nitrogen for 10 minutes. Then was added Tetrakis(triphenylphosphine)palladium(0), (0.5 g, 0.431 mmol, 0.1 equiv.) at same temperature and again purged the reaction mass with nitrogen for 10 minutes. Resultant reaction mixture was heated to 100°C and stirred for 4 hours. After completion of the reaction, the reaction mass was concentrated under reduced pressure. To the obtained crude mass, water (20 mL) was added and extracted with ethyl acetate (2 x 30 mL). The combined organic layer was washed with saturated brine solution (30 mL). Then the organic layer was dried over anhydrous sodium sulphate and concentrated to get the crude product. Obtained crude product was purified by column chromatography using silica gel (230-400 mesh) and 90% ethyl acetate in petroleum ether to get (4-(5-aminopyrazin-2-yl)-3,6-dihydropyridin-1(2H)-yl)(4-chlorophenyl)methanone (D2) (0.80 g, 59%). LC-MS (m/z): 315.11 [M+H]⁺ ion present.

### Step 2: Synthesis of phenyl (5-(1-(4-chlorobenzoyl)-1,2,3,6-tetrahydropyridin-4-yl)pyrazin-2-yl)carbamate (D4)

To a stirred solution of (4-(5-aminopyrazin-2-yl)-3,6-dihydropyridin-1(2H)-yl)(4-chlorophenyl)methanone (D2) (0.8 g, 2.542 mmol, 1.0 equiv.) in dichloromethane (8 mL) was added pyridine (0.308 mL, 3.812 mmol, 1.5 equiv.) at room temperature. Resultant reaction mixture was cooled to 0°C, then was added phenyl carbonochloridate (3) (0.438 g, 2.796 mmol, 1.1equiv.) in dichloromethane (4 mL). The resultant reaction mixture was warmed to room temperature and stirred for 1 hour. After completion of reaction, reaction mass was concentrated to get crude reaction mass. The obtained crude mass was dissolved in ethyl acetate (20 mL) then the organic layer washed with 1N hydrochloric acid (20 mL), saturated sodium bicarbonate (20 mL) and saturated brine solution (10 mL). Then the organic layer was separated, dried over anhydrous sodium sulphate and concentrated to get the crude product. To the obtained crude product was added diethyl ether (10 mL) and stirred for 5 minutes at room temperature. After filtration, the obtained solid was dried for 30 minutes under vacuum to get phenyl (5-(1-(4-chlorobenzoyl)-1,2,3,6-tetrahydropyridin-4-yl)pyrazin-2-yl)carbamate (4) (0.8 g, 72%).
LC-MS (m/z): 435.14 [M+H]⁺ ion present.

### Step 3: Synthesis of (E/Z)-1-(5-(1-(4-chlorobenzoyl)-1,2,3,6-tetrahydropyridin-4-yl)pyrazin-2-yl)-3-(3-(2-isopropyl-5-methylphenyl)-4-oxothiazolidin-2-ylidene)urea (compound I-12)

To a stirred solution of phenyl (5-(1-(4-chlorobenzoyl)-1,2,3,6-tetrahydropyridin-4-yl)pyrazin-2-yl)carbamate (D4) (0.5 g, 1.150 mmol, 1.0 equiv.) in tetrahydrofuran (10.0 mL, 20 V) were added 2-imino-3-(2-isopropyl-5-methylphenyl)thiazolidin-4-one (B8) (0.286 g, 1.150 mmol, 1.0 equiv.) and N,N-diisopropylethylamine (0.601 mL, 3.449 mmol, 3 equiv.) at room temperature. The reaction mixture was heated to 65°C, stirred at same temperature for 4 hours. After completion of the reaction, the reaction mass was concentrated to get crude mass to the obtained mass was added water (10 mL) and extracted the compound with ethyl acetate (2 x 15 mL). The combined organic layer was washed with saturated brine solution (15 mL), then dried over anhydrous sodium sulphate and concentrated to get the crude product. The crude product was slurred with mixture of diethyl ether (10 mL) and pentane (20 mL) at room temperature for 5 minutes. After filtration, the obtained solid was dried to get (E/Z)-1-(5-(1-(4-chlorobenzoyl)-1,2,3,6-tetrahydropyridin-4-yl)pyrazin-2-yl)-3-(3-(2-isopropyl-5-methylphenyl)-4-oxothiazolidin-2-ylidene)urea (compound I-12) (0.350 g, 52%). LC-MS (m/z): 589.31 [M+H]⁺ ion present

¹H NMR (400 MHz, DMSO-*d*₆): δ 10.47 (s, 1H), 9.05 (brs, 1H), 8.51 (brs, 1H), 7.55-7.48 (m, 4H), 7.37 (d, *J* = 8.0 Hz, 1H), 7.27 (d, *J* = 7.6 Hz, 1H), 7.04 (s, 1H), 6.83-6.54 (m, 1H), 4.31-4.09 (m, 4H), 3.93-3.51 (m, 2H), 2.67-2.56 (m, 3H), 2.30 (s, 3H), 1.14-1.07 (m, 6H).

### Example 6: Synthesis of (E/Z)-3-(2-isopropyl-5-methylphenyl)-2-(((E)-(1'-(4-(trifluoromethoxy)benzoyl)-1',2',3',6'-tetrahydro-[2,4'-bipyridin]-5-yl)methylene)hydrazine ylidene)thiazolidin-4-one (compound I-10)

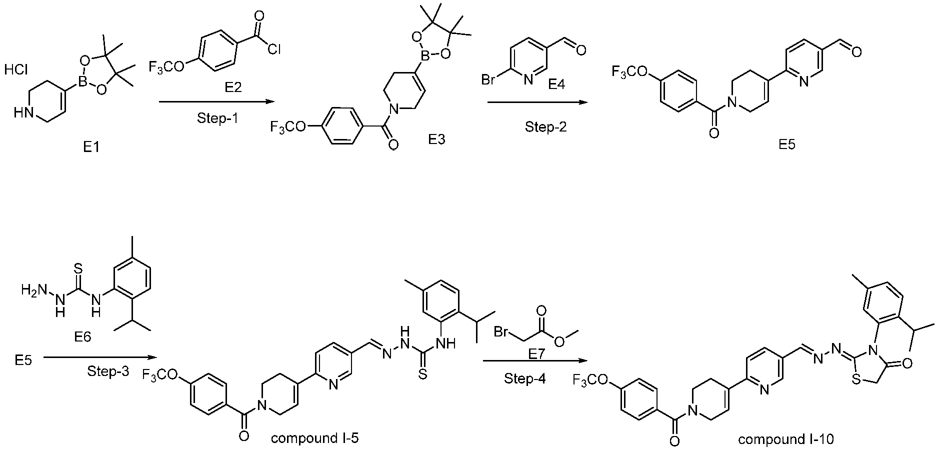

### Step-1: Synthesis of (4-(4,4,5,5-tetramethyl-1,3,2-dioxaborolan-2-yl)-3,6-dihydropyridin-1(2H)-yl)(4-(trifluoromethoxy)phenyl)methanone (E3):

A stirred solution of 4-(4,4,5,5-tetramethyl-1,3,2-dioxaborolan-2-yl)-1,2,3,6-tetrahydropyridine hydrochloride (E1) (2 g, 8.145 mmol, 1.0 equiv.) and triethylamine (3.387 mL, 24.435 mmol, 3.0 equiv.) in DCM (40 mL) was cooled to 0 °C, slowly added 4-(trifluoromethoxy) benzoyl chloride (E2) (2.195 g, 9.774 mmol, 1.2 equiv.) in DCM (5 mL). After that the reaction mixture was stirred at room temperature for 3 hours. After completion of reaction, water (50 mL) was added and extracted with DCM (2 x 50 mL). The combined organic layer was washed with saturated brine solution (50 mL), dried over anhydrous sodium sulphate and concentrated under reduced pressure to get the crude product. The crude product was washed and triturated with pentane (30 mL), filtered and dried to afford (4-(4,4,5,5-tetramethyl-1,3,2-dioxaborolan-2-yl)-3,6-dihydropyridin-1(2H)-yl)(4-(trifluoromethoxy)phenyl)methanone (E3) (2.2 g, 68%).
LC-MS (m/z): 398.73 [M+H]⁺ ion present

### Step-2: Synthesis of 1'-(4-(trifluoromethoxy)benzoyl)-1',2',3',6'-tetrahydro-[2,4'-bipyridine]-5-carbaldehyde (E5):

To a stirred solution of 6-bromonicotinaldehyde (E4) (1 g, 5.376 mmol, 1 equiv.), (4-(4,4,5,5-tetramethyl-1,3,2-dioxaborolan-2-yl)-3,6-dihydropyridin-1(2H)-yl) (4-(trifluoromethoxy) phenyl) methanone (E3) (2.349 g, 5.914 mmol, 1.1 equiv.) and sodium carbonate, anhydrous (1.425 g, 13.440 mmol, 2.5 equiv.) in mixture of 1,4-dioxane (10 mL), water (2 mL) was degassed with nitrogen for 5 minutes. After that, tetrakis(triphenylphosphine)palladium(0), (0.311 g, 0.269 mmol, 0.05 equiv.) was added and the resulting mixture was degassed with N₂ again for 5 minutes and stirred at 100 °C for 16 hours. After completion of the reaction, water (100 mL) was added and extracted with ethyl acetate (2 x 11 mL). The combined organic layer was washed with brine (50 mL), dried over sodium sulphate, filtered and concentrated under reduced pressure to get the crude product. The crude product was purified by silica gel (100-200 mesh) column chromatography using 50% ethyl acetate in petroleum ether as an eluent to afford 1'-(4-(trifluoromethoxy)benzoyl)-1',2',3',6'-tetrahydro-[2,4'-bipyridine]-5-carbaldehyde (5) (1.4 g, 69%).
LC-MS (m/z): 377.68 [M+H]⁺ ion present

### Step-3: Synthesis of (E)-N-(2-isopropyl-5-methylphenyl)-2-((1'-(4-(trifluoromethoxy) benzoyl)-1',2',3',6'-tetrahydro-[2,4'-bipyridin]-5-yl)methylene)hydrazine-1-carbothioamide (compound I-5)

To stirred solution of 1'-(4-(trifluoromethoxy)benzoyl)-1',2',3',6'-tetrahydro-[2,4'-bipyridine]-5-carbaldehyde (E5) (1.4 g, 3.720 mmol, 1 equiv.) and N-(2-isopropyl-5-methylphenyl)hydrazinecarbothioamide (E6) (0.831 g, 3.720 mmol, 1 equiv.) in methanol (14 mL) was added acetic acid (0.7 mL) at room temperature. The resulting mixture was stirred at the same temperature for 3 hours. After completion of the reaction, the precipitated solid was filtered and washed with petroleum ether (10 mL) and dried to afford (E)-N-(2-isopropyl-5-methylphenyl)-2-((1'-(4-(trifluoromethoxy)benzoyl)-1',2',3',6'-tetrahydro-[2,4'-bipyridin]-5-yl)methylene)hydrazine-1-carbothioamide (compound I-5) (1.6 g; 74%).

LC-MS (m/z): 582.34 [M+H] ⁺ ion present, ¹H NMR (400 MHz, DMSO-*d*₆): δ 11.88 (s, 1H), 10.02 (s, 1H), 8.91 (d, *J* = 1.6 Hz, 1H), 8.40 (d, *J* = 7.6 Hz, 1H), 8.14 (s, 1H), 7.67-7.58 (m, 3H), 7.46 (d, *J* = 8.0 Hz, 2H), 7.23 (d, *J* = 8.0 Hz, 1H), 7.11 (d, *J* = 8.0 Hz, 1H), 6.99 (s, 1H), 6.92-6.69 (m, 1H), 4.41-3.78 (m, 3H), 3.61-3.47 (m, 1H), 3.12-2.99 (m, 1H), 2.71 -2.62 (m, 2H), 2.28 (s, 3H), 1.15 (d, *J* = 6.8 Hz, 6H).

### Step-4: Synthesis of (E/Z)-3-(2-isopropyl-5-methylphenyl)-2-(((E)-(1'-(4-(trifluoromethoxy) benzoyl)-1',2',3',6'-tetrahydro-[2,4'-bipyridin]-5-yl)methylene)hydrazineylidene)thiazolidin-4-one (compound I-10)

To a stirred solution of (E)-N-(2-isopropyl-5-methylphenyl)-2-((1'-(4-(trifluoromethoxy) benzoyl)-1',2',3',6'-tetrahydro-[2,4'-bipyridin]-5-yl)methylene)hydrazine-1-carbothioamide (compound I-5) (1 g, 1.719 mmol, 1 equiv.) and sodium acetate (0.564 g, 6.877 mmol, 4 equiv.) in ethanol (20 mL) was added methyl 2-bromoacetate (E7) (0.394 g, 2.579 mmol, 1.5 equiv.) at room temperature. The resulting mixture was stirred at 70 °C for 3 hours. After completion of the reaction, the reaction mass was poured into water (200 mL), the precipitated solid was filtered and dried to get crude product. The crude product was purified by silica gel (100-200 mesh) column chromatography using 30% ethyl acetate in petroleum ether as an eluent to afford (E/Z)-3-(2-isopropyl-5-methylphenyl)-2-(((E)-(1'-(4-(trifluoromethoxy)benzoyl)-1',2',3',6'-tetrahydro-[2,4'-bipyridin]-5-yl)methylene)hydrazineylidene)thiazolidin-4-one (compound I-10) (0.49 g, 45%).

LC-MS (m/z): 622.49 [M+H] + ion present; ¹H NMR (400 MHz, DMSO-*d*₆): δ 8.81 (d, *J =* 1.6 Hz, 1H), 8.38 (s, 1H), 8.09 (d, *J* = 8.0 Hz, 1H), 7.72-7.58 (m, 3H), 7.46 (d, *J* = 8.0 Hz, 2H), 7.38 (d, *J* = 8.0 Hz, 1H), 7.27 (d, *J =* 8.0 Hz, 1H), 7.05 (s, 1H), 6.94-6.68 (m, 1H), 4.41-4.08 (m, 4H), 3.91-3.48 (m, 2H), 2.76-2.62 (m, 3H), 2.31 (s, 3H) 1.17-1.05 (m, 6H).

### Example 7: Synthesis of (E/Z)-2-(((E)-3-chloro-4-(1-(4-chlorobenzoyl)-1,2,3,6-tetrahydropyridin-4-yl)benzylidene)hydrazineylidene)-3-(2-isopropyl-5-methylphenyl) thiazolidin-4-one (compound I-9)

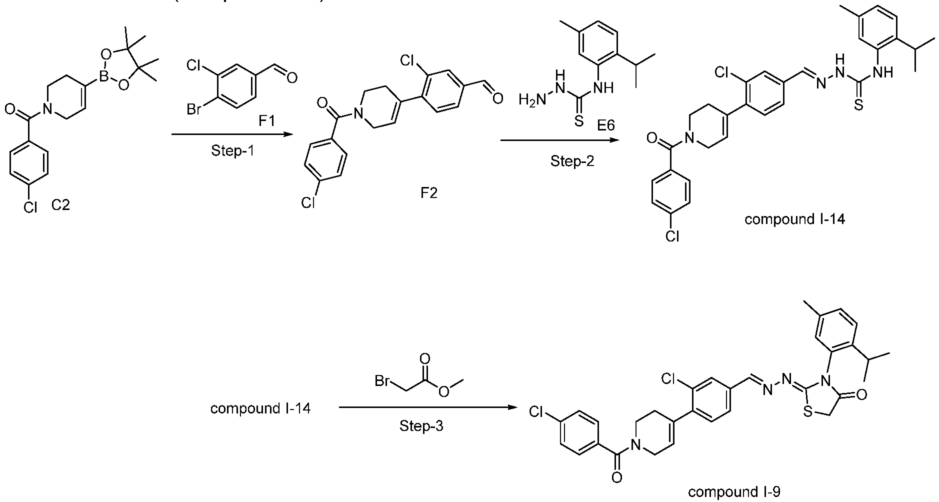

### Step-1: Synthesis of 3-chloro-4-(1-(4-chlorobenzoyl)-1,2,3,6-tetrahydropyridin-4-yl) benzaldehyde (F3):

To stirred solution of (4-chlorophenyl) (4-(4,4,5,5-tetramethyl-1,3,2-dioxaborolan-2-yl)-3,6-dihydropyridin-1(2H)-yl) methanone (C2) (1.2 g, 3.452 mmol, 1 equiv.), 4-bromo-3-chlorobenzaldehyde (F1) (0.758 g, 3.452 mmol, 1 equiv.) and sodium carbonate (0.915 g, 8.629 mmol, 2.5 equiv.) in 1,4 dioxane (10.2 mL), water (1.8 mL) was degassed with nitrogen for 5 minutes. After that, tetrakis(triphenylphosphine)palladium(0), (0.199 g, 0.173 mmol, 0.05 equiv.) was added and resulting mixture was degassed with N₂ again for 5 minutes and stirred at 100 °C for 16 hours. After completion of the reaction, water (200 mL) was added and extracted with ethyl acetate (2 x 100 mL). The combined organic layer was washed with brine (50 mL), dried over sodium sulphate, filtered and concentrated under reduced pressure to get the crude product. The crude product was purified by silica gel (100-200 mesh) column chromatography using 40% ethyl acetate in petroleum ether as an eluent to afford 3-chloro-4-(1-(4-chlorobenzoyl)-1,2,3,6-tetrahydropyridin-4-yl) benzaldehyde (F2) (1.2 g, 96%). LCMS (m/z): 360.04 [M+H] + ion present

### Step-2: Synthesis of (E)-2-(3-chloro-4-(1-(4-chlorobenzoyl)-1,2,3,6-tetrahydropyridin-4-yl) benzylidene)-N-(2-isopropyl-5-methylphenyl) hydrazine-1-carbothioamide (compound I-14):

To a stirred solution of 3-chloro-4-(1-(4-chlorobenzoyl)-1,2,3,6-tetrahydropyridin-4-yl) benzaldehyde (F2) (1.2 g, 3.331 mmol, 1 equiv.) and N-(2-isopropyl-5-methylphenyl) hydrazinecarbothioamide (E6) (0.744 g, 3.331 mmol, 1 equiv.) in methanol (12 mL) was added acetic acid (0.6 mL) at room temperature. The resulting mixture was stirred at room temperature for 3 hours. After completion of the reaction, the precipitated solid was filtered and washed with petroleum ether (10 mL) and dried to afford (E)-2-(3-chloro-4-(1-(4-chlorobenzoyl)-1,2,3,6-tetrahydropyridin-4-yl) benzylidene)-N-(2-isopropyl-5-methylphenyl) hydrazine-1-carbothioamide (compound I-14) (1.2 g; 63%). LCMS (m/z): 565.29 [M+H]⁺ ion present

¹H NMR (400 MHz, DMSO-*d*₆): δ 11.82 (s, 1H), 10.04 (s, 1H), 8.16 (s, 1H), 8.08 (s, 1H), 7.72 (d, *J =* 7.6 Hz, 1H), 7.61-7.44 (m, 4H), 7.41-7.29 (m, 1H), 7.23 (d, *J =* 8.0 Hz, 1H), 7.11 (d, *J =* 8.0 Hz, 1H), 6.99 (s, 1H), 5.90-5.64 (m, 1H), 4.32-3.73 (m, 3H), 3.59-3.48 (m, 1H), 3.11-2.99 (m, 1H), 2.49-2.41 (m, 2H), 2.29 (s, 3H), 1.16 (d, *J* = 6.8 Hz, 6H).

### Step-3: Synthesis of (E/Z)-2-(((E)-3-chloro-4-(1-(4-chlorobenzoyl)-1,2,3,6-tetrahydropyridin-4-yl)benzylidene)hydrazineylidene)-3-(2-isopropyl-5-methylphenyl) thiazolidin-4-one (compound I-9):

To a stirred solution of (E)-2-(3-chloro-4-(1-(4-chlorobenzoyl)-1,2,3,6-tetrahydropyridin-4-yl)benzylidene)-N-(2-isopropyl-5-methylphenyl) hydrazine-1-carbothioamide (compound I-14) (0.8 g, 1.415 mmol, 1 equiv.) and sodium acetate (0.464 g, 5.658 mmol, 4 equiv.) in ethanol (16 mL) was added methyl 2-bromoacetate (0.325 g, 2.122 mmol, 1.5 equiv.) at room temperature. The resulting reaction mixture was stirred at 70 °C for 3 hours. After completion of the reaction, reaction mass was poured into water (200 mL), the precipitated solid was filtered and dried to get crude product. The crude product was purified by silica gel (100-200 mesh) column chromatography using 30% ethyl acetate in petroleum ether as an eluent to afford (E/Z)-2-(((E)-3-chloro-4-(1-(4-chlorobenzoyl)-1,2,3,6-tetrahydropyridin-4-yl)benzylidene)hydrazineylidene)-3-(2-isopropyl-5-methylphenyl) thiazolidin-4-one (compound I-9) (0.58 g, 67%). LC-MS (m/z): 605.43 [M+H] + ion present

¹H NMR (400 MHz, DMSO-*d*₆): δ 8.32 (s, 1H), 7.78 (s, 1H), 7.69 (d, *J* = 8.0 Hz, 1H), 7.61-7.44 (m, 4H), 7.43-7.33 (m, 2H), 7.27 (d, *J =* 8.0 Hz, 1H), 7.06 (s, 1H), 5.92-5.61 (m, 1H), 4.32-3.48 (m, 6H), 2.78-2.64 (m, 1H), 2.48-2.42 (m, 2H), 2.30 (s, 3H), 1.11 (t, *J =* 7.2 Hz, 6H).

### Example 8: Synthesis of (E/Z)-2-(((E)-(5-(1-(4-chlorobenzoyl)-1,2,3,6-tetrahydropyridin-4-yl)pyrazin-2-yl)methylene)hydrazineylidene)-3-(2-isopropyl-5-methylphenyl)thiazolidin-4-one (compound I-11)

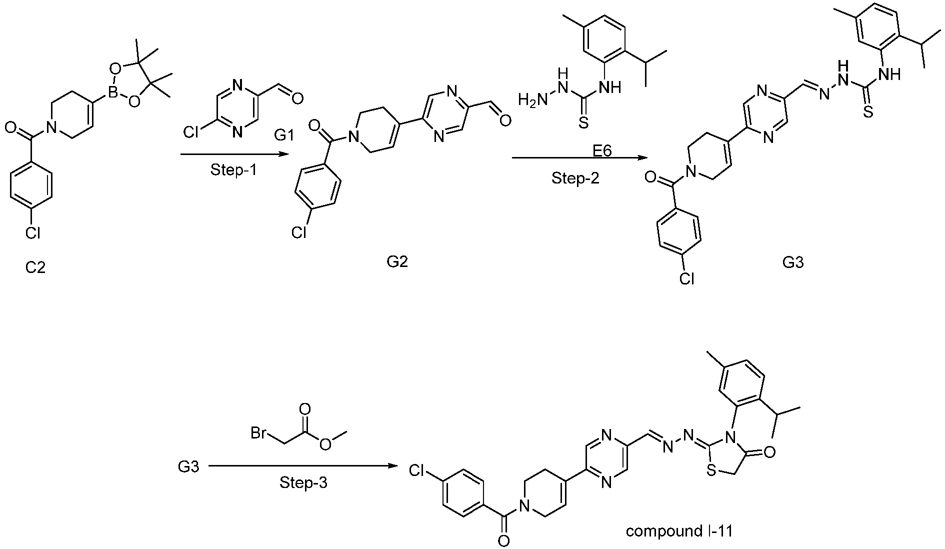

### Step-1: Synthesis of 5-(1-(4-chlorobenzoyl)-1,2,3,6-tetrahydropyridin-4-yl)pyrazine-2-carbaldehyde (G2):

To a stirred solution of (4-chlorophenyl)(4-(4,4,5,5-tetramethyl-1,3,2-dioxaborolan-2-yl)-3,6-dihydropyridin-1(2H)-yl)methanone (C2) (2.0 g, 5.753 mmol, 1.0 equiv.) in 1,4-dioxane (17.000 mL, 8.5 V) Water (3.000 mL, 1.5 V) were added 5-chloropyrazine-2-carbaldehyde (G1) (0.902 g, 6.328 mmol, 1.1 equiv.) and potassium carbonate (1.988 g, 14.382 mmol, 2.5 equiv.) at room temperature. The reaction mass was purged with nitrogen for 10 minutes. Then was added tetrakis(triphenylphosphine)palladium(0), (0.5 g, 0.575 mmol, 0.1 equiv.) at same temperature and again purged the reaction mas with nitrogen for 10 minutes. Resultant reaction mixture was heated to 100°C and stirred for 4 hours. After completion of the reaction, water (20 mL) was added and extracted with ethyl acetate (2 x 20 mL). The combined organic layer was washed with brine (20 mL), dried over sodium sulphate, filtered and concentrated under reduced pressure to get the crude product. The crude product was purified by silica gel (100-200 mesh) column chromatography using 50% ethyl acetate in pet ether as an eluent to afford 5-(1-(4-chlorobenzoyl)-1,2,3,6-tetrahydropyridin-4-yl)pyrazine-2-carbaldehyde (G2) (0.50 g, 27%).
LC-MS (m/z): 328.09 [M+H]⁺ ion present

### Step-2: Synthesis of (E)-2-((5-(1-(4-chlorobenzoyl)-1,2,3,6-tetrahydropyridin-4-yl)pyrazin-2-yl)methylene)-N-(2-isopropyl-5-methylphenyl)hydrazine-1-carbothioamide (G3):

To a stirred solution of 5-(1-(4-chlorobenzoyl)-1,2,3,6-tetrahydropyridin-4-yl)pyrazine-2-carbaldehyde (G2) (0.5 g, 1.525 mmol, 1.0 equiv.) in methanol (10.0 mL), was added acetic acid (0.050 mL, 0.1 V) and N-(2-isopropyl-5-methylphenyl)hydrazinecarbothioamide (E6) (0.341 g, 1.525 mmol, 1.0 equiv.) at 0° C, After that, reaction mixture was stirred at room temperature for 3 hours. After completion of the reaction, water (10 mL) was added and extracted with ethyl acetate (2 x 10 mL). The combined organic layer washed with brine (10 mL), dried over sodium sulphate, filtered and concentrated under reduced pressure to get the crude product. The crude product was slurried with mixture of diethyl ether (15 mL) and pentane (30 mL), filtered to get (E)-2-((5-(1-(4-chlorobenzoyl)-1,2,3,6-tetrahydropyridin-4-yl)pyrazin-2-yl)methylene)-N-(2-isopropyl-5-methylphenyl)hydrazine-1-carbothioamide (G3) (0.60 g, 74%).
LC-MS (m/z): 533.17 [M+H]⁺ ion present

### Step-3: Synthesis of (E/Z)-2-(((E)-(5-(1-(4-chlorobenzoyl)-1,2,3,6-tetrahydropyridin-4-yl)pyrazin-2-yl)methylene)hydrazineylidene)-3-(2-isopropyl-5-methylphenyl)thiazolidin-4-one (compound I-11):

To a stirred solution of (E)-2-((5-(1-(4-chlorobenzoyl)-1,2,3,6-tetrahydropyridin-4-yl)pyrazin-2-yl)methylene)-N-(2-isopropyl-5-methylphenyl)hydrazine-1-carbothioamide (G3) (0.60 g, 1.126 mmol, 1.0 equiv.) in ethanol (6.000 mL, 10 V) were added methyl 2-bromoacetate (0.258 g, 1.688 mmol, 1.5 eq.) and sodium acetate (0.369 g, 4.502 mmol, 4 equiv.) at room temperature. The resulting reaction mixture was stirred at 70°C for 3 hours. After completion of reaction, the reaction mass was concentrated under reduced pressure to get crude mass, added water (10 mL) and extracted with ethyl acetate (2 x 20 mL). Combined organic layer washed with saturated brine solution (10 mL). Then the organic layer dried over anhydrous sodium sulphate and concentrated to get the crude product. The crude product was slurried with mixture of diethyl ether (10 mL), pentane (20 mL) and filtered to afford (E/Z)-2-(((E)-(5-(1-(4-chlorobenzoyl)-1,2,3,6-tetrahydropyridin-4-yl)pyrazin-2-yl)methylene)hydrazineylidene)-3-(2-isopropyl-5-methylphenyl)thiazolidin-4-one (compound I-11) (173 mg, 26%).

LC-MS (m/z): 573.35 [M+H]⁺ ion present; ¹H NMR (400 MHz, DMSO-*d*₆): δ 9.04 (s, 1H), 8.92 (brs, 1H), 8.25 (s, 1H), 7.55-7.49 (m, 4H), 7.39 (d, *J* = 8.0 Hz, 1H), 7.28 (d, *J* = 7.6 Hz, 1H), 7.07-6.85 (m, 2H), 4.38-4.11 (m, 4H), 3.86-3.54 (m, 2H), 2.76-2.69 (m, 3H), 2.31 (s, 3H), 1.14-1.09 (m, 6H).

### Example 9: Synthesis of (E/Z)-1-(2-chloro-4-(1-(4-(trifluoromethoxy)benzoyl)-1,2,3,6-tetrahydropyridin-4-yl)phenyl)-3-(3-(2-isopropyl-5-methylphenyl)-4-oxothiazolidin-2-ylidene)urea (compound I-8):

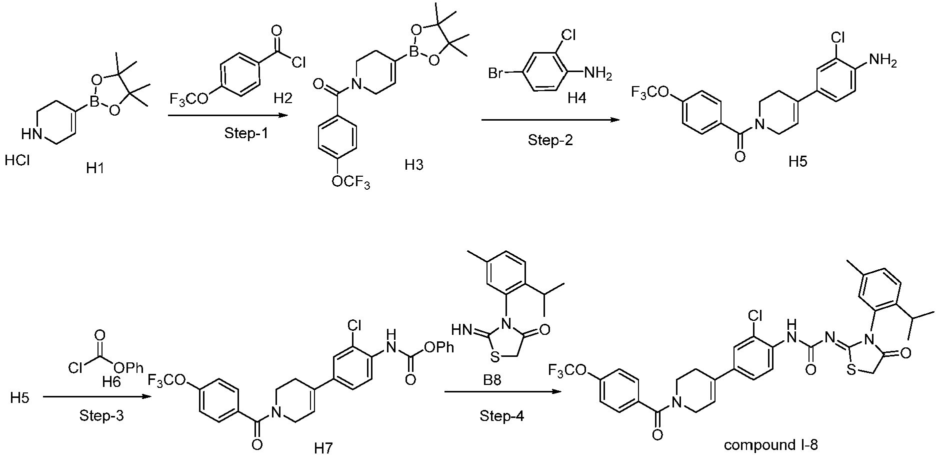

### Step 1: Synthesis of (4-(4,4,5,5-tetramethyl-1,3,2-dioxaborolan-2-yl)-3,6-dihydropyridin-1(2H)-yl)(4-(trifluoromethoxy)phenyl)methanone (H3):

To a stirred solution of 4-(4,4,5,5-tetramethyl-1,3,2-dioxaborolan-2-yl)-1,2,3,6-tetrahydropyridine hydrochloride (H1) (2 g, 8.145 mmol, 1.0 equiv.) in dichloromethane (20 mL) were added triethyl amine (1.694 mL, 12.217 mmol, 1.5 equiv.) and 4-(trifluoromethoxy)benzoyl chloride (H2) (1.920 g, 8.552 mmol, 1.05 equiv.) at 0 °C, After that, the reaction mixture was stirred at room temperature for 3 hours. After completion of reaction, the reaction mixture was quenched with water (50 mL) and extracted with dichloromethane (2 x 30 mL). The organic layer was washed with brine (20 mL) and dried over anhydrous sodium sulfate, filtered, and concentrated under reduced pressure to get crude product. The crude product was triturated with diethyl ether (30 mL), filtered and dried to afford (4-(4,4,5,5-tetramethyl-1,3,2-dioxaborolan-2-yl)-3,6-dihydropyridin-1(2H)-yl)(4-(trifluoromethoxy)phenyl)methanone (H3) (2.5 g, 77%).
LC-MS (m/z): 398.16 [M+H]⁺ ion present

### Step 2: Synthesis of (4-(4-amino-3-chlorophenyl)-3,6-dihydropyridin-1(2H)-yl)(4-(trifluoromethoxy)phenyl)methanone (H5):

To a stirred solution of (4-(4,4,5,5-tetramethyl-1,3,2-dioxaborolan-2-yl)-3,6-dihydropyridin-1(2H)-yl)(4-(trifluoromethoxy)phenyl)methanone (H3) (2 g, 5.035 mmol, 1.0 equiv.) in 1,4-dioxane (17 mL, 8.5 V) and water (3.0 mL, 1.5 V) were added 4-bromo-2-chloroaniline (H4) (1.040g, 5.035 mmol, 1.1 equiv.) and potassium carbonate (1.740 g, 12.588 mmol, 2.5 equiv.) at room temperature, degassed the reaction mass with nitrogen for 10 minutes. Then was added tetrakis(triphenylphosphine)palladium(0), (0.5 g, 0.504 mmol, 0.1 equiv.) at same temperature and again degassed the reaction mass with nitrogen for 10 minutes. The resultant reaction mixture was heated to 100 °C and stirred for 16 hours. After completion of reaction, the reaction mass was concentrated under reduced pressure. To the obtained crude mass, water (20 mL) was added and extracted with ethyl acetate (2 x 20 mL). The combined organic layer washed with saturated brine solution (20 mL). Then the organic layer was dried over anhydrous sodium sulphate and concentrated to get the crude product. Obtained crude product was purified by column chromatography using silica gel (230-400 mesh) and 25% ethyl acetate in petroleum ether to get (4-(4-amino-3-chlorophenyl)-3,6-dihydropyridin-1(2H)-yl)(4-(trifluoromethoxy)phenyl)methanone (H5) (1 g, 50%). LC-MS (m/z): 397.10 [M+H]⁺ ion present.

### Step 3: Synthesis of phenyl (2-chloro-4-(1-(4-(trifluoromethoxy)benzoyl)-1,2,3,6-tetrahydropyridin-4-yl)phenyl)carbamate (H7):

To a stirred solution of (4-(4-amino-3-chlorophenyl)-3,6-dihydropyridin-1(2H)-yl)(4-(trifluoromethoxy)phenyl)methanone (H5) (1 g, 2.520 mmol, 1.0 equiv.) in dichloromethane (10 mL, 10 V) was added pyridine (0.305 mL, 3.708 mmol, 1.5 equiv.) at room temperature. The resultant reaction mixture was cooled to 0 °C, then was added phenyl carbonochloridate (H6) (0.434 g, 2.772 mmol, 1.1 equiv.) in dichloromethane (5 mL). The resultant reaction mixture was warmed to room temperature and stirred for 1 hour. After completion of the reaction, the reaction mass was concentrated to get crude mass. The obtained crude mass was dissolved in ethyl acetate (20 mL), then organic layer washed with 1N hydrochloric acid (20 mL), saturated sodium bicarbonate (20 mL) and saturated brine solution (10 mL). Then organic layer was dried over anhydrous sodium sulphate and concentrated to get the crude product. To the obtained crude product was added diethyl ether (10 mL) and stirred for 5 minutes at room temperature. Filtered the solid and dried for 30 minutes to get phenyl (2-chloro-4-(1-(4-(trifluoromethoxy)benzoyl)-1,2,3,6-tetrahydropyridin-4-yl)phenyl)carbamate (H7) (1 g, 77%).
LC-MS (m/z): 517.12 [M+H]⁺ ion present.

### Step 4: Synthesis of (E/Z)-1-(2-chloro-4-(1-(4-(trifluoromethoxy)benzoyl)-1,2,3,6-tetrahydropyridin-4-yl)phenyl)-3-(3-(2-isopropyl-5-methylphenyl)-4-oxothiazolidin-2-ylidene)urea (compound I-8):

To a stirred solution of phenyl (2-chloro-4-(1-(4-(trifluoromethoxy)benzoyl)-1,2,3,6-tetrahydropyridin-4-yl)phenyl)carbamate (H7) (1 g, 1.935 mmol, 1 equiv.) in tetrahydrofuran (20 mL, 20 V) were added 2-imino-3-(2-isopropyl-5-methylphenyl)thiazolidin-4-one (B8) (0.480 g, 1.935 mmol, 1 equiv.) and N,N-diisopropylethylamine (1.011 mL, 5.804 mmol, 3 equiv.) at room temperature. The reaction mixture was heated to 65 °C and stirred at same temperature for 16 hours. After completion of the reaction, the mass was concentrated, to the obtained crude mass was added water (10 mL) and extracted the compound with ethyl acetate (2 x 20 mL). The combined organic layer was washed with saturated brine solution (10 mL), then dried over anhydrous sodium sulphate and concentrated to get the crude product. The crude product was purified by silica gel (230-400 mesh) column chromatography using 50% ethyl acetate in petroleum ether, concentrated the pure product to get (E/Z)-1-(2-chloro-4-(1-(4-(trifluoromethoxy)benzoyl)-1,2,3,6-tetrahydropyridin-4-yl)phenyl)-3-(3-(2-isopropyl-5-methylphenyl)-4-oxothiazolidin-2-ylidene)urea (compound I-8) (0.307 g, 24%).

LC-MS (m/z): 671.36 [M+H]⁺ ion present; ¹H NMR (400 MHz, DMSO-*d*₆): δ 8.99 (s, 1H), 7.68-7.35 (m, 8H), 7.26 (d, *J =* 7.6 Hz, 1H), 7.05 (s, 1H), 6.35-6.04 (m, 1H), 4.35-4.05 (m, 4H), 3.91-3.46 (m, 2H), 3.45-3.38 (m, 2H), 2.67-2.56 (m, 1H), 2.30 (s, 3H), 1.24-1.05 (m, 6H).

### Example 10: Synthesis of (E/Z)-1-(2-chloro-4-(1-(4-chlorobenzoyl)-2,5-dihydro-1H-pyrrol-3-yl)phenyl)-3-(3-(2-isopropyl-5-methylphenyl)-4-oxothiazolidin-2-ylidene)urea (compound I-13):

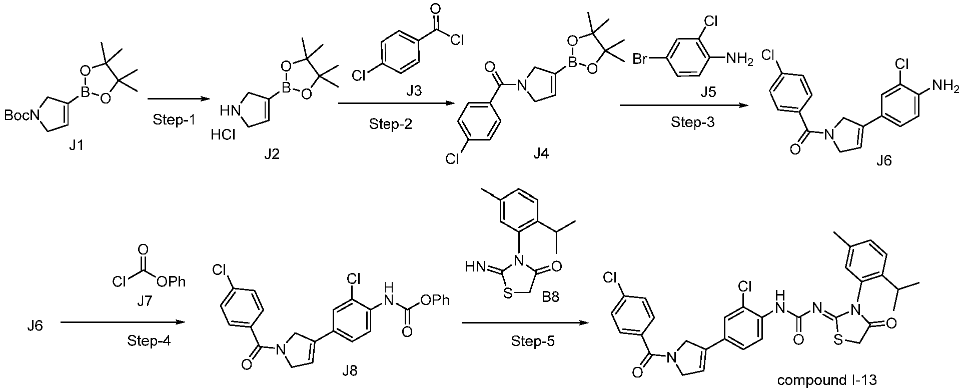

### Step 1: Synthesis of 3-(4,4,5,5-tetramethyl-1,3,2-dioxaborolan-2-yl)-2,5-dihydro-1H-pyrrole hydrochloride (J2):

To a stirred solution of tert-butyl 3-(4,4,5,5-tetramethyl-1,3,2-dioxaborolan-2-yl)-2,5-dihydro-1H-pyrrole-1-carboxylate (J1) (5 g, 16.939 mmol, 1.0 equiv.) in 1,4-dioxane (10 mL, 2 V) were added hydrogen chloride 4M in 1,4-dioxane (40 mL) at 0 °C, after that, the reaction mixture stirred at room temperature for 3 hours. After completion of reaction, the reaction mass was concentrated under reduced pressure to get the crude product. The crude product was triturated with diethyl ether (20 mL) and n-pentane (30 mL), filtered and dried to afford 3-(4,4,5,5-tetramethyl-1,3,2-dioxaborolan-2-yl)-2,5-dihydro-1H-pyrrole hydrochloride (J2) (3.5 g, 89%).
LC-MS (m/z): 196.15 [M-HCl+H]+ ion present

### Step 2: Synthesis of (4-chlorophenyl)(3-(4,4,5,5-tetramethyl-1,3,2-dioxaborolan-2-yl)-2,5-dihydro-1H-pyrrol-1-yl)methanone (J4):

To a stirred solution of 3-(4,4,5,5-tetramethyl-1,3,2-dioxaborolan-2-yl)-2,5-dihydro-1H-pyrrole hydrochloride (J2) (3.5 g, 15.117 mmol, 1.0 equiv.) in dichloromethane (35 mL, 10 V) where added triethyl amine (3.143 mL, 22.676 mmol, 1.5 equiv.) and 4-chlorobenzoyl chloride (J3) (2.778 g, 15.873 mmol, 1.05 equiv.) at 0°C, after that, the reaction mixture was stirred at room temperature for 3 hours. After completion of the reaction, the reaction mixture was quenched with water (50 mL) and extracted with dichloromethane (2 x 30 mL). The organic layer was washed with brine (20 mL) and dried over anhydrous sodium sulfate, filtered, and concentrated under reduced pressure to get the crude product. The crude product was triturated with diethyl ether (10 mL), n-pentane (20 mL), filtered and dried to afford (4-chlorophenyl)(3-(4,4,5,5-tetramethyl-1,3,2-dioxaborolan-2-yl)-2,5-dihydro-1H-pyrrol-1-yl)methanone (J4) (2.0 g, 40%).
LC-MS (m/z): 334.13 [M+H]+ ion present

### Step 3: Synthesis of (3-(4-amino-3-chlorophenyl)-2,5-dihydro-1H-pyrrol-1-yl)(4-chlorophenyl)methanone (J6):

To a stirred solution of (4-chlorophenyl)(3-(4,4,5,5-tetramethyl-1,3,2-dioxaborolan-2-yl)-2,5-dihydro-1H-pyrrol-1-yl)methanone (J4) (2.0 g, 5.995 mmol, 1.0 equiv.) in 1,4-dioxane (17 mL, 8.5 V) and water (3.0 mL, 1.5 V) were added 4-bromo-2-chloroaniline (J5) (1.362 g, 6.594 mmol, 1.1 equiv) and potassium carbonate (2.071 g, 14.987 mmol, 2.5 equiv.) at room temperature, degassed the reaction mass with nitrogen for 10 minutes. Then was added tetrakis(triphenylphosphine)palladium(0), (0.693 g, 0.599 mmol, 0.1 equiv.) at the same temperature and again degassed the reaction mass with nitrogen for 10 minutes. Resultant reaction mixture was heated to 100 °C and stirred for 4 hours. After completion of the reaction, the reaction mass was concentrated under reduced pressure. To the obtained crude mass, water (20 mL) was added and extracted with ethyl acetate (2 x 20 mL). The combined organic layer washed with saturated brine solution (20 mL). Then the organic layer was dried over anhydrous sodium sulphate and concentrated to get the crude product. The crude product was triturated with diethyl ether (20 mL), filtered and dried to afford (3-(4-amino-3-chlorophenyl)-2,5-dihydro-1H-pyrrol-1-yl)(4-chlorophenyl)methanone (J6) (1 g, 50%). LC-MS (m/z): 332.95 [M+H]+ ion present.

### Step 4: Synthesis of phenyl (2-chloro-4-(1-(4-chlorobenzoyl)-2,5-dihydro-1H-pyrrol-3-yl)phenyl)carbamate (J8):

To a stirred solution of (3-(4-amino-3-chlorophenyl)-2,5-dihydro-1H-pyrrol-1-yl)(4-chlorophenyl)methanone (J6) (1.0 g, 3.001 mmol, 1.0 equiv.) in dichloromethane (10 mL) was added pyridine (0.363 mL, 4.502 mmol, 1.5 equiv.) at room temperature. The resultant reaction mixture was cooled to 0 °C, then was added phenyl carbonochloridate (J7) (0.517 g, 3.301 mmol, 1.1 equiv.) in dichloromethane (5 mL). The resultant reaction mixture was stirred at room temperature for 1 hour. After completion of the reaction, the reaction mass was concentrated. The obtained crude mass was dissolved in ethyl acetate (20 mL), then organic layer washed with 1N hydrochloric acid (20 mL), saturated sodium bicarbonate (20 mL) and saturated brine solution (10 mL). Then the organic layer was dried over anhydrous sodium sulphate and concentrated to get the crude product. To the obtained crude product was added diethyl ether (10 mL) and n-pentane stirred for 5 minutes at room temperature. Filtered the solid and dried for 30 minutes to get phenyl (2-chloro-4-(1-(4-chlorobenzoyl)-2,5-dihydro-1H-pyrrol-3-yl)phenyl)carbamate (J8) (0.9 g, 66%).
LC-MS (m/z): 453.05 [M+H]+ ion present

### Step 5: Synthesis of (E/Z)-1-(2-chloro-4-(1-(4-chlorobenzoyl)-2,5-dihydro-1H-pyrrol-3-yl)phenyl)-3-(3-(2-isopropyl-5-methylphenyl)-4-oxothiazolidin-2-ylidene)urea (compound I-13):

To a stirred solution of phenyl (2-chloro-4-(1-(4-chlorobenzoyl)-2,5-dihydro-1H-pyrrol-3-yl)phenyl)carbamate (J8) (0.6 g, 1.324 mmol, 1.0 equiv.) in tetrahydrofuran (12 mL, 20 V) were added 2-imino-3-(2-isopropyl-5-methylphenyl)thiazolidin-4-one (B8) (0.329 g, 1.324 mmol, 1.0 equiv.) and N,N-diisopropylethylamine (0.692 mL, 3.971 mmol, 3 equiv.) at room temperature. The reaction mixture was heated to 65 °C and stirred at same temperature for 4 hours. After completion of the reaction, the mass was concentrated. To the obtained crude product was added water (10 mL) and extracted the compound with ethyl acetate (2 x 20 mL). Combined organic layer was washed with saturated brine solution (10 mL) then dried over anhydrous sodium sulphate and concentrated to get the crude product. Obtained crude product was slurried with diethyl ether (10 mL) and pentane (20 mL), stirred for 5 minutes at room temperature. Filtered the solid and dried to get (E/Z)-1-(2-chloro-4-(1-(4-chlorobenzoyl)-2,5-dihydro-1H-pyrrol-3-yl)phenyl)-3-(3-(2-isopropyl-5-methylphenyl)-4-oxothiazolidin-2-ylidene)urea (compound I-13) (0.256 g, 32%).

LC-MS (m/z): 607.28 [M+H]+ ion present; ¹H NMR (400 MHz, DMSO-*d*₆): δ 9.00 (d, J = 15.2 Hz, 1H), 7.79-7.33 (m, 8H), 7.26 (d, J = 7.6 Hz, 1H), 7.05 (s, 1H), 6.65-6.39 (m, 1H), 4.63 (d, J = 22.4 Hz, 2H), 4.48-4.39 (m, 2H), 4.20 (dd, J = 18.0, 3.6 Hz, 1H), 4.07 (dd, J = 18.0, 4.0 Hz, 1H), 2.67-2.55 (m, 1H), 2.31 (s, 3H), 1.24-0.98 (m, 6H).

### Example 11: (E/Z)-1-(2-chloro-4-(4-(4-(trifluoromethoxy)benzoyl)piperazin-1-yl)phenyl)-3-(3-(2-isopropyl-5-methylphenyl)-4-oxothiazolidin-2-ylidene)urea (compound I-17):

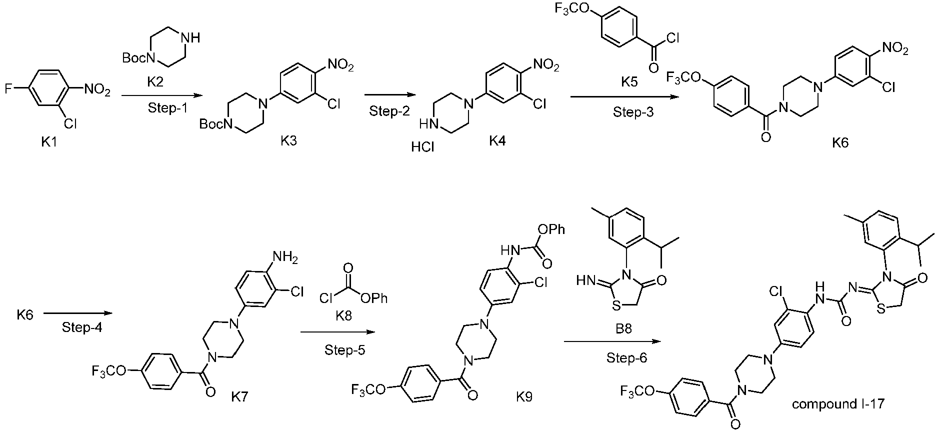

### Step 1: Synthesis of tert-butyl 4-(3-chloro-4-nitrophenyl)piperazine-1-carboxylate (K3):

To a stirred solution of 2-chloro-4-fluoro-1-nitrobenzene (K1) (10 g, 56.966 mmol, 1.0 equiv.) in acetonitrile (250.0 mL) were added tert-butyl piperazine-1-carboxylate (K2) (10.610 g, 56.966 mmol, 1.1 equiv) and potassium carbonate (19.682 g, 142.415 mmol, 2.5 equiv.) at room temperature. The reaction mixture was stirred at 80 °C for 5 hours. After completion of the reaction, the reaction mixture was quenched with water (100 mL) and extracted with ethyl acetate (2 x 100 mL). The combined organic layers were washed with brine (100 mL), dried over anhydrous sodium sulphate, filtered and concentrated to get the crude product. The crude product was purified by silica gel (230-400 mesh) column chromatography using 25% ethyl acetate in petroleum ether to give tert-butyl 4-(3-chloro-4-nitrophenyl)piperazine-1-carboxylate (3) (9 g, 46%). LC-MS (m/z): 242.08 [M+H-Boc]+ ion present

### Step 2: Synthesis of 1-(3-chloro-4-nitrophenyl) piperazine hydrochloride (K4):

To a stirred solution of (tert-butyl 4-(3-chloro-4-nitrophenyl) piperazine-1-carboxylate (K3) (9 g, 26.332 mmol, 1.0 equiv.) in 1,4-dioxane (18 mL) were added hydrogen chloride solution (4.0 M in dioxane) (72.0 mL) at 0 °C, the reaction mixture was stirred at room temperature for 3 hours. After completion of the reaction, the reaction mass was concentrated under reduced pressure to get the crude product. The crude product was triturated with diethyl ether (50 mL) to get 1-(3-chloro-4-nitrophenyl) piperazine hydrochloride (K4) (6.7 g, 91%) as a yellow solid (¹H NMR complies). The obtained product was used as such in the next step without further purification.

### Step 3: Synthesis of (4-(3-chloro-4-nitrophenyl)piperazin-1-yl)(4-(trifluoromethoxy)phenyl) methanone (K6):

To a stirred solution of 1-(3-chloro-4-nitrophenyl) piperazine hydrochloride (K4) (3.3 g, 11.865 mmol, 1.0 equiv.) in dichloromethane (33 mL) where added triethyl amine (4.934 mL, 35.594 mmol, 3.0 equiv.) and 4-(trifluoromethoxy) benzoyl chloride (K5) (3.997 g, 17.797 mmol, 1.5 equiv.) at 0 °C. The reaction mixture was stirred at room temperature for 3 hours. After completion of the reaction, was added water (50 mL) and extracted with dichloromethane (2 x 30 mL). Combined organic layer was washed with saturated brine solution (50 mL), dried over anhydrous sodium sulphate and concentrated to get the crude product. The crude product was triturated with mixture of diethyl ether (50 mL) and n-pentane to get (4-(3-chloro-4-nitrophenyl)piperazin-1-yl)(4-(trifluoromethoxy)phenyl)methanone (K6) (5 g, 98%). LC-MS (m/z): 430.45 [M+H]⁺ ion present

### Step 4: Synthesis of (4-(4-amino-3-chlorophenyl)piperazin-1-yl)(4-(trifluoromethoxy)phenyl) methanone (K7):

To a stirred solution of (4-(3-chloro-4-nitrophenyl)piperazin-1-yl)(4-(trifluoromethoxy)phenyl) methanone (K6) (3 g, 6.980 mmol, 1.0 equiv.) in mixture of ethanol (22.50 mL), water (7.50 mL) were ammonium chloride (1.120 g, 20.941 mmol, 3.0 equiv) and Iron powder(2.729 g, 48.862 mmol, 7 equiv.) at room temperature. The reaction mixture was stirred at 70 °C for 2 hours. After completion of reaction, the reaction mixture was filtered through celite bed. The organic layer was quenched with water and extracted with ethyl acetate (2 x 50 mL). Combined organic layer was washed with saturated brine solution (20 mL). Then the organic layer was dried over anhydrous sodium sulphate and concentrated to get the crude product. The obtained crude product was slurried with mixture of diethyl ether (10 mL) and pentane (20 mL) to get (4-(4-amino-3-chlorophenyl)piperazin-1-yl)(4-(trifluoromethoxy)phenyl)methanone (K7) (2 g, 71%).
LC-MS (m/z): 400.23 [M+H]⁺ ion present

### Step 5: Synthesis of phenyl (2-chloro-4-(4-(4-(trifluoromethoxy)benzoyl)piperazin-1-yl)phenyl) carbamate (K9):

To a stirred solution of (4-(4-amino-3-chlorophenyl)piperazin-1-yl)(4-(trifluoromethoxy) phenyl)methanone (K7) (2 g, 5.003 mmol, 1.0 equiv.) in dichloromethane (20 mL) were added pyridine (0.606 mL, 7.504 mmol, 1.5 equiv) and phenyl carbonochloridate (K8) (0.862 g, 5.503 mmol, 1.2 equiv.) in dichloromethane (10 mL) at 0°C. The reaction mixture was stirred at room temperature for 3 hours. The reaction mixture was quenched with 1N HCl solution in water (20 mL) and extracted with dichloromethane (2x 10 mL). The combined organic layers were washed with saturated aqueous NaHCO₃ solution (2 x 10 mL) and brine (10 mL), dried over anhydrous sodium sulphate, filtered and concentrated to get the crude product. The crude product was triturated with diethyl ether in (10 mL) to get phenyl (2-chloro-4-(4-(4-(trifluoromethoxy)benzoyl)piperazin-1-yl)phenyl)carbamate (K9) (2 g, 77%).
LC-MS (m/z): 520.55 [M+H]⁺ ion present

### Step 6: Synthesis of (E/Z)-1-(2-chloro-4-(4-(4-(trifluoromethoxy)benzoyl)piperazin-1-yl)phenyl)-3-(3-(2-isopropyl-5-methylphenyl)-4-oxothiazolidin-2-ylidene)urea (compound I-17):

To a stirred solution of phenyl (2-chloro-4-(4-(4-(trifluoromethoxy)benzoyl)piperazin-1-yl)phenyl)carbamate (K9) (0.6 g, 1.154 mmol, 1.0 equiv.) in tetrahydrofuran (12.0 mL) were added 2-imino-3-(2-isopropyl-5-methylphenyl)thiazolidin-4-one (B8) (0.344 g, 1.385 mmol, 1.2 equiv) and N,N-diisopropylethylamine (0.603 mL, 3.462 mmol, 3 equiv) at room temperature. The reaction mixture was heated to 65 °C and stirred at same temperature for 4 hours. After completion of the reaction, the reaction mixture was quenched with water (20 mL) and extracted with EtOAc (2 x 20 mL). The combined organic layers were washed with brine (20 mL), dried over anhydrous sodium sulphate, filtered and concentrated to get the crude product. The crude product was triturated with mixture of diethyl ether (10 mL) and pet ether (20 mL) to get (E/Z)-1-(2-chloro-4-(4-(4-(trifluoromethoxy)benzoyl)piperazin-1-yl)phenyl)-3-(3-(2-isopropyl-5-methylphenyl)-4-oxothiazolidin-2-ylidene)urea (compound I-17) (0.430 g, 55%).

LC-MS (m/z): 674.26 [M+H]⁺ ion present; ¹H NMR (400 MHz, DMSO-*d*₆): δ 8.91 (s, 1H), 7.59 (d, *J* = 8.4 Hz, 2H), 7.46 (d, *J* = 8.4 Hz, 2H), 7.38 (d, *J* = 7.6 Hz, 1H), 7.29-7.23 (m, 2H), 7.05 (s, 1H), 6.98 (s, 1H), 6.90 (d, *J* = 9.2 Hz, 1H), 4.16 (d, *J* = 18.0 Hz, 1H), 4.04 (d, *J* = 18.0 Hz, 1H), 3.85-3.41 (m, 4H), 3.29-3.05 (m, 4H), 2.69-2.55 (m, 1H), 2.31 (s, 3H), 1.17 (d, *J* = 6.8 Hz, 3H), 1.08 (d, *J* = 6.8 Hz, 3H).

### Example 12: Synthesis of (E/Z)-1-(4-(4-(4-chlorobenzoyl)piperazin-1-yl)-2-fluorophenyl)-3-(3-(2-isopropyl-5-methylphenyl)-4-oxothiazolidin-2-ylidene)urea (compound I-21):

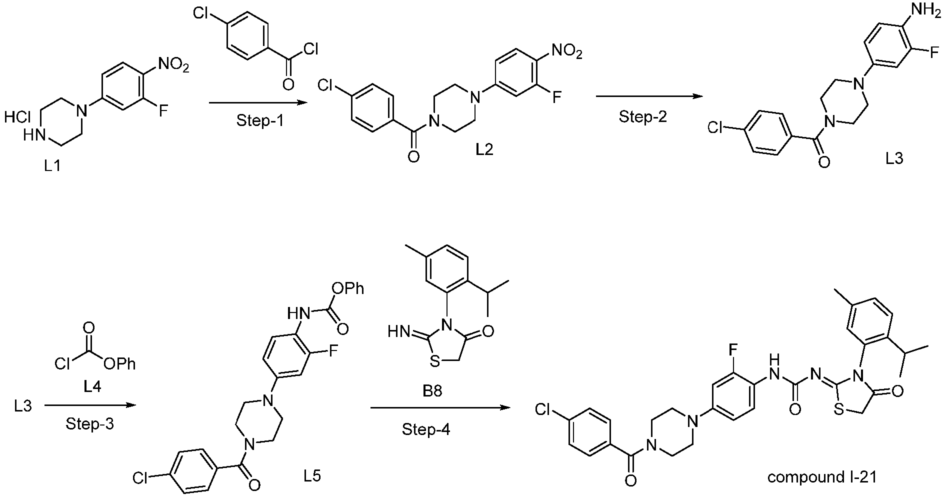

### Step 1: Synthesis of (4-chlorophenyl)(4-(3-fluoro-4-nitrophenyl)piperazin-1-yl)methanone (L2):

To a stirred solution of 1-(3-fluoro-4-nitrophenyl)piperazine hydrochloride (L1) (2.5 g, 9.554 mmol, 1 equiv.) in dichloromethane (25 mL) were added triethyl amine (3.973 mL, 28.661 mmol, 3.0 equiv.) and 4-chlorobenzoyl chloride (2.508 g, 14.330 mmol, 1.5 equiv.) at 0 °C. After that, the reaction mixture was stirred at room temperature for 3 hours. After completion of the reaction, the reaction mixture was quenched with water (100 mL) and extracted with dichloromethane (2 x 50 mL). The combined organic layer was washed with saturated brine solution (30 mL), dried over anhydrous sodium sulphate and concentrated to get the crude product. The crude product was washed with diethyl ether (20 mL) and dried to afford (4-chlorophenyl)(4-(3-fluoro-4-nitrophenyl)piperazin-1-yl)methanone (L2) (3.3 g, 95%) (¹H NMR complies).

### Step 2: Synthesis of (4-(4-amino-3-fluorophenyl)piperazin-1-yl)(4-chlorophenyl)methanone (L3):

To a stirred solution of (4-chlorophenyl)(4-(3-fluoro-4-nitrophenyl)piperazin-1-yl)methanone (L2) (3.3 g, 9.072 mmol, 1 equiv.) in ethanol (33 mL) and water (8.250 mL), ammonium chloride (4.852 g, 90.716 mmol, 10 equiv.) and iron powder (2.533 g, 45.358 mmol, 5 equiv.) were added at room temperature, after that, the reaction mixture was stirred at 80 °C for 16 hours. After completion of the reaction, the reaction mixture was filtered through celite bed. The filtrate was diluted with water (100 mL) and extracted with ethyl acetate (2 x 100 mL). The combined organic layers were washed with saturated brine solution (100 mL). Then the organic layer was separated, dried over anhydrous sodium sulphate and concentrated to get the crude product. The obtained crude product was triturated with diethyl ether (10 mL) and pentane (20 mL), stirred for 5 minutes at room temperature. After filtration, the obtained solid was dried to get (4-(4-amino-3-fluorophenyl)piperazin-1-yl)(4-chlorophenyl)methanone (L3) (2.4 g, 79%).
LC-MS (m/z): 334.66 [M+H]⁺ ion present

### Step 3: Synthesis of phenyl (4-(4-(4-chlorobenzoyl)piperazin-1-yl)-2-fluorophenyl)carbamate (L5):

To a stirred solution of (4-(4-amino-3-fluorophenyl)piperazin-1-yl)(4-chlorophenyl) methanone (L3) (2.4 g, 7.190 mmol, 1 equiv.) in dichloromethane (24 mL) was added pyridine (0.871 mL, 10.785 mmol, 1.5 equiv.) at room temperature. The resultant reaction mixture was cooled to 0 °C, then was added phenyl carbonochloridate (L4) (0.992 mL, 7.909 mmol, 1.1 equiv.) in dichloromethane (12 mL). Resultant reaction mixture was warm to room temperature and stirred for 2 hours (reaction monitored by TLC). After completion of the reaction, the reaction mass was concentrated. The obtained crude product was dissolved in ethyl acetate (200 mL), then organic layer was washed with 1N hydrochloric acid (50 mL), saturated sodium bicarbonate (100 mL) and saturated brine solution (100 mL). Then organic layer was dried over anhydrous sodium sulphate and concentrated to get the crude product. To the obtained crude product was added diethyl ether (10 mL) and n-pentane (10 mL) and stirred for 5 minutes at room temperature. Filtered the solid and dried to get phenyl (4-(4-(4-chlorobenzoyl)piperazin-1-yl)-2-fluorophenyl)carbamate (L5) (2.7 g, 83%). LC-MS (m/z): 454.66 [M+H]⁺ ion present

### Step 4: Synthesis of (E/Z)-1-(4-(4-(4-chlorobenzoyl)piperazin-1-yl)-2-fluorophenyl)-3-(3-(2-isopropyl-5-methylphenyl)-4-oxothiazolidin-2-ylidene)urea (compound I-21):

To a stirred solution of phenyl (4-(4-(4-chlorobenzoyl)piperazin-1-yl)-2-fluorophenyl) carbamate (L5) (0.7 g, 1.542 mmol, 1 equiv.) in tetrahydrofuran (14 mL) were added 2-imino-3-(2-isopropyl-5-methylphenyl)thiazolidin-4-one (B8) (0.460 g, 1.851 mmol, 1.2 equiv.) and N,N-diisopropylethylamine (0.806 mL, 4.627 mmol, 3 equiv.) at room temperature. The reaction mixture was heated to 65 °C and stirred at the same temperature for 16 hours. After completion of the reaction, the reaction mass poured into water (100 mL) and extracted with ethyl acetate (2 x 200 mL). The combined organic layer was washed with saturated brine solution (100 mL), then dried over anhydrous sodium sulphate and concentrated to get the crude product. To the obtained crude product was twice slurried with diethyl ether (20 mL x 2) and pentane (20 mL), stirred for 5 minutes at room temperature. Filtered the solid and dried to get (E/Z)-1-(4-(4-(4-chlorobenzoyl)piperazin-1-yl)-2-fluorophenyl)-3-(3-(2-isopropyl-5-methylphenyl)-4-oxothiazolidin-2-ylidene)urea (compound I-21) (0.37 g, 39.45%).

LC-MS (m/z): 608.37 [M+H]⁺ ion present; ¹H NMR (400 MHz, DMSO-*d*₆): δ 9.08 (s, 1H), 7.53 (d, *J* = 8.0 Hz, 2H), 7.47 (d, *J* = 8.4 Hz, 2H), 7.38 (d, *J* = 8.0 Hz, 1H), 7.29-7.21 (m, 2H), 7.04 (s, 1H), 6.78 (d, *J =* 13.6 Hz, 1H), 6.71 (d, *J =* 8.8 Hz, 1H), 4.16 (d, *J =* 18.0 Hz, 1H), 4.03 (d, *J =* 18.0 Hz, 1H), 3.78-3.41 (m, 4H), 3.27-3.05 (m, 4H), 2.69-2.60 (m, 1H), 2.30 (s, 3H), 1.16 (d, *J =* 6.8 Hz, 3H), 1.09 (d, *J* = 6.8 Hz, 3H).

### Example 13: Synthesis of (E/Z)-1-(2-fluoro-4-(4-(4-(trifluoromethoxy)benzoyl)piperazin-1-yl)phenyl)-3-(3-(2-isopropyl-5-methylphenyl)-4-oxothiazolidin-2-ylidene)urea (compound I-20):

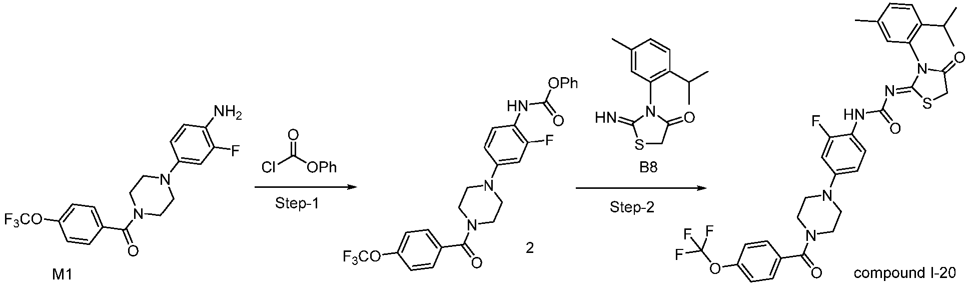

### Step 1: Synthesis of phenyl (2-fluoro-4-(4-(4-(trifluoromethoxy)benzoyl)piperazin-1-yl)phenyl)carbamate (M2):

To a stirred solution of (4-(4-amino-3-fluorophenyl) piperazin-1-yl)(4-(trifluoromethoxy)phenyl)methanone (M1) (1 g, 2.609 mmol, 1 equivin dichloromethane (10.0 mL) was added pyridine (0.316 mL, 3.913 mmol, 1.5 equiv.) at room temperature. The resultant reaction mixture was cooled to 0 °C, then was added phenyl carbonochloridate (0.449 g, 2.869 mmol, 1.1 equiv.) in dichloromethane (5 mL). The resultant reaction mixture was warmed to room temperature and stirred for 2 hours. After completion of the reaction, the mixture was concentrated in vacuum. The resulting crude product was dissolved in ethyl acetate (100 mL), then washed with 1N hydrochloric acid (50 mL), saturated sodium bicarbonate (30 mL) and saturated brine solution (50 mL). Then the organic layer was dried over anhydrous sodium sulphate and concentrated to get the crude product. To the obtained crude product was triturated with diethyl ether (10 mL) and n-pentane (10 mL) to get phenyl (2-fluoro-4-(4-(4-(trifluoromethoxy)benzoyl)piperazin-1-yl)phenyl)carbamate (M2) (1.1 g, 83.76%).

### Step 2: Synthesis (E/Z)-1-(2-fluoro-4-(4-(4-(trifluoromethoxy)benzoyl)piperazin-1-yl)phenyl)-3-(3-(2-isopropyl-5-methylphenyl)-4-oxothiazolidin-2-ylidene)urea (compound I-20):

To a stirred solution of phenyl (2-fluoro-4-(4-(4-(trifluoromethoxy) benzoyl) piperazin-1-yl)phenyl)carbamate (M2) (0.6 g, 1.192 mmol, 1.0 equiv.) in tetrahydrofuran (12 mL, 20 V) were added 2-imino-3-(2-isopropyl-5-methylphenyl)thiazolidin-4-one (B8) (0.355 g, 1.430 mmol, 1.2 equiv.) and N,N-diisopropylethylamine (0.623 mL, 3.575 mmol, 3 equiv.) at room temperature. The reaction mixture was heated to 65 °C and stirred at same temperature for 4 hours. After completion of the reaction, the reaction mixture was quenched with water (20 mL) and extracted with ethyl acetate (2x 20 mL). The combined organic layers were washed with brine (20 mL), dried over anhydrous sodium sulphate, filtered and concentrated to get the crude product. The crude product was triturated with mixture of diethyl ether (10 mL) and petrol ether (20 mL) to get (E/Z)-1-(2-fluoro-4-(4-(4-(trifluoromethoxy)benzoyl)piperazin-1-yl)phenyl)-3-(3-(2-isopropyl-5-methylphenyl)-4-oxothiazolidin-2-ylidene)urea (compound I-20) (0.455 g, 58%).

LC-MS (m/z): 658.35 [M+H]⁺ ion present; ¹H NMR (400 MHz, DMSO-*d*₆): δ 9.08 (s, 1H), 7.59 (d, *J* = 8.8 Hz, 2H), 7.46 (d, *J* = 8.4 Hz, 2H), 7.38 (d, *J* = 8.0 Hz, 1H), 7.25 (d, *J* = 9.2 Hz, 2H), 7.04 (s, 1H), 6.78 (d, *J =* 13.6 Hz, 1H), 6.71 (d, *J =* 8.8 Hz, 1H), 4.16 (d, *J =* 18.0 Hz, 1H), 4.03 (d, *J =* 18.0 Hz, 1H), 3.85-3.61 (m, 4H), 3.27-3.05 (m, 4H), 2.72-2.61 (m, 1H), 2.30 (s, 3H), 1.16 (d, *J* = 6.8 Hz, 3H), 1.09 (d, *J* = 6.8 Hz, 3H).

### Example 14: Synthesis of (E/Z)-1-(2-chloro-4-(4-(4-chlorobenzoyl)piperazin-1-yl)phenyl)-3-(3-(2-isopropyl-5-methylphenyl)-4-oxothiazolidin-2-ylidene)urea (compound I-18) :

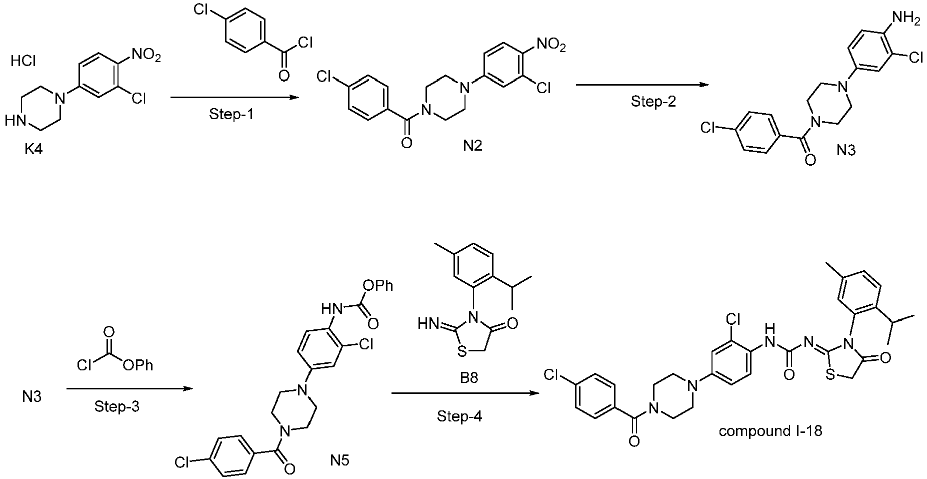

### Step 1: Synthesis of (4-(3-chloro-4-nitrophenyl) piperazin-1-yl)(4-chlorophenyl)methanone (N2):

To a stirred solution of 1-(3-chloro-4-nitrophenyl)piperazine hydrochloride (3.3 g, 11.865 mmol, 1.0 equiv. (K4) in dichloromethane (33 mL) were added triethyl amine (4.934 mL, 35.594 mmol, 3.0 equiv.) and 4-chlorobenzoyl chloride (3.115 g, 17.797 mmol, 1.5 equiv.) at 0 °C. The reaction mixture was stirred at room temperature for 3 hours. After completion of reaction, water (50 mL) was added and extracted with dichloromethane (2 x 30 mL). The combined organic layer was washed with saturated brine solution (50 mL). Then the organic layer was dried over anhydrous sodium sulphate and concentrated to get the crude product. The crude product was triturated with mixture of diethyl ether (10 mL) and n- pentane (20 mL) to get (4-(3-chloro-4-nitrophenyl) piperazin-1-yl)(4-chlorophenyl)methanone (N2) (4.2 g, 93%).
LC-MS (m/z): 380.40 [M+H]⁺ ion present.

### Step 2: Synthesis of (4-(4-amino-3-chlorophenyl)piperazin-1-yl)(4-chlorophenyl)methanone (N3):

To a stirred solution of (4-(3-chloro-4-nitrophenyl) piperazin-1-yl)(4-chlorophenyl) methanone (N2) (3 g, 7.890 mmol, 1 equiv.) in ethanol (22.50 mL, 7.5 V) water (7.50 mL, 2.5 V) were added ammonium chloride (1.266 g, 23.670 mmol, 3.0 equiv.) and iron powder (3.085 g, 55.230 mmol,, 7 equiv) at room temperature. The reaction mixture was stirred at 70°C for 2 hours. After completion of reaction, the reaction mixture was filtered through celite bed. The organic layer was diluted with water and extracted with ethyl acetate (2 x 50 mL). Combined organic layer was washed with saturated brine solution (20 mL). Then the organic layer was dried over anhydrous sodium sulphate and concentrated to get the crude product. The obtained crude product was slurried with mixture of diethyl ether (10 mL) and n-pentane (30 mL) to get (4-(4-amino-3-chlorophenyl)piperazin-1-yl)(4-chlorophenyl)methanone (N3) (2 g, 72%).
LC-MS (m/z): 350.20 [M+H]⁺ ion present

### Step 3: Synthesis of phenyl (2-chloro-4-(4-(4-chlorobenzoyl) piperazin-1-yl) phenyl) carbamate (N5):

To a stirred solution of (4-(4-amino-3-chlorophenyl)piperazin-1-yl)(4-chlorophenyl) methanone (N3) (2 g, 5.710 mmol, 1.0 equiv.) in dichloromethane (20 mL) was added pyridine (0.691 mL, 8.565 mmol, 1.5 equiv) at room temperature. The resultant reaction mixture was cooled to 0°C, then was added phenyl carbonochloridate (1.073 g, 6.852 mmol, 1.2 equiv.) in dichloromethane (10 mL). The reaction mixture was stirred at room temperature for 3 hours. The reaction mixture was quenched with 1N HCl solution in water (20 mL) and extracted with dichloromethane (2x 10 mL). The combined organic layers were washed with saturated aqueous NaHCO₃ solution (2 x 15 mL) and brine (20 mL), dried over anhydrous sodium sulphate, filtered and concentrated to get the crude product. The crude product was triturated with diethyl ether in (20 mL) to get phenyl (2-chloro-4-(4-(4-chlorobenzoyl) piperazin-1-yl) phenyl) carbamate (N5) (2 g, 74%).
LC-MS (m/z): 470.51 [M+H]⁺ ion present

### Step 4: Synthesis of (E/Z)-1-(2-chloro-4-(4-(4-chlorobenzoyl)piperazin-1-yl)phenyl)-3-(3-(2-isopropyl-5-methylphenyl)-4-oxothiazolidin-2-ylidene)urea (compound I-18):

To a stirred solution of phenyl (2-chloro-4-(4-(4-chlorobenzoyl)piperazin-1-yl)phenyl) carbamate (N5) (0.6 g, 1.276 mmol, 1.0 equiv.) in tetrahydrofuran (12.0 mL) were added 2-imino-3-(2-isopropyl-5-methylphenyl)thiazolidin-4-one (B8) (0.380 g, 1.531 mmol, 1.2 equiv.) and N,N-diisopropylethylamine (0.667 mL, 3.827 mmol, 3 equiv.) at room temperature. The reaction mixture was heated to 65 °C and stirred at the same temperature for 4 hours. After completion of the reaction, the reaction mixture was quenched with water (20 mL) and extracted with EtOAc (2 x 20 mL). The combined organic layers were washed with brine (20 mL), dried over anhydrous sodium sulphate, filtered and concentrated to get the crude product. The crude product was triturated with mixture of diethyl ether (10 mL) and petroleum ether (20 mL) to get (E/Z)-1-(2-chloro-4-(4-(4-chlorobenzoyl)piperazin-1-yl)phenyl)-3-(3-(2-isopropyl-5-methylphenyl)-4-oxothiazolidin-2-ylidene)urea (compound I-18) (0.310 g, 39%).

LC-MS (m/z): 624.27 [M+H]⁺ ion present; ¹H NMR (400 MHz, DMSO-*d*₆): δ 8.91 (s, 1H), 7.53 (d, *J* = 8.4 Hz, 2H), 7.47 (d, *J* = 8.4 Hz, 2H), 7.38 (d, *J* = 7.6 Hz, 1H), 7.32-7.21 (m, 2H), 7.05 (s, 1H), 6.99 (s, 1H), 6.90 (d, *J* = 8.8 Hz, 1H), 4.16 (d, *J* = 18.0 Hz, 1H), 4.04 (d, *J* = 17.6 Hz, 1H), 3.83-3.39 (m, 4H), 3.30-3.01 (m, 4H), 2.69-2.59 (m, 1H), 2.31 (s, 3H), 1.18 (d, *J* = 6.4 Hz, 3H), 1.08 (d, *J* = 6.8 Hz, 3H).

### Example 15: Synthesis of intermediate 2-imino-3-(2-isopropyl-5-methylphenyl)thiazolidin-4-one (B8)

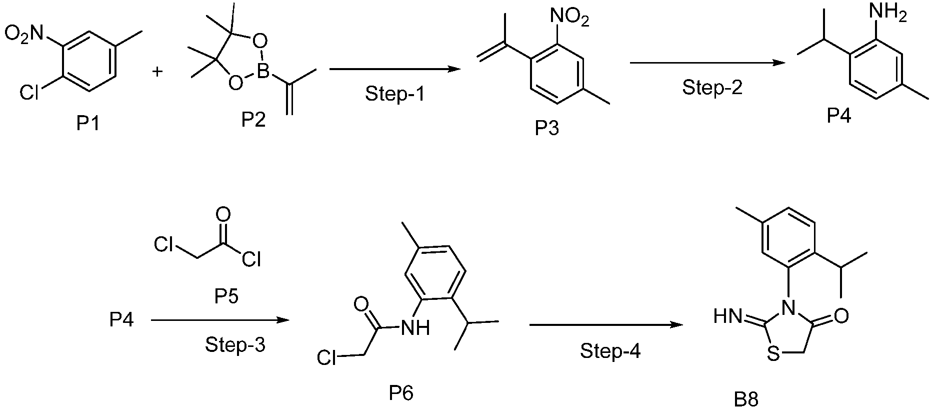

### Step-1: Synthesis of 4-methyl-2-nitro-1-(prop-1-en-2-yl)benzene (P3):

To a stirred solution of 1-chloro-4-methyl-2-nitrobenzene (P1) (50.0 g, 291.408 mmol, 1 equiv.) in 1,4-dioxane (500 mL, 10 V) were added sodium carbonate (92.659 g, 874.223 mmol, 3 equiv.), 4,4,5,5-tetramethyl-2-(prop-1-en-2-yl)-1,3,2-dioxaborolane (P2) (58.763 g, 349.689 mmol, 1.2 equiv.) and water (250 mL, 5 V) at room temperature. The reaction mixture was degassed with N2 atmosphere about 15 minutes. After that bis (triphenylphosphine) palladium (II) dichloride (10.227 g, 14.570 mmol, 0.05 equiv.) was added and again degassed with N2 atmosphere about 5 minutes. The reaction mass was heated to 80°C and stirred at same temperature for 16 hours. After completion of the reaction, the reaction mass was filtered through celite bed and washed with ethyl acetate (250 mL), added water (250 mL) to the filtrate and extracted with ethyl acetate (2 x 250 mL). The combined organic layer was washed with saturated NaCl solution (250 mL). The organic layer was dried over anhydrous MgSO₄, filtered and concentrated under reduced pressure to get the crude product. The crude product was purified by silica gel (230-400 mesh) column chromatography using 20% ethyl acetate in petroleum ether to afford a 4-methyl-2-nitro-1-(prop-1-en-2-yl)benzene (P3) (42 g, 81%). LC-MS (m/z): 178.11 [M+H]+ ion present

### Step-2: Synthesis of 2-isopropyl-5-methylaniline (P4):

To a stirred solution of 4-methyl-2-nitro-1-(prop-1-en-2-yl)benzene (P3) (42 g, 237.016 mmol, 1 equiv.) in ethyl acetate (420 mL) was charged 10% Palladium on activated carbon (50% wet) (8.4 g, 0.2 w/w) and degassed with H2 gas. The reaction mass was stirred at RT under hydrogen (80 psi) for 16 h. After completion of the reaction, the reaction mass was filtered through celite bed and washed with ethyl acetate (150 mL) concentrated under reduced pressure to get the crude product. The crude product was purified by silica gel (230-400 mesh) column chromatography using 20% ethyl acetate in petroleum ether to afford 2-isopropyl-5-methylaniline (P4) (30 g, 85%).
LC-MS (m/z): 150.41 [M+H]+ ion present

### Step-3: Synthesis of 2-chloro-N-(2-isopropyl-5-methylphenyl)acetamide (P6):

To a stirred solution of 2-isopropyl-5-methylaniline (P4) (20 g, 134.015 mmol, 1 equiv.) in dichloromethane (100 mL, 5 V) were added sodium bicarbonate (19.139 g, 227.826 mmol, 1.7 equiv.), water (100 mL, 5 V) at room temperature, then added 2-chloroacetyl chloride (P5) (10.7 mL, 134.015 mmol) in dichloromethane (Lot-2) (100 mL, 5 V) to the reaction mass at 0°C. The reaction mass was allowed to room temperature and stirred for 1 hour at same temperature. After completion of the reaction the reaction mass was quenched with water (200 mL) and extracted with DCM (2 x 200 mL). The combined organic layer was washed with saturated NaCl solution (200 mL). The organic layer was dried over anhydrous MgSO4, filtered and concentrated under reduced pressure to get the crude product of 2-chloro-N-(2-isopropyl-5-methylphenyl)acetamide (P6) (20 g, crude, 66%). LC-MS (m/z): 226.14 [M+H] + ion present

### Step-4: Synthesis of 2-imino-3-(2-isopropyl-5-methylphenyl)thiazolidin-4-one (E6):

To a stirred solution of 2-chloro-N-(2-isopropyl-5-methylphenyl) acetamide (6) (20 g, 88.607 mmol, and 1 equiv.) in acetone (200 mL, 10 V) was added potassium thiocyanate (17.222 g, 177.214 mmol, 2 equiv.) at room temperature. The reaction mixture was heated to 55°C and stirred at same temperature for 3 hours. Reaction mass was cooled to room temperature and added cesium carbonate (1.443 g, 4.430 mmol, 0.05 equiv.) and stirred for 30 minutes at same temperature. After completion of the reaction the reaction mass was diluted with water (200 mL) and extracted with ethyl acetate (2 x 200 mL). The combined organic layer was washed with saturated NaCl solution (200 mL). The organic layer was dried over anhydrous MgSO4, filtered and concentrated under reduced pressure to get the crude product. The crude product was purified by silica gel (230-400 mesh) column chromatography using 30% ethyl acetate in petroleum ether to afford 2-imino-3-(2-isopropyl-5-methylphenyl)thiazolidin-4-one (E6) (12.5 g, crude, 57% yield).

LC-MS (m/z): 249.17 [M+H] + ion present; ¹H NMR (400 MHz, DMSO-*d*₆): δ 9.20 (s, 1H), 7.32 (d, J = 8.0 Hz, 1H), 7.22 (d, J = 7.6 Hz, 1H), 6.88 (s, 1H), 4.26-4.13 (m, 2H), 2.67-2.62 (m, 1H), 2.28 (s, 3H), 1.10-1.06 (m, 6H).

### Example 16: Synthesis of intermediate N-(2-isopropyl-5-methylphenyl) hydrazine carbothioamide (E6)

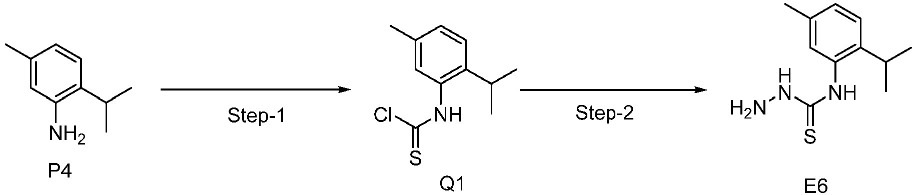

### Step-1: Synthesis of (2-isopropyl-5-methylphenyl)carbamothioic chloride (Q1):

To a stirred solution of 2-isopropyl-5-methylaniline (P4) (20 g, 134.015 mmol, 1 equiv.) in dichloromethan) (200 mL) were added sodium bicarbonate (19.140 g, 227.826 mmol, 1.7 equiv.), water (100 mL) at room temperature then added thiophosgene (10.273 mL, 134.015 mmol, 1 equiv.) in dichloromethane (100 mL) The reaction was stirred at 0°C for 5 minutes. After that, the mixture was allowed to reach room temperature and stirred for 1 hour at same temperature. After completion of the reaction, the reaction mass was quenched with water (200 mL) and extracted with DCM (2 x 150 mL). The combined organic layer was washed with saturated NaCl solution (100 mL). The organic layer was dried over anhydrous MgSO₄, filtered and concentrated under reduced pressure to get the crude product of (2-isopropyl-5-methylphenyl)carbamothioic chloride (Q1), this crude was used for next step (20 g crude, 66% yield). The mixture was used directly in the next step without further purification.

### Step-2: Synthesis of N-(2-isopropyl-5-methylphenyl) hydrazinecarbothioamide (E6):

To a stirred solution of (2-isopropyl-5-methylphenyl) carbamothioic chloride (1) (20 g, 87.816 mmol, 1 equiv.) in ethanol (200 mL) was added hydrazine hydrate, 55% (6.139 mL, 105.379 mmol, 1.2 equiv.) at room temperature. The reaction mass was stirred at same temperature for 16 hours. After completion of the reaction, added n-pentane (400 mL) to the reaction mass at room temperature stirred for 1 hour at same temperature and filtered the slurry mass and bed washed with n-pentane (100 mL). The solid compound was dried under vacuum at 40 °C for 1 hour to afford an N-(2-isopropyl-5-methylphenyl) hydrazinecarbothioamide (E6) (14.5 g, 72% yield).

LC-MS (m/z): 224.17 [M+H] + ion present; ¹H NMR (400 MHz, DMSO-*d*₆): δ 9.34-8.99 (m, 2H), 7.16 (d, J = 8.0 Hz, 2H), 7.01 (d, J = 8.0 Hz, 1H), 4.75 (brs, 2H), 3.05-2.98 (m, 1H), 2.25 (s, 3H), 1.13 (d, J = 7.2 Hz, 6H).

### Example 17: Synthesis of (E/Z)-2-(1-(2-chloro-4-(4-(3-chloro-4-fluorobenzoyl)piperazin-1-yl)phenyl)ethyl)-N-(3-(2-isopropyl-5-methylphenyl)-4-oxothiazolidin-2-ylidene)hydrazine-1-carboxamide (Compound I-29)

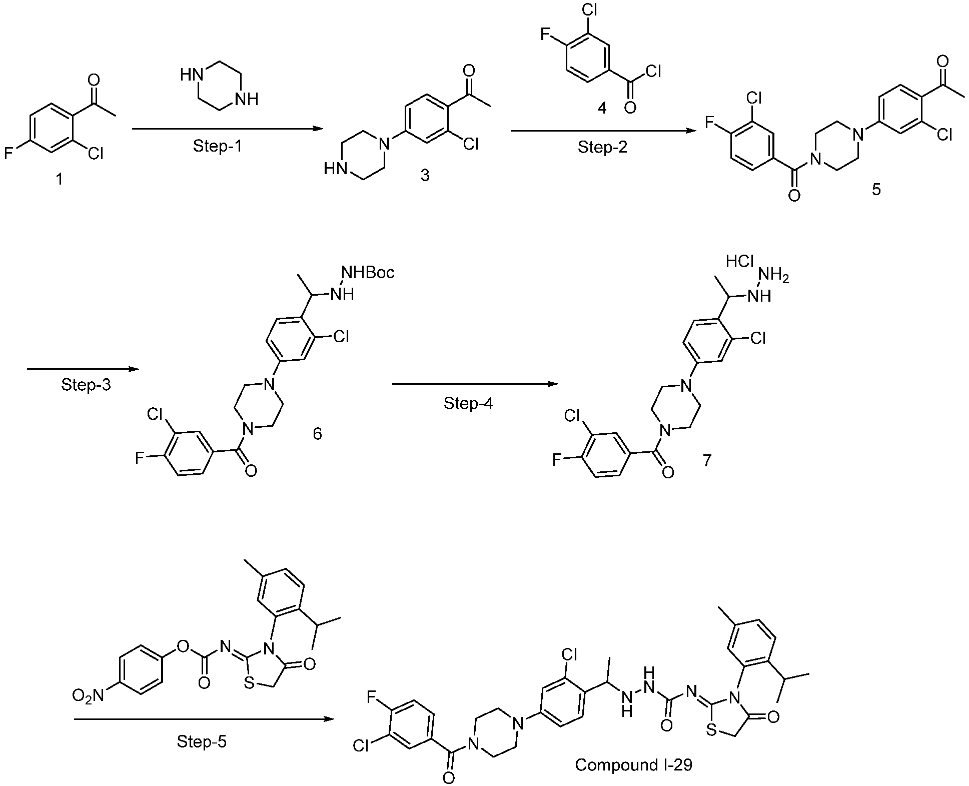

### Step 1: Synthesis of 1-(2-chloro-4-(piperazin-1-yl)phenyl)ethan-1-one:

To a stirred solution of 1-(2-chloro-4-fluorophenyl) ethan-1-one (1) (5 g, 28.972 mmol, 1 equiv.) in acetonitrile (150 mL) was added piperazine (2) (7.487 g, 86.915 mmol, 3 equiv.) at room temperature. After that, the reaction mixture was stirred at 70 °C for 16 hours. After completion of the reaction, the reaction mixture was quenched with water (200 mL) and extracted with ethyl acetate (2 x 200 mL). The combined organic layer was washed with saturated brine solution (300 mL), dried over anhydrous sodium sulphate and concentrated to get 1-(2-chloro-4-(piperazin-1-yl)phenyl)ethan-1-one (3) (6 g crude product), which was taken to next step without purification. LC-MS (m/z): 239.29 [M+H] + ion present

### Step 2: Synthesis of 1-(2-chloro-4-(4-(3-chloro-4-fluorobenzoyl)piperazin-1-yl)phenyl)ethan-1-one:

To a stirred solution of 1-(2-chloro-4-(piperazin-1-yl)phenyl)ethan-1-one (3) (6 g, 25.135 mmol, 1 equiv.) in dichloromethane (60 mL) were added triethylamine (10.510 mL, 75.404 mmol, 3 equiv.) and 3-chloro-4-fluorobenzoyl chloride (4) (7.276 g, 37.702 mmol, 1.5 equiv.) at 0 °C. The resultant reaction mixture was warm to room temperature and stirred at the same temperature for 3 hours. After completion of the reaction, the reaction mass was quenched with water (500 mL) and extracted with DCM (2 x 500 mL). The combined organic layer was washed with saturated brine solution (500 mL), dried over anhydrous sodium sulphate and concentrated under reduced pressure to get the crude product. The crude material was purified by column chromatography using 40% ethyl acetate in petroleum ether as an eluent to afford 1-(2-chloro-4-(4-(3-chloro-4-fluorobenzoyl)piperazin-1-yl)phenyl)ethan-1-one (5) (6 g, 60%). LC-MS (m/z): 395.61 [M+H] + ion present

### Step 3: Synthesis of tert-butyl 2-(1-(2-chloro-4-(4-(3-chloro-4-fluorobenzoyl)piperazin-1-yl)phenyl)ethyl)hydrazine-1-carboxylate:

To a solution of 1-(2-chloro-4-(4-(3-chloro-4-fluorobenzoyl)piperazin-1-yl)phenyl)ethan-1-one (5) (3 g, 7.590 mmol, 1 equiv.) in ethanol (30 mL) was added tert-butyl carbazate (1.204 g, 9.108 mmol, 1.2 equiv.). The reaction mixture was heated to 80 °C. After 6 hours, the reaction mixture was cooled to room temperature and acetic acid (1.367 g, 22.770 mmol, 3 equiv.) and sodium cyanoborohydride (2.862 g, 45.540 mmol, 6 equiv.) were added. Then the reaction mixture was stirred at 80 °C for 16 hours. The reaction mixture was quenched with saturated NaHCO₃ solution (100 mL) and extracted with EtOAc (2 x 100 mL). The combined organic layer was washed with brine solution (100 mL), dried over anhydrous Na₂SO₄ filtered and concentrated under vacuum to get the crude product. The crude material was purified by flash column chromatography using 60% ethyl acetate in petroleum ether as an eluent to afford tert-butyl 2-(1-(2-chloro-4-(4-(3-chloro-4-fluorobenzoyl)piperazin-1-yl)phenyl)ethyl)hydrazine-1-carboxylate (6) (3 g, 77%). LC-MS (m/z): 509.51 [M-H]⁻ ion present

### Step 4: Synthesis of (4-(3-chloro-4-(1-hydrazineylethyl)phenyl)piperazin-1-yl)(3-chloro-4-fluorophenyl)methanone hydrochloride:

To a stirred solution of tert-butyl 2-(1-(2-chloro-4-(4-(3-chloro-4-fluorobenzoyl)piperazin-1-yl)phenyl)ethyl)-2-methylhydrazine-1-carboxylate (6) (1 g, 1.903 mmol, 1 equiv.) in trifluoroethanol (15 mL) was added trimethylsilyl chloride (8 mL, 8 V) slowly at room temperature. The reaction mixture was stirred at room temperature for 1 hour (progress of the reaction was monitored by TLC). After completion of the reaction, (disappearance of Starting material in TLC), the reaction mass was concentrated at room temperature to get crude product, which was triturated with pentane (10 mL), filtered and dried to get (4-(3-chloro-4-(1-hydrazineylethyl)phenyl)piperazin-1-yl)(3-chloro-4-fluorophenyl)methanone hydrochloride (7) (0.8 g) as a white solid. The crude compound was carried to next step.

### Step-5: Synthesis of (E/Z)-2-(1-(2-chloro-4-(4-(3-chloro-4-fluorobenzoyl)piperazin-1-yl)phenyl)ethyl)-N-(3-(2-isopropyl-5-methylphenyl)-4-oxothiazolidin-2-ylidene)hydrazine-1-carboxamide (Compound I-29)

To a stirred solution of (4-(3-chloro-4-(1-hydrazineylethyl)phenyl)piperazin-1-yl)(3-chloro-4-fluorophenyl)methanone hydrochloride (7) (0.8 g, 1.945 mmol, 1 equiv.) and 4-nitrophenyl (E/Z)-(3-(2-isopropyl-5-methylphenyl)-4-oxothiazolidin-2-ylidene)carbamate (0.965 g, 2.334 mmol, 1.2 equiv.) in THF (16 mL) was added N,N-diisopropylethylamine (1.699 mL, 9.725 mmol, 5 equiv.). The reaction mixture was stirred at 70 °C for 2 hours. After completion of the reaction, reaction mass was concentrated to get crude product. The crude product was initially purified by silica gel (230-400) flash column chromatography using 70% ethyl acetate in petroleum ether as an eluent and re-purified by reverse phase preparative HPLC to afford (E/Z)-2-(1-(2-chloro-4-(4-(3-chloro-4-fluorobenzoyl)piperazin-1-yl)phenyl)ethyl)-N-(3-(2-isopropyl-5-methylphenyl)-4-oxothiazolidin-2-ylidene)hydrazine-1-carboxamide (0.062 g, 5%).

### Reverse phase preparative HPLC condition:

Column/dimensions: X BRIDGE C18 (19*250*5µ), Mobile phase A :0.1% FA IN WATER , Mobile phase B: ACN Gradient , (Time/%B): 0/40,1/40,9/70,15/70,15.1/100,20/100,20.1/40,22/40, Flow rate: 15ml/min, Solubility: Acetonitrile+THF+WATER
LC-MS (m/z): 683.20 [M-H]⁻ ion present.; ¹H NMR (400 MHz, DMSO-*d*₆): δ 8.92-8.76 (m, 1H), 7.70 (d, *J* = 7.2 Hz, 1H), 7.59-7.41 (m, 3H), 7.35-7.25 (m, 1H), 7.19 (d, *J =* 8.0 Hz, 1H), 6.94 (s, 1H), 6.91-6.83 (m, 2H), 4.88 (s, 1H), 4.49-4.35 (m, 1H), 4.17-3.95 (m, 2H), 3.81-3.38 (m, 4H), 3.27-3.04 (m, 4H), 2.61-2.52 (m, 1H), 2.29-2.18 (m, 3H), 1.19-0.95 (m, 9H).

### Example 17: Synthesis of intermediate (E/Z)-(3-(2-isopropyl-5-methylphenyl)-4-oxothiazolidin-2-ylidene)carbamate

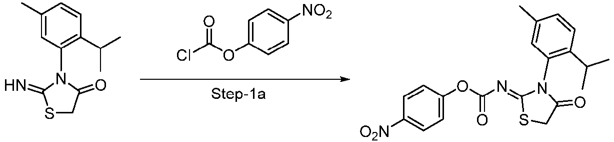

To a stirred solution of 2-imino-3-(2-isopropyl-5-methylphenyl)thiazolidin-4-one (0.5 g, 2.013 mmol, 1 equiv.) in acetonitrile (4 mL, 8 V) was added cesium carbonate (0.525 g, 1.611 mmol, 0.8 equiv.) at room temperature. After that 4-nitrophenyl carbonochloridate (0.406 g, 2.013 mmol, 1 equiv.) in acetonitrile (1 mL, 2 V) was added dropwise at room temperature. The resultant reaction mixture was stirred for 1 h at same temperature. After completion of the reaction, the reaction mass was diluted with water (5 mL) and extracted the product with ethyl acetate (2 X 10 mL). The combined organic layer was washed with saturated brine solution (5 mL), then dried over anhydrous sodium sulphate, filtered and concentrated to get crude product. Obtained crude product was washed with n-pentane (2 X 5 mL) and dried in rotatory evaporator at 40°C for 20 min to get 4-nitrophenyl (E/Z)-(3-(2-isopropyl-5-methylphenyl)-4-oxothiazolidin-2-ylidene)carbamate (810 mg, 97%). LC-MS (m/z): 414.10 [M+H]+ ion present

### Example 18: Synthesis of intermediate 2-imino-3-(5-methyl-2-((2,2,2-trifluoroethoxy)methyl)phenyl)thiazolidin-4-one:

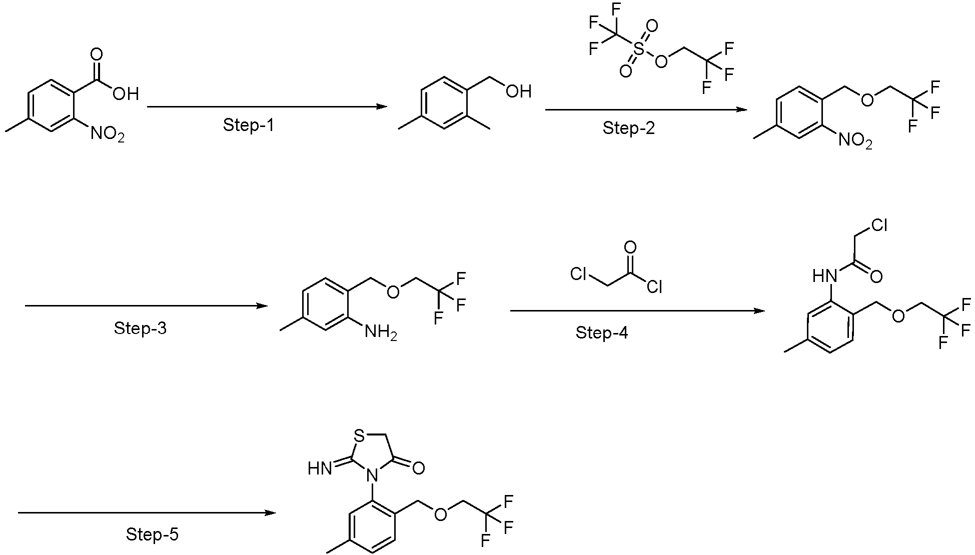

### Step 1: Synthesis of (4-methyl-2-nitrophenyl)methanol:

To a solution of 4-methyl-2-nitrobenzoic acid (20 g, 110.406 mmol, 1 equiv.) in tetrahydrofuran (200 mL) was added borane - tetrahydrofuran complex (1M in tetrahydrofuran) (154.568 mL, 154.568 mmol, 1.4 equiv.) at 0 °C, and the reaction mixture was stirred at 70°C for 4 h. Then cooled to room temperature and quenched slowly with saturated sodium bicarbonate solution (500 mL) and extracted with EtOAc (2 x 300 mL). The organic layer was washed with brine (200 mL) and dried over anhydrous sodium sulfate, filtered, and concentrated under reduced pressure to get the crude compound. The crude product was purified by column chromatography (silica gel 100-200 mesh) eluting with 20% EtOAc-petroleum ether to get (4-methyl-2-nitrophenyl)methanol (16 g, 87%).

### Step 2: Synthesis of 4-methyl-2-nitro-1-((2,2,2-trifluoroethoxy)methyl)benzene:

To a solution of (4-methyl-2-nitrophenyl)methanol (6 g, 35.893 mmol, 1 equiv.) in tetrahydrofuran (60 mL) was added sodium hydride, 60% dispersion in mineral oil (2.871 g, 71.787 mmol, 2 equiv.) portion-wise at 0°C, and the reaction mixture was stirred for 30 min. 2,2,2-Trifluoroethyl trifluoromethanesulfonate (16.662 g, 71.787 mmol, 2 equiv.) was added drop-wise, and the reaction mixture was stirred at room temperature for 2 h.. The reaction mixture was quenched with ice-cold water (300 mL) and extracted with ethyl acetate (2 x 300 mL). The organic layer was washed with brine (500 mL) and dried over anhydrous sodium sulfate, filtered, and concentrated under reduced pressure to get crude product (7.5 g), which was mixed with another 10 g batch and combinedly purified by column chromatography (silica gel 230-400 mesh) eluting with 3-4% EtOAc-petroleum ether to get 4-methyl-2-nitro-1-((2,2,2-trifluoroethoxy)methyl)benzene (5.4 g, 23%).

### Step 3: Synthesis of 5-methyl-2-((2,2,2-trifluoroethoxy)methyl)aniline:

To a stirred solution of 4-methyl-2-nitro-1-((2,2,2-trifluoroethoxy)methyl)benzene (5.4 g, 21.670 mmol, 1 equiv.) in ethanol (37.8 mL) and ethyl acetate (37.8 mL) was added palladium 10% on carbon (50% wet basis) (4.612 g) under nitrogen atmosphere. The heterogeneous mixture was placed under an atmosphere of hydrogen (15 psi) and stirred at room temperature for 16 h (reaction followed by TLC). After completion of reaction, the reaction mixture was filtered and concentrated onto celite bed and concentrated under reduced pressure to get crude product. The crude product was purified by column chromatography (silica gel 100-200 mesh) eluting with 6-7% EtOAc-petroleum ether to get 5-methyl-2-((2,2,2-trifluoroethoxy)methyl)aniline (4.3 g, 91%). LC-MS (m/z): 220.09 [M+H]⁺ ion present

### Step 4: Synthesis of 2-chloro-N-(5-methyl-2-((2,2,2-trifluoroethoxy)methyl)phenyl)acetamide:

To a solution of 5-methyl-2-((2,2,2-trifluoroethoxy)methyl)aniline (4.3 g, 19.616 mmol, 1 equiv.) in ethyl acetate (43 mL) were added sodium bicarbonate (4.120 g, 49.040 mmol, 2.5 equiv.) and 2-chloroacetyl chloride (1.899 mL, 23.539 mmol, 1.2 equiv.) drop-wise at 0°C, and the reaction mixture was stirred at room temperature for 3 h. The reaction mixture was quenched with water (100 mL) and was extracted with EtOAc (2 x 100 mL). The organic layer was dried over anhydrous Na₂SO₄, filtered and concentrated under reduced pressure to afford 2-chloro-N-(5-methyl-2-((2,2,2-trifluoroethoxy)methyl)phenyl)acetamide (5.8 g crude), which was used directly for next step. LC-MS (m/z): 296.63 [M+H]⁺ ion present

### Step 5: Synthesis of 2-imino-3-(5-methyl-2-((2,2,2-trifluoroethoxy)methyl)phenyl)thiazolidin-4-one:

To a stirred solution of 2-chloro-N-(5-methyl-2-((2,2,2-trifluoroethoxy)methyl)phenyl)acetamide (5.8 g, 19.615 mmol, 1 equiv.) in acetone (46.4 mL) was added potassium thiocyanate, 98% (3.81 g, 39.231 mmol, 2 equiv.). The reaction mixture was stirred at 55°C for 3 h. The reaction mixture was cooled to room temperature and cesium carbonate (1.278 g, 3.923 mmol, 0.2 equiv.) was added. Then, the reaction mixture was stirred at room temperature for 30 min. After completion of the reaction, the reaction mixture was filtered over a celite bed and concentrated under reduced pressure to get crude product. The crude product was purified by column chromatography (silica gel 100-200 mesh) using 35% ethyl acetated in petroleum ether to get product. The product was further re-purified by achiral SFC method. The clean fractions were concentrated to get 2-imino-3-(5-methyl-2-((2,2,2-trifluoroethoxy)methyl)phenyl)thiazolidin-4-one (2.8 g, 45%).

LC-MS (m/z): 319.27 [M+H]⁺ ion present; ¹H NMR (400 MHz, DMSO-*d*₆): δ 9.25 (s, 1H), 7.40 (d, *J =* 7.6 Hz, 1H), 7.29 (d, *J = 7.6* Hz, 1H), 7.03 (s, 1H), 4.46 (dd, *J* = 18.8, 12.8 Hz, 2H), 4.14 (s, 2H), 3.94 (q, *J =* 9.2 Hz, 2H), 2.33 (s, 3H).

### Example 19: Synthesis of intermediate (E/Z)-(3-(5-methyl-2-((2,2,2-trifluoroethoxy)methyl)phenyl)-4-oxothiazolidin-2-ylidene)carbamate

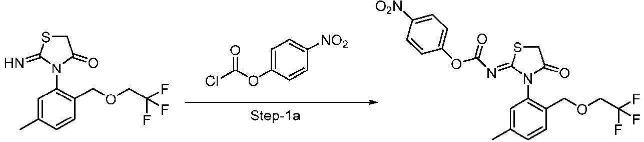

To a stirred solution of 2-imino-3-(5-methyl-2-((2,2,2-trifluoroethoxy)methyl)phenyl)thiazolidin-4-one (2 g, 6.283 mmol, 1.0 equiv.) in acetonitrile (16 mL) were added cesium carbonate (1.638 g, 5.026 mmol, 0.8 equiv.) and 4-nitrophenyl carbonochloridate (1.266 g, 6.283 mmol, 1.0 equiv.) solution in acetonitrile (4 mL) slowly at room temperature. The reaction mixture was stirred at same temperature for 1 h. The reaction mixture was diluted with water (20 mL) and extracted with ethyl acetate (2 x 30 mL). The combined organic layer was washed with saturated brine (10 mL), then dried over anhydrous sodium sulphate, filtered and concentrated to get the crude product. The crude product was washed with n-pentane (2 x 5 mL) to get 4-nitrophenyl (E/Z)-(3-(5-methyl-2-((2,2,2-trifluoroethoxy)methyl)phenyl)-4-oxothiazolidin-2-ylidene)carbamate (2.5 g, 82%). LC-MS (m/z): 484.65 [M+H] ⁺ ion present

### Example 20: Synthesis of (E/Z)-2-(1-(2-chloro-4-(4-(4-(trifluoromethoxy)benzoyl)piperazin-1-yl)phenyl)ethyl)-N-(3-(2-isopropyl-5-methylphenyl)-4-oxothiazolidin-2-ylidene)-2-methylhydrazine-1-carboxamide (Compound I-32)

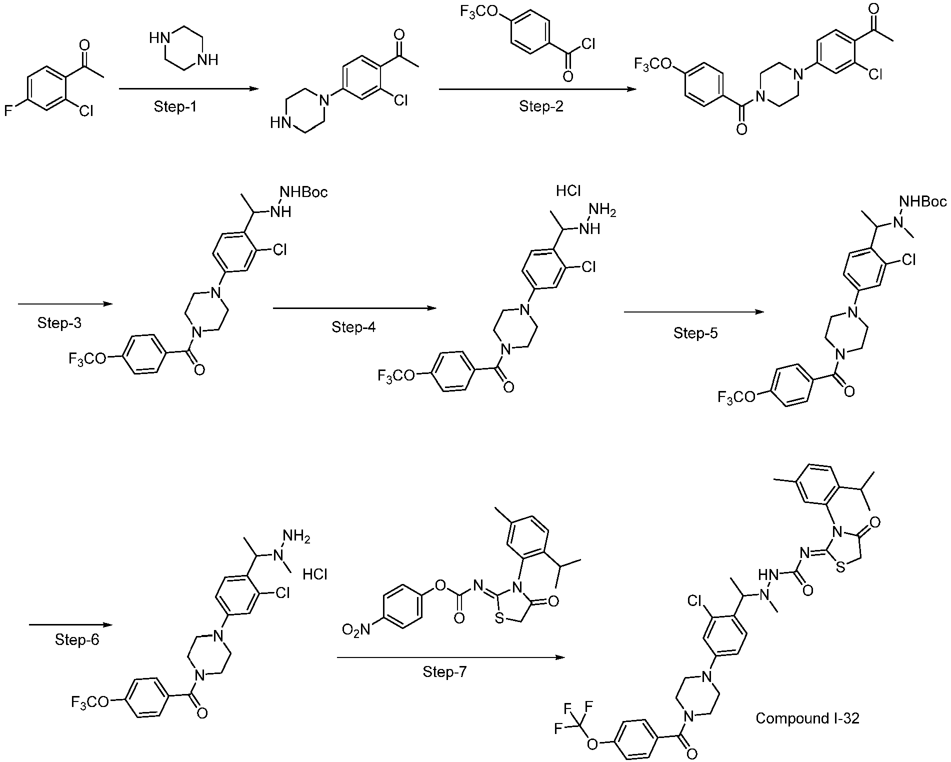

### Step 1: Synthesis of 1-(2-chloro-4-(piperazin-1-yl)phenyl)ethan-1-one

To a stirred solution of 1-(2-chloro-4-fluorophenyl)ethan-1-one (2 g, 11.589 mmol, 1 equiv.) in acetonitrile (60 mL, 30 V) was added piperazine (2) (2.995 g, 34.766 mmol, 3 equiv.) at room temperature. The resultant reaction mixture was stirred at 70°C for 20 h. After completion of reaction, the reaction mixture was quenched with water (100 mL) and extracted with dichloromethane (2 x 50 mL). The combined organic layer was washed with saturated brine solution (30 mL). Then the organic layer was dried over anhydrous sodium sulphate and concentrated to get the crude product. To the obtained crude product was added diethyl ether (20 mL) and stirred for 10 minutes. After filtration the solid was washed with diethyl ether (10 mL) and the obtained solid was dried in rotatory evaporator at 45°C for 10 minutes to get 1-(2-chloro-4-(piperazin-1-yl)phenyl)ethan-1-one (1.2 g, 43%). LC-MS (m/z): 239.42 [M+H]⁺ ion present

### Step 2: Synthesis of 1-(2-chloro-4-(4-(4-(trifluoromethoxy)benzoyl)piperazin-1-yl)phenyl)ethan-1-one

To a stirred solution of 1-(2-chloro-4-(piperazin-1-yl)phenyl)ethan-1-one (1.2 g, 5.027 mmol, 1 equiv.) and triethylamine (2.06 mL, 15.081 mmol, 3 equiv.) in dichloromethane (lot-1) (9.6 mL) at 0 °C was added 4-(trifluoromethoxy)benzoyl chloride (1.693 g, 7.540 mmol, 1.5 equiv.) in dichloromethane (2.4 mL). The resultant reaction mixture was warmed to room temperature and stirred for 3 h. After completion of reaction, the resulting reaction mass was concentrated. To the obtained crude product was added water (100 mL) and extracted with ethyl acetate (2 x 100 mL). Combined organic layers were washed with saturated sodium bicarbonate (50 mL) and saturated brine solution (50 mL). Then dried over anhydrous sodium sulphate and concentrated to get the crude product. Obtained crude product was purified by column chromatography using 30% ethyl acetate in petroleum ether as a eluent to get 1-(2-chloro-4-(4-(4-(trifluoromethoxy)benzoyl)piperazin-1-yl)phenyl)ethan-1-one (1.5 g, 70%).
LC-MS (m/z): 427.60 [M+H]⁺ ion present

### Step 3: Synthesis of tert-butyl 2-(1-(2-chloro-4-(4-(4-(trifluoromethoxy)benzoyl)piperazin-1-yl)phenyl)ethyl)hydrazine-1-carboxylate

To a stirred solution of 1-(2-chloro-4-(4-(4-(trifluoromethoxy)benzoyl)piperazin-1-yl)phenyl)ethan-1-one (1.5 g, 3.514 mmol, 1 equiv.) in ethanol (15 mL, 10 V) was added tert-butyl carbazate (1.393 g, 10.543 mmol, 3 equiv.). The resulting mixture was heated at 80 °C and stirred at same temperature for 3 hours. After that, the reaction mixture was cooled to room temperature and added acetic acid (0.609 mL, 10.543 mmol, 3 equiv.) followed by sodium cyanoborohydride (1.546 g, 24.601 mmol, 7 equiv.) at room temperature, the resulting mixture was heated to 80 °C and stirred for 16 hours. After completion of reaction, the reaction mixture was quenched with water (200 mL) and extracted with ethyl acetate (2 x 200 mL). The combined organic layer was washed with saturated brine solution (300 mL). Then the organic layer was dried over anhydrous sodium sulphate and concentrated to get the crude product. Obtained crude product was purified by column chromatography using 30% ethyl acetate in petroleum ether as a eluent to get tert-butyl 2-(1-(2-chloro-4-(4-(4-(trifluoromethoxy)benzoyl)piperazin-1-yl)phenyl)ethyl)hydrazine-1-carboxylate (1.6 g, 84%).

### Step 4: Synthesis of (4-(3-chloro-4-(1-hydrazineylethyl)phenyl)piperazin-1-yl)(4-(trifluoromethoxy)phenyl)methanone hydrochloride

To stirred solution of tert-butyl 2-(1-(2-chloro-4-(4-(4-(trifluoromethoxy)benzoyl)piperazin-1-yl)phenyl)ethyl)hydrazine-1-carboxylate (1 g, 1.842 mmol, 1 equiv.) in 2,2,2-trifluoroethanol (20 mL, 20 V) was added trimethylsilyl chloride (4 mL, 4 V) at room temperature. The reaction mixture was stirred at room temperature for 1 h. After completion of the reaction, the reaction mixture was concentrated to get the crude product. To the obtained crude product was added 30% methyl tert-butyl ether in petroleum ether (~10 mL) and stirred for 10 minutes a room temperature. Filtered the solid and solid was washed with 30% methyl tert-butyl ether in petroleum ether (~5 mL). Obtained solid was dried in rotatory evaporator at 45°C for 10 min to get (4-(3-chloro-4-(1-hydrazineylethyl)phenyl)piperazin-1-yl)(4-(trifluoromethoxy)phenyl)methanone hydrochloride (0.580 g crude) as white solid. Crude product was taken for next step without purification.

### Step 5: Synthesis of 2-(1-(2-chloro-4-(4-(4-(trifluoromethoxy)benzoyl)piperazin-1-yl)phenyl)ethyl)-2-methylhydrazine-1-carboxylate:

To a stirred solution of tert-butyl 2-(1-(2-chloro-4-(4-(4-(trifluoromethoxy)benzoyl)piperazin-1-yl)phenyl)ethyl)hydrazine-1-carboxylate (2.4 g, 4.420 mmol, 1 equiv) in methanol (24 mL, 10 V) were added 37% formaldehyde solution in water (1.076 mL, 13.260 mmol, 3 equiv.) and acetic acid (0.510 mL, 8.840 mmol, 2 equiv.) at room temperature. The resultant reaction mixture was stirred at the same temperature for 60 min. After that, sodium cyanoborohydride (1.111 g, 17.680 mmol, 4 equiv.) was added at 0°C. Then the resultant reaction mixture was warmed to room temperature and stirred for 4 h. After completion of the reaction, the reaction mixture was quenched with water (14 mL) and extracted with ethyl acetate (2 x 28 mL). The combined organic layer was washed with water (2 X 14 mL), brine (14 mL), dried over anhydrous sodium sulphate, filtered and concentrated under reduced pressure to obtained the crude product. The crude product was purified by column chromatography (silica gel 230-400 mesh) eluting with 30% EtOAc in petroleum ether to get tert-butyl 2-(1-(2-chloro-4-(4-(4-(trifluoromethoxy)benzoyl)piperazin-1-yl)phenyl)ethyl)-2-methylhydrazine-1-carboxylate (2 g).

### Step 6: Synthesis of (4-(3-chloro-4-(1-(1-methylhydrazineyl)ethyl)phenyl)piperazin-1-yl)(4-(trifluoromethoxy)phenyl)-methanone hydrochloride:

To stirred solution of tert-butyl 2-(1-(2-chloro-4-(4-(4-(trifluoromethoxy)benzoyl)piperazin-1-yl)phenyl)ethyl)-2-methylhydrazine-1-carboxylate (1.3 g, 2.33 mmol, 1 equiv.) in 2,2,2-trifluoroethanol (26 mL, 20 V) was added chlorotrimethylsilane (5.2 mL, 4 V) at 5 to 10 °C. The reaction mixture was stirred at same temperature for 4 h. The reaction mixture was concentrated under vacuum at room temperature to get the crude product. The crude product was triturated with MTBE (10 mL) to get (4-(3-chloro-4-(1-(1-methylhydrazineyl)ethyl)phenyl)piperazin-1-yl)(4-(trifluoromethoxy)phenyl)methanone hydrochloride (1.15 g, crude). LC-MS (m/z): 457.76 [M-HCl+H]⁺ ion present.

### Step 7: Synthesis of (E/Z)-2-(1-(2-chloro-4-(4-(4-(trifluoromethoxy)benzoyl)piperazin-1-yl)phenyl)ethyl)-N-(3-(2-isopropyl-5-methylphenyl)-4-oxothiazolidin-2-ylidene)-2-methylhydrazine-1-carboxamide (Compound I-32):

To a stirred solution of (4-(3-chloro-4-(1-(1-methylhydrazineyl)ethyl)phenyl)piperazin-1-yl)(4-(trifluoromethoxy)phenyl)methanone hydrochloride (1.15 g, 2.331 mmol, 1.0 equiv.) and 4-nitrophenyl (E/Z)-(3-(2-isopropyl-5-methylphenyl)-4-oxothiazolidin-2-ylidene)carbamate (1.156 g, 2.797 mmol, 1.2 equiv.) in tetrahydrofuran (23 mL) was added *N,N-*diisopropylethylamine (2.022 mL, 11.655 mmol, 5 equiv.) at room temperature. The reaction mixture was stirred at 70 °C for 2 h. The reaction mixture was diluted with water (20 mL) and extracted with ethyl acetate (2x 20 mL). The combined organic layers were washed with brine (10 mL), dried over anhydrous sodium sulphate, filtered and concentrated to get the crude product. The crude product was purified by silica gel chromatography using 30% of ethyl acetate in petroleum ether and further by re-purified with reverse phase preparative HPLC to get (E/Z)-2-(1-(2-chloro-4-(4-(4-(trifluoromethoxy)benzoyl)piperazin-1-yl)phenyl)ethyl)-N-(3-(2-isopropyl-5-methylphenyl)-4-oxothiazolidin-2-ylidene)-2-methylhydrazine-1-carboxamide (0.115 g, 7%).

### Reverse phase preparative HPLC conditions:

Column/dimensions: X BRIDGE C18 (19*150*5um); Mobile phase A: 10mM ABC IN WATER; Mobile phase B: ACN (org); Gradient (Time/%B): 0/50,1/50,9/75,13/75,13.1/100,15/100,15.1/50,18/50; Flow rate: 16ml/min; Solubility: THF+WATER+ACN.
LC-MS (m/z): 729.27 [M-H]⁻ ion present; ¹H NMR (400 MHz, DMSO-*d*₆): δ 7.59 (d, *J =* 8.4 Hz, 2H), 7.49-7.33 (m, 3H), 7.32-6.80 (m, 5H), 5.44-4.01 (m, 4H), 3.87-3.39 (m, 4H), 3.25-3.05 (m, 4H), 2.76-2.55 (m, 4H), 2.26 (s, 3H), 1.20-0.98 (m, 7H), 0.58-0.43 (m, 2H).

### Example 21: of (E/Z)-1-(5-(4-(3-chloro-4-fluorobenzoyl) piperazin-1-yl) pyrazin-2-yl)-3-(3-(2-isopropyl-5-methylphenyl)-4-oxothiazolidin-2-ylidene) urea (Compound I-31)

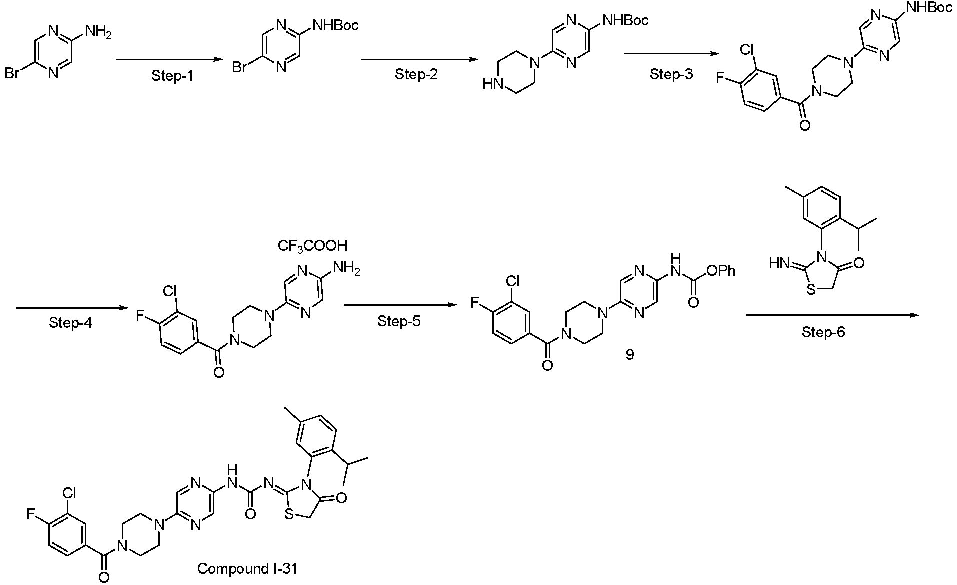

### Step 1: Synthesis of tert-butyl (5-bromopyrazin-2-yl)carbamate

To a stirred solution of 5-bromopyrazin-2-amine (1) (5 g, 28.735 mmol, 1 equiv.) in dichloromethane (80 mL, 16 V) were added di-tert-butyl dicarbonate (6.272 g, 28.735 mmol, 1 equiv.) and 4-dimethylaminopyridine (0.351 g, 2.874 mmol, 0.1 equiv.) at room temperature. The reaction mass was stirred at the same temperature for 16 hours. After completion of the reaction, the reaction mixture was quenched with water (100 mL) and extracted with ethyl acetate (2 x 50 mL). The combined organic layers were washed with brine (100 mL), dried over anhydrous sodium sulphate, filtered and concentrated to get the crude product. The crude product was purified by silica gel (230-400 mesh) column chromatography using 20% ethyl acetate in petroleum ether to get tert-butyl (5-bromopyrazin-2-yl)carbamate (3 g, 38%). LC-MS (m/z): 274.41 [M+H]⁺ ion present

### Step 2: Synthesis of tert-butyl (5-(piperazin-1-yl) pyrazin-2-yl) carbamate

A mixture of tert-butyl (5-bromopyrazin-2-yl) carbamate (1 g, 3.648 mmol, 1 equiv) and piperazine (3.142 g, 36.482 mmol, 10 equiv) was heated to 90°C and stirred for 16 h at the same temperature. After completion of the reaction, the mixture was cooled to room temperature, quenched with water (100 mL) and extracted with ethyl acetate (2 x 50 mL). The combined organic layers were washed with brine (100 mL), dried over anhydrous sodium sulphate, filtered and concentrated to get the crude product. The crude product was purified by silica gel (230-400 mesh) column chromatography using 70% ethyl acetate in petroleum ether to get tert-butyl (5-(piperazin-1-yl) pyrazin-2-yl) carbamate (0.45 g, 64%). LC-MS (m/z): 280.73 [M+H]⁺ ion present

### Step 3: Synthesis of tert-butyl (5-(4-(3-chloro-4-fluorobenzoyl) piperazin-1-yl) pyrazin-2-yl) carbamate

To a stirred solution of tert-butyl (5-(piperazin-1-yl) pyrazin-2-yl)carbamate (0.45 g, 1.611 mmol, 1 equiv) in dichloromethane (4.5 mL, 10 V) were added 3-chloro-4-fluorobenzoyl chloride (0.342 g, 1.772 mmol, 1.1 equiv) and pyridine (0.325 mL, 4.027 mmol, 2.5 equiv) at room temperature. The reaction mass was stirred for 3 h at the same temperature. After completion of the reaction, the reaction mass was quenched with 1N hydrogen chloride solution in water (20 mL) and extracted with dichloromethane (2 x 10 mL). The combined organic layers were washed with saturated aqueous NaHCO₃ solution (2 x 10 mL) and brine (10 mL), dried over anhydrous sodium sulphate, filtered and concentrated to get the crude product. The crude product was purified by silica gel (230-400 mesh) column chromatography using 30% ethyl acetate in petroleum ether to get tert-butyl (5-(4-(3-chloro-4-fluorobenzoyl) piperazin-1-yl) pyrazin-2-yl) carbamate (0.3 g, 43%). LC-MS (m/z): 434.71 [M-H]⁻ ion present

### Step 4: Synthesis of (4-(5-aminopyrazin-2-yl) piperazin-1-yl) (3-chloro-4-fluorophenyl) methanone TFA salt

To a stirred solution of tert-butyl (5-(4-(3-chloro-4-fluorobenzoyl) piperazin-1-yl) pyrazin-2-yl) carbamate (0.3 g, 0.668 mmol, 1 equiv) in dichloromethane (6 mL, 20 V) was added trifluoroacetic acid (1.2 mL, 4 V) at 0 °C. The resulting reaction mixture was stirred at room temperature for 3 h. After completion of the reaction, the reaction mixture was concentrated under reduced pressure to get the crude product. Obtained crude product was triturated with n-pentane (30 mL) to get (4-(5-aminopyrazin-2-yl) piperazin-1-yl) (3-chloro-4-fluorophenyl) methanone TFA salt (0.250 g). LC-MS (m/z): 336.68 [M-TFA+H] ⁺ ion present

### Step 5: Synthesis of phenyl (5-(4-(3-chloro-4-fluorobenzoyl) piperazin-1-yl) pyrazin-2-yl) carbamate

To a stirred solution of (4-(5-aminopyrazin-2-yl) piperazin-1-yl) (3-chloro-4-fluorophenyl)methanone TFA salt (0.250 g, 0.556 mmol, 1 equiv) in dichloromethane (5 mL, 20 V) was added pyridine (0.3 mL, 2.78 mmol, 5 equiv.) at room temperature. Then the reaction mass was cooled to 0°C, after that was added phenyl chloroformate (0.350 g, 1.667 mmol, 3 equiv). The resulting reaction mixture was stirred at room temperature for 1 h. After completion of the reaction, quenched with 1N hydrogen chloride solution in water (20 mL) and extracted with dichloromethane (2 x 10 mL). The combined organic layers were washed with saturated aqueous NaHCO₃ solution (2 x 10 mL) and brine (10 mL), dried over anhydrous sodium sulphate, filtered and concentrated to get the crude product. The crude compound was triturated with n-pentane (10 mL) to get phenyl (5-(4-(3-chloro-4-fluorobenzoyl) piperazin-1-yl) pyrazin-2-yl) carbamate (0.339 g, 88%). LC-MS (m/z): 456.68 [M+H]⁺ ion present

### Step 6: Synthesis of (E/Z)-1-(5-(4-(3-chloro-4-fluorobenzoyl) piperazin-1-yl) pyrazin-2-yl)-3-(3-(2-isopropyl-5-methylphenyl)-4-oxothiazolidin-2-ylidene) urea (Compound I-31)

To a stirred solution of phenyl (5-(4-(3-chloro-4-fluorobenzoyl) piperazin-1-yl) pyrazin-2-yl) carbamate (9) (0.300 g, 0.658 mmol, 1 equiv.) in tetrahydrofuran (6 mL, 20 V) were added 2-imino-3-(2-isopropyl-5-methylphenyl) thiazolidin-4-one (ES5103-2) (0.196 g, 0.790 mmol, 1.2 equiv) (synthesized following the protocol adapted for ES5103-2) and *N,N-*diisopropylethylamine (0.255 g, 1.974 mmol, 3 equiv) at room temperature. The reaction mixture was heated to 65°C and stirred at the same temperature for 16 h. After completion of the reaction, the reaction mixture was quenched with water (20 mL) and extracted with ethyl acetate (2 x 20 mL). The combined organic layers were washed with brine (20 mL), dried over anhydrous sodium sulphate, filtered and concentrated to get the crude product. The crude product was purified by reverse phase preparative HPLC to get (E/Z)-1-(5-(4-(3-chloro-4-fluorobenzoyl) piperazin-1-yl) pyrazin-2-yl)-3-(3-(2-isopropyl-5-methylphenyl)-4-oxothiazolidin-2-ylidene) urea (210 mg, 52%).

### Reverse phase preparative HPLC method:

Column/dimensions: SUNFIRE C18(19*150) 5um Mobile phase A: 0.1% FA in water (aq) Mobile phase B: Acetonitrile (org) Gradient (Time/%B): 0/40,1/40,8/60,13/60,13.1/100,15/100,15.1/40,17/40. Flow rate: 16ml/min Solubility: Acetonitrile + THF+ WATER
LC-MS (m/z): 610.35 [M+H]⁺ ion present; ¹H NMR (400 MHz, DMSO-d₆): δ 9.94 (s, 1H), 8.59 (s, 1H), 8.04 (s, 1H), 7.71 (dd, *J* = 7.2 & 1.6 Hz, 1H), 7.57-7.44 (m, 2H), 7.36 (d, *J* = 8.0 Hz, 1H), 7.25 (d, *J* = 8.0 Hz, 1H), 7.02 (s, 1H), 4.21 (d, *J =* 18.0 Hz, 1H), 4.07 (d, *J =* 18.0 Hz, 1H), 3.78-3.39 (m, 8H), 2.67-2.58 (m, 1H), 2.30 (s, 3H), 1.19-0.95 (m, 6H).

### Example 22: Synthesis of (E/Z)-1-(2-chloro-4-(4-(3,4-dichlorobenzoyl) piperazin-1-yl) phenyl)-3-(3-(5-(dimethylamino)-2-isopropylphenyl)-4-oxothiazolidin-2-ylidene) urea (Compound I-35)

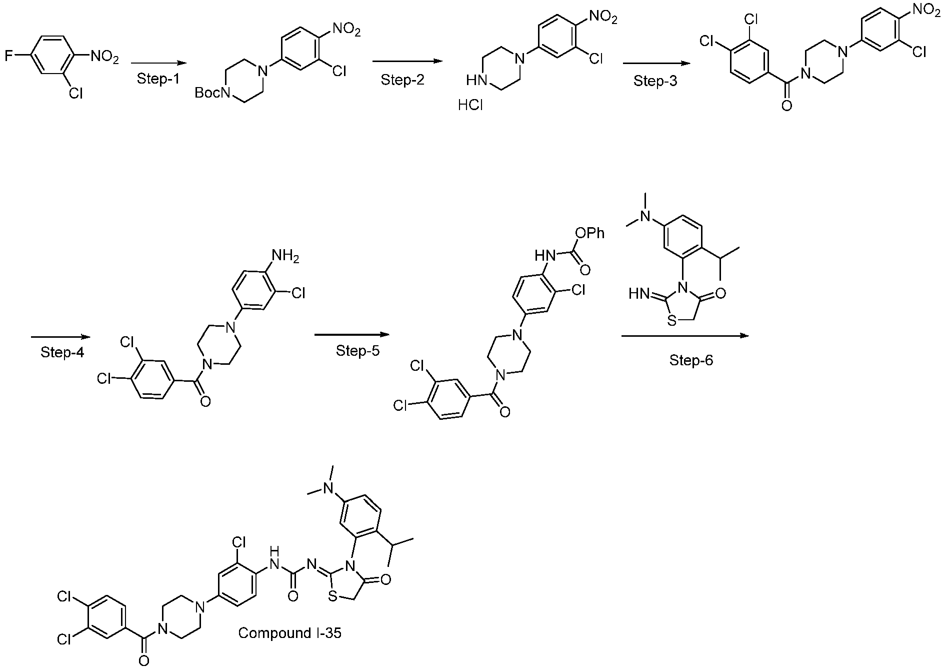

### Step 1: Synthesis of tert-butyl 4-(3-chloro-4-nitrophenyl) piperazine-1-carboxylate

To a stirred solution of 2-chloro-4-fluoro-1-nitrobenzene (5 g, 28.483 mmol, 1 equiv) in acetonitrile (125 mL, 25 V) were added potassium carbonate (9.841 g, 71.208 mmol, 2.5 equiv) and tert-butyl piperazine-1-carboxylate (5.836 g, 31.331 mmol, 1.1 equiv) at room temperature. The reaction mass was heated to 80°C and stirred at the same temperature for 16 h. After completion of the reaction, the reaction mixture was quenched with water (100 mL) and extracted with ethyl acetate (2 x 100 mL). The combined organic layers were washed with brine (100 mL), dried over anhydrous sodium sulphate, filtered and concentrated to get the crude product. The crude product was triturated with n-pentane (150 mL) to get tert-butyl 4-(3-chloro-4-nitrophenyl) piperazine-1-carboxylate (3.5 g, 36%).
LC-MS (m/z): 286.66 [M-tBu+H] ⁺ ion present

### Step 2: Synthesis of 1-(3-chloro-4-nitrophenyl) piperazine hydrochloride

To a stirred solution of tert-butyl 4-(3-chloro-4-nitrophenyl) piperazine-1-carboxylate (3.5 g, 10.240 mmol, 1 equiv) in 1,4-dioxane (17.50 mL, 5 V) was added 4M HCl in dioxane (35 mL, 10 V) at room temperature. The resulting reaction mixture was stirred at the same temperature for 6 hours. After completion of the reaction, the reaction mixture was concentrated under reduced pressure to get the crude product. The obtained crude product was triturated with n-pentane (150 mL) to get 1-(3-chloro-4-nitrophenyl) piperazine hydrochloride (3 g).
LC-MS (m/z): 242.35 [M+H-HCl]⁺ ion present

Step 3: Synthesis of (4-(3-chloro-4-nitrophenyl) piperazin-1-yl) (3,4-dichlorophenyl) methanone

To a stirred solution of 1-(3-chloro-4-nitrophenyl) piperazine hydrochloride (3 g, 10.786 mmol, 1 equiv) in dichloromethane (30 mL, 10 V) were added 3,4-dichlorobenzoyl chloride (3.389 g, 16.179 mmol, 1.5 equiv) and pyridine (2.612 mL, 32.359 mmol, 3 equiv) at room temperature. The reaction mass was stirred for 3 h at the same temperature. After completion of the reaction, the reaction mass was quenched with 1N hydrogen chloride solution in water (50 mL) and extracted with dichloromethane (2 x 50 mL). The combined organic layers were washed with saturated aqueous NaHCO₃ solution (2 x 50 mL) and brine (50 mL), dried over anhydrous sodium sulphate, filtered and concentrated to get the crude product. The crude product was triturated with n-pentane (100 mL) to get (4-(3-chloro-4-nitrophenyl) piperazin-1-yl) (3,4-dichlorophenyl) methanone (4 g, 89%). LC-MS (m/z): 414.58 [M+H]⁺ ion present

### Step 4: Synthesis of (4-(4-amino-3-chlorophenyl) piperazin-1-yl) (3,4-dichlorophenyl) methanone

To a stirred solution of (4-(3-chloro-4-nitrophenyl) piperazin-1-yl) (3,4-dichlorophenyl) methanone (4 g, 9.646 mmol, 1 equiv) in ethanol (32 mL, 8 V)) and water (8 mL, 2 V) were added Iron powder (3.771 g, 67.524 mmol, 7 equiv) and ammonium chloride (1.548 g, 28.939 mmol, 3 equiv) at room temperature. The reaction mass was heated to 70°C and stirred at same temperature for 2 h. After completion of the reaction, the reaction mass was filter through celite and extracted with EtOAc (2 x 50 mL). The combined organic layer was washed with saturated brine solution (2 x 50 mL). The organic layer was dried over anhydrous MgSO4, filtered and concentrated under reduced pressure to get the crude product. The obtained crude product was triturated with n-pentane (30 mL) to get (4-(4-amino-3-chlorophenyl) piperazin-1-yl) (3,4-dichlorophenyl) methanone (7) (3.5 g, 94%). LC-MS (m/z): 384.49 [M+H]⁺ ion present

### Step 5: Synthesis of phenyl (2-chloro-4-(4-(3,4-dichlorobenzoyl) piperazin-1-yl) phenyl)carbamate

To a stirred solution of (4-(4-amino-3-chlorophenyl) piperazin-1-yl) (3,4-dichlorophenyl) methanone (3.5 g, 9.098 mmol, 1 equiv) in dichloromethane (52 mL, 15 V) was added pyridine (1.469 mL, 18.197 mmol, 2 equiv) at room temperature. Then the reaction mass was cooled to 0°C. After that was added phenyl chloroformate (1.709 g, 10.918 mmol, 1.2 equiv) The resulting reaction mixture was stirred at room temperature for 3 hours. After completion of the reaction, quenched with 1N hydrogen chloride solution in water (50 mL) and extracted with dichloromethane (2 x 50 mL). The combined organic layers were washed with saturated aqueous NaHCO₃ solution (2 x 50 mL) and brine (50 mL), dried over anhydrous sodium sulphate, filtered and concentrated to get the crude product. The crude compound was triturated with n-pentane (150 mL) to get phenyl (2-chloro-4-(4-(3,4-dichlorobenzoyl) piperazin-1-yl) phenyl)carbamate (2 g, 44%). LC-MS (m/z): 504.71 [M+H] ⁺ ion present

### Step 6: Synthesis of (E/Z)-1-(2-chloro-4-(4-(3,4-dichlorobenzoyl) piperazin-1-yl) phenyl)-3-(3-(5-(dimethylamino)-2-isopropylphenyl)-4-oxothiazolidin-2-ylidene) urea (Compound I-35)

To a stirred solution of phenyl (2-chloro-4-(4-(3,4-dichlorobenzoyl)piperazin-1-yl)phenyl)carbamate (1.5 g, 2.972 mmol, 1 equiv) in tetrahydrofuran (20 mL, 20 V) were added 3-(5-(dimethylamino)-2-isopropylphenyl)-2-iminothiazolidin-4-one (0.989 g, 3.566 mmol, 1.2 equiv) and *N, N*-diisopropylethylamine (1.152 g, 8.915 mmol, 3 equiv) at room temperature. The reaction mixture was heated to 65°C and stirred at the same temperature for 6 h. After completion of the reaction, the reaction mixture was quenched with water (20 mL) and extracted with ethyl acetate (2 x 20 mL). The combined organic layers were washed with brine (20 mL), dried over anhydrous sodium sulphate, filtered and concentrated to get the crude product. The crude product was purified by silica gel (230-400 mesh) column chromatography using 40% ethyl acetate in petroleum ether to get (E/Z)-1-(2-chloro-4-(4-(3,4-dichlorobenzoyl) piperazin-1-yl) phenyl)-3-(3-(5-(dimethylamino)-2-isopropylphenyl)-4-oxothiazolidin-2-ylidene) urea (565 mg, 28%).

LC-MS (m/z): 687.41 [M+H]+ ion present; ¹H NMR (400 MHz, DMSO-d6): δ 8.90 (s, 1H), 7.79-7.70 (m, 2H), 7.44 (dd, *J* = 8.4, 2.0 Hz, 1H), 7.31-7.24 (m, 2H), 6.99 (d, *J* = 2.0 Hz, 1H), 6.90 (d, *J* = 8.8 Hz, 1H), 6.82 (dd, *J* = 8.4, 2.0 Hz, 1H), 6.57 (d, *J =* 2.0 Hz, 1H), 4.14 (d, *J =* 18.0 Hz, 1H), 4.02 (d, *J* = 18.0 Hz, 1H), 3.81-3.65 (m, 2H), 3.53-3.37 (m, 2H), 3.28-3.05 (m, 4H), 2.87 (s, 6H), 2.62-2.54 (m, 1H), 1.20-0.95 (m, 6H).

### Example 23: Synthesis of intermediate 3-(5-(dimethylamino)-2-isopropylphenyl)-2-iminothiazolidin-4-one

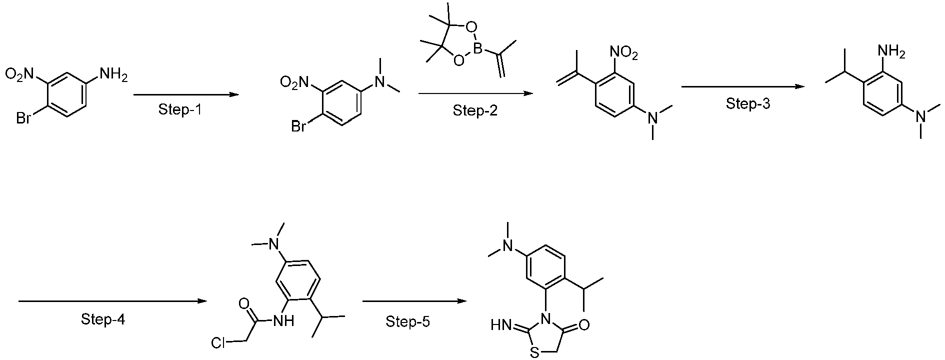

### Step 1: Synthesis of 4-bromo-N, N-dimethyl-3-nitroaniline

To a stirred solution of 4-bromo-3-nitroaniline (5 g, 23.039 mmol, 1 equiv) in DMF (50 mL, 10 V) at 0 °C was added sodium hydride (1.843 g, 46.079 mmol, 2 equiv) and stirred for 30 min. After, that was added iodomethane, (16.351 g, 115.197 mmol, 5 equiv) at 0 °C. The reaction mass was stirred at room temperature for 6 h. After completion of the reaction, the reaction mass was quenched with water (150 mL) and extracted with EtOAc (2 x 100 mL). The combined organic layer was washed with saturated NaCl solution (2 X 150 mL). The organic layer was dried over anhydrous MgSO4, filtered and concentrated under reduced pressure to get the crude product. The crude product was purified by silica gel (230-400 mesh) column chromatography using 10% ethyl acetate in petroleum ether to get 4-bromo-N, N-dimethyl-3-nitroaniline (2.5 g, 44% yield). LC-MS (m/z): 245.58 [M+H] ⁺ ion present

### Step 2: Synthesis of N,N-dimethyl-3-nitro-4-(prop-1-en-2-yl)aniline

To a stirred solution of 4-bromo-N, N-dimethyl-3-nitroaniline (2.5 g, 10.201 mmol, 1 equiv) in 1,4-dioxane (25 mL, 10 V) and water (12.50 mL, 5 V) were added sodium carbonate (3.244 g, 30.604 mmol, 3 equiv) and 4,4,5,5-tetramethyl-2-(prop-1-en-2-yl)-1,3,2-dioxaborolane (2.057 g, 12.241 mmol, 1.2 equiv) at room temperature. The reaction mixture was degassed with N₂ atmosphere for about 10 min. After that was added bis(triphenylphosphine)palladium (II) dichloride (0.143 g, 0.204 mmol, 0.02 equiv) again the reaction mixture degassed for 5 minutes. The reaction mass was heated to 80°C and stirred at same temperature for 16 h. After completion of the reaction, the reaction mass was diluted water (50 mL) and extracted with EtOAc (2 x 50 mL). The combined organic layer was washed with saturated NaCl solution (2 x 50 mL). The organic layer was dried over anhydrous MgSO₄, filtered and concentrated under reduced pressure to get the crude product. The crude product was passed through a short plug of silica gel (230-400 mesh) bed using 10% ethyl acetate in petroleum ether to get N,N-dimethyl-3-nitro-4-(prop-1-en-2-yl)aniline (2.6 g, crude). The crude product taken to next step without purification. LC-MS (m/z): 207.30 [M+H] ⁺ ion present

### Step 3: Synthesis of 4-isopropyl-N1,N1-dimethylbenzene-1,3-diamine:

To a stirred solution of N,N-dimethyl-3-nitro-4-(prop-1-en-2-yl) aniline (2.6 g, 12.606 mmol, 1 equiv) in ethyl acetate (26 mL, 10 V) was charged 10% palladium on activated carbon (50% wet) (0.520 g, 0.2 w/w) and degassed with H₂ gas. The reaction mass was stirred at room temperature under H₂ (80 psi) for 16 h. After completion of the reaction, the reaction mass was filtered through celite bed and washed with ethyl acetate (50 mL) concentrated under reduced pressure to get the crude product. The crude product was passed through short silica gel (230-400 mesh) bed using 10% ethyl acetate in petroleum ether to get 4-isopropyl-N1,N1-dimethylbenzene-1,3-diamine (2.4 g, crude). LC-MS (m/z): 179.68 [M+H]⁺ ion present

### Step-4: Synthesis of 2-chloro-N-(5-(dimethylamino)-2-isopropylphenyl) acetamide

To a stirred solution of 4-isopropyl-N1,N1-dimethylbenzene-1,3-diamine (2.4 g, 13.462 mmol, 1 equiv) in dichloromethane (24 mL, 10 V) were added sodium bicarbonate (1.923 g, 22.885 mmol, 1.7 equiv), water (12 mL, 5 V) at room temperature, then added 2-chloroacetyl chloride (16) (1.520 g, 13.462 mmol, 1 equiv) to the reaction mass at 0°C. The reaction mass was allowed to room temperature and stirred for 1 h at same temperature. After completion of the reaction, the reaction mass was diluted with water (50 mL) and extracted with DCM (2 x 50 mL). The combined organic layer was washed with saturated NaCl solution (50 mL). The organic layer was dried over anhydrous MgSO₄, filtered and concentrated under reduced pressure to get 2-chloro-N-(5-(dimethylamino)-2-isopropylphenyl) acetamide (2.5 g). LC-MS (m/z): 255.37 [M+H] ⁺ ion present

### Step 4: Synthesis of 3-(5-(dimethylamino)-2-isopropylphenyl)-2-iminothiazolidin-4-one

To a stirred solution of 2-chloro-N-(5-(dimethylamino)-2-isopropylphenyl) acetamide (2.5 g, 9.813 mmol, 1 equiv) in acetone (25 mL, 10 V) was added potassium thiocyanate (1.907 g, 19.626 mmol, 2 equiv) at room temperature. The reaction mixture was heated to 55°C and stirred at the same temperature for 3 h. The reaction mass was cooled to room temperature and added cesium carbonate (0.160 g, 0.491 mmol, 0.05 equiv) and stirred for 30 min at same temperature. After completion of the reaction, the reaction mass was diluted with water (50 mL) and extracted with ethyl acetate (2 x 50 mL). The combined organic layer was washed with saturated NaCl solution (50 mL), dried over anhydrous MgSO₄, filtered and concentrated under reduced pressure to get the crude product. The crude product was purified by silica gel (230-400 mesh) column chromatography using 30% ethyl acetate in petroleum ether to get 3-(5-(dimethylamino)-2-isopropylphenyl)-2-iminothiazolidin-4-one (1.5 g, 55% yield). LC-MS (m/z): 278.45 [M+H] + ion present

### Example 24: Synthesis of (E/Z)-1-(4-(4-(4-ethoxybenzoyl) piperazin-1-yl) phenyl)-3-(3-(2-isopropyl-5-methylphenyl)-4-oxothiazolidin-2-ylidene) urea (Compound I-36)

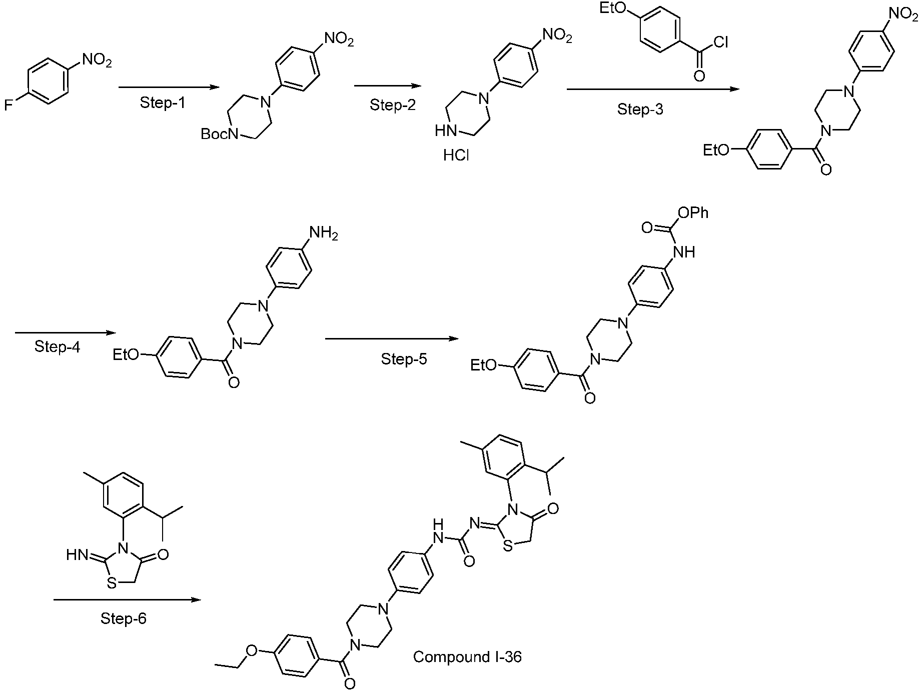

### Step 1: Synthesis of tert-butyl 4-(4-nitrophenyl) piperazine-1-carboxylate

To a stirred solution of 1-fluoro-4-nitrobenzene (5 g, 35.436 mmol, 1 equiv.) in N,N-dimethylformamide (50 mL, 10 V) were added tert-butyl piperazine-1-carboxylate (6.6 g, 35.436 mmol, 1 equiv) and triethylamine (12.550 g, 124.026 mmol, 3.5 equiv) at room temperature. The reaction mass was heated to 90°C and stirred at same temperature for 16 h. After completion of the reaction, the reaction mixture was quenched with water (100 mL) and extracted with ethyl acetate (2 × 100 mL). The combined organic layers were washed with brine (100 mL), dried over anhydrous sodium sulphate, filtered and concentrated to get the crude product. The crude product was triturated with n-pentane (~50 mL) to get tert-butyl 4-(4-nitrophenyl) piperazine-1-carboxylate (3) (5 g, 46%). LC-MS (m/z): 308.75 [M+H]⁺ ion present

### Step 2: Synthesis of 1-(4-nitrophenyl) piperazine hydrochloride

To a stirred solution of tert-butyl 4-(4-nitrophenyl) piperazine-1-carboxylate (3 g, 9.761 mmol, 1 equiv) in 1,4-dioxane (15 mL, 5 V) was added 4M HCl in dioxane (30 mL, 10 V) at 0 °C. The resulting reaction mixture was stirred at room temperature for 16 h. After completion of the reaction, the reaction mixture was concentrated under reduced pressure to get the crude product. The obtained crude product was triturated with n-pentane (150 mL) to get 1-(4-nitrophenyl) piperazine hydrochloride (2.5 g) as a yellow solid. LC-MS (m/z): 208.32 [M-HCl+H]⁺ ion present

### Step 3: Synthesis of (4-ethoxyphenyl) (4-(4-nitrophenyl) piperazin-1-yl) methanone

To a stirred solution of 1-(4-nitrophenyl) piperazine hydrochloride (2.5 g, 10.259 mmol, 1 equiv) in dichloromethane (25 mL, 10 V) were added 4-ethoxybenzoyl chloride (2.841 g, 15.388 mmol, 1.5 equiv) and pyridine (2.484 mL, 30.777 mmol, 3 equiv) at room temperature. The reaction mass was stirred for 3 h at the same temperature. After completion of the reaction, the reaction mass was quenched with 1N hydrogen chloride solution in water (50 mL) and extracted with dichloromethane (2 x 50 mL). The combined organic layers were washed with saturated aqueous NaHCO₃ solution (2 x 50 mL) and brine (50 mL), dried over anhydrous sodium sulphate, filtered and concentrated to get the crude product. The crude product was triturated with n-pentane (~50 mL) to get (4-ethoxyphenyl) (4-(4-nitrophenyl) piperazin-1-yl) methanone (3 g, 82%). LC-MS (m/z): 356.58 [M+H]⁺ ion present

### Step 4: Synthesis of (4-(4-aminophenyl) piperazin-1-yl) (4-ethoxyphenyl) methanone

To a stirred solution of (4-ethoxyphenyl) (4-(4-nitrophenyl) piperazin-1-yl) methanone (3 g, 8.441 mmol, 1 equiv) in ethanol (24 mL, 8 V) and water (6 mL, 2 V) were added Iron powder (3.3 g, 59.089 mmol, 7 equiv) and ammonium chloride (1.355 g, 25.324 mmol, 3 equiv) at room temperature. The reaction mass was heated to 70°C and stirred at same temperature for 2 h. After completion of the reaction, the reaction mass was filter through celite and extracted with ethyl acetate (2 x 50 mL). The combined organic layer was washed with saturated brine solution (2 X 50 mL). The organic layer was dried over anhydrous MgSO₄, filtered and concentrated under reduced pressure to get the crude product. The obtained crude product was triturated with n-pentane (30 mL) to get (4-(4-aminophenyl) piperazin-1-yl) (4-ethoxyphenyl) methanone (2.5 g, 91%). LC-MS (m/z): 326.53 [M+H] ⁺ ion present

### Step 5: Synthesis of phenyl (4-(4-(4-ethoxybenzoyl) piperazin-1-yl) phenyl)carbamate

To a stirred solution of (4-(4-aminophenyl) piperazin-1-yl) (4-ethoxyphenyl) methanone (2.5 g, 7.683 mmol, 1 equiv) in dichloromethane (37 mL, 15 V) was added pyridine (1.24 mL, 15.365 mmol, 2 equiv) at room temperature. Then the reaction mass was cooled to 0 °C, after that was added phenyl chloroformate (1.443 g, 9.219 mmol, 1.2 equiv). The resulting reaction mixture was stirred at same temperature for 3 hours. After completion of the reaction, the reaction was quenched with 1N hydrogen chloride solution in water (20 mL) and extracted with dichloromethane (2 x 25 mL). The combined organic layers were washed with saturated aqueous NaHCO₃ solution (2 x 25 mL) and brine (25 mL), dried over anhydrous sodium sulphate, filtered and concentrated to get the crude product. The crude compound was triturated with n-pentane (30 mL) to get phenyl (4-(4-(4-ethoxybenzoyl) piperazin-1-yl) phenyl)carbamate (2 g, 58%).
LC-MS (m/z): 446.78 [M+H] ⁺ ion present

### Step 6: Synthesis of (E/Z)-1-(4-(4-(4-ethoxybenzoyl) piperazin-1-yl) phenyl)-3-(3-(2-isopropyl-5-methylphenyl)-4-oxothiazolidin-2-ylidene) urea (Compound I-36)

To a stirred solution of phenyl (4-(4-(4-ethoxybenzoyl) piperazin-1-yl) phenyl)carbamate (1 g, 2.245 mmol, 1 equiv) in tetrahydrofuran (20 mL, 20 V) were added 2-imino-3-(2-isopropyl-5-methylphenyl) thiazolidin-4-one (0.669 g, 2.693 mmol, 1.2 equiv) and N,N-diisopropylethylamine (0.870 g, 6.734 mmol, 3 equiv) at room temperature. The reaction mixture was heated to 65 °C and stirred at the same temperature for 6 hours. After completion of the reaction, the reaction mixture was quenched with water (20 mL) and extracted with ethyl acetate (2 x 20 mL). The combined organic layers were washed with brine (20 mL), dried over anhydrous sodium sulphate, filtered and concentrated to get the crude product. The crude product was purified by silica gel (230-400 mesh) column chromatography using 40% ethyl acetate in petroleum ether to get (E/Z)-1-(4-(4-(4-ethoxybenzoyl) piperazin-1-yl) phenyl)-3-(3-(2-isopropyl-5-methylphenyl)-4-oxothiazolidin-2-ylidene) urea (0.414 g).

LC-MS (m/z): 600.47 [M+H] ⁺ ion present; ¹H NMR (400 MHz, DMSO-d₆): δ 7.44-7.35 (m, 5H), 7.33-7.29 (m, 1H), 7.16 (s, 1H), 6.94-6.83 (m, 5H), 4.06 (q, *J =* 7.2 Hz, 2H), 4.01-3.62 (m, 6H), 3.12 (s, 4H), 2.72-2.61 (m, 1H), 2.38 (s, 3H), 1.43 (t, *J* = 7.2 Hz, 3H), 1.22-1.11 (m, 6H).

### Example 25: Synthesis of (E/Z)-3-(2-isopropyl-5-methylphenyl)-2-(((E)-4-(4-(4-(trifluoromethoxy)benzoyl)piperazin-1-yl)benzylidene)hydrazineylidene)thiazolidin-4-one

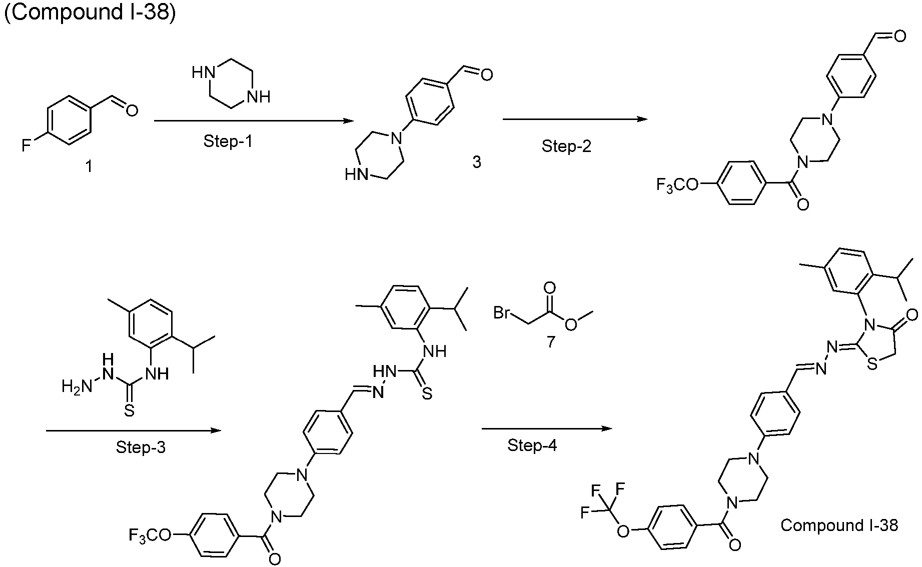

### Step 1: Synthesis of 4-(piperazin-1-yl)benzaldehyde

A stirred solution of piperazine (4.164 g, 48.344 mmol, 4 equiv.) in water (49.50 mL, 33 V) and 2-methoxyethanol (55.50 mL, 37 V) was heated to 100 °C and stirred for 30 minutes. The resultant reaction mass was cooled to room temperature and added 4-fluorobenzaldehyde (1.5 g, 12.086 mmol, 1 equiv.) in 2-methoxyethanol (13.50 mL, 9 V). The resultant reaction mass was again heated to 100°C and stirred for 5 h. After completion of the reaction, the reaction mass was quenched in ice water (15 mL) and extracted the product with dichloromethane (15 mL x 10 times). The combined organic layer was washed with saturated brine solutions (30 mL) and dried over anhydrous sodium sulphate and concentrated. To the obtained crude product added methyl tert-butyl ether (15 V) and stirred for 10 minutes, then filtered to get 4-(piperazin-1-yl)benzaldehyde (3) (2 g, 87%). LC-MS (m/z): 191.39 [M+H]⁺ ion present

### Step 2: Synthesis of 4-(4-(4-(trifluoromethoxy)benzoyl)piperazin-1-yl)benzaldehyde

To a stirred solution of 4-(piperazin-1-yl)benzaldehyde (1 g, 5.256 mmol, 1 equiv.) and triethylamine (2.198 mL, 15.769 mmol, 3 equiv.) in dichloromethane (8 mL, 8 V) at 0 °C was added 4-(trifluoromethoxy)benzoyl chloride (1.534 g, 6.833 mmol, 1.3 equiv.) in dichloromethane (2 mL, 2 V). Resultant reaction mixture was warm to room temperature and stirred for 3 h. After completion of the reaction, concentrated the reaction mass. To the obtained crude product was added water (10 mL) and extracted the product with 10% methanol in ethyl acetate (2 x 50 mL). The combined organic layer was washed with saturated brine solution (10 mL), dried over anhydrous sodium sulphate and concentrated to get the crude product. To the obtained crude product added petroleum ether (20 mL) and stirred at room temperature for 10 minutes. Decant the petroleum ether and dried in rotatory evaporator for 15 minutes at 45 °C to get 4-(4-(4-(trifluoromethoxy)benzoyl)piperazin-1-yl)benzaldehyde (5) (1.5 g, 75%) as a pale yellow liquid.
LC-MS (m/z): 379.73 [M+H] + ion present

### Step 3: Synthesis of 6

To a stirred solution of 4-(4-(4-(trifluoromethoxy)benzoyl)piperazin-1-yl)benzaldehyde (5) (1 g, 2.643 mmol, 1 equiv.) in ethanol (20 mL, 20 V) was added N-(2-isopropyl-5-methylphenyl)hydrazinecarbothioamide (ES5103-3) (synthesized following the protocol adapted for ES5103-3) (0.531 g, 2.379 mmol, 0.9 equiv.) at room temperature. The reaction mass was heated to 78 °C and stirred at same temperature for 3 hours. After completion of the reaction, concentrated the reaction mass under reduced pressure to get the crude product. To the obtained crude product added methyl tert-butyl ether (10 mL) at room temperature and stirred the mass for 10 minutes at same temperature. Decant the solvent and obtained solid was dried in rotatory evaporator for 15 minutes at 40 °C to get (E)-N-(2-isopropyl-5-methylphenyl)-2-(4-(4-(4-(trifluoromethoxy)benzoyl)piperazin-1-yl)benzylidene)hydrazine-1-carbothioamide (6) (1.3 g, 84%) as a pale yellow solid.

### Step 4: Synthesis of (E/Z)-3-(2-isopropyl-5-methylphenyl)-2-(((E)-4-(4-(4-(trifluoromethoxy)benzoyl)piperazin-1-yl)benzylidene)hydrazineylidene)thiazolidin-4-one (Compound I-38)

To a stirred solution (E)-N-(2-isopropyl-5-methylphenyl)-2-(4-(4-(4-(trifluoromethoxy)benzoyl)piperazin-1-yl)benzylidene)hydrazine-1-carbothioamide (6) (0.8 g, 1.371 mmol, 1 equiv.) in ethanol (8 mL, 10 V) were added methyl 2-bromoacetate (7) (0.315 g, 2.056 mmol, 1.5 equiv.) and sodium acetate (0.450 g, 5.483 mmol, 4 equiv.) at room temperature. The resulting reaction mixture was heated to 78 °C and stirred at same temperature for 3 hours. After completion of the reaction, the reaction mass was concentrated. To the obtained crude product was added water (10 mL) and extracted with hot ethyl acetate (~50 °C) (40 mL). The combined organic layer was washed with saturated brine solution (10 mL), then dried over anhydrous sodium sulphate and concentrated to get the crude product. To the obtained crude product was added methyl tert-butyl ether (10 mL) at room temperature and stirred for 10 minutes and filtered the solid and washed with methyl tert-butyl ether (2 mL). Obtained solid was dried in rotatory evaporator for 30 minutes at 40 °C to get (E/Z)-3-(2-isopropyl-5-methylphenyl)-2-(((E)-4-(4-(4-(trifluoromethoxy)benzoyl)piperazin-1-yl)benzylidene)hydrazineylidene)thiazolidin-4-one (0.45 g, 53%).

LC-MS (m/z): 624.34 [M+H]⁺ ion present; ¹H NMR (400 MHz, DMSO-d₆): δ 8.16 (s, 1H), 7.65-7.53 (m, 4H), 7.46 (d, *J* = 7.6 Hz, 2H), 7.36 (d, *J* = 8.0 Hz, 1H), 7.31-7.19 (m, 1H), 7.03 (d, *J =* 1.2 Hz, 1H), 6.98 (d, *J* = 8.8 Hz, 2H), 4.19 (d, *J =* 17.2 Hz, 1H), 4.08 (d, *J* = 17.2 Hz, 1H), 3.89-3.38 (m, 8H), 2.80-2.67 (m, 1H), 2.30 (s, 3H), 1.13-1.03 (m, 6H).

With appropriate modification of the starting materials, the procedures given in the synthesis descriptions were used to obtain further compounds I. The compounds obtained in this manner are listed in the table that follows (Table I), together with physical data.

**Table I:**

| No. | R^{1a} | R^{1b} | Q | B¹ | B² | B³ | B⁴ | R² | R³ | phys. Data | | |
|---|---|---|---|---|---|---|---|---|---|---|---|---|
| | | | | | | | | | | M e t h o d | HP LC RT [min] | M+H [m/z] |
| I-1 | H | Cl | Q¹ | CH | CH | CH | N | R²-1 | R³-29 | E | 5.39 | 574.3 |
| I-2 | H | Cl | Q¹ | CH | CH | CH | N | R²-4 | R³-29 | E | 5.23 | 534.3 |
| I-3 | H | Cl | Q⁵ | CH | CH | CH | CH | R²-2 | R³-29 | E | 4.71 | 604.3 |
| I-4 | H | Cl | Q³ | CH | CH | CH | N | R²-2 | R³-29 | F | 2.13 | 588.3 |
| I-5 | H | Cl | Q³ | CH | CH | CH | N | R²-4 | R³-29 | F | 2.20 | 532.5 |
| I-6 | H | Cl | Q³ | CH | CH | CH | N | R²-1 | R³-29 | F | 2.26 | 572.4 |
| I-7 | H | Cl | Q³ | CH | CCl | CH | CH | R²-2 | R³-29 | F | 2.33 | 621.4 |
| I-8 | H | OCF₃ | Q³ | CH | CCl | CH | CH | R²-2 | R³-29 | E | 5.92 | 671.5 |
| I-9 | H | Cl | Q³ | CCl | CH | CH | CH | R²-1 | R³-29 | D | 9.38 | 605.4 |
| I-10 | H | OCF₃ | Q³ | CH | CH | CH | N | R²-1 | R³-29 | D | 8.72 | 622.5 |
| I-11 | H | Cl | Q³ | CH | N | CH | N | R²-1 | R³-29 | E | 5.47 | 573.4 |
| I-12 | H | Cl | Q³ | CH | N | CH | N | R²-2 | R³-29 | E | 5.17 | 589.3 |
| I-13 | H | Cl | Q⁴ | CH | CCl | CH | CH | R²-2 | R³-29 | E | 5.66 | 607.3 |
| I-14 | H | Cl | Q³ | CCl | CH | CH | CH | R²-4 | R³-29 | E | 5.90 | 565.3 |
| I-15 | H | Cl | Q³ | CH | CF | CH | CH | R²-2 | R³-29 | E | 5.50 | 605.3 |
| I-16 | H | OCF₃ | Q³ | CH | CH | CH | N | R²-4 | R³-29 | E | 5.49 | 582.3 |
| I-17 | H | OCF₃ | Q² | CH | CCl | CH | CH | R²-2 | R³-29 | E | 5.60 | 674.3 |
| I-18 | H | Cl | Q² | CH | CCl | CH | CH | R²-2 | R³-29 | E | 5.41 | 624.4 |
| I-19 | H | OCF₃ | Q² | CH | CF | CH | CH | R²-3 | R³-29 | D | 8.83 | 618.4 |
| I-20 | H | OCF₃ | Q² | CH | CF | CH | CH | R²-2 | R³-29 | E | 5.39 | 658.4 |
| I-21 | H | Cl | Q² | CH | CF | CH | CH | R²-2 | R³-29 | E | 5.25 | 608.4 |
| I-22 | Cl | F | Q² | CH | CF | CH | CH | R²-2 | R³-29 | E | 5.34 | 626.3 |
| I-23 | Cl | F | Q² | CH | CCl | CH | CH | R²-2 | R³-29 | E | 5.51 | 642.3 |
| I-24 | H | OCF₃ | Q² | CH | CF | CH | CH | R²-2 | R³-31 | E | 5.27 | 728.5 |
| I-25 | OCF ₃ | H | Q² | CH | CCl | CH | CH | R²-2 | R³-29 | D | 1.39 | 674.2 |
| I-26 | OCF ₃ | Cl | Q² | CH | CCl | CH | CH | R²-2 | R³-29 | D | 1.43 | 709.1 |
| I-27 | OCF ₃ | F | Q² | CH | CCl | CH | CH | R²-2 | R³-29 | D | 1.39 | 692.1 |
| I-28 | H | OCF₃ | Q² | CH | CCl | CH | CH | R²-7 | R³-29 | E | 5.36 | 717.3 |
| | | | | | | | | | | | 5.31 | 717.3 |
| I-29 | Cl | F | Q² | CH | CCl | CH | CH | R²-7 | R³-29 | E | 5.32 | 683.2 |
| | | | | | | | | | | | 5.37 | 683.2 |
| I-30 | H | Cl | Q² | CH | CCl | CH | CH | R²-7 | R³-29 | D | 8.14 | 667.5 |
| | | | | | | | | | | | 8.20 | 667.5 |
| I-31 | Cl | F | Q² | CH | N | CH | N | R²-2 | R³-29 | E | 4.99 | 610.4 |
| I-32 | H | OCF₃ | Q² | CH | CCl | CH | CH | R²-9 | R³-29 | E | 5.86 | 729.3 |
| | | | | | | | | | | | 5.88 | 729.3 |
| I-33 | Cl | F | Q² | CH | CCl | CH | CH | R²-9 | R³-29 | E | 5.75 | 697.2 |
| | | | | | | | | | | | 5.77 | 697.2 |
| I-34 | H | OCF₃ | Q² | CH | CF | CH | CH | R²-7 | R³-29 | E | 5.14 | 701.4 |
| | | | | | | | | | | | 5.18 | 702.4 |
| I-35 | Cl | Cl | Q² | CH | CCl | CH | CH | R²-2 | R³-30 | E | 5.60 | 687.4 |
| I-36 | H | OCH₂ -CH₃ | Q² | CH | CH | CH | CH | R²-2 | R³-29 | D | 7.65 | 600.5 |
| I-37 | Cl | F | Q² | CH | CF | CH | CH | R²-7 | R³-29 | E | 5.16 | 667.2 |
| | | | | | | | | | | | 5.20 | 667.2 |
| I-38 | H | OCF₃ | Q² | CH | CH | CH | CH | R²-1 | R³-29 | E | 5.83 | 624.3 |
| I-39 | H | OCF₃ | Q² | CCl | CH | CH | CH | R²-1 | R³-29 | E | 6.10 | 658.3 |
| I-40 | H | OCF₃ | Q² | CH | CF | CH | CH | R²-9 | R³-29 | D | 8.68 | 715.5 |
| I-41 | H | Br | Q² | N | CH | CH | CH | R²-1 | R³-1 | D | 8.10 | 605.5 |
| I-42 | H | Cl | Q² | CH | CCl | CH | CH | R²-7 | R³-29 | E | 5.67 | 681.4 |
| I-43 | H | Cl | Q² | CCl | CH | CH | CH | R²-1 | R³-30 | E | 5.85 | 637.3 |
| I-44 | Cl | F | Q² | CH | CF | CH | CH | R²-9 | R³-29 | D | 8.52 | 683.5 |
| I-45 | H | CN | Q² | CH | CBr | CH | CH | R²-2 | R³-29 | E | 5.05 | 659.3 |
| I-46 | H | OCF₃ | Q² | CCl | CH | CH | CH | R²-4 | R³-29 | G | 3.72 | 618.5 |
| I-47 | H | OCF₃ | Q² | CH | CH | CH | CH | R²-2 | R³-29 | E | 5.32 | 640.5 |
| I-48 | H | 2,2-Cl₂-cyclopr o-pyl | Q² | CH | CBr | CH | CH | R²-2 | R³-1 | D | 8.48 | 730.6 |
| I-49 | H | OCF₃ | Q² | CH | CCl | CH | CH | R²-3 | R³-29 | E | 5.90 | 634.3 |
| I-50 | Cl | Cl | Q² | CCl | CH | CH | CH | R²-1 | R³-7 | D | 9.23 | 656.4 |
| I-51 | -OCF₂-O- | | Q² | CH | CF | CH | CH | R²-2 | R³-29 | D | 8.07 | 654.6 |

| | | | | | | | | | | | | |
|---|---|---|---|---|---|---|---|---|---|---|---|---|
| wherein R^{1c} is H, W is O; or an N-oxide, stereoisomer, tautomer, agriculturally or veterinarily acceptable salt thereof. | | | | | | | | | | | | |

### B. Biological Examples

If not otherwise specified, the test solutions were prepared as follows:
The active compound was dissolved at the desired concentration in a mixture of 1:1 (vol:vol) distilled water:acetone. The test solution was prepared on the day of use.
The activity of the compounds of formula I of the present invention can be demonstrated and evaluated by the following biological tests.

### B.1. Boll weevil (Anthonomus grandis)

For evaluating control of boll weevil, the test unit consisted of 96-well-microtiter plates containing an insect diet and 5-10 *A. grandis* eggs.

The compounds were formulated using a solution containing 75% v/v water and 25% v/v DMSO. Different concentrations of formulated compounds were sprayed onto the insect diet at 5µl, using a custom-built micro atomizer, at two replications.

After application, microtiter plates were incubated at about 25 ± 1°C and about 75 ± 5 % relative humidity for 5 days. Egg and larval mortality were then visually assessed.

In this test, compounds I-1, I-2, I-3, I-4, I-5, 1-6, I-7, I-8, I-9, I-10, I-11, I-12, I-13, I-14, I-15, I-16, I-17, I-18, I-19, I-20, I-21, I-22, I-23, I-24, I-25 at 800 ppm showed at least 75% mortality in comparison with untreated controls.

### B.2. Green peach aphid (Myzus persicae) (mixed life stages)

For evaluating control of Green Peach Aphid through systemic means the test unit consisted of 96-well-microtiter plates containing liquid artificial diet under an artificial membrane. The compounds were formulated using a solution containing 75% v/v water and 25% v/v DMSO. Different concentrations of formulated compounds were pipetted into the aphid diet, using a custom-built pipette, at two replications. After application, 5 - 8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed.

In this test, compounds: I-2, I-4, I-5, I-7, I-8, I-10, I-11, I-16, I-17, I-18, I-19, I-20, I-21, I-22, I-23, I-24, I-25 at 800 ppm showed at least 75% mortality in comparison with untreated controls.

### B.3. Tobacco budworm (Heliothis virescens)

For evaluating control of tobacco budworm (*Heliothis virescens*)*,* the test unit consisted of 96-well-microtiter plates containing an insect diet and 15-25 *H. virescens* eggs.

The compounds were formulated using a solution containing 75% v/v water and 25% v/v DMSO. Different concentrations of formulated compounds were sprayed onto the insect diet at 10µl, using a custom-built micro atomizer, at two replications.

After application, microtiter plates were incubated at about 28 ± 1°C and about 80 ± 5 % relative humidity for 5 days. Egg and larval mortality was then visually assessed.

In this test, compounds I-1, I-2, I-3, I-4, I-5, I-6, I-7, I-8, I-9, I-10, I-11, I-12, I-13, I-15, I-16, I-17, I-18, I-19, I-20, I-21, I-22, I-23, I-24, I-25 at 800 ppm showed at least 75% mortality in comparison with untreated controls.

### B.4. Southern armyworm (Spodoptera eridania), 2^{nd} instar larvae

The active compounds were formulated by a Tecan liquid handler in 100% cyclohexanone as a 10,000-ppm solution supplied in tubes. The 10,000-ppm solution was serially diluted in 100% cyclohexanone to make interim solutions. These served as stock solutions for which final dilutions were made by the Tecan in 50% acetone:50% water (v/v) into 10 or 20ml glass vials. A non-ionic surfactant (Kinetic^{®}) was included in the solution at a volume of 0.01% (v/v). The vials were then inserted into an automated electrostatic sprayer equipped with an atomizing nozzle for application to plants/insects. Lima bean plants (variety Sieva) were grown 2 plants to a pot and selected for treatment at the 1^{st} true leaf stage. Test solutions were sprayed onto the foliage by an automated electrostatic plant sprayer equipped with an atomizing spray nozzle. The plants were dried in the sprayer fume hood and then removed from the sprayer. Each pot was placed into perforated plastic bags with a zip closure. Ten to 11 armyworm larvae were placed into the bag and the bags zipped closed. Test plants were maintained in a growth room at about 25°C and about 20-40% relative humidity for 4 days, avoiding direct exposure to fluorescent light (14:10 light:dark photoperiod) to prevent trapping of heat inside the bags. Mortality and reduced feeding were assessed 4 days after treatment, compared to untreated control plants.

In this test, compounds I-1, I-2, I-3, I-4, I-5, 1-6, I-7, I-8, I-9, I-10, I-11, I-12, I-13, I-15, I-16, I-17, I-18, I-19, I-20, I-21, I-22, I-23 at 100 ppm showed at least 75% mortality in comparison with untreated controls.

### B.5. Yellow fever mosquito (Aedes aegypti)

For evaluating control of yellow fever mosquito (Aedes aegypti) the test unit consisted of 96-well-microtiter plates containing 200µl of tap water per well and 5-15 freshly hatched A. aegypti larvae.

The active compounds were formulated using a solution containing 75% (v/v) water and 25% (v/v) DMSO. Different concentrations of formulated compounds or mixtures were sprayed onto the insect diet at 2.5µl, using a custom built micro atomizer, at two replica-tions. After application, microtiter plates were incubated at 28 + 1°C, 80 + 5 % RH for 2 days. Larval mortality was then visually assessed.

In this test, compounds I-2, I-3, I-4, I-5, 1-6, I-7, I-8, I-9, I-10, I-11, I-12, I-13, I-15, I-16, I-17, I-18, I-19, I-20, I-21, I-22, I-23, I-24, I-25 at 100 ppm showed at least 75% mortality in comparison with untreated controls.

## Claims

1. Compounds of formula I 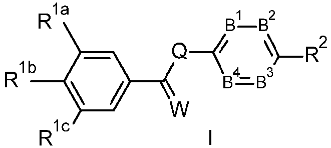 wherein
B¹ is N or CR^{B1};
B² is N or CR^{B2};
B³ is N or CR^{B3};
B⁴ is N or CR^{B4};
with the proviso that not more than two of B¹, B², B³, or B⁴ are N;
W is O, S, or N-R^{N};
R^{N} is H, or C₁-C₄-alkyl, wherein the alkyl moiety is unsubstituted or substituted with halogen;
Q is a group Q¹, Q², Q³, Q⁴, or Q⁵ 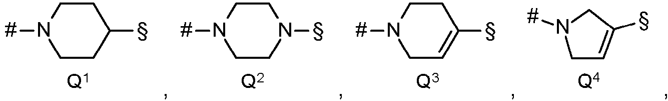 or 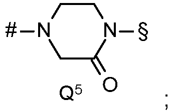 wherein # is the bond to the -C(W)- spacer, and § is the bond to the 6-membered ring;
R^{B1}, R^{B2}, R^{B3}, and R^{B4} independently of each other are H, halogen, N₃, OH, CN, NO₂, - SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₁-C₄-alkyl-C₃-C₆-cycloalkoxy, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e}, phenyl, phenoxy, phenylcarbonyl, phenylthio, or -CH₂-phenyl, wherein the phenyl rings are unsubstituted or substituted with R^{f};
R^{1a}, R^{1b}, and R^{1c} independently of each other are H, halogen, N₃, OH, CN, NO₂, - SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e}, phenyl, phenoxy, phenylcarbonyl, phenylthio or -CH₂-phenyl, wherein phenyl rings are unsubstituted or substituted with R^{f}; or
R^{1a} and R^{1b}, or R^{1b} and R^{1c}, form together with the carbon atoms to which they are bonded a 5- or 6-membered partially unsaturated heterocyclic ring, which is unsubstituted or substituted with one or more halogen, CN, C₁-C₄-alkyl, or C₁-C₄-haloalkyl;
m is 0, 1, or 2;
R² is a moiety of formula X-Y-Z-T-R³; wherein
X is a single bond;
-(C(R^{xa})₂)ₚ-, wherein p is an integer of 1 to 3, preferably 1 or 2;
-(C(R^{xa})₂)ₒ-NR^{xc}-, wherein o is an integer of 1 or 2 and N is bound to Y; or
-NR^{xc}-;
Y is -CR^{ya}=N-, wherein the N is bound to Z;
-NR^{yc}-C(=O)-, wherein C(=O) is bound to Z; or
-NR^{yc}-C(=S)-, wherein C(=S) is bound to Z;
Z is -NR^{zc}-C(=O)-, wherein C(=O) is bound to T;
-NR^{zc}-C(=S)-, wherein C(=S) is bound to T;
-N=C(S-R^{za})-, wherein T is bound to the carbon atom; or
-NR^{zc}-C(S-R^{za})=, wherein T is bound to the carbon atom;
T is O, N or N-R^{T};
R³ is aryl, aryl-C₁-C₄-alkyl, hetaryl, or hetaryl-C₁-C₄-alkyl, wherein the aryl or hetaryl rings are unsubstituted or substituted with one or more R^{g} and wherein the hetaryl is a 5- or 6-membered monocyclic hetaryl or a 8-, 9- or 10-membered bicyclic hetaryl;
and wherein
R^{xa}, R^{ya} are, identical or different, H, halogen, C₁-C₆-alkyl, C₁-C₆-alkoxy, C₂-C₆-alkenyl, C₂-C₆-alkynyl, C₁-C₆-alkoxy-C₁-C₄-alkyl, C₃-C₆-cycloalkyl, C₁-C₄-alkyl-C₃-C₆-cycloalkyl, C₁-C₄-alkyl-C₃-C₆-cycloalkoxy, wherein the alkyl, alkoxy, alkenyl, alkynyl, cycloalkyl and cycloalkoxy moieties are unsubstituted or substituted with halogen;
R^{xc}, R^{yc}, R^{zc} are, identical or different, H, C₁-C₆-alkyl, C₂-C₆-alkenyl, C₂-C₆-alkynyl, C₁-C₄-alkyl-C₁-C₆-alkoxy, C₃-C₆-cycloalkyl, C₁-C₄-alkyl-C₃-C₆-cycloalkyl, C₁-C₄-alkyl-C₃-C₆-cycloalkoxy, or -NR^{b}R^{c}, wherein the alkyl, alkoxy, alkenyl, alkynyl, cycloalkyl and cycloalkoxy moieties are unsubstituted or substituted with halogen;
R^{T} is H, C₁-C₆-alkyl, C₂-C₆-alkenyl, C₂-C₆-alkynyl, C₁-C₄-alkyl-C₁-C₆-alkoxy, C₃-C₆-cycloalkyl, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen;
R^{za} is H, C₁-C₆-alkyl, C₁-C₆-alkoxy, C₂-C₆-alkenyl, tri-C₁-C₆-alkylsilyl, C₂-C₆-alkynyl, C₁-C₄-alkyl-C₁-C₆-alkoxy, C₃-C₆-cycloalkyl, C₁-C₄-alkyl-C₃-C₆-cycloalkoxy, C₁-C₄-alkyl-C₃-C₆-cycloalkyl, wherein the alkyl, alkoxy, alkenyl, alkynyl, cycloalkyl and cycloalkoxy moieties are unsubstituted or substituted with halogen; C₁-C₆-alkylene-NR^{b}R^{c}, C₁-C₆-alkylene-CN, C(=O)-NR^{b}R^{c}, C(=O)-R^{d}, phenyl, phenylcarbonyl, or -CH₂-phenyl, wherein the phenyl rings are unsubstituted or substituted with R^{f};
R^{za} together with R^{T} or R^{zc} if present, may form a linear C₁-C₆-alkylene or a linear C₂-C₆-alkenylene group, where in the linear C₁-C₆-alkylene and the linear C₂-C₆-alkenylene a CH₂ moiety is optionally replaced by a carbonyl and/or wherein 1 or 2 CH₂ moieties are optionally replaced by O or S and/or wherein the linear C₁-C₆-alkylene and the linear C₂-C₆-alkenylene are unsubstituted or substituted with one or more R^{h};
R^{a}, R^{b} and R^{c} are, identical or different, H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen, C₁-C₆-alkylene-CN, phenyl, or -CH₂-phenyl, wherein the phenyl rings are unsubstituted or substituted with one or more R^{f};
R^{d} is H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen; phenyl, or -CH₂-phenyl, wherein the phenyl rings are unsubstituted or substituted with one or more R^{f};
R^{e} is C₁-C₆-alkyl, C₃-C₆-cycloalkyl, C₃-C₆-cycloalkyl-C₁-C₄-alkyl, wherein the alkyl, cycloalkyl moieties are unsubstituted or substituted with halogen; phenyl or -CH₂-phenyl, wherein the phenyl rings are unsubstituted or substituted with one or more R^{f};
R^{f} is halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, wherein the alkyl, alkoxy, alkenyl, alkynyl, cycloalkyl and cycloalkoxy moieties are unsubstituted or substituted with halogen;
R^{g} is halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₄-alkyl-C₁-C₆-alkoxy, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, wherein the alkyl, alkoxy, alkenyl, alkynyl, cycloalkyl and cycloalkoxy moieties are unsubstituted or substituted with halogen; C(=O)-OR^{a}, NR^{b}R^{c}, C₁-C₆-alkylene-NR^{b}R^{c}, O-C₁-C₆-alkylene-NR^{b}R^{c}, C₁-C₆-alkylene-CN, NH-C₁-C₆-alkylene-NR^{b}R^{c}, C(=O)-NR^{b}R^{c}, C(=O)-R^{d}, SO₂NR^{b}R^{c}, or S(=O)ₘR^{e};
R^{h} is halogen, OH, C₁-C₆-alkyl, C₃-C₆-cycloalkyl, or CN;
and the N-oxides, stereoisomers, tautomers, and agriculturally or veterinarily acceptable salts thereof.

2. The compounds of formula I according to claim 1, wherein
a) B¹ is CR^{B1}, B² is CR^{B2}, B³ is CR^{B3}, and B⁴ is CR^{B4}; or
b) B¹ is N, B² is CR^{B2}, B³ is CR^{B3}, and B⁴ is CR^{B4}; or
c) B¹ is CR^{B1}, B² is N, B³ is CR^{B3}, and B⁴ is CR^{B4}; or
d) B¹ is N, B² is CR^{B2}, B³ is CR^{B3}, and B⁴ is N; or
e) B¹ is N, B² is CR^{B2}, B³ is N, and B⁴ is CR^{B4}; or
f) B¹ is CR^{B1}, B² is N, B³ is N, and B⁴ is CR^{B4}.

3. The compounds of formula I according to any of claims 1 or 2, wherein
(1) X is a single bond, Y is -CR^{ya}=N-, wherein the N is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and R^{ya} is preferably H; or
(2) X is a single bond, Y is -NR^{yc}-C(=O)-, wherein C(=O) is bound to Z, Z is - N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and R^{yc} is preferably H; or
(3) X is -(C(R^{xa})₂)ₚ-, wherein p is 2, Y is -NR^{yc}-C(=O)-, wherein C(=O) is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and all R^{xa} and R^{yc} are preferably H; or
(4) X is -(C(R^{xa})₂)ₒ-NR^{xc}-, wherein o is 1 and N is bound to Y, Y is -NR^{yc}-C(=O)-, wherein C(=O) is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, wherein one R^{xa}, R^{xc} and R^{yc} are preferably H and the other R^{xa} is preferably methyl, or wherein one R^{xa} and R^{yc} are preferably H and the other R^{xa} and R^{xc} are preferably methyl; or
(5) X is -(C(R^{xa})₂)ₒ-NR^{xc}-, wherein o is 1 and N is bound to Y, Y is -NR^{yc}-C(=O)-, wherein C(=O) is bound to Z, Z is -NR^{zc}-C(=S)-, wherein C(=S) is bound to T, and T is N-R^{T}, wherein one R^{xa}, R^{xc}, R^{yc}, R^{zc} and R^{T} are preferably H and the other R^{xa} is preferably methyl, or wherein one R^{xa}, R^{yc}, R^{zc} and R^{T} are preferably H and the other R^{xa} and R^{xc} are preferably methyl; or
(6) X is -(C(R^{xa})₂)ₚ-, wherein p is 1, Y is -NR^{yc}-C(=O)-, wherein C(=O) is bound to Z, Z is -NR^{zc}-C(=S)-, wherein C(=S) is bound to T, and T is N-R^{T}, wherein one R^{xa}, R^{zc} and R^{T} are preferably H, the other R^{xa} is preferably methyl, and R^{yc} is preferably -NH₂; or
(7) X is -(C(R^{xa})₂)ₒ-NR^{xc}-, wherein o is 1 and N is bound to Y, Y is -NR^{yc}-C(=O)-, wherein C(=O) is bound to Z, Z is -NR^{zc}-C(=S)-, wherein C(=S) is bound to T, and T is N-R^{T}, wherein one R^{xa}, R^{yc}, R^{zc} and R^{T} are preferably H and the other R^{xa} and R^{xc} are preferably methyl; or
(8) X is -(C(R^{xa})₂)ₒ-NR^{xc}-, wherein o is 1 and N is bound to Y, Y is -NR^{yc}-C(=O)-, wherein C(=O) is bound to Z, Z is -N=C(S-R^{za})-, wherein T is bound to the carbon atom, and T is N-R^{T}, wherein R^{za} together with R^{T} forms a linear C₁-C₆-alkylene group, preferably C₂ alkylene group, where in the linear C₁-C₆-alkylene a CH₂ moiety is replaced by a carbonyl and wherein the linear C₁-C₆-alkylene is unsubstituted or substituted with R^{h}, and one R^{xa} and R^{yc} are preferably H and the other R^{xa} and R^{xc} are preferably methyl;
(9) X is a single bond, Y is -CR^{ya}=N-, wherein the N is bound to Z, Z is -NR^{zc}-C(=S)-, wherein C(=S) is bound to T and T is N-R^{T}, wherein R^{ya}, R^{zc}, and R^{T} are preferably H; or
(10) X is a single bond, Y is -NR^{yc}-C(=O)-, wherein C(=O) is bound to Z, Z is -NR^{zc}-C(=S)-, wherein C(=S) is bound to T and T is N-R^{T}, wherein R^{yc}, R^{zc}, and R^{T} are preferably H.

4. The compounds of formula I according to any of claims 1 to 3, wherein
R³ is monocyclic or bicyclic aryl substituted with one or more R^{g}, preferably phenyl, naphthyl, or pyridinyl substituted with one or more R^{g}.

5. The compounds of formula I according to any of claims 1 to 4, wherein
R^{1a}, R^{1b}, and R^{1c} independently of each other are halogen, OH, C₁-C₆-alkyl, C₁-C₆-haloalkyl, C₁-C₆-alkoxy, C₁-C₆-haloalkoxy, or S-R^{e};
R^{e} is C₁-C₆-alkyl, C₃-C₆-cycloalkyl, C₃-C₆-cycloalkyl-C₁-C₄-alkyl, wherein the alkyl, cycloalkyl moieties are unsubstituted or substituted with halogen; and
R^{B1}, R^{B2}, R^{B3}, and R^{B4} independently of each other are H, halogen, C₁-C₆-alkyl, C₁-C₆-alkoxy, C₂-C₆-alkenyl, C₂-C₆-alkynyl, wherein the alkyl, alkoxy, alkenyl, alkynyl, moieties are unsubstituted or substituted with halogen.

6. The compounds of formula I according to any of claims 1 to 5, wherein R² is selected from the following groups:

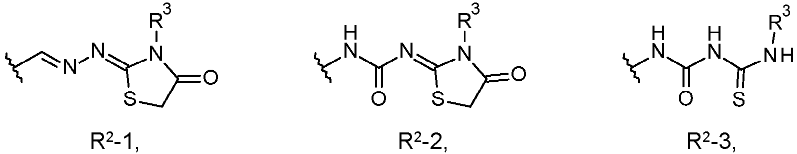

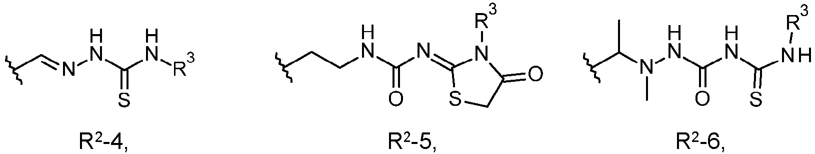

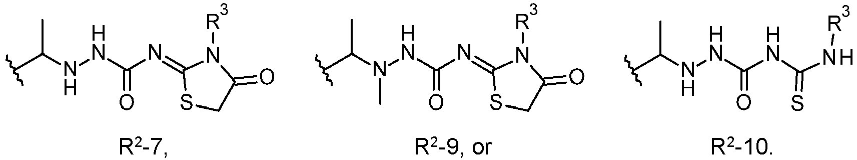

7. The compounds of formula I according to any of claims 1 to 6, wherein R³ is selected from the following groups: 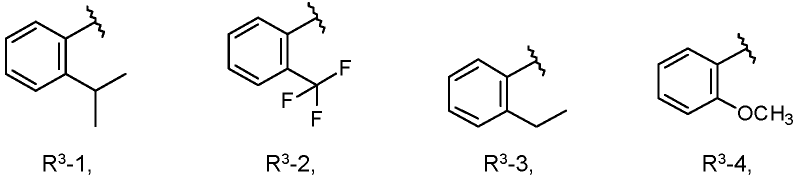 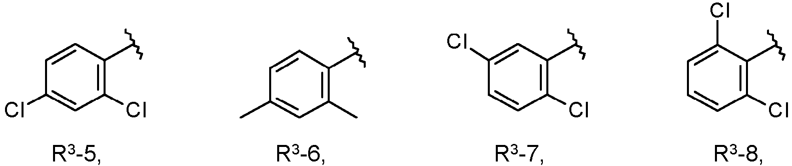 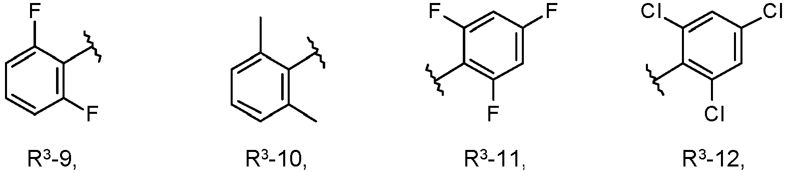 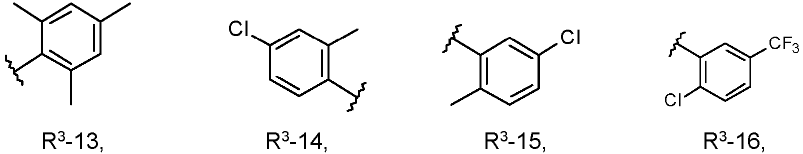 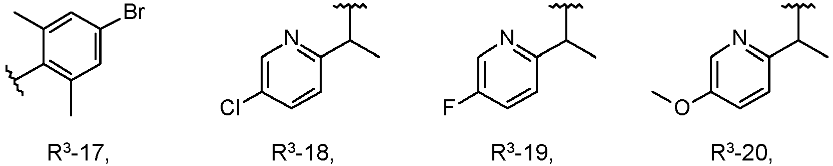 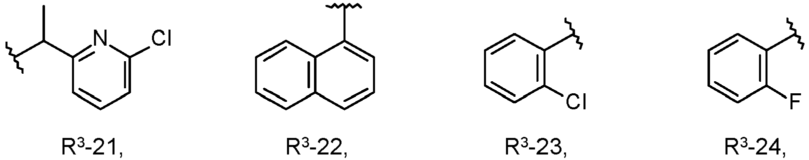 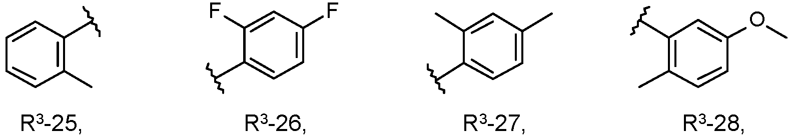 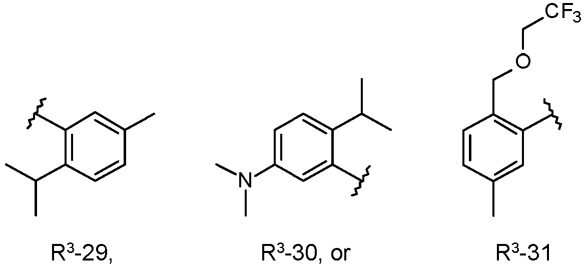 preferably, wherein R³ is R³-1, R³-29, R³-30, or R³-31.

8. The compounds of formula I according to any of claims 1 to 7, wherein the compounds are selected from the following compounds:
| No. | R^{1a} | R^{1b} | Q | B¹ | B² | B³ | B⁴ | R² | R³ |
|---|---|---|---|---|---|---|---|---|---|
| I-1 | H | Cl | Q¹ | CH | CH | CH | N | R²-1 | R³-29 |
| I-2 | H | Cl | Q¹ | CH | CH | CH | N | R²-4 | R³-29 |
| I-3 | H | Cl | Q⁵ | CH | CH | CH | CH | R²-2 | R³-29 |
| I-4 | H | Cl | Q³ | CH | CH | CH | N | R²-2 | R³-29 |
| I-5 | H | Cl | Q³ | CH | CH | CH | N | R²-4 | R³-29 |
| I-6 | H | Cl | Q³ | CH | CH | CH | N | R²-1 | R³-29 |
| I-7 | H | Cl | Q³ | CH | CCl | CH | CH | R²-2 | R³-29 |
| I-8 | H | OCF₃ | Q³ | CH | CCl | CH | CH | R²-2 | R³-29 |
| I-9 | H | Cl | Q³ | CCl | CH | CH | CH | R²-1 | R³-29 |
| I-10 | H | OCF₃ | Q³ | CH | CH | CH | N | R²-1 | R³-29 |
| I-11 | H | Cl | Q³ | CH | N | CH | N | R²-1 | R³-29 |
| I-12 | H | Cl | Q³ | CH | N | CH | N | R²-2 | R³-29 |
| I-13 | H | Cl | Q⁴ | CH | CCl | CH | CH | R²-2 | R³-29 |
| I-14 | H | Cl | Q³ | CCl | CH | CH | CH | R²-4 | R³-29 |
| I-15 | H | Cl | Q³ | CH | CF | CH | CH | R²-2 | R³-29 |
| I-16 | H | OCF₃ | Q³ | CH | CH | CH | N | R²-4 | R³-29 |
| I-17 | H | OCF₃ | Q² | CH | CCl | CH | CH | R²-2 | R³-29 |
| I-18 | H | Cl | Q² | CH | CCl | CH | CH | R²-2 | R³-29 |
| I-19 | H | OCF₃ | Q² | CH | CF | CH | CH | R²-3 | R³-29 |
| I-20 | H | OCF₃ | Q² | CH | CF | CH | CH | R²-2 | R³-29 |
| I-21 | H | Cl | Q² | CH | CF | CH | CH | R²-2 | R³-29 |
| I-22 | Cl | F | Q² | CH | CF | CH | CH | R²-2 | R³-29 |
| I-23 | Cl | F | Q² | CH | CCl | CH | CH | R²-2 | R³-29 |
| I-24 | H | OCF₃ | Q² | CH | CF | CH | CH | R²-2 | R³-31 |
| No. | R^{1a} | R^{1b} | Q | B¹ | B² | B³ | B⁴ | R² | R³ |
| I-25 | OCF₃ | H | Q² | CH | CCl | CH | CH | R²-2 | R³-29 |
| I-26 | OCF₃ | Cl | Q² | CH | CCl | CH | CH | R²-2 | R³-29 |
| I-27 | OCF₃ | F | Q² | CH | CCl | CH | CH | R²-2 | R³-29 |
| I-28 | H | OCF₃ | Q² | CH | CCl | CH | CH | R²-7 | R³-29 |
| I-29 | Cl | F | Q² | CH | CCl | CH | CH | R²-7 | R³-29 |
| I-30 | H | Cl | Q² | CH | CCl | CH | CH | R²-7 | R³-29 |
| I-31 | Cl | F | Q² | CH | N | CH | N | R²-2 | R³-29 |
| I-32 | H | OCF₃ | Q² | CH | CCl | CH | CH | R²-9 | R³-29 |
| I-33 | Cl | F | Q² | CH | CCl | CH | CH | R²-9 | R³-29 |
| I-34 | H | OCF₃ | Q² | CH | CF | CH | CH | R²-7 | R³-29 |
| I-35 | Cl | Cl | Q² | CH | CCl | CH | CH | R²-2 | R³-30 |
| I-36 | H | OCH₂ CH₃ | Q² | CH | CH | CH | CH | R²-2 | R³-29 |
| I-37 | Cl | F | Q² | CH | CF | CH | CH | R²-7 | R³-29 |
| I-38 | H | OCF₃ | Q² | CH | CH | CH | CH | R²-1 | R³-29 |
| I-39 | H | OCF₃ | Q² | CCl | CH | CH | CH | R²-1 | R³-29 |
| I-40 | H | OCF₃ | Q² | CH | CF | CH | CH | R²-9 | R³-29 |
| I-41 | H | Br | Q² | N | CH | CH | CH | R²-1 | R³-1 |
| I-42 | H | Cl | Q² | CH | CCl | CH | CH | R²-7 | R³-29 |
| I-43 | H | Cl | Q² | CCl | CH | CH | CH | R²-1 | R³-30 |
| I-44 | Cl | F | Q² | CH | CF | CH | CH | R²-9 | R³-29 |
| I-45 | H | CN | Q² | CH | CBr | CH | CH | R²-2 | R³-29 |
| I-46 | H | OCF₃ | Q² | CCl | CH | CH | CH | R²-4 | R³-29 |
| I-47 | H | OCF₃ | Q² | CH | CH | CH | CH | R²-2 | R³-29 |
| I-48 | H | 2,2-Cl₂-cyclo-propyl | Q² | CH | CBr | CH | CH | R²-2 | R³-1 |
| I-49 | H | OCF₃ | Q² | CH | CCl | CH | CH | R²-3 | R³-29 |
| I-50 | Cl | Cl | Q² | CCl | CH | CH | CH | R²-1 | R³-7 |
| I-51 | -OCF₂-O- | | Q² | CH | CF | CH | CH | R²-2 | R³-29 |
wherein R^{1c} is H, and W is O;
or the N-oxide, stereoisomer, tautomer, agriculturally or veterinarily acceptable salt thereof.

9. An agricultural or veterinary composition comprising at least one compound according to any one of claims 1 to 8 and/or at least one agriculturally or veterinarily acceptable salt thereof, and at least one inert liquid and/or solid agriculturally or veterinarily acceptable carrier.

10. An agricultural composition for combating animal pests comprising at least one compound as defined in any of claims 1 to 8 and at least one inert liquid and/or solid acceptable carrier and, if desired, at least one surfactant.

11. A method for combating or controlling invertebrate pests, which method comprises contacting said pest or its food supply, habitat or breeding grounds with a pesticidally effective amount of at least one compound as defined in any one of claims 1 to 8.

12. A method for protecting growing plants from attack or infestation by invertebrate pests, which method comprises contacting a plant, or soil or water in which the plant is growing, with a pesticidally effective amount of at least one compound as defined in any of claims 1 to 8.

13. Seed comprising a compound as defined in any of claims 1 to 8, or the enantiomers, diastereomers or salts thereof, in an amount of from 0.1 g to 10 kg per 100 kg of seed.

14. A method for treating or protecting an animal from infestation or infection by invertebrate pests which comprises bringing the animal in contact with a pesticidally effective amount of at least one compound of the formula I as defined in any of claims 1 to 8, a stereoisomer thereof and/or at least one veterinarily acceptable salt thereof.
